# EUROPEAN PATENT SPECIFICATION

(11) **EP 1 040 098 B1**
(45) Date of publication and mention of the grant of the patent: **27.04.2005**
(21) Application number: 98963136.1
(22) Date of filing: 14.12.1998
(51) Int. Cl.: C07D 213/30, C07D 235/12, C07D 239/26, C07D 257/00, C07D 277/00, C07D 471/04, C07D 471/14, A61K 31/155, A61K 31/415, A61K 31/44, A61K 31/505, C07D 401/12, C07D 491/04, C07D 401/06, C07D 519/00, C07D 213/74

(54) **INTEGRIN RECEPTOR ANTAGONISTS**
INTEGRIN-REZEPTOR-ANTAGONISTEN
ANTAGONISTES DU RECEPTEUR DE L'INTEGRINE

(30) Priority: 17.12.1997 US 69899 P; 06.04.1998 GB 9807382; 27.04.1998 US 83209 P; 26.05.1998 GB 9811295; 13.07.1998 US 92622 P
(43) Date of publication of application: 04.10.2000
(73) Proprietor: Merck & Co., Inc. (a New Jersey corp.), Rahway, N.J. 07065 (US)
(72) Inventor: ASKEW, Ben, C., Rahway, NJ 07065 (US); COLEMAN, Paul, J., Rahway, NJ 07065 (US); DUGGAN, Mark, E., Rahway, NJ 07065 (US); HALCZENKO, Wasyl, Rahway, NJ 07065 (US); HARTMAN, George, D., Rahway, NJ 07065 (US); HUNT, Cecilia, Rahway, NJ 07065 (US); HUTCHINSON, John, H., Rahway, NJ 07065 (US); MEISSNER, Robert, S., Rahway, NJ 07065 (US); PATANE, Michael, A., Rahway, NJ 07065 (US); SMITH, Garry, R., Rahway, NJ 07065 (US); WANG, Jiabing, Rahway, NJ 07065 (US)
(74) Representative: Horgan, James Michael Frederic
(86) International application number: PCT/US1998/026484
(87) International publication number: WO 1999/031061

(56) References cited:
- EP-A- 0 737 204
- WO-A-92/17490
- WO-A-94/04503
- WO-A-96/30036
- WO-A-97/01540
- WO-A1-94/08577
- WO-A1-95/32710
- WO-A1-97/26250
- FR-A- 2 466 252
- US-A- 3 843 798
- US-A- 5 021 443
- US-A- 5 025 025
- US-A- 5 352 667
- LABAUDINIERE R ET AL: "OMEGA-U(4,6-DIPHENYL-2-PYRIDYL)OXY)ALKANO IC DERIVATIVES: A NEW FAMILY OF POTENT AND ORALLY ACTIVE LTB4 ANTAGONISTS" JOURNAL OF MEDICINAL CHEMISTRY, AMERICAN CHEMICAL SOCIETY. WASHINGTON, US, vol. 35, 1992, pages 4315-4324, XP002065466 ISSN: 0022-2623
- PAOLO COZZI ET AL: "Agents combining thromboxane receptor antagonism with thromboxane synthase inhibition: (((2-(1H-Imidazol-1-yl)ethylidene)amino)ox y)alk anoic acids" JOURNAL OF MEDICINAL CHEMISTRY, AMERICAN CHEMICAL SOCIETY. WASHINGTON, US, vol. 37, no. 21, 1994, pages 3588-3604, XP002144611 ISSN: 0022-2623
- LABAUDINIERE ET AL.: "Omega[(Omega-Aralakyl)thienyl]alkanoic Acids: From Specific LTA4 Hydrolase Inhibitors to LTB4 Receptor Antagonists" JOURNAL OF MEDICINAL CHEMISTRY, vol. 35, 1992, pages 3170-3179, XP002209551
- XUE C-B ET AL: "DISCOVERY OF AN ORALLY ACTIVE SERIES OF ISOXAZOLINE GLYCOPROTEIN IIB/IIIA ANTAGONISTS" JOURNAL OF MEDICINAL CHEMISTRY, AMERICAN CHEMICAL SOCIETY. WASHINGTON, US, vol. 40, 1997, pages 2064-2084, XP000941935 ISSN: 0022-2623
- WITYAK ET AL: "Discovery of Potent Isoxazoline Glycoprotein IIb/IIIa Receptro Antagonists" JOURNAL OF MEDICINAL CHEMISTRY, AMERICAN CHEMICAL SOCIETY. WASHINGTON, US, vol. 40, no. 1, 1997, pages 50-60, XP002149693 ISSN: 0022-2623

## Description

### CROSS-REFERENCE TO RELATED APPLICATIONS

The present invention is related to U.S. provisional applications Serial No. 60/069,899, filed December 17, 1997; 60/083,209, filed April 27, 1998; 60/092,622, filed July 13, 1998; and 60/108,063, filed November 12, 1998; the contents of all of which are hereby incorporated by reference.

### FIELD OF THE INVENTION

The present invention relates to compounds and derivatives thereof, their synthesis, and their use as integrin receptor antagonists. More particularly, the compounds of the present invention are antagonists of the integrin receptors αvβ3, αvβ5, and/or αvβ6 and are useful for inhibiting bone resorption, treating and preventing osteoporosis, and inhibiting vascular restenosis, diabetic retinopathy, macular degeneration, angiogenesis, atherosclerosis, inflammation, wound healing, viral disease, tumor growth, and metastasis.

### BACKGROUND OF THE INVENTION

It is believed that a wide variety of disease states and conditions can be mediated by acting on integrin receptors and that integrin receptor antagonists represent a useful class of drugs. Integrin receptors are heterodimeric transmembrane receptors through which cells attach and communicate with extracellular matrices and other cells. (*See* S.B. Rodan and G.A. Rodan, "Integrin Function In Osteoclasts", *Journal of Endocrinology*, Vol. 154, S47- S56 (1997), which is incorporated by reference herein in its entirety).

In one aspect of the present invention, the compounds herein are useful for inhibiting bone resorption. Bone resorption is mediated by the action of cells known as osteoclasts. Osteoclasts are large multinucleated cells of up to about 400 mm in diameter that resorb mineralized tissue, chiefly calcium carbonate and calcium phosphate, in vertebrates. Osteoclasts are actively motile cells that migrate along the surface of bone, and can bind to bone, secrete necessary acids and proteases, thereby causing the actual resorption of mineralized tissue from the bone. More specifically, osteoclasts are believed to exist in at least two physiological states, namely. the secretory state and the migratory or motile state. In the secretory state, osteoclasts are flat, attach to the bone matrix via a tight attachment zone (sealing zone), become highly polarized, form a ruffled border, and secrete lysosomal enzymes and protons to resorb bone. The adhesion of osteoclasts to bone surfaces is an important initial step in bone resorption. In the migratory or motile state, the osteoclasts migrate across bone matrix and do not take part in resorption until they again attach to bone.

Integrins are involved in osteoclast attachment, activation and migration. The most abundant integrin in osteoclasts, e.g., in rat, chicken, mouse and human osteoclasts, is an integrin receptor known as αvβ3, which is thought to interact in bone with matrix proteins that contain the RGD sequence. Antibodies to αvβ3 block bone resorption *in vitro* indicating that this integrin plays a key role in the resorptive process. There is increasing evidence to suggest that αvβ3 ligands can be used effectively to inhibit osteoclast mediated bone resorption *in vivo* in mammals.

The current major bone diseases of public concern are osteoporosis, hypercalcemia of malignancy, osteopenia due to bone metastases, periodontal disease, hyperparathyroidism, periarticular erosions in rheumatoid arthritis, Paget's disease, immobilization-induced osteopenia, and glucocorticoid-induced osteoporosis. All of these conditions are characterized by bone loss, resulting from an imbalance between bone resorption, i.e. breakdown, and bone formation, which continues throughout life at the rate of about 14% per year on the average. However, the rate of bone turnover differs from site to site; for example, it is higher in the trabecular bone of the vertebrae and the alveolar bone in the jaws than in the cortices of the long bones. The potential for bone loss is directly related to turnover and can amount to over 5% per year in vertebrae immediately following menopause, a condition which leads to increased fracture risk.

In the United States, there are currently about 20 million people with detectable fractures of the vertebrae due to osteoporosis. In addition, there are about 250,000 hip fractures per year attributed to osteoporosis. This clinical situation is associated with a 12% mortality rate within the first two years, while 30% of the patients require nursing home care after the fracture.

Individuals suffering from all the conditions listed above would benefit from treatment with agents which inhibit bone resorption.

Additionally, αvβ3 ligands have been found to be useful in treating and/or inhibiting restenosis, i.e. recurrence of stenosis after corrective surgery on the heart valve, atherosclerosis, diabetic retinopathy, macular degeneration, and angiogenesis, i.e. formation of new blood vessels, and inhibiting viral disease. Moreover, it has been postulated that the growth of tumors depends on an adequate blood supply, which in turn is dependent on the growth of new vessels into the tumor; thus, inhibition of angiogenesis can cause tumor regression in animal models (*See* Harrison's Principles of Internal Medicine, 12th ed., 1991, which is incorporated by reference herein in its entirety). Therefore, αvβ3 antagonists which inhibit angiogenesis can be useful in the treatment of cancer by inhibiting tumor growth (*See*, e.g., Brooks et al., Cell, 79:1157-1164 (1994), which is incorporated by reference herein in its entirety).

Moreover, compounds of this invention can also inhibit neovascularization by acting as antagonists of the integrin receptor, ανβ5. A monoclonal antibody for ανβ5 has been shown to inhibit VEGF-induced angiogenesis in rabbit cornea and the chick chorioallantoic membrane model (*See* M.C. Friedlander, et.al., Science 270:1500-1502 (1995), which is incorporated by reference herein in its entirety). Thus, compounds that antagonize ανβ5 are useful for treating and preventing macular degeneration, diabetic retinopathy, tumor growth, and metastasis.

Additionally, compounds of the instant invention can inhibit angiogenesis and inflammation by acting as antagonists of the integrin receptor, αvβ6, which is expressed during the later stages of wound healing and remains expressed until the wound is closed (*See* Christofidou-Solomidou, et al., "Expression and Function of Endothelial Cell αv Integrin Receptors in Wound-Induced Human Angiogenesis in Human Skin/SCID Mice Chimeras, American Journal of Pathology, Vol. 151, No. 4, pp. 975-983 (October 1997), which is incorporated by reference herein in its entirety). It is postulated that αvβ6 plays a role in the remodeling of the vasculature during the later stages of angiogenesis. Also, αvβ6 participates in the modulation of epithelial inflammation and is induced in response to local injury or inflammation (*See* Xiao-Zhu Huang, et al., "Inactivation of the Integrin β6 Subunit Gene Reveals a Role of Epithelial Integrins in Regulating Inflammation in the Lungs and Skin," Journal of Cell Biology, Vol. 133, No.4, pp. 921-928 (May 1996), which is incorporated by reference herein in its entirety). Accordingly, compounds that antagonize αvβ6 are useful in treating or preventing cancer by inhibiting tumor growth and metastasis.

In addition, certain compounds of this invention antagonize both the αvβ3 and αvβ5 receptors. These compounds, referred to as "dual αvβ3/αvβ5 antagonists," are useful for inhibiting bone resorption, treating and preventing osteoporosis, and inhibiting vascular restenosis, diabetic retinopathy, macular degeneration, angiogenesis, atherosclerosis, inflammation, viral disease, tumor growth, and metastasis.

In addition, certain compounds of this invention are useful as mixed αvβ3, αvβ5, and αvβ6 receptor antagonists.

It is therefore an object of the present invention to provide compounds which are useful as integrin receptor antagonists.

It is another object of the present invention to provide compounds which are useful as αvβ3 receptor antagonists.

It is another object of the present invention to provide compounds which are useful as αvβ5 receptor antagonists.

It is another object of the present invention to provide compounds which are useful as αvβ6 receptor antagonists.

It is another object of the present invention to provide compounds which are useful as dual αvβ3/αvβ5 receptor antagonists.

It is another object of the present invention to provide compounds which are useful as mixed αvβ3, αvβ5, and αvβ6 receptor antagonists.

It is another object of the present invention to provide pharmaceutical compositions comprising integrin receptor antagonists.

It is another object of the present invention to provide methods for making the pharmaceutical compositions of the present invention.

It is another object of the present invention to provide a use of the compounds and pharmaceutical compositions of the present invention for eliciting an integrin receptor antagonizing effect.

It is another object of the present invention to provide compounds and pharmaceutical compositions useful for inhibiting bone resorption, restenosis, atherosclerosis, inflammation, viral disease, diabetic retinopathy, macular degeneration, angiogenesis, tumor growth, and metastasis.

It is another object of the present invention to provide compounds and pharmaceutical compositions useful for treating osteoporosis.

These and other objects will become readily apparent from the detailed description which follows.

### SUMMARY OF THE INVENTION

The present invention relates to compounds of the formula 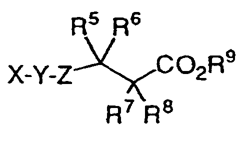 wherein X is 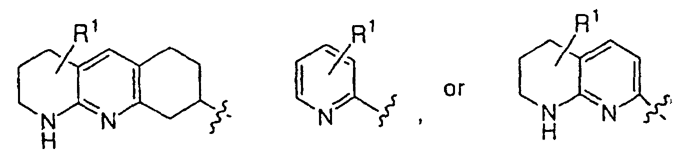 Y is selected from the group consisting of
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH2)m-O-(CH2)n-O-(CH2)p-,
-(CH2)m-O-(CH2)n-NR⁴-(CH2)p-,
-(CH2)m-NR⁴-(CH2)n-NR⁴-(CH2)p-,
-(CH2)m-O-(CH2)n-S-(CH2)p-,
-(CH2)m-S-(CH2)n-S-(CH2)p-,
-(CH2)m-NR⁴-(CH2)n-S-(CH2)p-,
-(CH2)m-NR⁴-(CH2)n-O-(CH2)p-,
-(CH2)m-S-(CH2)n-O-(CH2)p-, and
-(CH2)m-S-(CH2)n-NR⁴-(CH2)p-,
wherein any methylene (CH₂) carbon atom in Y, other than in R⁴, can be substituted by one or two R³ substituents with the proviso that when Y is -(CH₂)ₘ-NR⁴-(CH₂)ₙ- and n is 1, then the R³ substituent on the methylene carbon in -(CH₂)ₘ- adjacent to the nitrogen cannot be oxo;
Z is selected from the group consisting of 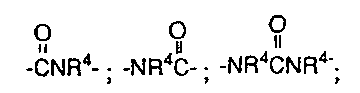 -CH₂CH₂- and -CH=CH-, wherein either carbon atom can be substituted by one or two R³ substituents;
R¹ is selected from the group consisting of
hydrogen, halogen, C₁₋₁₀ alkyl, C₃₋₈ cycloalkyl, C₃₋₈ cycloheteroalkyl, C₃₋₈ cycloalkyl C₁₋₆ alkyl, C₃₋₈ cycloheteroalkyl C₁₋₆ alkyl, aryl, aryl C₁₋₈ alkyl, amino, amino C₁₋₈ alkyl, C₁₋₃ acylamino, C₁₋₃ acylamino C₁₋₈ alkyl, (C₁₋₆ alkyl)pamino, (C₁₋₆ alkyl)ₚamino C₁₋₈ alkyl, C₁₋₄ alkoxy, C₁₋₄ alkoxy C₁₋₆ alkyl, hydroxycarbonyl, hydroxycarbonyl C₁₋₆ alkyl, C₁₋₃ alkoxycarbonyl, C₁₋₃ alkoxycarbonyl C₁₋₆ alkyl, hydroxycarbonyl-C₁₋₆ alkyloxy, hydroxy, hydroxy C₁₋₆ alkyl, C₁₋₆ alkyloxy-C₁₋₆ alkyl, nitro, cyano, trifluoromethyl, trifluoromethoxy, trifluoroethoxy, C₁₋₈ alkyl-S(O)ₚ, (C₁₋₈ alkyl)ₚaminocarbonyl, C₁₋₈ alkyloxycarbonylamino, (C₁₋₈ alkyl)ₚaminocarbonyloxy, (aryl C₁₋₈ alkyl)ₚamino, (aryl)ₚamino, aryl C₁₋₈ alkylsulfonylamino, and C₁₋₈ alkylsulfonylamino;
each R³, R⁵, R⁶, R⁷ and R⁸ is independently selected from the group consisting of
hydrogen,
aryl,
C₁₋₁₀ alkyl,
aryl-(CH₂)ᵣ-O-(CH₂)ₛ-,
aryl-(CH₂)ᵣS(O)ₚ-(CH₂)ₛ-,
aryl-(CH₂)ᵣ-C(O)-(CH₂)ₛ-,
aryl-(CH₂)ᵣ-C(O)-N(R⁴)-(CH₂)ₛ-,
aryl-(CH₂)ᵣ-N(R⁴)-C(O)-(CH₂)ₛ-,
aryl-(CH₂)ᵣ-N(R⁴)-(CH₂)ₛ-,
halogen,
hydroxyl,
aryl C₁₋₅ alkoxy,
C₁₋₅ alkoxycarbonyl,
(C₁₋₈ alkyl)ₚaminocarbonyl,
C₁₋₆ alkylcarbonyloxy,
C₃₋₈ cycloalkyl,
(C₁₋₆ alkyl)ₚamino,
arylaminocarbonyl,
aryl C₁₋₅ alkylaminocarbonyl,
aminocarbonyl,
aminocarbonyl C₁₋₆ alkyl,
hydroxycarbonyl,
hydroxycarbonyl C₁₋₆ alkyl,
HC≡C-(CH₂)ₜ-,
C₁₋₆ alkyl-C≡C-(CH₂)ₜ-,
C₃₋₇ cycloalkyl-C≡C-(CH₂)ₜ-,
aryl-C≡C-(CH₂)ₜ-,
C₁₋₆ alkylaryl-C≡C-(CH₂)ₜ-,
CH₂=CH-(CH₂)ₜ-,
C₁₋₆ alkyl-CH=CH-(CH₂)ₜ-,
C₃₋₇ cycloalkyl-CH=CH-(CH₂)ₜ-,
aryl-CH=CH-(CH₂)ₜ-,
C₁₋₆ alkylaryl-CH=CH-(CH₂)ₜ-,
C₁₋₆ alkyl-SO₂-(CH₂)ₜ-,
C₁₋₆ alkylaryl-SO₂-(CH₂)ₜ-,
C₁₋₆ alkoxy,
aryl C₁₋₆ alkoxy,
aryl C ₁₋₆ alkyl,
(C₁₋₆ alkyl)ₚamino C₁₋₆ alkyl,
(aryl)ₚamino,
(aryl)ₚamino C₁₋₆ alkyl,
(aryl C₁₋₆ alkyl)ₚamino,
(aryl C₁₋₆ alkyl)ₚamino C₁₋₆ alkyl,
arylcarbonyloxy,
aryl C₁₋₆ alkylcarbonyloxy,
(C₁₋₆ alkyl)ₚaminocarbonyloxy,
C₁₋₈ alkylsulfonylamino,
arylsulfonylamino,
C₁₋₈ alkylsulfonylamino C₁₋₆ alkyl,
aryl C₁₋₆ alkylsulfonylamino,
aryl C₁₋₆ alkylsulfonylamino C₁₋₆ alkyl,
aminocarbonylamino C₁₋₆ alkyl,
(C₁₋₈ alkyl)ₚaminocarbonylamino,
(C₁₋₈ alkyl)ₚaminocarbonylamino C₁₋₆ alkyl,
(aryl)ₚaminocarbonylamino C₁₋₆ alkyl,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylsulfonyl,
C₁₋₆ alkylsulfonyl C₁₋₆ alkyl,
arylsulfonyl C₁₋₆ alkyl,
aryl C₁₋₆ alkylsulfonyl,
aryl C₁₋₆ alkylsulfonyl C₁₋₆ alkyl,
C₁₋₆ alkylcarbonyl,
C₁₋₆ alkylcarbonyl C₁₋₆ alkyl,
arylcarbonyl C₁₋₆ alkyl,
aryl C₁₋₆ alkylcarbonyl,
aryl C₁₋₆ alkylcarbonyl C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino,
aryl C₁₋₆ alkylthiocarbonylamino C₁₋₆ alkyl,
(C₁₋₈ alkyl)ₚaminocarbonyl C₁₋₆ alkyl,
(aryl)ₚaminocarbonyl C₁₋₆ alkyl,
(aryl C₁₋₈ alkyl)ₚaminocarbonyl, and
(aryl C₁₋₈ alkyl)ₚaminocarbonyl C₁₋₆ alkyl;
or R³ is oxo, trifluoromethyl or C₁₋₈ alkylcarbonylamino;
or R⁵ and R⁶ are independently amino C₁₋₆ alkyl, arylsulfonylamino C₁₋₆ alkyl, or C₁₋₈ allcylcarbonylamino;
or R⁷ and R⁸ are independently amino C₁₋₆ alkyl, arylcarbonylamino, arylsulfonylamino C₁₋₆ alkyl, arylaminocarbonylamino or C₇₋₂₀ polycyclyl C₀₋₈ alkylsulfonylamino;
or two R³ substituents, when on the same carbon atom are taken together with the carbon atom to which they are attached to form a carbonyl group or a cyclopropyl group,
or R⁵ and R⁶ when on the same carbon atom are taken together with the carbon atom to which they are attached to form a carbonyl group,
wherein any of the alkyl groups of R³, R⁵, R⁶, R⁷ and R⁸ are either unsubstituted or substituted with one to three R¹ substituents, and provided that each R³, each R⁵ and R⁶, and each R⁷ and R⁸, are selected such that in the resultant compound the carbon atom or atoms to which R³, R⁵ and R⁶, and R⁷ and R⁸, are attached are themselves attached to no more than one heteroatom;
or R⁵ and R⁶ when on the same carbon atom are taken together with the carbon atom to which they are attached to form a carbonyl group,
wherein any of the alkyl groups of R³, R⁵, R⁶, R⁷ and R⁸ are either unsubstituted or substituted with one to three R¹ substituents, and provided that each R³, each R⁵ and R⁶, and each R⁷ and R⁸, are selected such that in the resultant compound the carbon atom or atoms to which R³, R⁵ and R⁶, and R⁷ and R⁸, are attached are themselves attached to no more than one heteroatom;
each R⁴ is independently selected from the group consisting of
hydrogen,
aryl,
aminocarbonyl,
C₃₋₈ cycloalkyl,
amino C₁₋₆ alkyl,
(aryl)ₚaminocarbonyl,
(aryl C₁₋₅ alkyl)ₚaminocarbonyl,
hydroxycarbonyl C₁₋₆ alkyl,
C₁₋₈ alkyl,
aryl C₁₋₆ alkyl,
(C₁₋₆ alkyl)ₚamino C₂₋₆ alkyl,
(aryl C₁₋₆ alkyl)ₚamino C₂₋₆ alkyl,
C₁₋₈ alkylsulfonyl,
C₁₋₈ alkoxycarbonyl,
aryloxycarbonyl,
aryl C₁₋₈ alkoxycarbonyl,
C₁₋₈ alkylcarbonyl,
arylcarbonyl,
aryl C₁₋₆ alkylcarbonyl,
(C₁₋₈ alkyl)ₚaminocarbonyl,
aminosulfonyl,
C₁₋₈ alkylaminosulfonyl,
(aryl)ₚaminosulfonyl,
(aryl C₁₋₈ alkyl)ₚaminosulfonyl,
arylsulfonyl,
arylC1-6 alkylsulfonyl,
C₁₋₆ alkylthiocarbonyl,
arylthiocarbonyl, and
aryl C₁₋₆ alkylthiocarbonyl,
wherein any of the alkyl groups of R⁴ are either unsubstituted or substituted with one to three R¹ substituents;
R⁹ is selected from, the group consisting of
hydrogen,
C₁₋₈ alkyl,
aryl,
aryl C₁₋₈ alkyl,
C₁₋₈ alkylcarbonyloxy C₁₋₄ alkyl,
aryl C₁₋₈ alkylcarbonyloxy C₁₋₄ alkyl,
C₁₋₈ alkylaminocarbonylmethylene, and
C₁₋₈ dialkylaminocarbonylmethylene;
wherein
each aryl as a group or part of a group is selected from the group consisting of phenyl, naphthyl, pyridyl, pyrryl, pyrazolyl, pyrazinyl, pyrimidinyl, imidazolyl, benzimidazolyl, benzthiazolyl, benzoxazolyl, indolyl, thienyl, furyl, dihydrobenzofuryl, benzo(1,3) dioxolane, oxazolyl, isoxazolyl and thiazolyl;
each m is independently an integer from 2 to 4;
each n is independently an integer from 0 to 6;
each p is independently an integer from 0 to 2;
each r is independently an integer from 1 to 3;
each s is independently an integer from 0 to 3; and
each t is independently an integer from 0 to 3;
and the pharmaceutically acceptable salts thereof.

The present invention also relates to pharmaceutical compositions comprising the compounds of the present invention and a pharmaceutically acceptable carrier.

The present invention also relates to methods for making the pharmaceutical compositions of the present invention.

The present invention also relates to a use of the compounds and pharmaceutical compositions of the present invention for eliciting an integrin receptor antagonizing effect.

The present invention also relates to a use of the compounds and pharmaceutical compositions of the present invention for inhibiting bone resorption, restenosis, atherosclerosis, inflammation, viral disease, diabetic retinopathy, macular degeneration, angiogenesis, wound healing, tumor growth, and metastasis.

The present invention also relates to a use of the compounds and pharmaceutical compositions of the present invention for treating osteoporosis.

### DETAILED DESCRIPTION OF THE INVENTION

The present invention relates to compounds useful as integrin receptor antagonists. Representative compounds of the present invention are described by the following structural formula: 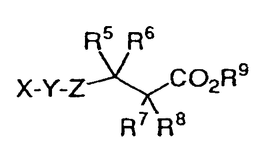 wherein X is 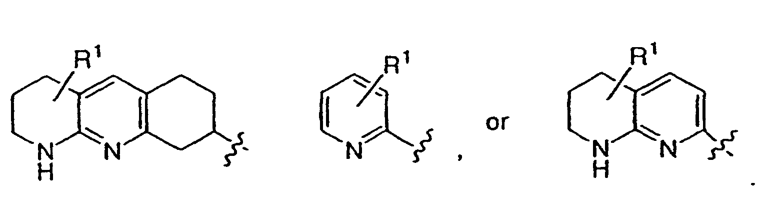 Y is selected from the group consisting of
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH2)m-O-(CH2)n-O-(CH2)p-,
-(CH2)m-O-(CH2)n-NR⁴-(CH2)p-,
-(CH2)m-NR⁴-(CH2)n-NR⁴-(CH2)p-,
-(CH2)m-O-(CH2)n-S-(CH2)p-,
-(CH2)m-S-(CH2)n-S-(CH2)p-,
-(CH2)m-NR⁴-(CH2)n-S-(CH2)p-,
-(CH2)m-NR⁴-(CH2)n-O-(CH2)p-,
-(CH2)m-S-(CH2)n-O-(CH2)p-, and
-(CH2)m-S-(CH2)n-NR⁴-(CH2)p-,
wherein any methylene (CH₂) carbon atom in Y, other than in R⁴, can be substituted by one or two R³ substituents, with the proviso that when Y is -(CH₂)ₘ-NR⁴-(CH₂)ₙ- and n is 1, then the R³ substituent on the methylene carbon in -(CH₂)ₘ- adjacent to the nitrogen cannot be oxo;
Z is selected from the group consisting of 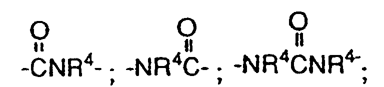 -CH₂CH₂-, and -CH=CH-, wherein either carbon atom can be substituted by one or two R³ substituents;
R¹ is selected from the group consisting of
hydrogen, halogen, C₁₋₁₀ alkyl, C₃₋₈ cycloalkyl, C₃₋₈ cycloheteroalkyl, C₃₋₈ cycloalkyl C₁₋₆ alkyl, C₃₋₈ cycloheteroalkyl C₁₋₆ alkyl, aryl, aryl C₁₋₈ alkyl, amino, amino C₁₋₈ alkyl, C₁₋₃ acylamino, C₁₋₃ acylamino C₁₋₈ alkyl, (C₁₋₆ alkyl)ₚamino, (C₁₋₆ alkyl)ₚamino C₁₋₈ alkyl, C₁₋₄ alkoxy, C₁₋₄ alkoxy C₁₋₆ alkyl, hydroxycarbonyl, hydroxycarbonyl C₁₋₆ alkyl, C₁₋₃ alkoxycarbonyl, C₁₋₃ alkoxycarbonyl C₁₋₆ alkyl, hydroxycarbonyl-C₁₋₆ alkyloxy, hydroxy, hydroxy C₁₋₆ alkyl, C₁₋₆ alkyloxy-C₁₋₆ alkyl, nitro, cyano, trifluoromethyl, trifluoromethoxy, trifluoroethoxy, C₁₋₈ alkyl-S(O)ₚ, (C₁₋₈ alkyl)ₚaminocarbonyl, C₁₋₈ alkyloxycarbonylamino, (C₁₋₈ alkyl)ₚaminocarbonyloxy, (aryl C₁₋₈ alkyl)ₚamino, (aryl)ₚamino, aryl C₁₋₈ alkylsulfonylamino, and C₁₋₈ alkylsulfonylamino;
each R³, R⁵, R⁶, R⁷ and R⁸ is independently selected from the group consisting of
hydrogen,
aryl,
C₁₋₁₀ alkyl,
aryl-(CH₂)ᵣ-O-(CH₂)ₛ-,
aryl-(CH₂)ᵣS(O)ₚ-(CH₂)ₛ-,
aryl-(CH₂)ᵣ-C(O)-(CH₂)ₛ-,
aryl-(CH₂)ᵣ-C(O)-N(R⁴)-(CH₂)ₛ-,
aryl-(CH₂)ᵣ-N(R⁴)-C(O)-(CH₂)ₛ-,
aryl-(CH₂)ᵣ-N(R⁴)-(CH₂)ₛ-,
halogen,
hydroxyl,
aryl C₁₋₅ alkoxy,
C₁₋₅ alkoxycarbonyl,
(C₁₋₈ alkyl)ₚaminocarbonyl,
C₁₋₆ alkylcarbonyloxy,
C₃₋₈ cycloalkyl,
(C₁₋₆ alkyl)ₚamino,
arylaminocarbonyl,
aryl C₁₋₅ alkylaminocarbonyl,
aminocarbonyl,
aminocarbonyl C₁₋₆ alkyl,
hydroxycarbonyl,
hydroxycarbonyl C₁₋₆ alkyl,
HC≡C-(CH₂)ₜ-,
C₁₋₆ alkyl-C≡C-(CH₂)ₜ-,
C₃₋₇ cycloalkyl-C≡C-(CH₂)ₜ-,
aryl-C≡C-(CH₂)ₜ-,
C₁₋₆ alkylaryl-C≡C-(CH₂)ₜ-,
CH₂=CH-(CH₂)ₜ-,
C₁₋₆ alkyl-CH=CH-(CH₂)ₜ-,
C₃₋₇ cycloalkyl-CH=CH-(CH₂)ₜ-,
aryl-CH=CH-(CH₂)ₜ-,
C₁₋₆ alkylaryl-CH=CH-(CH₂)ₜ-,
C₁₋₆ alkyl-SO₂-(CH₂)ₜ-,
C₁₋₆ alkylaryl-SO₂-(CH₂)ₜ-,
C₁₋₆ alkoxy,
aryl C₁₋₆ alkoxy,
aryl C₁₋₆ alkyl,
(C₁₋₆ alkyl)ₚamino C₁₋₆ alkyl,
(aryl)ₚamino,
(aryl)ₚamino C₁₋₆ alkyl,
(aryl C₁₋₆ alkyl)ₚamino,
(aryl C₁₋₆ alkyl)ₚamino C₁₋₆ alkyl,
arylcarbonyloxy,
aryl C₁₋₆ alkylcarbonyloxy,
(C₁₋₆ alkyl)ₚaminocarbonyloxy,
C₁₋₈ alkylsulfonylamino,
arylsulfonylamino,
C₁₋₈ alkylsulfonylamino C₁₋₆ alkyl,
aryl C₁₋₆ alkylsulfonylamino,
aryl C₁₋₆ alkylsulfonylamino C₁₋₆ alkyl,
aminocarbonylamino C₁₋₆ alkyl,
(C₁₋₈ alkyl)ₚaminocarbonylamino,
(C₁₋₈ alkyl)ₚaminocarbonylamino C₁₋₆ alkyl,
(aryl)ₚaminocarbonylamino C₁₋₆ alkyl,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylsulfonyl,
C₁₋₆ alkylsulfonyl C₁₋₆ alkyl,
arylsulfonyl C₁₋₆ alkyl,
aryl C₁₋₆ alkylsulfonyl,
aryl C₁₋₆ alkylsulfonyl C₁₋₆ alkyl,
C₁₋₆ alkylcarbonyl,
C₁₋₆ alkylcarbonyl C₁₋₆ alkyl,
arylcarbonyl C₁₋₆ alkyl,
aryl C₁₋₆ alkylcarbonyl,
aryl C₁₋₆ alkylcarbonyl C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino,
aryl C₁₋₆ alkylthiocarbonylamino C₁₋₆ alkyl,
(C₁₋₈ alkyl)ₚaminocarbonyl C₁₋₆ alkyl,
(aryl)ₚaminocarbonyl C₁₋₆ alkyl,
(aryl C₁₋₈ alkyl)ₚaminocarbonyl, and
(aryl C₁₋₈ alkyl)ₚaminocarbonyl C₁₋₆ alkyl;
or R³ is oxo, trifluoromethyl or C₁₋₈ alkylcarbonylamino;
or R⁵ and R⁶ are independently amino C₁₋₆ alkyl, arylsulfonylamino C₁₋₆ alkyl, or C₁₋₈ alkylcarbonylamino;
or R⁷ and R⁸ are independently amino C₁₋₆ alkyl, arylcarbonylamino, arylsulfonylamino C₁₋₆ alkyl, arylaminocarbonylamino or C₇₋₂₀ polycyclyl C₀₋₈ alkylsulfonylamino;
or two R³ substituents, when on the same carbon atom are taken together with the carbon atom to which they are attached to form a carbonyl group or a cyclopropyl group,
or R⁵ and R⁶ when on the same carbon atom are taken together with the carbon atom to which they are attached to form a carbonyl group,
wherein any of the alkyl groups of R³, R⁵, R⁶, R⁷ and R⁸ are either unsubstituted or substituted with one to three R¹ substituents, and provided that each R³, each R⁵ and R⁶, and each R⁷ and R⁸, are selected such that in the resultant compound the carbon atom or atoms to which R³, R⁵ and R⁶, and R⁷ and R⁸, are attached are themselves attached to no more than one heteroatom;
each R⁴ is independently selected from the group consisting of
hydrogen,
aryl,
aminocarbonyl,
C₃₋₈ cycloalkyl,
amino C₁₋₆ alkyl,
(aryl)ₚaminocarbonyl,
(aryl C₁₋₅ alkyl)ₚaminocarbonyl,
hydroxycarbonyl C₁₋₆ alkyl,
C₁₋₈ alkyl,
aryl C₁₋₆ alkyl,
(C₁₋₆ alkyl)ₚamino C₂₋₆ alkyl,
(aryl C₁₋₆ alkyl)ₚamino C₂₋₆ alkyl,
C₁₋₈ alkylsulfonyl,
C₁₋₈ alkoxycarbonyl,
aryloxycarbonyl,
aryl C₁₋₈ alkoxycarbonyl,
C₁₋₈ alkylcarbonyl,
arylcarbonyl,
aryl C₁₋₆ alkylcarbonyl,
(C₁₋₈ alkyl)ₚaminocarbonyl,
aminosulfonyl,
C₁₋₈ alkylaminosulfonyl,
(aryl)ₚaminosulfonyl,
(aryl C₁₋₈ alkyl)ₚaminosulfonyl,
arylsulfonyl,
arylC1-6 alkylsulfonyl,
C₁₋₆ alkylthiocarbonyl,
arylthiocarbonyl, and
aryl C₁₋₆ alkylthiocarbonyl,
wherein any of the alkyl groups of R⁴ are either unsubstituted or substituted with one to three R¹ substituents;
R⁹ is selected from the group consisting of
hydrogen,
C₁₋₈ alkyl,
aryl,
aryl C₁₋₈ alkyl,
C₁₋₈ alkylcarbonyloxy C₁₋₄ alkyl,
aryl C₁₋₈ alkylcarbonyloxy C₁₋₄ alkyl,
C₁₋₈ alkylaminocarbonylmethylene, and
C₁₋₈ dialkylaminocarbonylmethylene;
wherein
each aryl as a group or part of a group is selected from the group consisting of phenyl, naphthyl, pyridyl, pyrryl, pyrazolyl, pyrazinyl, pyrimidinyl, imidazolyl, benzimidazolyl, benzthiazolyl, benzoxazolyl, indolyl, thienyl, furyl, dihydrobenzofuryl, benzo(1,3) dioxolane, oxazolyl, isoxazolyl and thiazolyl;
each m is independently an integer from 2 to 4;
each n is independently an integer from 0 to 6;
each p is independently an integer from 0 to 2;
each r is independently an integer from 1 to 3;
each s is independently an integer from 0 to 3; and
each t is independently an integer from 0 to 3;
and the pharmaceutically acceptable salts thereof.

In the compounds of the present invention, Y is preferably selected from the group consisting of
-(CH₂)ₘ-,
-(CH₂)ₘ-O-(CH₂)n-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)m-SO₂-(CH₂)ₙ-,
-(CH2)m-O-(CH2)n-O-(CH2)p-,
-(CH2)m-O-(CH2)n-NR⁴-(CH2)p-,
-(CH2)m-NR⁴-(CH2)n-NR⁴-(CH2)p-, and
-(CH2)m-NR⁴-(CH2)n-O-(CH2)p-,
wherein any methylene (CH₂) carbon atom in Y, other than in R⁴, can be substituted by one or two R³ substituents. More preferably, Y is selected from the group consisting of
(CH₂)ₘ, (CH₂)ₘ-S-(CH₂)ₙ, and (CH₂)ₘ-NR⁴-(CH₂)ₙ,
wherein any methylene (CH₂) carbon atom in Y, other than in R⁴, can be substituted by one or two R³ substituents.

In the compounds of the present invention, Z is preferably selected from the group consisting of 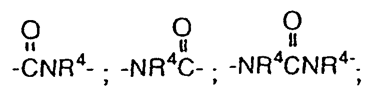 -CH₂CH₂ -, and -CH=CH-, wherein either carbon atom can be substituted by one or two R³ substituents.

More preferably, Z is selected from the group consisting of 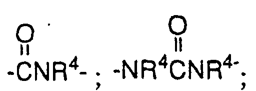 and
-CH₂CH₂-, wherein either carbon atom can be substituted by one or two R³ substituents.

In the compounds of the present invention, R¹ and R² are preferably selected from the group consisting of hydrogen, halogen, C₁₋₁₀ alkyl, C₃₋₈ cycloalkyl, C₃₋₈ cycloheteroalkyl, hydroxy, nitro, cyano, trifluoromethyl, and trifluoromethoxy.

More preferably, R¹ and R² are selected from the group consisting of hydrogen, halogen, C₁₋₁₀ alkyl, C₃₋₈ cycloalkyl, trifluoromethyl, and trifluoromethoxy.

In the compounds of the present invention, R³ is preferably selected from the group consisting of
hydrogen,
fluoro,
trifluoromethyl,
aryl,
C₁₋₈ alkyl,
arylC₁₋₆ alkyl
hydroxyl,
oxo,
arylaminocarbonyl,
aryl C₁₋₅ alkylaminocarbonyl,
aminocarbonyl, and
aminocarbonyl C₁₋₆ alkyl.

More preferably, R³ is selected from the group consisting of fluoro,
aryl,
C₁₋₈ alkyl,
arylC₁₋₆ alkyl
hydroxyl,
oxo, and
arylaminocarbonyl.

In the compounds of the present invention, R⁴ is preferably selected from the group consisting of
hydrogen,
aryl,
C₃₋₈ cycloalkyl,
C₁₋₈ alkyl,
C₁₋₈ alkylcarbonyl,
arylcarbonyl,
C₁₋₆ alkylsulfonyl,
arylsulfonyl,
arylC₁₋₆alkylsulfonyl,
arylC₁₋₆alkylcarbonyl,
C₁₋₈alkylaminocarbonyl,
arylC₁₋₅alkylaminocarbonyl,
arylC₁₋₈alkoxycarbonyl, and
C ₁₋₈alkoxycarbonyl.

More preferably, R⁴ is selected from the group consisting of hydrogen,
C₁₋₈ alkyl,
C₁₋₈ alkylcarbonyl,
arylcarbonyl,
arylC₁₋₆alkylcarbonyl,
C₁₋₆ alkylsulfonyl,
arylsulfonyl, and
arylC₁₋₆alkylsulfonyl.

In one embodiment of the present invention, R⁵ and R⁶ are each independently selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

In a class of this embodiment of the present invention, R⁶ is hydrogen and R⁵ is selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

In a subclass of this class of the present invention, R⁶ , R⁷ and R⁸ are each hydrogen and R⁵ is selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

In another embodiment of the present invention, R⁷ and R⁸ are each independently selected from the group consisting of
hydrogen,
aryl,
C₁₋₈ alkylcarbonylamino,
C₁₋₈ alkylsulfonylamino,
arylcarbonylamino,
arylsulfonylamino,
C₁₋₈ alkylsulfonylamino C₁₋₆ alkyl,
arylsulfonylamino C₁₋₆ alkyl,
aryl C₁₋₆ alkylsulfonylamino,
aryl C₁₋₆ alkylsulfonylamino C₁₋₆ alkyl,
C₁₋₈ alkoxycarbonylamino,
C₁₋₈ alkoxycarbonylamino C₁₋₈ alkyl,
aryloxycarbonylamino C₁₋₈ alkyl,
aryl C₁₋₈ alkoxycarbonylamino,
aryl C₁₋₈ alkoxycarbonylamino C₁₋₈ alkyl,
C₁₋₈ alkylcarbonylamino C₁₋₆ alkyl,
arylcarbonylamino C₁₋₆ alkyl,
aryl C₁₋₆ alkylcarbonylamino,
aryl C₁₋₆ alkylcarbonylamino C₁₋₆ alkyl,
aminocarbonylamino C₁₋₆ alkyl,
(C₁₋₈ alkyl)ₚaminocarbonylamino,
(C₁₋₈ alkyl)ₚaminocarbonylamino C₁₋₆ alkyl,
(aryl)paminocarbonylamino C₁₋₆ alkyl,
arylaminocarbonylamino,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino,
aryl C₁₋₆ alkylthiocarbonylamino C₁₋₆ alkyl, and
C₇₋₂₀ polycyclyl C₀₋₈ alkylsulfonylamino.

In a class of this embodiment of the present invention, R⁸ is hydrogen and R⁷ is selected from the group consisting of
hydrogen,
aryl,
C₁₋₈ alkylcarbonylamino,
aryl C₁₋₆ alkylcarbonylamino,
arylcarbonylamino,
C₁₋₈ alkylsulfonylamino,
aryl C₁₋₆ alkylsulfonylamino,
arylsulfonylamino,
C₁₋₈ alkoxycarbonylamino,
aryl C₁₋₈ alkoxycarbonylamino,
arylaminocarbonylamino,
(C₁₋₈ alkyl)ₚaminocarbonylamino,
(aryl C₁₋₈ alkyl)ₚaminocarbonylamino,
(C₁₋₈ alkyl)ₚaminosulfonylamino, and
(aryl C₁₋₈ alkyl)ₚaminosulfonylamino.

In a subclass of this class of the present invention, R⁵ , R⁶ and R⁸ are each hydrogen and R⁷ is selected from the group consisting of
hydrogen,
aryl,
C₁₋₈ alkylcarbonylamino,
aryl C₁₋₆ alkylcarbonylamino,
arylcarbonylamino,
C₁₋₈ alkylsulfonylamino,
aryl C₁₋₆ alkylsulfonylamino,
arylsulfonylamino,
C₁₋₈ alkoxycarbonylamino,
aryl C₁₋₈ alkoxycarbonylamino,
arylaminocarbonylamino,
(C₁₋₈ alkyl)ₚaminocarbonylamino,
(aryl C₁₋₈ alkyl)ₚaminocarbonylamino,
(C₁₋₈ alkyl)ₚaminosulfonylamino, and
(aryl C₁₋₈ alkyl)ₚaminosulfonylamino.

In the compounds of the present invention, R⁹ is preferably selected from the group consisting of hydrogen, methyl, and ethyl.

More preferably, R⁹ is hydrogen.

In the compounds of the present invention, m is preferably an integer from 0 to 4, more preferably from 2 to 4.

In the compounds of the present invention, n is preferably an integer from 0 to 4, more preferably from 2 to 4.

In the compounds of the present invention, r is preferably an integer from 1 to 2.

In the compounds of the present invention, s is preferably an integer from 0 to 2.

In the compounds of the present invention, t is preferably an integer from 0 to 2, more preferably from 0 to 1.

Illustrative but nonlimiting examples of compounds of the present invention that are useful as integrin receptor antagonists are the following:
3-(5-(5,6,7,8-Tetrahydro[1,8]naphthyridin-2-yl)-pentanoylamino)-propionic acid;
3(S)-(Pyridin-3-yl)-3-(5-(5,6,7,8-tetrahydro[1,8]naphthyridin-2-yl)-pentanoylamino)-propionic acid;
3(S)-(5,6,7,8-Tetrahydroquinolin-3-yl)-3-(5-(5,6,7,8-tetrahydro[1,8]naphthyridin-2-yl)-pentanoylamino)-propionic acid (trifluoroacetate);
2(S)-Benzenesulfonylamino-3-(5-(5,6,7,8-tetrahydro-[1,8]naphthyridin-2-yl)-pentanoylamino)-propionic acid trifluoroacetate;
3(S)-(Quinolin-3-yl)-3-(5-(5,6,7,8-tetrahydro-[1,8]naphthyridin-2-yl)-pentanoylamino)-propionic acid;
3(R)-(Quinolin-3-yl)-3-(5-(5,6,7,8-tetrahydro-[1,8]naphthyridin-2-yl)-pentanoylamino)-propionic acid;
3-(Quinolin-3-yl)-3-(7-(5,6,7,8-tetrahydro-[1,8]naphthyridin-2-yl)-heptanoylamino)-propionic acid bis(trifluoroacetate);
3-(Quinolin-3-yl)-3-(6-(5,6,7,8-tetrahydro-[1,8]naphthyridin-2-yl)-hexanoylamino)-propionic acid;
3(S)-(3-Fluorophenyl)-3-(4-(5,6,7,8-tetrahydro-[1,8]naphthyridin-2-ylamino)-butyrylamino)-propionic acid bis(trifluoroacetate);
3(S)-(5-(5,6,7,8-Tetrahydro-[1,8]naphthyridin-2-yl)-pentanoylamino)-pent-4-enoic acid;
3(S)-(3-Fluorophenyl)-3-(5-(5,6,7,8-tetrahydro-[1,8]naphthyridin-2-yl)-pentanoylamino)-propionic acid;
2-(3-Fluorophenyl)-3-(5-(5,6,7,8-tetrahydro-[1,8]naphthyridin-2-yl)-pentanoylamino)-propionic acid trifluoroacetate salt;
3(S)-(Benzo[1,3]dioxol-5-yl)-3-(5-(5,6,7,8-tetrahyclro-[1,8]naphthyridin-2-yl)-pentanoylamino)-propionic acid;
3(S)-(2,3-Dihydro-benzofuran-6-yl)-3-(5-(5,6,7,8-tetrahydro-[1,8]naphthyridin-2-yl)-pentanoylamino)-propionic acid;
3(S)-(2-Oxo-2,3-dihydro-benzoxazol-6-yl)-3-(5-(5,6,7,8-tetrahydro-[1,8]naphthyridin-2-yl)-pentanoyiamino)-propionic acid trifluoroacetate;
3(S)-(3-Fluorophenyl)-3-{3-[(5,6,7,8-tetrahydro-[1,8]naphthyridin-2-ylmethyl)-amino]-propionylamino}-propionic acid;
3(S)-(3-Fluorophenyl)-3-(2-{propyl-[2-(5,6,7,8-tetrahydro-[1,8]naphthyridin-2-yl)-ethyl]-amino}-acetylamino)-propionic acid trifluoroacetate;
3(S)-(3-Fluorophenyl)-3-(2-{phenethyl-[2-(5,6,7,8-tetrahydro-[1,8]naphthyridin-2-yl)-ethyl]-amino}-acetylamino)-propionic acid trifluoroacetate;
3(S)-(3-Fluorophenyl)-3-{3(S)-[(5,6,7,8-tetrahydro-[1,8]naphthyridin-2-ylmethyl)-amino]-pent-4-ynoylamino}-propionic acid;
3(S)-(3-Fluorophenyl)-3-{3(S)-(3-fluorophenyl)-3-[(5,6,7,8-tetrahydro-[1,8]naphthyridin-2-ylmethyl)-amino]-propionylamino}-propionic acid bis(trifluoroacetate);
3(S)-(3-Fluoro-4-phenyl-phenyl)-3-(5-(5,6,7,8-tetrahydro-[1,8]naphthyridin-2-yl)-pentanoylamino)-propionic acid trifluoroacetate;
2(S)-(2-Thienylsulfonylamino)-3-(5-(5,6,7,8-tetrahydro-[1,8]naphthyridin-2-yl)-pentanoylamino)-propionic acid trifluoroacetate;
3(S)-(3-Fluorophenyl)-3-{3-methyl-3-[(5,6,7,8-tetrahydro-[1,8]naphthyridin-2-ylmethyl)-amino]-propionylamino}-propionic acid;
3(S)-(3-Fluorophenyl)-3-{2-[2-(5,6,7,8-tetrahydro-[1,8]naphthyridin-2-yl)-ethylamino]-acetylamino}-propionic acid;
3(S)-(3-Fluorophenyl)-3{[3-(5,6,7,8-tetrahydro-[1,8]naphthyridin-2-yl)-propyl]-ureido}-propionic acid;
2(S)-(Methanesulfonylamino)-3-(5-(5,6,7,8-tetrahydro-[1,8]naphthyridin-2-yl)-pentanoylamino)-propionic acid;
3(S)-(2,3-Dihydro-benzofuran-6-yl)-3-[3-(1,2,3,4,6,7,8(R or S),9-octahydrobenzo[*b*][1,8]naphthyridin-8-yl)-propionylamino]-propionic acid;
3(S)-(2,3-Dihyclro-benzofuran-6-yl)-3-[3-(1,2,3,4,6,7,8(S or R),9-octahydrobenzo[*b*][1,8]naphthyridin-8-yl)-propionylamino]-propionic acid;
3(S)-(6-Methoxy-pyridin-3-yl)-3-[N-methyl-3-(1,2,3,4,6,7,8,9-octahydrobenzo[b][1,8]naphthyridin-8-yl-propionyl)-amino]propionic acid;
3(S)-(2,3-Dihydro-benzofuran-6-yl)-3-[3-(5,6,7,8-tetrahydro-[1,8]-naphthyridin-2-ylmethylsulfanyl)propionylamino]-propionic acid bis(trifluoroacetate);
3-(Quinolin-3-yl)-7-[(5,6,7,8-tetrahydro-[1,8]-naphthyridin-2-ylmethyl)amino]-heptanoic acid;
3-(Quinolin-3-yl)-7-[acetyl-(5,6,7,8-tetrahydro-[1,8]-naphthyridin-2-ylmethyl)amino]-heptanoic acid;
3-(Quinolin-3-yl)-7-[methanesulfonyl-(5,6,7,8-tetrahydro-[1,8]-naphthyridin-2-ylmethyl)amino]-heptanoic acid;
9-(5,6,7,8-Tetrahydro-[1,8]-naphthyridin-2-yl)-nonanoic acid;
2-(Benzenesulfonylamino)-9-(5,6,7,8-tetrahydro-[1,8]-naphthyridin-2-yl)-non-4-enoic acid bis(trifluoroacetate);
2(S)-(Benzenesulfonylamino)-9-(5,6,7,8-tetrahydro-[1,8]naphthyridin-2-yl)-nonanoic acid;
2(R)-(Benzenesulfonylamino)-9-(5,6,7,8-tetrahydro-[1,8]naphthyridin-2-yl)-nonanoic acid;
2(S)-(Benzenesulfonylamino)-10-(5,6,7,8-tetrahydro-[1,8]naphthyridin-2-yl)-decanoic acid;
2(S)-(Benzenesulfonylaniino)-8-(5,6,7,8-tetrahydro-[1,8]naphthyridin-2-yl)-octanoic acid;
2(S)-(Cyclohexylmethanesulfonylamino)-9-(5,6,7,8-tetrahydro-[1,8]naphthyridin-2-yl)-nonanoic acid hydrochloride;
2(S)-(7,7-Dimethyl-2-oxo-bicyclo[2.2.1]hept-1(S)-ylmethanesulfonylamino)-9-(5,6,7,8-tetrahydro-[1,8]naphthyridin-2-yl)-nonanoic acid hydrochloride;
2(S)-(Phenylmethanesulfonylamino)-9-(5,6,7,8-tetrahydro-[1,8]naphthyridin-2-yl)-nonanoic acid;
2(S)-(Cyclohezanesulfonylamino)-9-(5,6,7,8-tetrahydro-[1,8]naphthyridin-2-yl)-nonanoic acid hydrochloride;
2(S)-(Butane-1-sulfonylamino)-9-(5,6,7,8-tetrahydro-[1,8]naphthyridin-2-yl)-nonanoic acid hydrochloride;
2(S)-(3-Benzylureido)-9-(5,6,7,8-tetrahydro-[1,8]naphthyridin-2-yl)-nonanoic acid;
2(S)-(Benzyloxycarbonylamino)-9-(5,6,7,8-tetrahydro-[1,8]naphthyridin-2-yl)-nonanoic acid;
2(S)-(Phenylacetylamino)-9-(5,6,7,8-tetrahydro-[1,8]naphthyridin-2-yl)-nonanoic acid;
2(S)-(Acetylamino)-9-(5,6,7,8-tetrahydro-[1,8]naphthyridin-2-yl)-nonanoic acid;
2(S)-(Benzoylamino)-9-(5,6,7,8-tetrahydro-[1,8]naphthyridin-2-yl)-nonanoic acid;
3-(Quinolin-3-yl)-9-(5,6,7,8-tetrahydro-[1,8]-naphthyridin-2-yl)-nonanoic acid;
3(S)-(Quinolin-3-yl)-9-(5,6,7,8-tetrahydro-[1,8]-naphthyridin-2-yl)-nonanoic acid;
3(R)-(Quinolin-3-yl)-9-(5,6,7,8-tetrahydro-[1,8]-naphthyridin-2-yl)-nonanoic acid;
3-(Quinolin-3-yl)-7-(1,2,3,4,6,7,8,9-octahydro-benzo[b][1,8]-naphthyridin-8-yl)-heptanoic acid bis(hydrochloride);
6-Oxo-3-(quinolin-3-yl)-9-(5,6,7,8-tetrahydro-[1,8]-naphthyridin-2-yl)-nonanoic acid;
3-(N-Oxo-quinolin-3-yl)-9-(5,6,7,8-tetrahydro-[1,8]-naphthyridin-2-yl)-nonanoic acid;
3-(Phenyl)-9-(5,6,7,8-tetrahydro-[1,8]-naphthyridin-2-yl)-nonanoic acid;
3-(Benzo[b]thiophen-2-yl)-9-(5,6,7,8-tetrahydro-[1,8]-naphthyridin-2-yl)-nonanoic acid;
3(R)-(Benzo[b]thiophen-2-yl)-9-(5,6,7,8-tetrahydro-[1,8]-naphthyridin-2-yl)-nonanoic acid;
3(S)-(Benzo[b]thiophen-2-yl)-9-(5,6,7,8-tetrahydro-[1,8]-naphthyridin-2-yl)-nonanoic acid;
3-(Pyridin-3-yl)-9-(5,6,7,8-tetrahydro-[1,8]-naphthyridin-2-yl)-nonanoic acid;
3(R)-(Pyridin-3-yl)-9-(5,6,7,8-tetrahydro-[1,8]-naphthyridin-2-yl)-nonanoic acid;
3(S)-(Pyridin-3-yl)-9-(5,6,7,8-tetrahydro-[1,8]-naphthyridin-2-yl)-nonanoic acid;
3-(3-Fluorophenyl)-9-(5,6,7,8-tetrahydro-[1,8]-naphthyridin-2-yl)-nonanoic acid;
3(R)-(3-Fluorophenyl)-9-(5,6,7,8-tetrahydro-[1,8]-naphthyridin-2-yl)-nonanoic acid;
3(S)-(3-Fluorophenyl)-9-(5,6,7,8-tetrahydro-[1,8]-naphthyridin-2-yl)-nonanoic acid;
3-(2,3-Dihydro-benzofuran-6-yl)-9-(5,6,7,8-tetrahydro-[1,8]-naphthyridin-2-yl)-nonanoic acid;
3(S)-(2,3-Dihydro-benzofuran-6-yl)-9-(5,6,7,8-tetrahydro-[1,8]-naphthyridin-2-yl)-nonanoic acid;
3(R)-(2,3-Dihydro-benzofuran-6-yl)-9-(5,6,7,8-tetrahydro-[1,8]-naphthyridin-2-yl)-nonanoic acid;
3-(2,3-Dihydro-benzofuran-6-yl)-9-(5,6,7,8-tetrahydro-[1,8]-naphthyridin-2-yl)-non-4-enoic acid trifluoroacetate;
3-(2,3-Dihydro-furo[3,2-b)pyridin-5-yl)-9-(5,6,7,8-tetrahydro-[1,8] naphthyridin-2-yl)-nonanoic acid;
3(R)-(2,3-Dihydro-furo[3,2-b]pyridin-5-yl)-9-(5,6,7,8-tetrahydro-[1,8] naphthyridin-2-yl)-nonanoic acid;
3(S)-(2,3-Dihydro-furo[3,2-b]pyridin-5-yl)-9-(5,6,7,8-tetrahydro-[1,8] naphthyridin-2-yl)-nonanoic acid;
3-(Furo[2,3b]pyridin-5-yl)-9-(5,6,7,8-tetrahydro-[1,8]naphthyridin-2-yl)-nonanoic acid;
3(R)-(Furo[2,3b]pyridin-5-yl)-9-(5,6,7,8-tetrahydro-[1,8]naphthyridin-2-yl)-nonanoic acid;
3(S)-(Furo[2,3b]pyridin-5-yl)-9-(5,6,7,8-tetrahydro-[1,8]naphthyridin-2-yl)-nonanoic acid;
3-(2,3-Dihydro-furo[2,3-b]pyridin-5-yl)-9-(5,6,7,8-tetrahydro-[1,8] naphthyridin-2-yl)-nonanoic acid;
3(R)-(2,3-Dihydro-furo[2,3-b]pyridin-5-yl)-9-(5,6,7,8-tetrahydro-[1,8] naphthyridin-2-yl)-nonanoic acid;
3(S)-(2,3-Dihydro-furo[2,3-b]pyridin-5-yl)-9-(5,6,7,8-tetrahydro-[1,8] naphthyridin-2-yl)-nonanoic acid;
3-(6-Methoxy-pyridin-3-yl)-9-(5,6,7,8-tetrahydro-[1,8]-naphthyridin-2-yl)-nonanoic acid;
3(S)-(6-Methoxy-pyridin-3-yl)-9-(5,6,7,8-tetrahydro-[1,8]-naphthyridin-2-yl)-nonanoic acid;
3(R)-(6-Methoxy-pyridin-3-yl)-9-(5,6,7,8-tetrahydro-[1,8]-naphthyridin-2-yl)-nonanoic acid;
3-(Pyrimidin-5-yl)-9-(5,6,7,8-tetrahydro-[1,8]-naphthyridin-2-yl)-nonanaic acid trifluoroacetate;
3(R)-(Pyrimidin-5-yl)-9-(5,6,7,8-tetrahydro-[1,8]-naphthyridin-2-yl)-nonanoic acid trifluoroacetate;
3(S)-(Pyrimidin-5-yl)-9-(5,6,7,8-tetrahydro-[1,8]-naphthyridin-2-yl)-nonanoic acid trifluoroacetate;
3-(6-Amino-pyridin-3-yl)-9-(5,6,7,8-tetrahydro-[1,8]-naphthyridin-2-yl)-nonanoic acid;
3(R)-(6-Amino-pyridin-3-yl)-9-(5,6,7,8-tetrahydro-[1,8]-naphthyridin-2-yl)-nonanoic acid;
3(S)-(6-Amino-pyridin-3-yl)-9-(5,6,7,8-tetrahydro-[1,8]-naphthyridin-2-yl)-nonanoic acid;
3-(Benzo[b]thiazol-2-yl)-9-(5,6,7,8-tetrahydro-[1,8]-naphthyridin-2-yl)-nonanoic acid hydrochloride;
3(R)-(Benzo[b]thiazol-2-yl)-9-(5,6,7,8-tetrahydro-[1,8]-naphthyridin-2-yl)-nonanoic acid hydrochloride;
3(S)-(Benzo[b]thiazol-2-yl)-9-(5,6,7,8-tetrahydro-[1,8]-naphthyridin-2-yl)-nonanoic acid hydrochloride;
3-(6-Oxo-1,6-dihydro-pyridin-3-yl)-9-(5,6,7,8-tetrahydro-[1,8]-naphthyridin-2-yl)-nonanoic acid bis-(trifluoroacetate);
and the pharmaceutically acceptable salts thereof.

Further illustrative of the present invention are the compounds selected from the group consisting of
3(S)-(Pyridin-3-yl)-3-(5-(5,6,7,8-tetrahydro[1,8]naphthyridin-2-yl)-pentanoylamino)-propionic acid;
2(S)-Benzenesulfonylamino-3-(5-(5,6,7,8-tetrahydro-[1,8]naphthyridin-2-yl)-pentanoylamino)-propionic acid trifluoroacetate;
3(S)-(Quinolin-3-yl)-3-(5-(5,6,7,8-tetrahydro-[1,8]naphthyridin-2-yl)-pentanoylamino)-propionic acid;
3(R)-(Quinolin-3-yl)-3-(5-(5,6,7,8-tetrahydro-[1,8]naphthyridin-2-yl)-pentanoylamino)-propionic acid;
2(S)-(2-Thienylsulfonyiamino)-3-(5-(5,6,7,8-tetrahydro-[1,8]naphthyridin-2-yl)-pentanoylamino)-propionic acid trifluoroacetate;
3(S)-(2,3-Dihydro-benzofuran-6-yl)-3-[3-(1,2,3,4,6,7,8(R or S),9-octahydro-benzo[*b*][1,8]naphthyridin-8-yl)-propionylamino]-propionic acid;
3(S)-(2,3-Dihydro-benzofuran-6-yl)-3-[3-(1,2,3,4,6,7,8(S or R),9-octahydro-benzo[*b*][1,8]naphthyridin-8-yl)-propionylamino]-propionic acid;
3(S)-(6-Methoxy-pyridin-3-yl)-3-[N-methyl-3-(1,2,3,4,6,7,8,9-octahydro-benzo[b][1,8]naphthyridin-8-yl-propionyl)-amino]propionic acid;
2-(Benzenesulfonylamino)-9-(5,6,7,8-tetrahydro-[1,8]-naphthyridin-2-yl)-non-4-enoic acid bis(trifluoroacetate);
and the pharmaceutically acceptable salts thereof.

Yet further illustrative are the compounds selected from the group consisting of
3(R)-(Quinolin-3-yl)-9-(5,6,7,8-tetrahydro-[1,8]-naphthyridin-2-yl)-nonanoic acid;
3(S)-(Quinolin-3-yl)-9-(5,6,7,8-tetrahydro-[1,8]-naphthyridin-2-yl)-nonanoic acid;
3(R)-(Benzo[b]thiophen-2-yl)-9-(5,6,7,8-tetrahydro-[1,8]-naphthyridin-2-yl)-nonanoic acid;
3(S)-(Benzo[b]thiophen-2-yl)-9-(5,6,7,8-tetrahydro-[2,8]-naphthyridin-2-yl)-nonanoic acid;
3(R)-(Pyridin-3-yl)-9-(5,6,7,8-tetrahydro-[1,8]-naphthyridin-2-yl)-nonanoic acid;
3(S)-(Pyridin-3-yl)-9-(5,6,7,8-tetrahydro-[1,8]-naphthyridin-2-yl)-nonanoic acid;
3(R)-(3-Fluorophenyl)-9-(5,6,7,8-tetrahydro-[1,8]-naphthyridin-2-yl)-nonanoic acid;
3(S)-(3-Fluorophenyl)-9-(5,6,7,8-tetrahydro-[1,8]-naphthyridin-2-yl)-nonanoic acid;
3(R)-(2,3-Dihydro-benzofuran-6-yl)-9-(5,6,7,8-tetrahydro-[1,8]-naphthyridin-2-yl)-nonanoic acid;
3(S)-(2,3-Dihydro-benzofuran-6-yl)-9-(5,6,7,8-tetrahydro-[1,8]-naphthyridin-2-yl)-nonanoic acid;
3(R)-(2,3-Dihydro-furo[3,2-b]pyridin-5-yl)-9-(5,6,7,8-tetrahydro-[1,8] naphthyridin-2-yl)-nonanoic acid;
3(S)-(2,3-Dihydro-furo[3,2-b]pyridin-5-yl)-9-(5,6,7,8-tetrahydro-[1,8] naphthyridin-2-yl)-nonanoic acid;
3(R)-(Furo[2,3b]pyridin-5-yl)-9-(5,6,7,8-tetrahydro-[1,8]naphthyridin-2-yl)-nonanoic acid;
3(S)-(Furo[2,3b]pyridin-5-yl)-9-(5,6,7,8-tetrahydro-[1,8]naphthyridin-2-yl)-nonanoic acid;
3(R)-(2,3-Dihydro-furo[2,3-b]pyridin-5-yl)-9-(5,6,7,8-tetrahydro-[1,8] naphthyridin-2-yl)-nonanoic acid;
3(S)-(2,3-Dihydro-furo[2,3-b]pyridin-5-yl)-9-(5,6,7,8-tetrahydro-[1,8] naphthyridin-2-yl)-nonanoic acid;
3(R)-(6-Methoxy-pyridin-3-yl)-9-(5,6,7,8-tetrahydro-[1,8]-naphthyridin-2-yl)-nonanoic acid;
3(S)-(6-Methoxy-pyridin-3-yl)-9-(5,6,7,8-tetrahydro-[1,8]-naphthyridin-2-yl)-nonanoic acid;
3(R)-(Pyrimidin-5-yl)-9-(5,6,7,8-tetrahydro-[1,8]-naphthyridin-2-yl)-nonanoic acid;
3(S)-(Pyrimidin-5-yl)-9-(5,6,7,8-tetrahydro-[1,8]-naphthyridin-2-yl)-nonanoic acid;
3(R)-(6-Amino-pyridin-3-yl)-9-(5,6,7,8-tetrahydro-[1,8]-naphthyridin-2-yl)-nonanoic acid;
3(S)-(6-Amino-pyridin-3-yl)-9-(5,6,7,8-tetrahydro-[1,8]-naphthyridin-2-yl)-nonanoic acid;
3(R)-(Benzo[b]thiazol-2-yl)-9-(5,6,7,8-tetrahydro-[1,8]-naphthyridin-2-yl)-nonanoic acid hydrochloride;
3(S)-(Benzo[b]thiazol-2-yl)-9-(5,6,7,8-tetrahydro-[1,8]-naphthyridin-2-yl)-nonanoic acid hydrochloride;
and the pharmaceutically acceptable salts thereof.

For use in medicine, the salts of the compounds of this invention refer to non-toxic "pharmaceutically acceptable salts." Other salts may, however, be useful in the preparation of the compounds according to the invention or of their pharmaceutically acceptable salts. Salts encompassed within the term "pharmaceutically acceptable salts" refer to non-toxic salts of the compounds of this invention which are generally prepared by reacting the free base with a suitable organic or inorganic acid. Representative salts include the following: acetate, benzenesulfonate, benzoate, bicarbonate, bisulfate, bitartrate, borate, bromide, calcium, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, oxalate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

The compounds of the present invention can have chiral centers and can thus occur as racemates, racemic mixtures, single enantiomers, diastereomeric mixtures, and individual diastereomers, with all isomeric forms being included in the present invention. Therefore, where a compound is chiral, the separate enantiomers or diastereomers, substantially free of the other, are included within the scope of the invention; further included are all mixtures of the two enantiomers.

Some of the compounds described herein contain olefinic double bonds, and unless specified otherwise, are meant to include both E and Z geometric isomers.

Some of the compounds described herein may exist with different points of attachment of hydrogen, referred to as tautomers. Such an example may be a ketone and its enol form, known as keto-enol tautomers. The individual tautomers as well as mixtures thereof are encompassed within the compounds of the present invention.

Compounds of the present invention may be separated into diastereoisomeric pairs of enantiomers by, for example, fractional crystallization from a suitable solvent, for example, methanol or ethyl acetate or a mixture thereof. The pair of enantiomers thus obtained may be separated into individual stereoisomers by conventional means, for example, by the use of an optically active acid as a resolving agent, or by HPLC using a chiral stationary phase. Alternatively, any enantiomer of a compound of the present invention may be obtained by stereospecific synthesis using optically pure starting materials or reagents of known configuration.

Also included within the scope of the invention are polymorphs and hydrates of the compounds of the instant invention.

The present invention includes within its scope prodrugs of the compounds of this invention. In general, such prodrugs will be functional derivatives of the compounds of this invention which are readily convertible *in vivo* into the required compound. Thus, in the methods of treatment of the present invention, the term "administering" shall encompass the treatment of the various conditions described with the compound specifically disclosed or with a compound which may not be specifically disclosed, but which converts to the specified compound in vivo after administration to the patient. Conventional procedures for the selection and preparation of suitable prodrug derivatives are described, for example, in "Design of Prodrugs," ed. H. Bundgaard, Elsevier, 1985, which is incorporated by reference herein in its entirety. Metabolites of these compounds include active species produced upon introduction of compounds of this invention into the biological milieu.

The term "therapeutically effective amount" shall mean that amount of a drug or pharmaceutical agent that will elicit the biological or medical response of a tissue, system, animal or human that is being sought by a researcher or clinician.

The term "integrin receptor antagonist," as used herein, refers to a compound which binds to and antagonizes either the αvβ3 receptor, the αvβ5 receptor, or the αvβ6 receptor, or a compound which binds to and antagonizes combinations of these receptors (for example, a dual αvβ3/αvβ5 receptor antagonist).

The term "bone resorption," as used herein, refers to the process by which osteoclasts degrade bone.

The term "alkyl" shall mean straight or branched chain alkanes of one to ten total carbon atoms, or any number within this range (i.e., methyl, ethyl, 1-propyl, 2-propyl, n-butyl, s-butyl, t-butyl, etc.).

The term "alkenyl" shall mean straight or branched chain alkenes of two to ten total carbon atoms, or any number within this range.

The term "alkynyl" shall mean straight or branched chain alkynes of two to ten total carbon atoms, or any number within this range.

The term "cycloalkyl" shall mean cyclic rings of alkanes of three to eight total carbon atoms, or any number within this range (i.e., cyclopropyl, cyclobutyl, cyclopentyl, cyclohexyl, cycloheptyl or cyclooctyl).

The term "cycloheteroalkyl," as used herein, shall mean a 3- to 8-membered fully saturated heterocyclic ring containing one or two heteroatoms chosen from N, O, or S. Examples of cycloheteroalkyl groups include, but are not limited to piperidinyl, pyrrolidinyl, azetidinyl, morpholinyl, piperazinyl.

The term "alkoxy," as used herein, refers to straight or branched chain alkoxides of the number of carbon atoms specified (e.g., C₁₋₅ alkoxy), or any number within this range (i.e., methoxy, ethoxy, etc.).

Whenever the term "alkyl" or "aryl" or either of their prefix roots appears in a name of a substituent (e.g., aryl C₀₋₈ alkyl), it shall be interpreted as including those limitations given above for "alkyl" and "aryl." Designated numbers of carbon atoms (e.g., C₁₋₁₀) shall refer independently to the number of carbon atoms in an alkyl or cyclic alkyl moiety or to the alkyl portion of a larger substituent in which alkyl appears as its prefix root.

The terms "arylalkyl" and "alkylaryl" include an alkyl portion where alkyl is as defined above and to include an aryl portion where aryl is as defined above. Examples of arylalkyl include, but are not limited to, benzyl, fluorobenzyl, chlorobenzyl, phenylethyl, phenylpropyl, fluorophenylethyl, chlorophenylethyl, thienylmethyl, thienylethyl, and thienylpropyl. Examples of alkylaryl include, but are not limited to, toluene, ethylbenzene, propylbenzene, methylpyridine, ethylpyridine, propylpyridine and butylpyridine.

In the compounds of the present invention, two R³ substituents, when on the same carbon atom, can be taken together with the carbon atom to which they are attached to form a carbonyl group. In such instances, the limitation, that in the resultant compound the carbon atom or atoms at which R³ is attached is itself attached to no more than one heteroatom, does not apply. Also, two R³ substituents, when on the same carbon atom, can be taken together with the carbon atom to which they are attached to form a cyclopropyl group.

In the compounds of the present invention, R⁵ and R⁶ can be taken together to form a carbonyl group. In such instances, the limitation, that in the resultant compound the carbon atom at which R⁵ and R⁶ is attached is itself attached to no more than one heteroatom, does not apply.

The term "halogen" shall include iodine, bromine, chlorine, and fluorine.

The term "oxy" means an oxygen (O) atom. The term "thio" means a sulfur (S) atom. The term "oxo" means "=O". The term "carbonyl" means "C=O."

The term "substituted" shall be deemed to include multiple degrees of substitution by a named substitutent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

Under standard nonmenclature used throughout this disclosure, the terminal portion of the designated side chain is described first, followed by the adjacent functionality toward the point of attachment. For example, a C₁₋₅ alkylcarbonylamino C₁₋₆ alkyl substituent is equivalent to

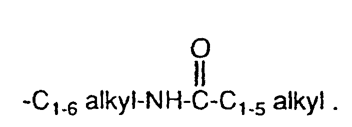

In choosing compounds of the present invention, one of ordinary skill in the art will recognize that the various substituents, i.e. X, Y, Z, R¹, R³, R⁴, R⁵, R6, R⁷, R⁸ and R⁹, and the subscripts m, n, p, r, s, and t are to be chosen in conformity with well-known principles of chemical structure connectivity.

Representative compounds of the present invention typically display submicromolar affinity for the integrin receptors, particularly the αvβ3, αvβ5, and/or αvβ6 receptors. Compounds of this invention are therefore useful for treating mammals suffering from a bone condition caused or mediated by increased bone resorption, who are in need of such therapy. Pharmacologically effective amounts of the compounds, including pharamaceutically acceptable salts thereof, are administered to the mammal, to inhibit the activity of mammalian osteoclasts.

The compounds of the present invention are administered in dosages effective to antagonize the αvβ3 receptor where such treatment is needed, as, for example, in the prevention or treatment of osteoporosis.

Further exemplifying the invention is the use wherein the integrin receptor antagonizing effect is an αvβ3 antagonizing effect. An illustration of the invention is the use wherein the αvβ3 antagonizing effect is selected from inhibition of bone resorption, restenosis, angiogenesis, diabetic retinopathy, macular degeneration, inflammation, viral disease, tumor growth, or metastasis. Preferably, the αvβ3 antagonizing effect is the inhibition of bone resorption.

An example of the invention is the use wherein the integrin receptor antagonizing effect is an αvβ5 antagonizing effect. More specifically, the αvβ5 antagonizing effect is selected from inhibition of restenosis, angiogenesis, diabetic retinopathy, macular degeneration, inflammation, tumor growth, or metastasis.

Illustrating the invention is the use wherein the integrin receptor antagonizing effect is a dual αvβ3/αvβ5 antagonizing effect. More particularly, the dual αvβ3/αvβ5 antagonizing effect is selected from inhibition of: bone resorption, restenosis, angiogenesis, diabetic retinopathy, macular degeneration, inflammation, viral disease, tumor growth, or metastasis.

Illustrating the invention is the use wherein the integrin receptor antagonizing effect is an αvβ6 antagonizing effect. More particularly, the αvβ6 antagonizing effect is selected from inhibition of angiogenesis, inflammatory response, or wound healing.

Illustrating the invention is the use wherein the αvβ3 antagonizing effect is selected from inhibition of bone resorption, inhibition of restenosis, inhibition of angiogenesis, inhibition of diabetic retinopathy, inhibition of macular degeneration, inhibition of atherosclerosis, inflammation, viral disease, or inhibition of tumor growth and metastasis. Preferably, the αvβ3 antagonizing effect is the inhibition of bone resorption.

More particularly illustrating the invention is a pharmaceutical composition comprising any of the compounds described above and a pharmaceutically acceptable carrier. Another example of the invention is a pharmaceutical composition made by combining any of the compounds described above and a pharmaceutically acceptable carrier. Another illustration of the invention is a process for making a pharmaceutical composition comprising combining any of the compounds described above and a pharmaceutically acceptable carrier.

Further illustrating the invention is the use of any of the compounds described above for the manufacture of a medicament for treating and/or preventing a condition mediated by antagonism of an integrin receptor. Preferably, the condition is selected from bone resorption, osteoporosis, restenosis, diabetic retinopathy, macular degeneration, angiogenesis, atherosclerosis, inflammation, viral disease, cancer, tumor growth, and metastasis. More preferably, the condition is selected from osteoporosis and cancer. Most preferably, the condition is osteoporosis.

More specifically exemplifying the invention is the use of any of the compounds or any of the pharmaceutical compositions described above for the manufacture of a medicament for eliciting an integrin antagonizing effect.

Preferably, the integrin antagonizing effect is an αvβ3 antagonizing effect; more specifically the αvβ3 antagonizing effect is selected from inhibition of bone resorption, inhibition of restenosis, inhibition of atherosclerosis, inhibition of angiogenesis, inhibition of diabetic retinopathy, inhibition of macular degeneration, inhibition of inflammation, inhibition of viral disease, or inhibition of tumor growth or metastasis. Most preferably, the αvβ3 antagonizing effect is inhibition of bone resorption. Alternatively, the integrin antagonizing effect is an αvβ5 antagonizing effect, an αvβ6 antagonizing effect, or a mixed αvβ3, αvβ5, and αvβ6 antagonizing effect. Examples of αvβ5 antagonizing effects are inhibition of restenosis, atherosclerosis, angiogenesis, diabetic retinopathy, macular degeneration, inflammation, or tumor growth. Examples of αvβ6 antagonizing effects are inhibition of angiogenesis, inflammatory response, and wound healing.

Additional examples of the invention are uses of any of the compounds or any of the pharmaceutical compositions described above for the manufacture of a medicament for inhibiting bone resorption and of treating and/or preventing osteoporosis.

Additional illustrations of the invention are uses of any of the compounds or any of the pharmaceutical compositions described above for the manufacture of a medicament for treating hypercalcemia of malignancy, osteopenia due to bone metastases, periodontal disease, hyperparathyroidism, periarticular erosions in rheumatoid arthritis, Paget's disease, immobilization-induced osteopenia, and glucocorticoid treatment.

More particularly exemplifying the invention is the use of any of the compounds described above in the preparation of a medicament for the treatment and/or prevention of osteoporosis.

Still further exemplifying the invention is the use of any of the compounds described above in the preparation of a medicament for the treatment and/or prevention of bone resorption, tumor growth, cancer, restenosis, atherosclerosis, diabetic retinopathy, macular degeneration, inflammation, viral disease, and/or angiogenesis.

Also exemplifying the invention are compositions further comprising an active ingredient selected from the group consisting of
a.) an organic bisphosphonate or a pharmaceutically acceptable salt or ester thereof,
b.) an estrogen receptor modulator,
c.) a cytotoxic/antiproliferative agent,
d.) a matrix metalloproteinase inhibitor,
e.) an inhibitor of epidermal-derived, fibroblast-derived, or platelet-derived growth factors,
f.) an inhibitor of VEGF,
g.) an inhibitor of Flk-1/KDR, Flt-1, Tck/Tie-2, or Tie-1,
h.) a cathepsin K inhibitor, and
i.) a prenylation inhibitor, such as a farnesyl transferase inhibitor or a geranylgeranyl transferase inhibitor or a dual farnesyl/geranylgeranyl transferase inhibitor; and mixtures thereof.
(*See* B. Millauer et al., "Dominant-Negative Inhibition of Flk-1 Suppresses the Growth of Many Tumor Types *in Vivo*", Cancer Research, 56, 1615-1620 (1996), which is incorporated by reference herein in its entirety).

Preferably, the active ingredient is selected from the group consisting of:
a.) an organic bisphosphonate or a pharmaceutically acceptable salt or ester thereof,
b.) an estrogen receptor modulator, and
c.) a cathepsin K inhibitor; and mixtures thereof.

Nonlimiting examples of such bisphosphonates include alendronate, etidronate, pamidronate, risedronate, ibandronate, and pharmaceutically acceptable salts and esters thereof. A particularly preferred bisphosphonate is alendronate, especially alendronate monosodium trihydrate.

Nonlimiting examples of estrogen receptor modulators include estrogen, progesterin, estradiol, droloxifene, raloxifene, and tamoxifene.

Nonlimiting examples of cytotoxic/antiproliferative agents are taxol, vincristine, vinblastine, and doxorubicin.

Cathepsin K, formerly known as cathepsin O2, is a cysteine protease and is described in PCT International Application Publication No. WO 96/13523, published May 9,1996; U.S. Patent No. 5,501,969, issued March 3, 1996; and U.S. Patent No. 5,736,357, issued April 7, 1998, all of which are incorporated by reference herein in their entirety. Cysteine proteases, specifically cathepsins, are linked to a number of disease conditions, such as tumor metastasis, inflammation, arthritis, and bone remodeling. At acidic pH's, cathepsins can degrade type-I collagen. Cathepsin protease inhibitors can inhibit osteoclastic bone resorption by inhibiting the degradation of collagen fibers and are thus useful in the treatment of bone resorption diseases, such as osteoporosis.

The present invention is also directed to combinations of the compounds of the present invention with one or more agents useful in the prevention or treatment of osteoporosis. For example, the compounds of the instant invention may be effectively administered in combination with effective amounts of other agents such as an organic bisphosphonate, an estrogen receptor modulator, or a cathepsin K inhibitor.

Additional illustrations of the invention are the combination of a compound described above and one or more agents known to be cytotoxic/antiproliferative for treating tumor growth or metastasis.

Also, the compounds of the present invention can be administered in combination with radiation therapy for treating tumor growth and metastasis.

In addition, the integrin αvβ3 antagonist compounds of the present invention may be effectively administered in combination with a growth hormone secretagogue in the therapeutic or prophylactic treatment of disorders in calcium or phosphate metabolism and associated diseases. These diseases include conditions which can benefit from a reduction in bone resorption. A reduction in bone resorption should improve the balance between resorption and formation, reduce bone loss or result in bone augmentation. A reduction in bone resorption can alleviate the pain associated with osteolytic lesions and reduce the incidence and/or growth of those lesions. These diseases include: osteoporosis (including estrogen deficiency, immobilization, glucocorticoid-induced and senile), osteodystrophy, Paget's disease, myositis ossificans, Bechterew's disease, malignant hypercalcemia, metastatic bone disease, periodontal disease, cholelithiasis, nephrolithiasis, urolithiasis, urinary calculus, hardening of the arteries (sclerosis), arthritis, bursitis, neuritis and tetany. Increased bone resorption can be accompanied by pathologically high calcium and phosphate concentrations in the plasma, which would be alleviated by this treatment. Similarly, the present invention would be useful in increasing bone mass in patients with growth hormone deficiency. Thus, preferred combinations are simultaneous or alternating treatments of an αvβ3 receptor antagonist of the present invention and a growth hormone secretagogue, optionally including a third component comprising an organic bisphosphonate, preferably alendronate monosodium trihydrate.

In accordance with the use of the present invention, the individual components of the combination can be administered separately at different times during the course of therapy or concurrently in divided or single combination forms. The instant invention is therefore to be understood as embracing all such regimes of simultaneous or alternating treatment, and the term "administering" is to be interpreted accordingly. It will be understood that the scope of combinations of the compounds of this invention with other agents useful for treating integrin-mediated conditions includes in principle any combination with any pharmaceutical composition useful for treating osteoporosis.

As used herein, the term "composition" is intended to encompass a product comprising the specified ingredients in the specified amounts, as well as any product which results, directly or indirectly, from combination of the specified ingredients in the specified amounts.

The compounds of the present invention can be administered in such oral dosage forms as tablets, capsules (each of which includes sustained release or timed release formulations), pills, powders, granules, elixirs, tinctures, suspensions, syrups and emulsions. Likewise, they may also be administered in intravenous (bolus or infusion), intraperitoneal, topical (e.g., ocular eyedrop), subcutaneous, intramuscular or transdermal (e.g., patch) form, all using forms well known to those of ordinary skill in the pharmaceutical arts. An effective but non-toxic amount of the compound desired can be employed as an αvβ3 antagonist.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 to 10 mg/kg/day, and most preferably 0.1 to 5.0 mg/kg/day. For oral administration, the compositions are preferably provided in the form of tablets containing 0.01, 0.05, 0.1, 0.5, 1.0, 2.5, 5.0, 10.0, 15.0, 25.0, 50.0, 100 and 500 milligrams of the active ingredient for the symptomatic adjustment of the dosage to the patient to be treated. A medicament typically contains from about 0.01 mg to about 500 mg of the active ingredient, preferably, from about 1 mg to about 100 mg of active ingredient. Intravenously, the most preferred doses will range from about 0.1 to about 10 mg/kg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

In the uses of the present invention, the compounds herein described in detail can form the active ingredient, and are typically administered in admixture with suitable pharmaceutical diluents, excipients or carriers (collectively referred to herein as 'carrier' materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxy-ethylaspartamide-phenol, or polyethyleneoxide-polylysine substituted with palmitoyl residues. Furthermore, the compounds of the present invention may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

In the schemes and examples below, various reagent symbols and abbreviations have the following meanings:

| | |
|---|---|
| AcOH | Acetic acid. |
| BH₃●DMS | Borane●dimethylsulfide. |
| BOC(Boc) | t-Butyloxycarbonyl. |
| BOP | Benzotriazol-1-yloxytris(dimethylamino)-phosphonium hexafluorophosphate. |
| CBZ(Cbz) | Carbobenzyloxy or benzyloxycarbonyl. |
| CDI | Carbonyldiimidazole. |
| CH₂Cl₂ | Methylene chloride. |
| CH₃CN | Acetonitrile |
| CHCl₃ | Chloroform. |
| DEAD | Diethyl azodicarboxylate. |
| DIAD | Diisopropyl azodicarboxylate. |
| DIBAH or DIBAL-H | Diisobutylaluminum hydride. |
| DIPEA | Diisopropylethylamine. |
| DMAP | 4-Dimethylaminopyridine. |
| DME | 1,2-Dimethoxyethane. |
| DMF | Dimethylformamide. |
| DMSO | Dimethylsulfoxide. |
| DPFN | 3,5-Dimethyl-1-pyrazolylformamidine nitrate. |
| EDC | 1-(3-Dimethylaminopropyl)-3-ethylcarbodiimide •HCl |
| EtOAc | Ethyl acetate. |
| EtOH | Ethanol. |
| HOAc | Acetic acid. |
| HOAT | 1-Hydroxy-7-azabenzotriazole |
| HOBT | 1-Hydroxybenzotriazole. |
| IBCF | Isobutylchloroformate |
| LDA | Lithium diisopropylamide. |
| MeOH | Methanol. |
| MMNG | 1,1-methyl-3-nitro-1-nitrosoguanidine |
| NEt₃ | Triethylamine. |
| NMM | N-methylmorpholine. |
| PCA●HCl | Pyrazole carboxamidine hydrochloride. |
| Pd/C | Palladium on activated carbon catalyst. |
| Ph | Phenyl. |
| PyCLU | Chloro-N,N,N',N'-(tetramethylene)-formamidinium hexafluorophosphate. |
| pTSA | p-Toluenesulfonic acid. |
| TEA | Triethylamine. |
| TFA | Trifluoroacetic acid. |
| THF | Tetrahydrofuran. |
| TLC | Thin Layer Chromatography. |
| TMEDA | N,N,N',N'-Tetramethylethylenediamine. |
| TMS | Trimethylsilyl. |

The novel compounds of the present invention can be prepared according to the procedure of the following schemes and examples, using appropriate materials and are further exemplified by the following specific examples. The compounds illustrated in the examples are not, however, to be construed as forming the only genus that is considered as the invention. The following examples further illustrate details for the preparation of the compounds of the present invention. Those skilled in the art will readily understand that known variations of the conditions and processes of the following preparative procedures can be used to prepare these compounds. All temperatures are degrees Celsius unless otherwise noted.

The following Schemes and Examples describe procedures for making representative compounds of the present invention. Moreover, by utilizing the procedures described in detail in PCT International Application Publication Nos. WO95/32710, published 7 December 1995, and WO95/17397, published 29 June 1995, both of which are incorporated by reference herein in their entirety, in conjunction with the disclosure contained herein, one of ordinary skill in the art can readily prepare additional compounds of the present invention claimed herein. Additionally, for a general review describing the synthesis of β-alanines which can be utilized as the C-terminus of the compounds of the present invention, see Cole, D.C., *Recent Stereoselective Synthetic Approaces to β-Amino Acids, Tetrahedron*, 1994, 50, 9517-9582; Juaristi, E, et al., *Enantioselective Synthesis of β-Amino Acids, Aldrichimica Acta,* 1994, 27, 3. In particular, synthesis of the 3-methyl-β-alanine is taught in Duggan, M.F. et al., *J. Med. Chem.,* 1995, 38, 3332-3341; the 3-ethynyl-β-alanine is taught in Zablocki, J.A., et al., *J. Med. Chem*., 1995, 38, 2378-2394; the 3-(pyridin-3-yl)-β-alanine is taught in Rico, J.G. et al., *J. Org. Chem.,* 1993, 58, 7948-7951; and the 2-amino- and 2-tosylamino-β-alanines are taught in Xue, C-B, et al., *Biorg. Med. Chem. Letts.,* 1996, 6, 339-344. The references described in this paragraph are all also incorporated by reference herein in their entirety.

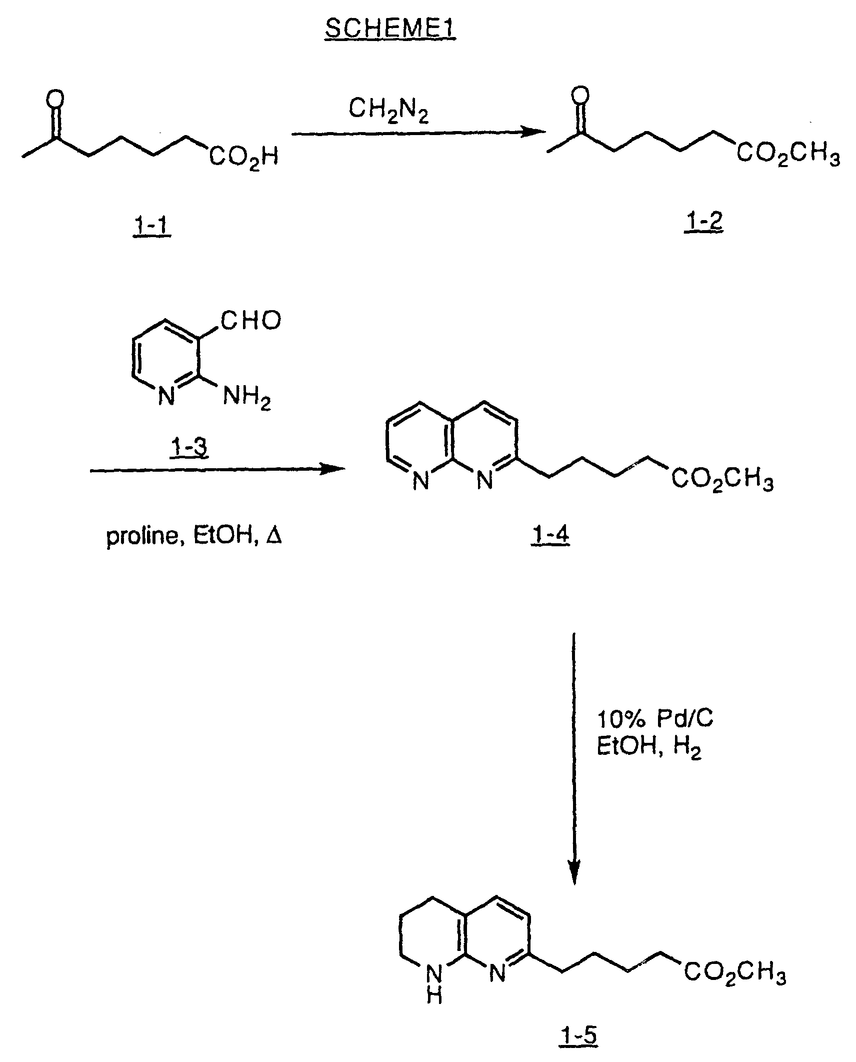

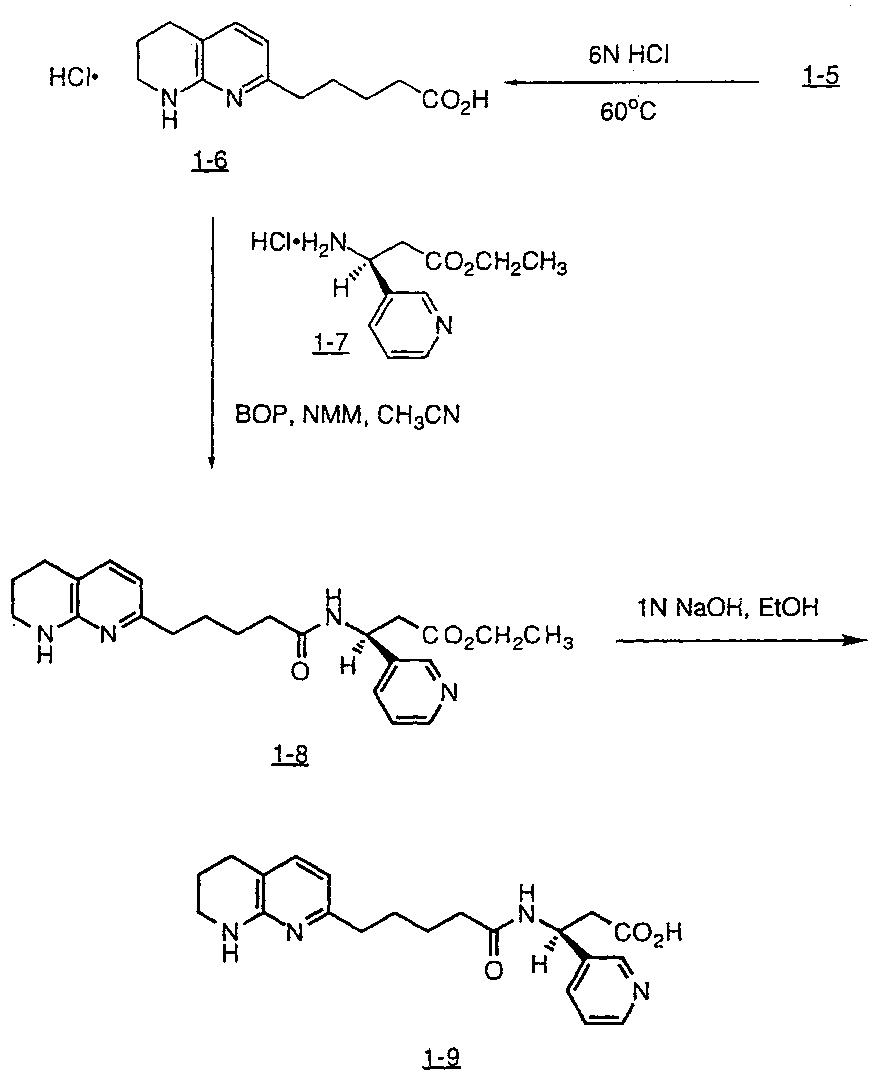

### 6-Oxo-heptanoic acid methyl ester (1-2)

To a rapidly stirred mixture of diethyl ether (175 ml) and 40% KOH (52 ml) at 0°C was added MNNG (15.4 g, 105 mmol). The mixture was stirred for 10 minutes. The ethereal layer was transferred to a solution of 6-oxo-heptanoic acid 1-1 (5.0 g, 34.68 mmol) and CH₂Cl₂ at 0°C. The solution was purged with argon for 30 minutes and then concentrated. Flash chromatography (silica, 30% to 50% EtOAc/hexanes) gave ester 1-2 as a clear oil.
TLC R_{f}= 0.88 (silica, EtOAc)
¹H NMR (300 MHz, CDCl₃) δ 3.67 (s, 3H), 2.46 (m,2H), 2.33 (m, 2H), 2.14 (s, 3H), 1.62 (m, 4H).

### 5-[1,8]-Naphthyridin-2-yl-pentanoic acid methyl ester (1-4)

A mixture of 1-2 (1.4 g, 9.04 mmol), 1-3, 2-amino-3-formylpyridine (552 mg, 4.52 mmol) (for preparation, see: *J. Org. Chem*., 1983, 48, 3401), and proline (260 mg, 2.26 mmol) in absolute ethanol (23 mL) was heated at reflux for 18 h. Following evaporative removal of the solvent, the residue was chromatographed (silica gel, 80% ethyl acetate/hexane, then ethyl acetate) to give ester 1-4 as a white solid.
TLC R_{f} = 0.38 (silica, EtOAc)
¹H NMR (300 MHz, CDCl₃) δ 9.08 (m, 1H), 8.16 (d, J=8.0 Hz, 1H), 8.10 (d, J=8.3 Hz, 1H), 7.45 (m, 1H), 7.39 (d, J=8.3 Hz, 1H), 3.66 (s, 3H), 3.08 (t, J=7.6 Hz, 2H), 2.39 (t, J=7.6 Hz, 2H), 1.94 (m,2H), 1.78 (m, 2H).

### 5-(5,6,7,8-Tetrahydro-[1,8]naphthyridin-2-yl)-pentanoic acid methyl ester (1-5)

A mixture of 1-4 (630 mg, 2.58 mmol) and 10% Pd/carbon (95 mg) in EtOH (25 mL) was stirred under a balloon of hydrogen for 72 h. Following filtration and evaporative removal of the solvent, the residue was chromatographed (silica gel, 70% ethyl acetate/hexanes) to give 1-5 as a colorless oil.
TLC R_{f} = 0.58 (silica, ethyl acetate).
¹H NMR (300 MHz, CDCl₃) δ 7.05 (d, J=7.3 Hz, 1H), 6.34 (d, J=7.3 Hz, 1H), 4.72 (s, 1H), 3.66 (s, 3H), 3.40 (m, 2H), 2.69 (t, J=6.3 Hz, 2H), 2.53 (m, 2H), 2.33 (m, 2H), 1.90 (m, 2H), 1.66 (m, 4H).

### 5-(5,6,7,8-Tetrahydro-[1,8]naphthyridin-2-yl)-pentanoic acid hydrochloride (1-6)

A mixture of 1-5 (620 mg, 2.50 mmol) and 6N HCl (12 mL) was heated at 50° C for 18 h. Evaporative removal of the solvent gave 1-6 as a yellow solid.
¹H NMR (300 MHz, CD₃OD) δ 7.59 (d, J=7.3 Hz, 1H), 6.63 (d, J=7.3 Hz, 1H), 3.48 (m, 2H), 2.82 (m, 2H), 2.72 (m, 2H), 2.35 (m, 2H),1.95 (m, 2H), 1.69 (m, 4H).

### 3(S)-(Pyridin-3-yl)-3-(5-(5,6,7,8-tetrahydro-[1,8]naphthyridin-2-yl)-pentanoylamino)propionic acid ethyl ester (1-8)

A mixture of 1-6 (50 mg, 0.1847 mmol), 1-7 (49 mg, 0.1847 mmol) (Rico et al., *J. Org. Chem*., 1993, 58, 7948), BOP (90 mg, 0.2032 mmol) and NMM (0.122 mL, 1.11 mmol) in CH₃CN (2 mL) was stirred for 18 h. The mixture was diluted with ethyl acetate, washed with sat. NaHCO₃, brine, and dried over MgSO₄. Following evaporative removal of the solvent, the residue was chromatographed (silica gel, 20% MeOH/ethyl acetate) to give 1-8 as a yellow oil.
TLC Rf = 0.23 (20% MeOH/ethyl acetate).
¹H NMR (300 MHz, CDCl₃) δ 8.54 (s,1H), 8.43 (d, J=4.9Hz, 1H), 8.15 (d, J=8.3 Hz, 1H), 7.85 (m, 1H), 7.39 (m, 1H), 7.15 (d, J=7.3Hz, 1H), 6.34 (d, J=7.3 Hz, 1H), 5.37 (t, J=7.3 Hz, 1H), 4.05 (q, J=7.1Hz, 2H), 3.38 (t, J=5.5 Hz, 2H), 2.64 (m,4H),2.52 (m, 2H), 2.22 (m, 2H), 1.86 (m, 2H), 1.64 (m, 4H), 1.16 (t, J=7.1 Hz,3H).

### 3(S)-(Pyridin-3-yl)-3-(5-(5,6,7,8-tetrahydro-[1,8]naphthyridin-2-yl)-pentanoylamino)propionic acid (1-9)

To a solution of 1-8 (0.1847 mmol) in EtOH (2 mL) was added 1N NaOH (0.250 ml, 0.250 mmol). After stirring for 2 h, the solvents were evaporated and the residue was chromatographed (silica gel, 15:10:1:1 ethyl acetate/EtOH/water/NH₄OH) to give 1-9 as a white solid.
TLC R_{f} = 0.15 (15:10:1:1 ethyl acetate/EtOH/water/NH₄OH).
¹H NMR (300 MHz, CD₃OD) δ 8.55 (s, 1H), 8.42 (d, J=4.9Hz, 1H), 7.84 (d, J=8.1 Hz, 1H), 7.46 (d, J=7.3 Hz, 1H), 7.41 (m, 1H), 6.50 (d,J=7.1 Hz, 1H), 5.42 (m, 1H,), 3.47 (t, J=5.6 Hz, 2H), 2.55 to 2.81 (m, 6H), 2.41 (m, 1H), 2.34 (m, 1H), 2.93 (m, 2H), 1.71 (m, 4H).

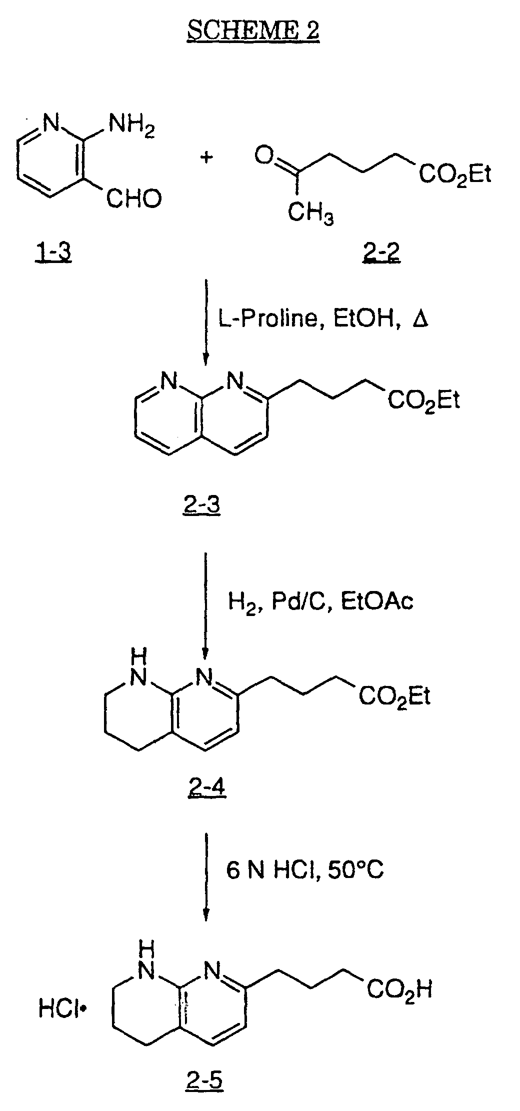

### Ethyl 4-(1,8-naphthyridin-2-yl)butanoate (2-3)

Aminoaldehyde 1-3 (2.02 g, 16.6 mmol, prepared according to *Het.* 1993, 36, 2513), ketone 2-2 (5.3 mL, 33.1 mmol) and L-proline (0.48 g, 4.17 mmol) were combined in 75 mL EtOH. After heating at reflux overnight, the reaction was concentrated. Flash chromatography (silica, EtOAc) provided 2-3 as an off-white crystalline solid.
TLC R_{f} 0.23 (silica, EtOAc)
¹H NMR (300 MHz, CDCl₃): δ 9.09 (dd, J=4, 2Hz, 1H), 8.17 (dd, J=8, 2Hz, 1H), 8.12 (d, J=8Hz, 1H), 7.46 (dd, J=8, 4Hz, 1H), 7.42 (d, J=8Hz, 1H), 4.12 (q, J=7Hz, 2H), 3.11 (t, J=8Hz, 2H), 2.44 (t, J=7Hz, 1H), 2.26 (qn, J=8Hz, 2H), 1.25 (t, J=7Hz, 3H).

### Ethyl 4-(1,2,3,4-tetrahydro-1,8-naphthyridin-7-yl)butanoate (2-4)

A solution of 2-3 (2.3 g, 9.4 mmol) in 50 mL EtOAc was treated with 10% Pd/C (230 mg) and a hydrogen balloon. After 4 days the reaction filtered through celite, concentrated, and purified by flash chromatography (silica, 70% EtOAc/hexane), providing 2-4 as a yellow oil.
TLC R_{f} 0.40 (silica, EtOAc)
¹H NMR (300 MHz, CDCl₃): δ 7.05 (d, J=7Hz, 1H), 6.35 (d, J=7Hz, 1H), 4.73 (br s, 1H), 4.12 (q, J=7Hz, 2H), 2.69 (t, J=6Hz, 2H), 2.57 (t, J=8Hz, 2H), 2.33 (t, J=7Hz, 2H), 1.98 (m, 2H), 1.90 (m, 2H), 1.25 (t, J=7Hz, 3H).

### 4-(1,2,3,4-Tetrahydro-1,8-naphthyridin-7-yl)butanoic acid hydrochloride (2-5)

Ester 2-4 (1.8 g, 7.25 mmol) in 36 mL 6 N HCl was heated at 50°C for 4 h, then concentrated, providing 2-5 as a yellow solid.
¹H NMR (300 MHz, CD₃OD): δ 7.59 (d, J=7Hz, 1H), 6.63 (d, J=7Hz, 1H), 3.50 (t, J=5Hz, 2H), 2.82 (t, J=6Hz, 2H), 2.74 (t, J=8Hz, 2H), 2.38 (t, J=7Hz, 2H), 2.02-1.90 (m, 4H).

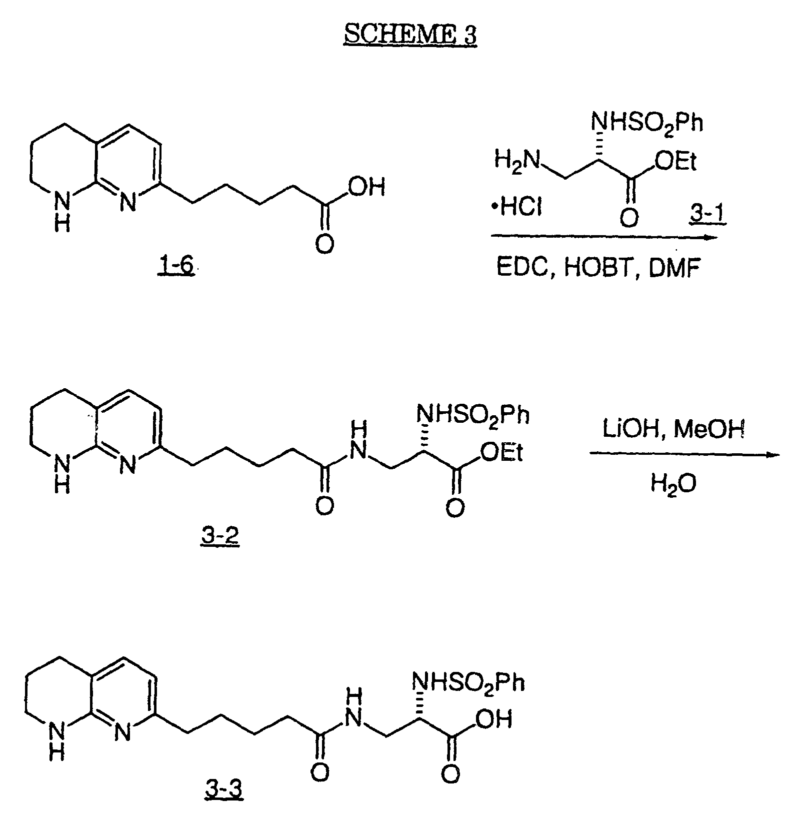

### 2(S)-Benzenesulfonylamino-3-(5-(5,6,7,8-tetrahydro-[1,8]naphthyridin-2-yl)-pentanoylamino)-gropionic acid ethyl ester ditrifluoroacetate salt (3-2)

A solution of 5-(5,6,7,8-tetrahydro-[1,8]naphthyridin-2-yl)-pentanoic acid hydrochloride (1-6) (304 mg, 0.85 mmol) in DMF (5 mL) was treated successively with HOBT (115 mg, 0.85 mmol), the amine 3-1 (prepared in a similar fashion as intermediate A-4 but substituting benzenesulfonyl chloride in place of 4-iodo-benzenesulfonyl chloride) (263 mg, 0.85 mmol), EDC (195 mg, 1.02 mmol) and triethylamine (0.24 mL, 1.71 mmol). The resulting solution was stirred at room temperature for 18hr., then poured into saturated NaHCO₃ and extracted twice with EtOAc. After washing with brine, the solvent was evaporated and the residue chromatographed (silica gel; 5% MeOH in CHCl₃) to give an oil. Further purification using reverse phase HPLC afforded the title compound as a white solid.
FAB mass spectrum, found (M+H)⁺ = 489.3

### 2(S)-Benzenesulfonylamino-3-(5-(5,6,7,8-tetrahydro-[1,8]naphthyridin-2-yl)-pentanoylamino)-propionic acid trifluoroacetate salt (3-3)

To a solution of the ester 3-2 (180 mg, 0.37 mmol) in methanol (2 mL) and water (2 mL) was added 1N LiOH (0.74 mL, 0.74 mmol) and the mixture was stirred for 4hr. After this time, 1N HCl (1 mL) was added, and the solution was purified by reverse phase HPLC to provide the title compound as a white solid.
FAB mass spectrum, found (M+H)⁺ = 461.21

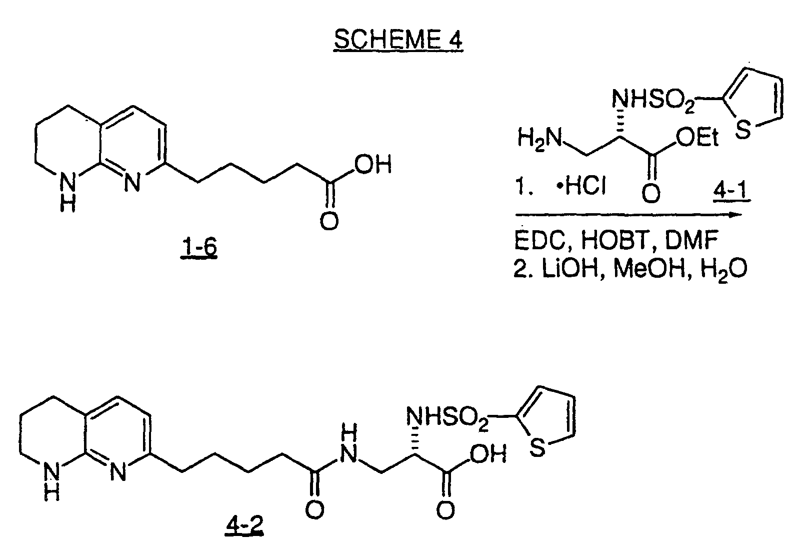

### 2(S)-(2-Thienylsulfonylamino)-3-(5-(5,6,7,8-tetrahydro-[1,8]naphthyridin-2-yl)-pentanoylamino)-propionic acid trifluoroacetate salt (4-2)

A solution of 5-(5,6,7,8-tetrahydro-[1,8]naphthyridin-2-yl)-pentanoic acid hydrochloride 1-6 (0.164 mg, 0.7 mmol) in DMF (5 mL) was treated successively with HOBT (104 mg, 0.77 mmol), the amine 4-1 (Egbertson, et al. *Bioorg Med Chem. Letts.,* 1996, 6, 2519; 211 mg, 0.85 mmol), EDC (161 mg, 0.84 mmol) and N-methylmorpholine (0.23 mL, 2.1 mmol). The resulting solution was stirred at room temperature for 18hr., then poured into saturated NaHCO₃ and extracted twice with EtOAc. After washing with brine, the solvent was evaporated and the residue was used without further purification in the next step. To a solution of this ester (290 mg, 0.6 mmol) in 20 mL of THF, methanol and water (1:1:1) was added LiOH●H₂O (100 mg, 2.4 mmol) and the mixture was stirred for 20 hr. After this time, the THF and methanol were removed in vacuo and the aqueous solution was purified by reverse phase HPLC to provide the title compound as a white solid.
¹H NMR (CD₃OD) δ 7.73-7.78 (1H, dd), 7.54-7.62 (2H, m), 7.08-7.15 (1H, m), 6.60-6.67 (1H, d), 4.08-4.16 (1H, m), 3.57-3.67 (1H, dd), 3.40-3.50 (2H, t), 3.20-3.30 (1H, m), 2.75-2.85 (2H, t), 2.65-2.75 (2H, t), 2.20-2.30 (2H, t), 1.85-1.97 (2H, m), 1.60-1.80 (4H, m).

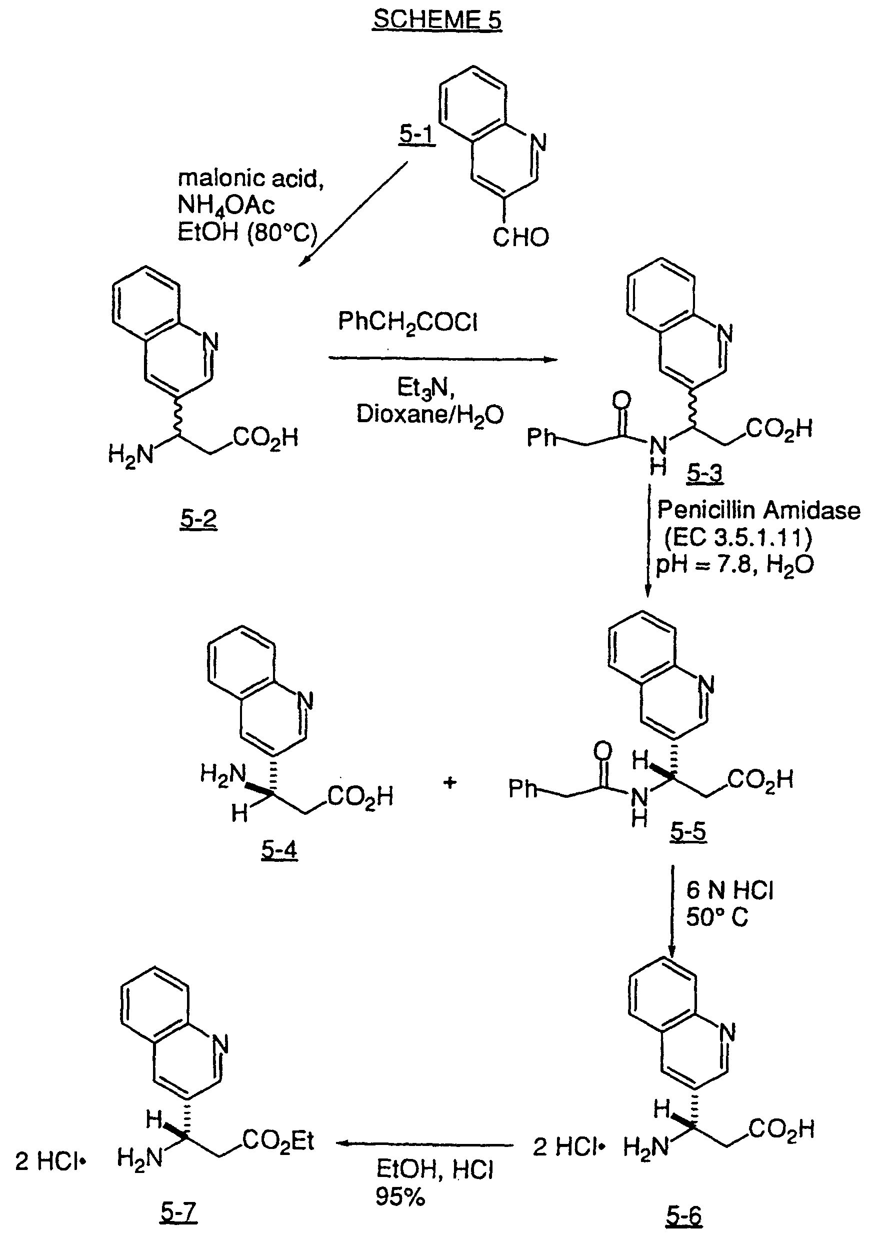

### 3-Amino-3-(quinolin-3-yl)-propionic acid (5-2)

A solution containing quinoline-3-carboxaldehyde 5-1 (5 g, 31.8 mmol), malonic acid (3.6 g, 35.0 mmol), and ammonium acetate (5.0 g, 63.6 mmol) in anhydrous ethanol (125 mL) was heated at reflux for 12 h. After cooling to room temperature, the resulting white solid was collected by filtration and washed with cold ethanol (150 mL) and then dried under vacuum to provide 5-2 as a white solid (3.84 g, 17.8 mmol, 56%).
¹H NMR (300 MHz, D₂O): δ 8.91 (d, J = 2 Hz 1H), 8.21 (d, J = 2 Hz, 1H), 8.12 (d, J = 8 Hz, 1H), 7.84 (d, J = 7 Hz, 1H), 7.72 (t, J = 7 Hz, 1H), 7.54 (t, J = 7 Hz, 1,H), 4.72 (m, 1H), 2.73 (m, 2H).

### 3-Phenylacetylamino-3-(quinolin-3-yl)-propionic (5-3)

A 0° solution of 5-2 (3.5 g, 16.2 mmol) and NaHCO₃ (2.7 g, 32.4 mmol) in 50% aqueous dioxane (100 mL) was treated dropwise with a solution of phenylacetyl chloride (3.00 g, 19.4 mmol) in 25 mL of dioxane. The resulting solution was stirred at 0° for 2.5h then warmed to room temperature, diluted with H₂O (50 mL) and washed with ether (2 x 100 mL). The aqueous layer was adjusted to pH = 3 with 3N HCl and then extracted with CH₂Cl₂ (3 x 150 mL). The pooled organic extracts were dried, filtered and concentrated to afford 5-3 as an off white solid.
¹H NMR (300 MHz, CD₃OD): δ 8.85 (d, J = 2 Hz 1H), 8.20 (d, J = 2 Hz, 1H), 8.00 (d, J = 8 Hz, 1H), 7.86 (d, J = 7 Hz, 1H), 7.76 (t, J = 7 Hz, 1H), 7.52 (t, J = 7 Hz, 1,H), 7.28 (m, 6H), 5.53 (t, J = 6.8 Hz, 1H), 3.57 (s, 2H), 2. 96 (m, 2H).

### 3-Amino-3(S)-(quinolin-3-yl)-propionic acid dihydrochloride (5-6)

Acid 5-3 (5.0 g, 15 mmol) was suspended in water (3.5 L) then treated with 1N NaOH (15 mL) to afford a clear solution. Penicillin amidase (Sigma, EC 3.5.1.11, 10,000 U) in 0.1 M phosphate buffer was added. The pH of the mixture was adjusted to 7.8 with 1N NaOH and the solution was stirred at room temperature for 4 days. The reaction was monitored periodically by HPLC and the reaction stopped once the 50% conversion was reached. Next, the reaction solution was cooled to 0°C and adjusted to pH = 3 with 3N HCl. An oily yellow precipitate formed and was collected by filtration then washed with water to afford crude 5-5 (1.8 g, 5.3 mmol). The filtrate was extracted with CH₂Cl₂ (3 x 500 mL) to afford additional 5-5 contaminated by phenylacetic acid. Both batches of crude 5-5 were combined and stirred in 3 N HCl (200 mL) at 50° for 12 h then cooled, washed with ether (2 x 100 mL) and evaporated to afford 5-6.

### 3-Amino-3-(quinolin-3-yl)-propionic acid ethyl ester dihydrochloride (5-7).

The resolved acid 5-6 was converted to 5-7 by refluxing in ethanolic HCl.
¹H NMR (300 MHz, CD₃OD): δ 9.25 (d, J = 2 Hz 1H), 8.31 (d, J = 2 Hz, 1H), 8.15 (d, J = 8 Hz, 1H), 7.84 (d, J =7 Hz, 1H), 7.72 (t, J = 7 Hz, 1H), 7.54 (t, J = 7 Hz, 1,H), 4.72 (m, 1H), 4.15 (q, J = 6 Hz, 2H), 2.73 (m, 2H) 1.18 (t, J= 6 Hz, 3H).

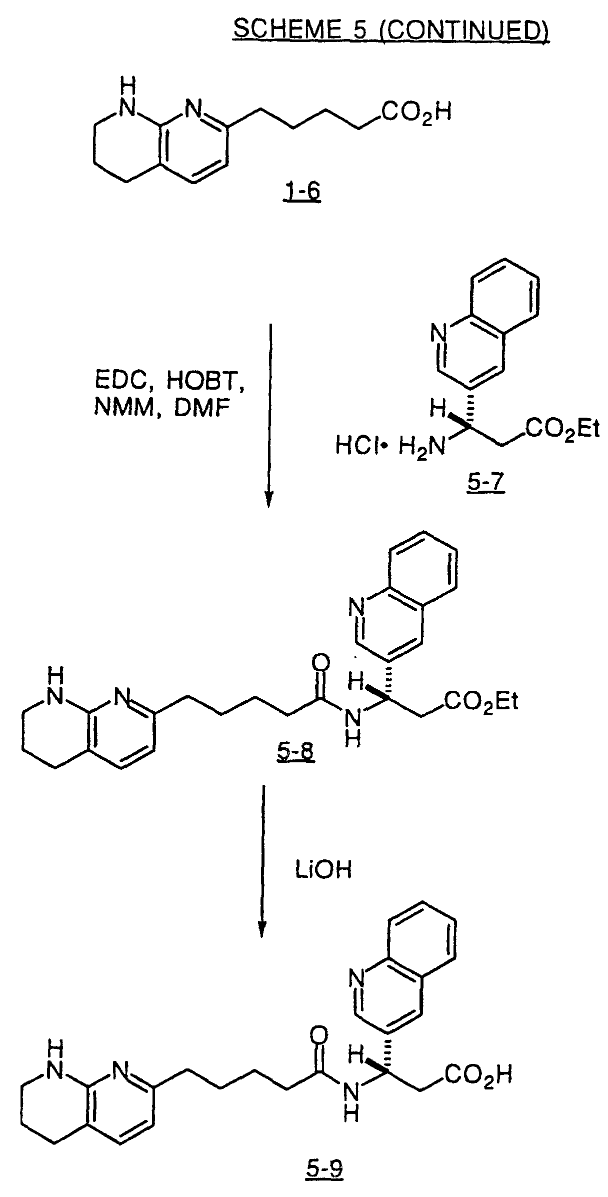

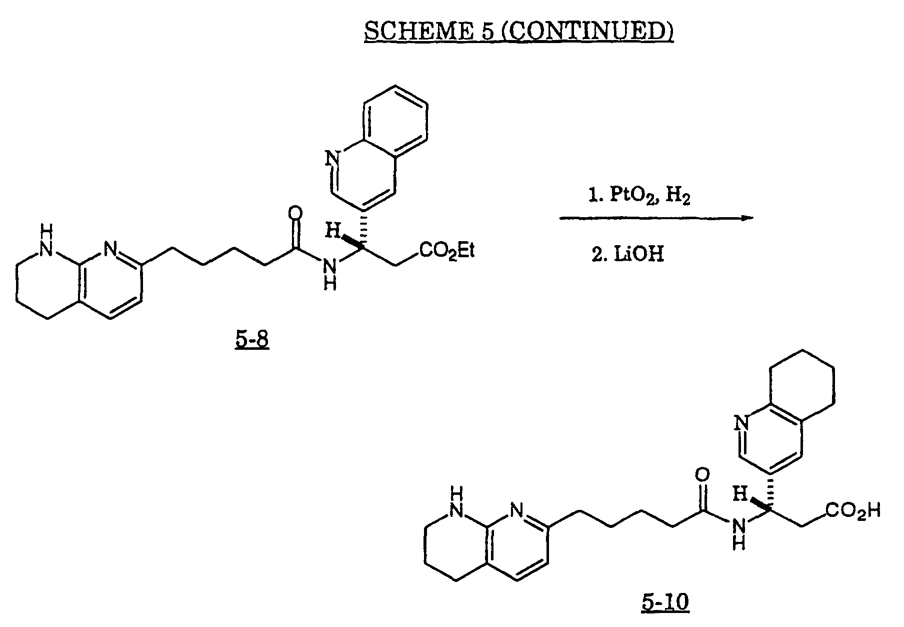

### Ethyl 3-(5-(5,6,7,8-tetrahydro-[1,8]-naphthyridin-2-yl)pentanoylamino)-3(S)-(quinolin-3-yl)-propionate (5-8)

A mixture of 1-6 (200 mg, 0.74 mmol), 5-7 (202 mg, 0.74 mmol), NMM (366 mL, 3.33 mmol), HOBT (130 mg, 0.96 mmol) and EDC (184 mg, 0.96 mmol) in 2 mL DMF was stirred overnight. After diluting with EtOAc (100 mL), the mixture was washed with sat. NaHCO₃, water, and brine, dried (MgSO₄), filtered and concentrated, and chromatographed on silica (10% EtOH/EtOAc) providing 5-8 as a colorless glass.
TLC R_{f} = 0.6 (10% EtOH/EtOAc).
¹H NMR (300 MHz, CDCl₃) δ 8.80 (d, J = 2 Hz 1H), 8.31 (d, J = 2 Hz, 1H), 8.15 (d, J = 8 Hz, 1H), 7.84 (d, J =7 Hz, 1H), 7.72 (t, J = 7 Hz, 1H), 7.54 (t, J = 7 Hz, 1,H), 7.00 (d, J=8.1 Hz, 1H), 6.53 (d, J=7.5 Hz, 1H), 5.34 (m, 1H), 4.06 (q, J= 7,5 Hz, 2H),3.48 (t, J=5.7 Hz, 2H), 2.79 (m, 4H), 2.63 (m, 2H), 2.25 (m, 2H), 1.94 (m, 2H), 1.64 (m, 4H) 1.12 (t, J= 7,5 Hz, 3H).

### 3(S)-(Quinolin-3-yl)-3-(5-(5,6,7,8-tetrahydro-[1,8]-naphthyridin-2-yl)pentanoylamino)-gropionic acid (5-9)

Ester 5-8 (145 mg, 0.320 mmol) was dissolved in 1 mL EtOH and treated with 1N LiOH (352 mL, 0.35 mmol) and stirred at room temperature overnight. The reaction solution was neutralized with 1N HCl (352 mL), evaporated, and purified by chromatography on silica gel (60% 20:1:1 EtOH/NH₄OH/H₂O - 40% EtOAc) to afford 5-9 as a white solid.
TLC R_{f} = 0.5 (60% 20:1:1 EtOH/NH₄OH/H₂O - 40% EtOAc)
¹H NMR (300 MHz, CD₃OD) δ 8.80 (d, J = 2 Hz 1H), 8.31 (d, J = 2 Hz, 1H), 8.15 (d, J = 8 Hz, 1H), 7.84 (d, J =7 Hz, 1H), 7.72 (t, J = 7 Hz, 1H), 7.54 (t, J = 7 Hz, 1,H), 7.00 (d, J=8.1 Hz, 1H), 6.53 (d, J=7.5 Hz, 1H), 5.34 (m, 1H), 3.48 (t, J=5.7 Hz, 2H), 2.79 (m, 4H), 2.63 (m, 2H), 2.25 (m, 2H), 1.94 (m, 2H), 1.64 (m, 4H).

### 3(S)-(5,6,7,8-Tetrahydro-quinolin-3-yl)-3-(5-(5,6,7,8-tetrahydro-[1,8]naphthyridin-2-yl)-pentanoylamino)-propionic acid trifluoroacetate (5-10)

A mixture of 5-8 (0.1 g, 0.3 mmol) and PtO₂ (0.04 g) in 10 mL TFA was purged with argon 3 times under vacuum, and treated under balloon hydrogenation condition for 18 hr. It was then filtered through a celite pad. The solution was concentrated. EtOH (3 mL) and LiOH (1 mL, 1 M, 1 mmol) were added. After stirring for 3 hr, the reaction mixture was treated with 2N HCl (2 mL), concentrated and purified by reverse phase HPLC (C18 column; gradient: H₂O /CH₃CN/TFA from 95:5:0.1 to 5:95:0.1 over 45 min) to give the desired product 5-10 as the TFA salt.
¹H NMR (300 MHz, CD₃OD) δ 8.50 (s, 1H), 8.23 (s, 1H), 7.52 (d, 1H), 6.54 (d, 1H), 5.33 (t, 1H), 3.50 (t, 2H), 3.08 (t, 2H), 2.94 (m, 4H), 2.80 (t, 2H), 2.68 (t. 2H), 2.28 (m, 2H), 1.96 (m, 6H), 1.66 (m, 4H).

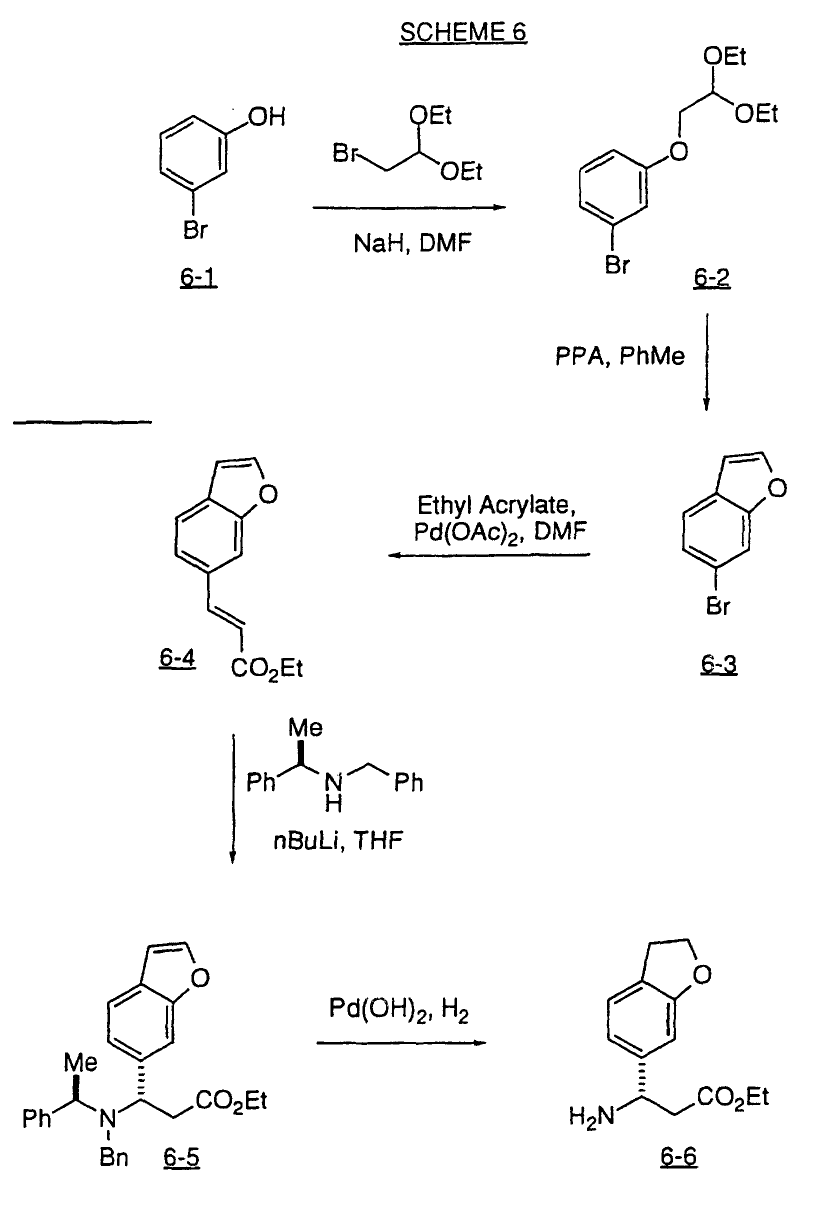

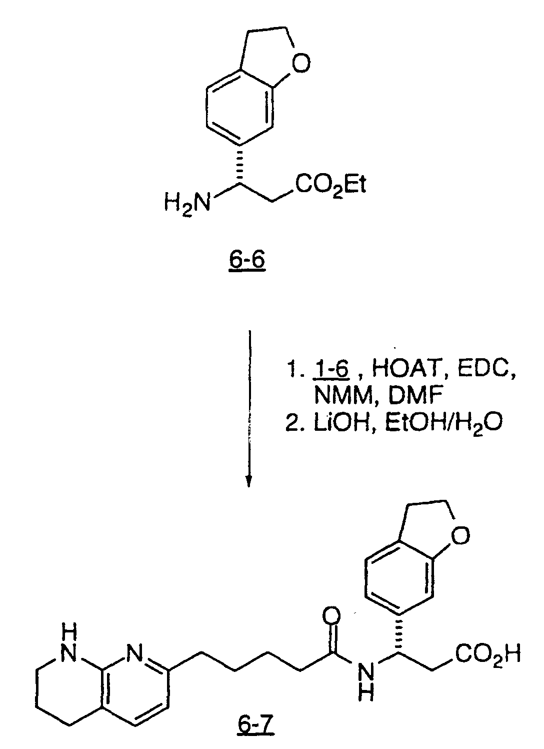

### 1-Bromo-3-(2,2-diethoxy-ethoxy)-benzene (6-2)

To a suspension of NaH (2.77 g, 115.6 mmol) in DMF (100 mL) at 0°C was added a solution of 3-bromophenol 6-1 in DMF (40 mL) over 40 min. After the addition was complete, the solution was stirred for an additional 30 min. The solution was then treated with neat bromoacetaldehyde diethyl acetal (17.36 g, 115.6 mmol). The solution was heated at 100°C for 8 h, cooled to room temperature, and extracted with Et₂O (3 x 200 mL). The combined organic extracts were washed with 10% aq. NaOH (100 mL) and brine (100 mL), dried over MgSO₄, filtered and concentrated to give 6-2 as a yellow oil.
TLC Rf = 0.4 (10% ethyl acetate/hexanes).
¹H NMR (300 MHz, CHCl₃) δ 7.19-7.05 (m, 3H), 6.85 (d, 1H), 4.81 (t, 1H, J=6.8 Hz), 3.99 (d, 2H, J=6.8 Hz), 3.71 (m, 4H), 1.22 (t, 6H, J=7.1 Hz) ppm.

### 6-Bromo-benzofuran (6-3)

To a solution of the acetal 6-2 in toluene (200 mL) was added polyphosphoric acid (20 g). The biphasic mixture was heated to 100°C and stirred at this temperature for 4 h. The mixture was cooled to room temperature, poured onto ice, and extracted with Et₂O (2 x 200 mL). The combined organic extracts were washed with saturated aq. NaHCO₃ and brine. The solution was dried over MgSO₄, filtered, and concentrated. The residue was purified by flash chromatography (100% hexanes) to give the product 6-3 as a yellow oil.
TLC Rf = 0.3 (100% hexanes).
¹H NMR (300 MHz, CHCl₃) δ 7.68 (s, 1H), 7.60 (d, 1H, J=2.1 Hz), 7.46 (d, 1H, J=8.4 Hz), 7.36 (dd, 1H, J=8.1, 1.5 Hz), 6.75 (dd, 1H, J=7.1, 0.9 Hz) ppm.

### 3-(Benzofuran-6-yl)-acrylic acid ethyl ester (6-4)

A mixture of the 6-bromo-benzofuran 6-3 (1.74 g, 8.79 mmol), ethyl acrylate (1.09 g, 10.98 mmol), Pd(OAc)₂ (0.099 g, 0.44 mmol), tri-o-tolylphosphine (0.268 g, 0.880 mmol), and sodium acetate (3.60 g, 43.9 mmol) in DMF (10 mL) was heated to 100°C in a sealed tube for 4 h. The mixture was cooled to room temperature, diluted with water, and extracted with Et₂O (2 x 40 mL). The combined organic extracts were washed with brine (30 mL), dried over MgSO₄, filtered, and concentrated. The residue was purified by flash chromatography (10% ethyl acetate/hexanes) to give the ester 6-4 as an off-white solid.
TLC Rf = 0.3 (10% ethyl acetate/hexanes).
¹H NMR (300 MHz, CHCl₃) δ 7.78 (d, 1H, J=15.9 Hz), 7.68 (d, 1H, J=2.4 Hz), 7.66 (s, 1H), 7.59 (d, 1H, J=8.4 Hz), 7.43 (dd, 1H, J=9.0, 1.5 Hz), 6.78 (m, 1H), 6.47 (d, 1H, J=15.9 Hz), 4.27 (q, 2H, J=7.2 Hz), 1.34 (t, 3H, J=7.2 Hz) ppm.

### 3(S)-(Benzofuran-6-yl)-3-[benzyl-(1(R)-phenyl-ethyl)-amino]-propionic acid ethyl ester (6-5)

A solution of N-benzyl-α-(R)-methylbenzylamine (1.32 g, 6.30 mmol) in THF (25 mL) at 0°C was treated with n-BuLi (2.52 mL of a 2.5 M soln in hexanes). The resulting solution was stirred at 0°C for 30 min and then cooled to -78°C. A solution of acrylate 6-4 (0.681 g, 3.15 mmol) in THF (5 mL) was added. After stirring for 15 min at -78 °C, satd. aq. NH₄Cl soln (5 mL) was added and the cold bath removed. The mixture was warmed to room temperature, and extracted with Et₂O (2 x 40 mL). The combined organic extracts were washed with brine (30 mL), dried over MgSO₄, filtered, and concentrated. The residue was purified by flash chromatography (10% ethyl acetate/hexanes) to give the β-aminoester 6-5 as a yellow oil.
TLC Rf = 0.8 (10% ethanol/dichloromethane).
¹H NMR (300 MHz, CHCl₃) δ 7.58 (m, 3H), 7.41 (m, 2H), 7.22 (m, 9H), 7.59 (s, 1H), 4.58 (m, 1H), 4.05 (m, 1H), 3.91 (q, 2H, J=7.1 Hz), 3.72 (m, 2H), 2.62 (m, 2H), 1.21 (d, 3H, J=7.2 Hz), 1.03 (t, 3H, J=7.1 Hz) ppm.

### 3(S)-Amino-3-(2,3-dihydro-benzofuran-6-yl)-propionic acid ethyl ester (6-6)

A mixture of the dibenzylamine 6-5 (1.19 g, 2.78 mmol) in EtOH/H₂O/AcOH (26 mL/3 mL/1.0 mL) was degassed with argon and treated with Pd(OH)₂ (1.19 g). The mixture was placed under 1 atm of H₂. After stirring for 18 h, the mixture was diluted with EtOAc, and filtered through celite. The filtrate was concentrated and the residue purified by flash chromatography (10% ethyl acetate/dichloromethane) to give the ester 6-6 as a white solid.
TLC Rf = 0.25 (10% ethanol/dichloromethane).
¹H NMR (300 MHz, CD₃OD) as the trifluoroacetate salt: δ 7.25 (d, 1H, J=8.1 Hz), 6.88 (m, 1H), 7.66 (s, 1H), 6.82 (s, 1H), 4.58 (m, 3H), 4.12 (m, 2H), 3.30 (m, 1H), 3.19 (m, 2H), 2.98 (m, 2H), 1.11 (t, 3H, J=7.2 Hz) ppm.

### 3(S)-(2,3-Dihydro-benzofuran-6-yl)-3-(5-(5,6,7,8-tetrahydro-[1,8]naphthyridin-2-yl)-pentanoylamino)-propionic acid (6-7)

A solution of the amine 6-6 (0.162 g, 0.596 mmol), acid 1-6 (0.183 g, 0.775 mmol), EDC (0.148 g, 0.775 mmol), NMM (0.156 g, 1.55 mmol), and HOAT (0.105 g, 0.775 mmol) in DMF (6 mL) was stirred at room temperature for 12 h. The solution was concentrated and the residue purified by preparative HPLC (gradient conditions: 95:05 to 50:50 H₂O/MeCN with 0.1% TFA) to give the ester (0.227 g) as a yellow oil. The ester was dissolved in a solution of EtOH/H₂O (6 mL of a 9:1 mixture) and treated with LiOH (0.065 g, 1.55 mmol). After stirring for 3 h, the solution was concentrated to a paste which was purified by preparative HPLC (gradient conditions: 95:05 to 50:50 H₂O/MeCN with 0.1% HCl) to give 6-7 as a white solid.
TLC R_{f} = 0.24 (5% MeOH/CH₂Cl₂).
¹H NMR (300 MHz, CD₃OD) δ 7.57 (d, J=7.2 Hz, 1H), 7.12 (d, J=7.8 Hz, 1H), 6.79 (dd, J=1.5, 7.2 Hz, 1H), 6.70 (s, 1H), 6.58 (d, J=7.2 Hz, 1H), 5.27 (t, J=8.4 Hz, 1H), 4.50 (t, J=8.7 Hz, 2H), 3.49 (t, J=5.7 Hz, 2H), 3.14 (t, J=8.7 Hz, 2H), 2.81 (t, J=6.0 Hz, 2H), 2.76 (m, 2H), 2.66 (m, 2H), 2.26 (m, 2H), 1.94 (m, 2H), 1.64 (m, 4H) ppm.

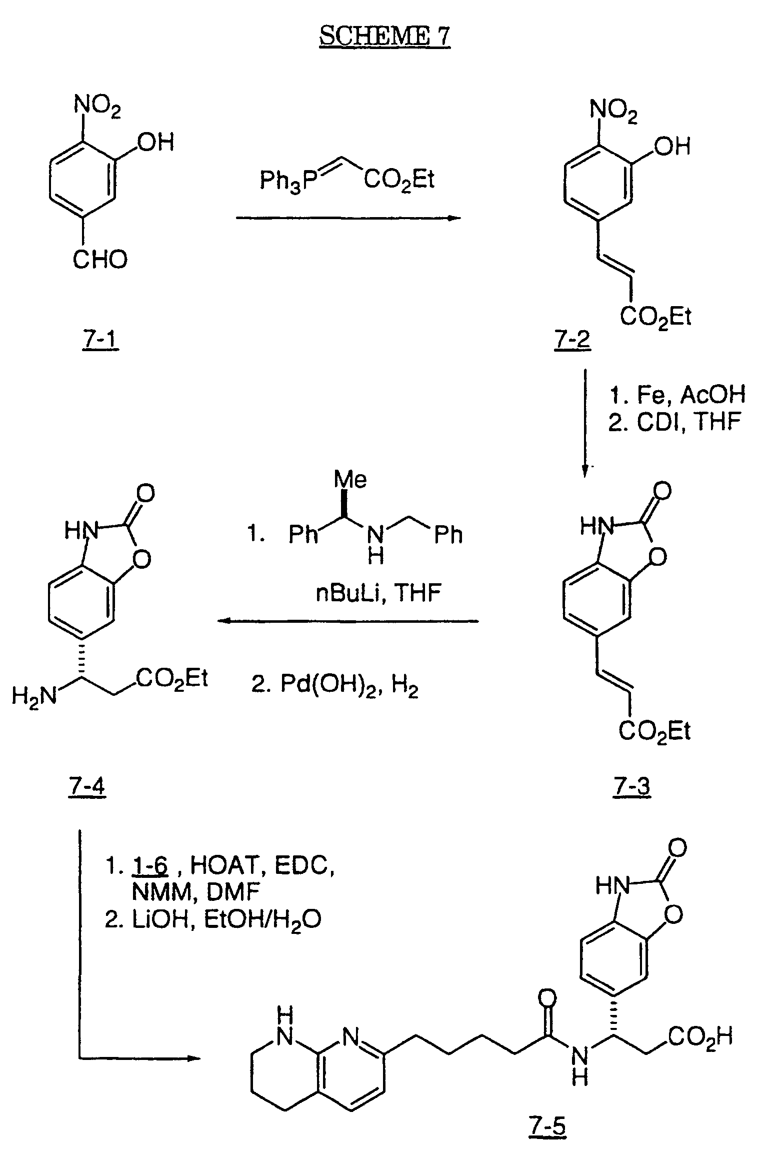

### 3-(3-Hydroxy-4-nitro-phenyl)-acrylic acid ethyl ester (7-2)

To a solution of aldehyde 7-1 (15.0 g, 98.0 mmol) in CH₂Cl₂ (300 mL) was slowly added carboethoxymethylenetriphenylphosphorane (34.1 g, 98.0 mmol). The orange solution was stirred for 12 h at ambient temperature. The solution was concentrated to a paste and purified by flash chromatography (10% EtOAc/CH₂Cl₂) to give 7-2 as a yellow solid.
TLC R_{f} = 0.51 (30% ethyl acetate/hexanes).
¹H NMR (300 MHz, CD₃OD) δ 8.08 (d, J=8.4 Hz, 1H), 7.63 (d, J=16.2 Hz, 1H), 7.35 (d, J=1.5 Hz, 1H), 7.27 (dd, J=8.4, 1.5 Hz, 1H), 6.65 (d, J=15.9 Hz, 1H), 4.25 (q, J=7.2 Hz, 2H), 1.32 (t, J=6.9 Hz, 3H) ppm.

### 3-(2-Oxo-2,3-dihydro-benzooxazol-6-yl)-acrylic acid ethyl ester (7-3)

To a solution of the nitrophenol 7-2 (12.0 g, 57.4 mmol) in warm (70°C) AcOH/H₂O (200 mL) was added iron dust (9.61 g, 172.2 mmol). The brown heterogeneous mixture was stirred for 30 min at 70-80°C. The mixture was filtered hot through celite, and the celite bed washed with EtOAc (2 x 200 mL). The filtrate was cautiously neutralized with satd. aq. NaHCO₃ (3 x 100 mL). The solution was dried over MgSO₄, filtered, and concentrated. The residue was purified by flash chromatography (5% MeOH in CH₂Cl₂) to give an orange solid (9.6 g, 81%). A portion of this solid (4.5 g, 21.7 mmol) was dissolved in THF (150 mL) and treated with 1,1-carbonyldiimidazole (3.87 g, 23.8 mmol), and the solution was stirred at ambient temperature for 24 h. The solution was diluted with EtOAc (100 mL) and washed with 10% HCl (50 mL) and brine (50 mL). The solution was dried over MgSO₄, filtered, and concentrated. The residue was purified by flash chromatography (5% MeOH in CH₂Cl₂) to give 7-3 as a yellow solid.
TLC R_{f} = 0.49 (5% MeOH/CH₂Cl₂).
¹H NMR (300 MHz, CD₃OD) δ 7.77 (d, J=15.9 Hz, 1H), 7.55 (s, 1H), 7.41 (d, J=8.4 Hz, 1H), 7.09 (d, J=8.1 Hz, 1H), 6.47 (d, J=15.9 Hz, 1H), 4.22 (q, J=7.2 Hz, 2H), 1.31 (t, J=7.2 Hz, 3H) ppm.

### 3(S)-Amino-3-(2-oxo-2,3-dihydro-benzooxazol-6-yl)-propionic acid ethyl ester (7-4)

A solution of N-benzyl-α-(R)-methylbenzylamine (4.08 g, 19.3 mmol) in THF (120 mL) at 0 °C was treated with n-BuLi (7.72 mL of a 2.5 M soln in hexanes). The resulting solution was stirred at 0 °C for 30 min and then cooled to -78°C. A solution of acrylate 7-3 (1.5 g, 6.43 mmol) in THF (20 mL) was added. After stirring for 15 min at -78°C, satd. aq. NH₄Cl soln (25 mL) was added and the cold bath removed. The mixture was warmed to room temperature, and extracted with Et₂O (2 x 40 mL). The combined organic extracts were washed with brine (30 mL), dried over MgSO₄, filtered, and concentrated. The residue was purified by flash chromatography (30% ethyl acetate/hexanes) to give 2.74 g of the β-aminoester as a yellow oil. The aminoester was dissolved in EtOH/H₂O/AcOH (54 mL/4.8 mL/1.2 mL), degassed with argon, and treated with Pd(OH)₂ (2.74 g). The mixture was placed under 1 atm of H₂. After stirring for 18 h, the mixture was diluted with EtOAc and filtered through celite. The filtrate was concentrated to give 7-4 as an off-white solid.
TLC R_{f} = 0.10 (5% MeOH/CH₂Cl₂).
¹H NMR (300 MHz, CD₃OD) δ 7.34 (s, 1H), 7.26 (dd, J=1.2, 8.1 Hz, 1H), 7.12 (d, J=8.1 Hz, 1H), 4.65 (t, J=7.2 Hz, 1H), 4.13 (q, J=6.9 Hz, 2H), 2.98 (m, 2H), 1.20 (t, J=7.2 Hz, 3H) ppm.

### 3(S)-(2-Oxo-2,3-dihydro-benzoxazol-6-yl)-3-(5-(5,6,7,8-tetrahydro-[1,8]naphthyridin-2-yl)-pentanoylamino)-propionic acid trifluoroacetate (7-5)

A solution of the amine 7-4 (0.196 g, 0.784 mmol), acid 1-6 (0.222 g, 0.941 mmol), EDC (0.189 g, 0.988 mmol), NMM (0.190 g, 1.88 mmol), and HOAT (0.134 g, 0.988 mmol) in DMF (6 mL) was stirred at room temperature for 12 h. The solution was concentrated and the residue purified by preparative HPLC (gradient conditions: 95:05 to 50:50 H₂O/MeCN with 0.1% TFA) to give the ester (0.144 g) as a yellow oil. The ester was dissolved in a solution of EtOH/H₂O (6 mL of a 9:1 mixture) and treated with LiOH (0.065 g, 1.55 mmol). After stirring for 3 h, the solution was concentrated to a paste which was purified by preparative HPLC (gradient conditions: 95:05 to 50:50 H₂O/MeCN with 0.1% TFA) to give 0.068 g (14% for two steps) of acid 7-5 as a white solid.
TLC R_{f} = 0.11 (5% MeOH/CH₂Cl₂).
¹H NMR (300 MHz, CD₃OD) δ 7.54 (d, J=8.7 Hz, 1H), 7.19 (s, 1H), 7.16 (d, J=8.4, 1H), 7.00 (d, J=8.1 Hz, 1H), 6.53 (d, J=7.5 Hz, 1H), 5.34 (t, J=6.9 Hz, 1H), 3.48 (t, J=5.7 Hz, 2H), 2.79 (m, 4H), 2.63 (m, 2H), 2.25 (m, 2H), 1.94 (m, 2H), 1.64 (m, 4H) ppm.

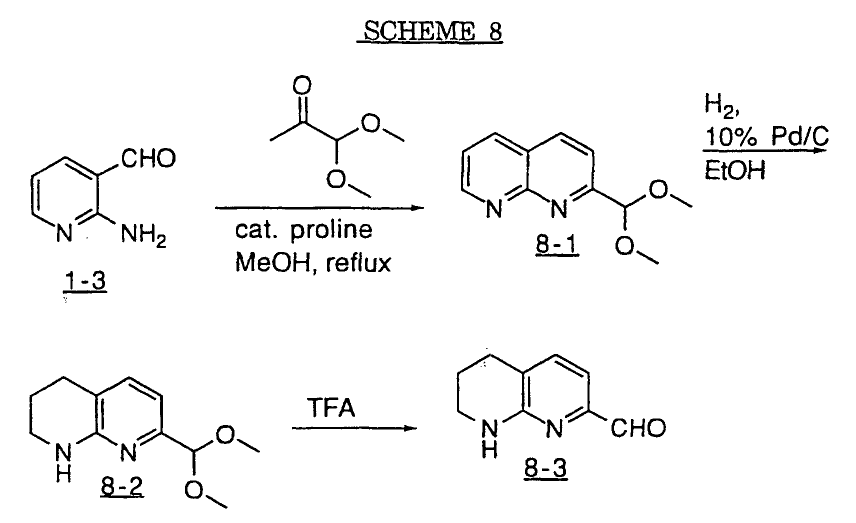

### 2-Dimethoxymethyl-[1,8]naphthyridine (8-1)

A mixture containing 1-3 (30 g, 0.245 mol), pyruvaldehyde dimethylacetal (87 g, 0.737 mol), and L-proline (7.0g, 0.062 mol) in MeOH (300 mL) was refluxed under argon for 16 h. The cooled solution was filtered, evaporated and the residue dissolved in CH₂Cl₂ (500 mL) and washed with water and brine then dried and concentrated to a volume of ca. 100 mL. Hexane (300 mL) was added and the mixture was kept at 0°C for 3 h, then filtered affording 8-1 as an off-white crystalline solid.
¹H NMR (300 MHz, CDCl₃) δ, 9.14 (d, J =2.2 Hz, 1H); 8.26 (d, J = 8.7 Hz, 1H); 8.21 (dd, J = 8.7, 2.2 Hz, 1H); 7.8 (d, J = 8.3 Hz, 1H); 7.5 (m, 1H); 5.48 (s, 1H); 3.53 (s, 6H).

### 2-Dimethoxymethyl-5,6,7,8-tetrahydro-[1,8]naphthyridine (8-2)

A solution of 8-1 (10 g, 0.049 mol) in ethanol (100 ml) was treated with 10% Pd on C (1.5 g) and the resulting mixture stirred under a H₂ filled balloon for 12.5 h. The catalyst was removed by filtration through celite and the solution concentrated to afford 8-2 as a yellow crystalline solid.
¹H NMR (300 MHz, CDCl₃) δ 7.18 (d, J = 7.12 Hz, 1H); 6.71 (d, J = 7.12 Hz, 1H); 5.18 (s, 1H); 4.96 (br, s, 1H); 3.43 (s, 6H); 3.4 (m, 2H); 2.65 (m, 2H); 1.91 (m, 2H).

### 5,6,7,8-tetrahydro-[1,8]naphthyridine-2-carboxaldehyde (8-3)

8-2 (10 g, 0.048 mol) was treated with trifluoroacetic acid (50 mL) and the resulting solution stirred under argon for 12.5 h. The TFA was removed at reduced pressure and the residue partitioned between sat. NaHCO₃ and CH₂Cl₂. The organic layer was dried, concentrated and passed through a 3 in. pad of silica gel (10% acetone/CH₂Cl₂) and concentrated to afford 8-3 as a yellow crystalline solid.
¹H NMR (300 MHz, CDCl₃) δ 9.80 (s, 1H); 7.31 (d, J = 7.32 Hz, 1H); 7.16 (d, J = 7.32 Hz, 1H); 5.31 (br, s, 1H); 3.48 (m, 2H); 2.81 (m, 2H); 1.94 (m, 2H).

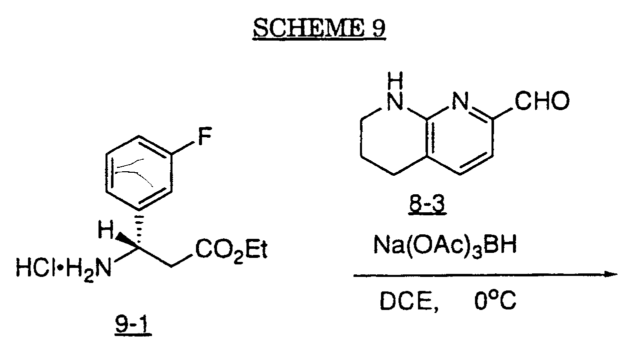

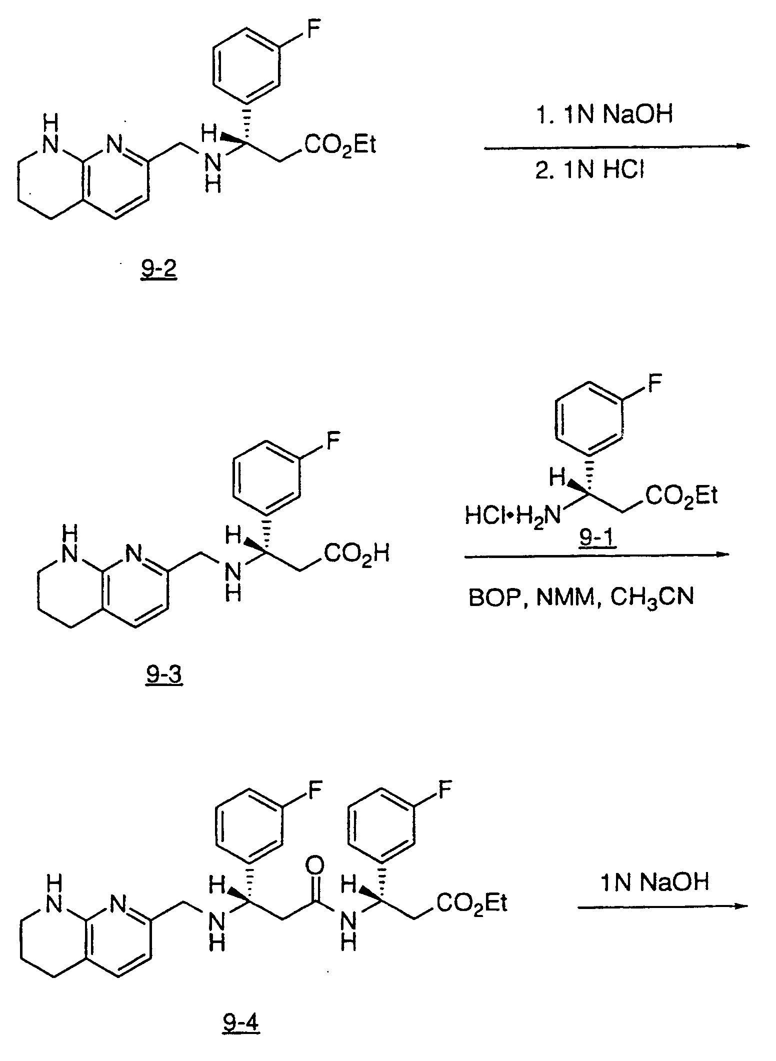

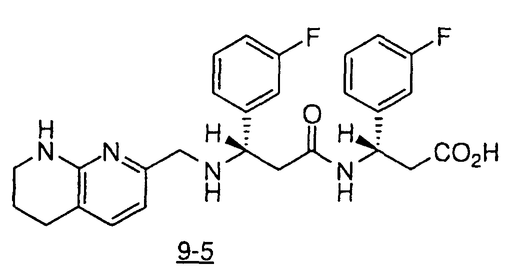

### 3(S)-Fluorophenyl-β-alanine ethyl ester hydrochloride (9-1)

The title compound 9-1 was prepared from 3-fluoro-benzaldehyde as described for preparing 7-4 from 7-1.
¹H NMR (CD₃OD) δ 1.21 (3H, t), 3.0-3.2 (2H, m), 4.16 (2H, q), 4,76 (1H, t), 7.2-7.35 (3H, m), 7.5 (1H, m).

### 3(S)-(3-Fluorophenyl)-3-[(5,6,7,8-tetrahydro-[1,8]naphthyridin-2-ylmethyl)-amino]-propionic acid ethyl ester (9-2)

To a stirred solution of 8-3 (300 mg, 1.85 mmol), 9-1 (458 mg, 1.85 mmol) in dichloroethane (10 ml) at 0°C was added sodium triacetoxyborohydride (570 mg, 2.59 mmol). After 1 hour, the reaction was diluted with EtOAc and then washed with 10% K₂CO₃, brine, and dried over MgSO₄. Following evaporative removal of the solvent, the residue was chromatographed (silica gel, 5% [10:1:1 ethanol/ NH₄OH/H₂O]: 95% [70:25:5 chloroform /ethyl acetate /methanol]) to give ester 9-2 as a yellow solid.
TLC R_{f} = 0.50 (silica, 5% [10:1:1 ethanol/ NH₄OH/ H₂O]: 95% [70:25:5 chloroform /ethyl acetate /methanol])
¹H NMR (300 MHz, CDCl₃) δ 7.26 (m,1H), 7.07 (m, 3H), 6.95 (m, 1H), 6.37 (d, J=7.1 Hz, 1H), 4.76 (bs, 1H), 4.10 (m, 3H), 3.47 (d, J=7.1 Hz, 2H), 3.38 (m, 2H), 2.68 (m, 4H), 1.90 (m, 2H), 1.18 (t, J=7.1 Hz, 3H).

### 3(S)-(3-Fluorophenyl)-3-[(5,6,7,8-tetrahydro-[1,8]naphthyridin-2-ylmethyl)-amino]-propionic acid (9-3)

To a solution of ester 9-2 (450 mg, 1.26 mmol) in EtOH (3 mL) was added 1N NaOH (1.39 ml, 1.39 mmol). After stirring for 1 h, the solvents were evaporated and the residue was dissolved in 1N HCl (1.39 ml, 1.39 mmol). The solution was concentrated and then azeotroped with CH₃CN to give the acid 9-3 as a brown solid.
¹H NMR (300 MHz, CD₃OD) δ 7.45 (m,1H), 7.10 to 7.28 (m, 4H), 6.36 (d, J=7.3 Hz, 1H), 4.41 (m, 1H), 3.80 (s, 2H), 3.31 (m, 2H), 2.62 to 2.85 (m, 4H), 1.90 (m, 2H).

### 3(S)-(3-Fluorophenyl)-3-{3(S)-(3-fluorophenyl)-3-[(5,6,7,8-tetrahydro-[1,8]naphthyridin-2-ylmethyl)-amino]-propionylamino}-propionic acid ethyl ester (9-4)

A mixture of acid 9-3 (235 mg, 0.6056 mmol), amine 9-1 (150 mg, 0.6056 mmol), BOP (350 mg, 0.7873 mmol) and NMM (0.333 mL, 3.03 mmol) in CH₃CN (5 mL) was stirred for 20 h. The mixture was diluted with ethyl acetate, washed with 10% K₂CO₃, brine, and dried over MgSO₄. Evaporative removal of the solvent gave 9-4 as a brown oil.
TLC R_{f}= 0.15 (75:10:15 chloroform/ ethyl acetate/ MeOH).
¹H NMR (300 MHz, CDCl₃) δ 6.85 to 7.28 (m, 9H), 6.34 (d, J=7.3 Hz, 1H), 5.40 (m, 1H), 4.92 (s, 1H), 4.10 (m,3H), 3.52 (d,J=5.4 Hz, 2H), 3.38 (m, 2H,), 2.48 to 2.84 (m, 7H), 1.26 (t, J=7.1 Hz, 3H).

### 3(S)-(3-Fluorophenyl)-3-(3(S)-(3-fluorophenyl)-3-[(5,6,7,8-tetrahydro-[1,8]naphthyridin-2-ylmethyl)-amino]-propionylamino}-propionic acid bis(trifluoroacetate) (9-5)

To a solution of 9-4 (0.6056 mmol) in EtOH (3 mL) was added 1N NaOH (1.21 ml, 1.21 mmol). After stirring for 1 h, the solvents were evaporated and the residue was purified by Preparative HPLC (Waters Delta Pak C18: 100:0:0.1 to 5:95:0.1 H₂O/CH₃CN/TFA) to give 9-5 as a white solid.
¹H NMR (300 MHz, CD₃OD) δ 7.45 (d, J=6.8 Hz, 1H), 7.38(d, J=6.1 Hz,
1H), 7.29 (m, 1H), 6.94 to 7.18 (m, 6H), 6.51 (d, J=7.6 Hz, 1H), 5.43 (m,1H), 4.26 (m, 1H,), 3.81 (m, 2H), 3.49 (m, 2H), 2.69 to 2.90 (m, 4H), 1.92 (m, 2H).

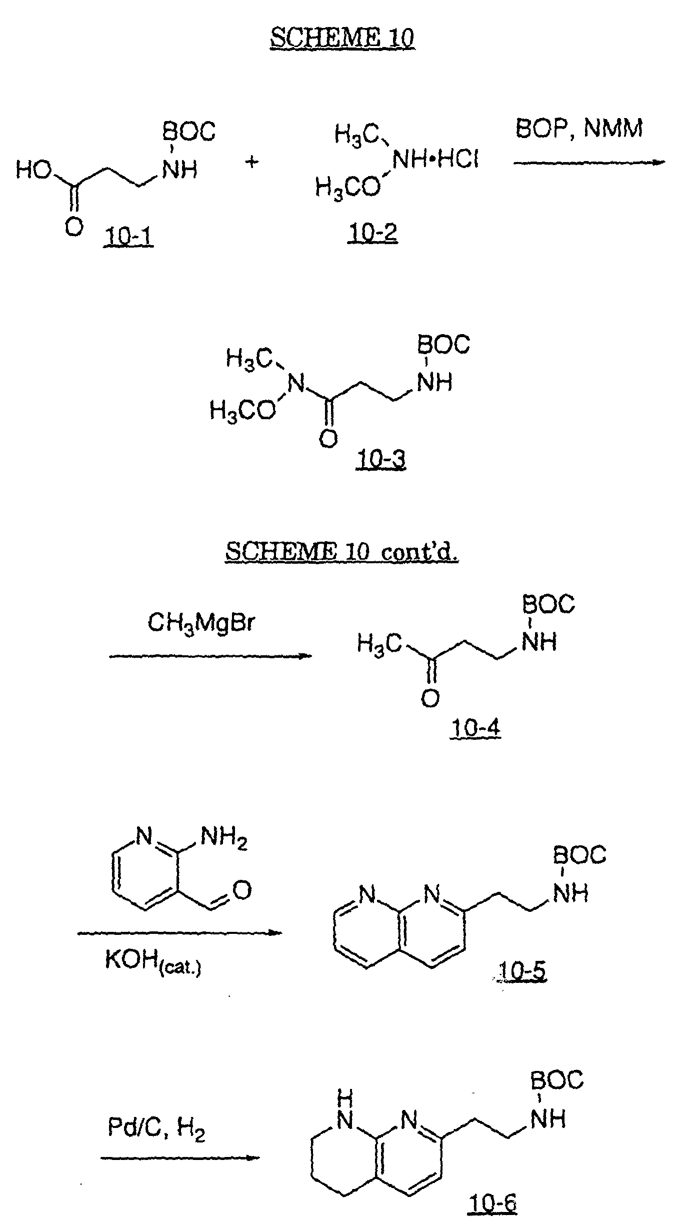

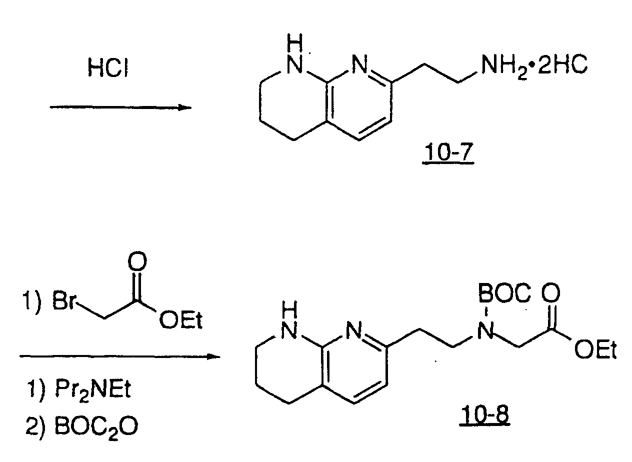

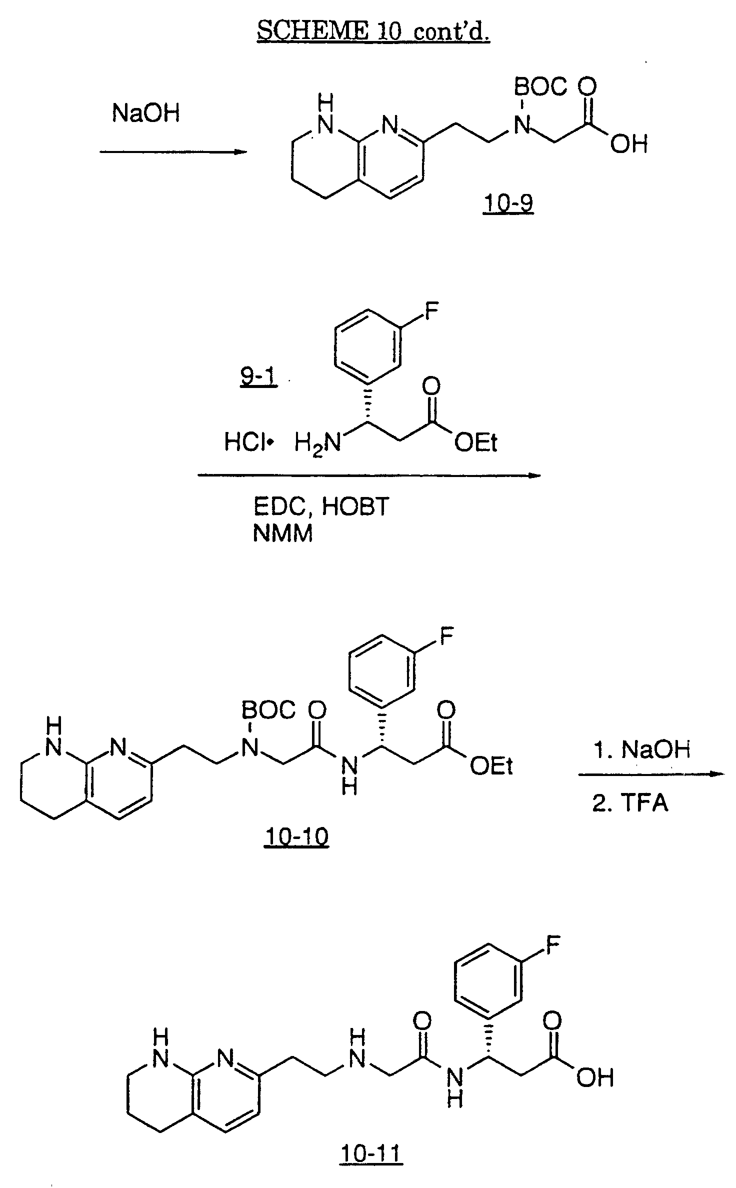

### (3-Oxo-butyl)-carbamic acid tert-butyl ester (10-4)

A solution of BOC-beta-alanine (20 g, 105 mmol), 10-2 (10.3 g, 105 mmol), BOP reagent (46.5 g, 105 mmol), and N-methylmorpholine (46 mL, 420 mmol) in acetonitrile (500 mL) was stirred for 15 h. The reaction was diluted with EtOAc, washed with H₂O, 10% KHSO₄(aq.), sat. NaHCO₃, dried (MgSO₄) and concentrated to give crude [2-(methoxymethyl-carbamoyl)-ethyl]-carbamic acid tert-butyl ester 10-3 as a yellow oil. TLC R_{f} = 0.42 (silica, 20% EtOAc/hexanes). To a solution of this crude amide in THF (500 mL) at 0°C was added CH₃MgBr (100 mL of 3M in ether, 300 mmol) over 30 minutes. After 2 h, 10% KHSO₄(aq.) was added, the mixture warmed to 25°C, and diluted with EtOAc. The organics were washed with sat. NaHCO₃, dried (MgSO₄) and concentrated. Flash chromatography (silica, 20% EtOAc/hexanes) gave 10-4 as a yellow oil.
TLC R_{f}= 0.35 (silica, 20% EtOAc/hexanes).
¹H NMR (300 MHz, CDCl₃) δ 5.02 (br s, 1H), 3.32 (q, 2H, J=6.8 Hz), 2.66 (q, 2H, J=6Hz), 2.16 (s, 3H), 1.43 (s, 9H).

### (2-[1,8]Naphthyridin-2-yl-ethyl)-carbamic acid tert-butyl ester (10-5)

A solution of 10-4 (10 g, 53.4 mmol), 2-amino-3-formylpyridine 1-3 (7.2 g, 64 mmol), 20% aq. KOH (1 mL), and ethanol (200 mL) was heated at reflux for 3 h. Evaporation of the solvents and flash chromatography (silica, 70 CHCl₃ /28 EtOAc/2 MeOH) gave 10-5 as a solid.
TLC R_{f}= 0.29 (silica, 70 CHCl₃ /28 EtOAc/2 MeOH).
¹H NMR (300 MHz, CDCl₃) δ 9.11 (m, 1H), 8.16 (m, 2H), 7.45 (m, 2H), 5.27 (s, 1H), 3.75 (m, 2H), 3.27 (m, 2H), 1.40 (s, 9H).

### [2-(5,6,7,8-Tetrahydro-[1,8]naphthyridin-2-yl)-ethyl]-carbamic acid tert-butyl ester (10-6)

A mixture of 10-5 (5.0 g, 20.4 mmol), 10% Pd/C (2.5 g), and EtOH (200 mL) was stirred under a balloon of hydrogen for 15 h. The mixture was filtered, and the filtrate concentrated to give 10-6 as a yellow oil.
TLC R_{f} = 0.29 (silica, 70 CHCl₃ /25 EtOAc/5 MeOH).
¹H NMR (300 MHz, CDCl₃) δ 7.16 (d, 1H, J=6.8 Hz), 6.48 (d, 1H, J=4.9 Hz), 5.12 (br s, 2H), 3.45 (m, 4H), 2.72 (m, 4H), 1.88 (m, 2H), 1.43 (s, 9H).

### 2-(5,6,7,8-Tetrahydro-[1,8]naphthyridin-2-yl)-ethylamine dihydrochloride (10-7)

Through a solution of 10-6 (4.0 g, 16 mmol) in EtOAc (200 mL) at 0°C was bubbled a stream of HCl gas for 10 minutes. After an additional 30 minutes, the mixture was purged with argon for 1 h and then concentrated. The residue was dissolved in acetonitrile and concentrated to give 10-7 as a yellow solid.
1H NMR (300 MHz, CD₃OD) δ 7.62 (d, 1H, J=7 Hz), 6.70 (d, 1H, J=7 Hz), 3.53 (t, 2H, J=6 Hz), 3.34 (m, 2H), 3.11 (m, 2H), 2.84 (m, 2H), 1.96 (m, 2H).

### [tert-Butoxycarbonyl-[2-(5,6,7,8-tetrahydro-[1,8]naphthyridin-2-yl)-ethyl]-amino]-acetic acid ethyl ester (10-8)

To a solution of 10-7 (2.0 g, 7.8 mmol) and N,N-diisopropylethylamine (6.8 mL) in acetonitrile (50 mL) at 0°C was added ethyl bromoacetate (0.975 mL, 8.6 mmol). The mixture was stirred at 50°C for 15 h, then cooled to 25°C and BOC₂O (1.7 g, 7.8 mmol) was added. After 1 h, the mixture was diluted with ethyl acetate, washed with sat. NaHCO₃, brine, dried (MgSO₄) and concentrated. Flash chromatography (silica, 50-60% EtOAc/hexanes) gave 10-8 as a colorless oil.
TLC R_{f} = 0.5 (silica, EtOAc).
¹H NMR (300 MHz, CDCl₃) mixture of rotamers: δ 7.04 (d, 2H, J=7Hz), 6.35 (dd, 1H, J=7Hz), 4.73 (br s, 1H), 4.16 (m, 2H), 3.89 (s, 1H), 3.82 (s, 1H), 3.56 (m, 2H), 3.39 (m, 2H), 2.76 (m, 4H), 1.90 (m, 2H), 1.44 (m, 9H), 1.25 (m, 3H).

### 3-(2-[tert-Butoxycarbonyl-[2-(5,6,7,8-tetrahyciro-[1,8]naphthyridin-2-yl)-ethyl]-amino]-acetylamino)-3(S)-(3-fluorophenyl)-propionic acid ethyl ester (10-10)

To a solution of 10-8 (0.8 g (2.20 mmol) in EtOH (10 mL) was added 1N NaOH (2.4 mL, 2.4 mmol) and the mixture stirred for 1 h. The solvents were evaporated, and the residue was dissolved in 1N HCl, evaporated, and additionally evaporated from acetonitrile to give crude [tert-butoxycarbonyl-[2-(5,6,7,8-tetrahydro-[1,8]naphthyridin-2-yl)-ethyl]-amino]-acetic acid 10-9 (0.820 g, 95%). To a mixture of the crude 10-9 in acetonitrile (2 mL) was added 3-amino-3-(S)[3-fluorophenyl]propionic acid (9-1) (0.1 g, 0.404 mmol), EDC (93 mg, 1.2 eq), HOBT (55 mg, 1 eq), and NMM (0.222 mL, 5 eq). After stirring for 12 h, the mixture was diluted with ethyl acetate, washed with water, sat. NaHCO₃, brine, dried (MgSO₄) and concentrated to give 10-10 as a yellow oil.
TLC R_{f} = 0.18 (silica, 70: CHCl₃ /25: EtOAc/5: MeOH).
¹H NMR (300 MHz, CDCl₃) mixture of retamers: δ 9.52 (m, 0.5 H), 9.07 (m, 0.5 H), 7.38-6.91 (m, 6H), 6.36 (m, 1H), 5.48 (m, 1H), 4.67 (m, 1H), 4.10 (m, 3H), 3.82 (m, 1H), 3.66 (m, 1H), 3.44 (m, 1H), 3.21 (m, 1H), 2.82 (m, 1H), 2.63 (m, 1H), 1.78 (m, 4H), 1.51-1.13 (m, 12H).

### 3(S)-(3-Fluorophenyl)-3-{2-[2-(5,6,7,8-tetrahydro-[1,8]naphthyridin-2-yl)-ethylamino]-acetylamino}-propionic acid (10-11)

To a solution of 10-10 (0.23 g (0.404 mmol) in EtOH (2 mL) was added 1N NaOH (0.6 mL, 0.6 mmol) and the mixture stirred for 1 h. The solvents were evaporated, and the residue was dissolved in dichloromethane (2 mL), and TFA (2 mL) added. After 1 h, the solution was concentrated from toluene. Flash chromatography (silica, 20:10:1:1 to 10:10:1:1 EtOAc / EtOH / NH₄OH / H₂O) gave 10-11 as a white solid.
TLC R_{f} = 0.21 (silica, 10:10:1:1 EtOAc / EtOH / NH₄OH / H₂0).
¹H NMR (300 MHz, CD₃OD): δ 7.28 (m, 1H), 7.13 (m, 3H), 6.91 (m, 1H), 6.38 (d, 1H, J=7Hz), 5.37 (m, 1H), 3.36 (m, 4H), 2.82 (m, 2H), 2.68 (m, 4H), 2.61 (m, 2H), 1.87 (m, 2H).

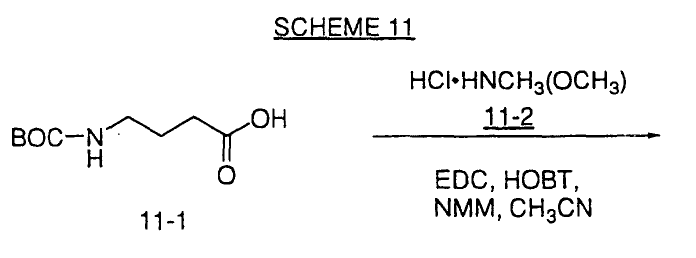

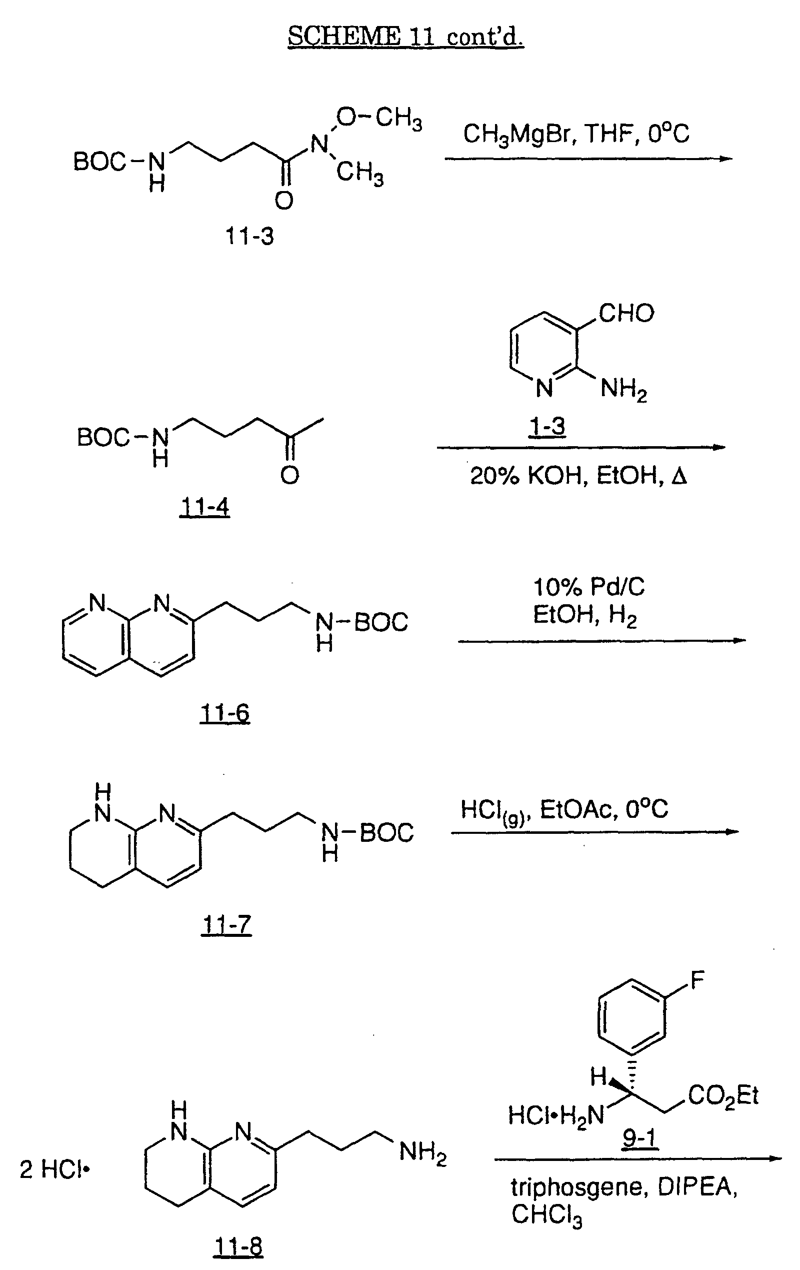

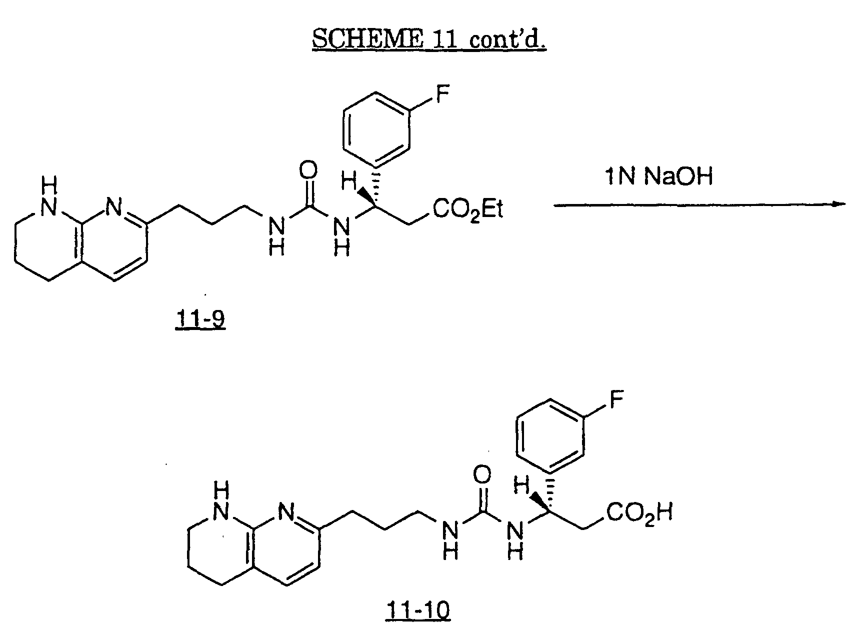

### [3-(N-Methoxy-N-methyl-carbamoyl)-propyl]carbamic acid tert-butyl ester (11-3)

A mixture of 11-1 (10 g, 49.2 mmol), 11-2 (4.8 mg, 49.2 mmol), EDC (9.40 g, 49.2 mmol), HOBT (6.6 g, 49.2 mmol) and NMM (2.7 mL, 246 mmol) in CH₃CN (200 mL) was stirred for 20 h. The reaction was concentrated. The residue was dissolved in ethyl acetate, washed with H₂O, 10% KHSO₄, sat. NaHCO₃, brine, and dried over MgSO₄. Evaporative removal of the solvent gave 11-3 as a colorless oil.
TLC R_{f} = 0.15 (50% ethyl acetate/ hexanes).
¹H NMR (300 MHz, CDCl₃) δ 4.96 (bs,1H), 3.55 (s, 3H), 3.46 (m, 5H), 2.48 (t, J=7.3 Hz, 2H), 1.83 (m, 2H), 1.46 (s, 9H).

### (4-Oxo-pentyl)carbamic acid tert-butyl ester (11-4)

To a stirred solution of 11-3 (10.0 g, 40.5 mmol) and THF (200ml) at 0°C was added methylmagnesium bromide (27.0 ml, 91.0 mmol; 3M in ether) dropwise over 20 minutes. After 2.0 hours, 10% KHSO₄ was added slowly. The mixture was extracted with EtOAc. The organic portion was washed with sat. NaHCO₃, brine, and dried over MgSO₄. Evaporative removal of the solvent gave 11-4 as a colorless oil.
TLC R_{f} = 0.53 (silica, 40% EtOAc/ hexanes)
¹H NMR (300 MHz, CDCl₃) δ 4.62 (bs, 1H), 3.13 (m, 2H), 2.49 (t, J=7.1 Hz, 2H), 2.16 (s, 3H), 1.78 (m, 2H), 1.44 (s, 9H).

### (3-[1,8]Naphthyridin-2-yl)-N-Boc-propylamine (11-6)

A mixture of 11-4 (5.0 g, 24.8 mmol), 1-3, 2-amino-3-formylpyridine (3.6 g, 29.8 mmol) and 20% KOH (1 ml) in absolute ethanol (100 mL) was heated at reflux for 8 h. Following evaporative removal of the solvent, the residue was chromatographed (silica gel, 70:28:2 chloroform /ethyl acetate /methanol) to give 11-6 as a yellow oil.
TLC R_{f} = 0.40 (silica, 70:20:10 chloroform /ethyl acetate /methanol)
¹H NMR (300 MHz, CDCl₃) δ 9.08 (m, 1H), 8.16 (d, J=8.1 Hz, 1H), 8.10 (d, J=8.1 Hz, 1H), 7.41 (m, 2H), 4.82 (bs, 1H), 3.21 (m, 2H), 3.06 (m, 2H), 2.12 (m,2H), 1.43 (s, 9H).

### 3-(5,6,7,8-Tetrahydro-[1,8]naphthyridin-2-yl)-N-Boc-propylamine (11-7)

A mixture of 11-6 (4.0 g, 13.9 mmol) and 10% Pd/carbon (4.0 g) in EtOH (100 mL) was stirred under a balloon of hydrogen for 4 h. Following filtration and evaporative removal of the solvent, the residue was chromatographed (silica gel, 70:28:2 chloroform /ethyl acetate /methanol) to give 11-7 as a white solid.
TLC R_{f} = 0.30 (silica, 70:25:5 chloroform /ethyl acetate /methanol)
¹H NMR (300 MHz, CDCl₃) δ 7.05 (d, J=7.3 Hz, 1H), 6.34 (d, J=7.3 Hz, 1H), 5.48 (s, 1H), 4.79 (s, 1H), 3.37 (m, 2H), 3.15 (m, 2H), 2.69 (t, J=6.3 Hz, 2H), 2.59 (t, J=7.3 Hz, 2H), 1.88 (m, 4H), 1.44 (s, 9H).

### 3-(5,6,7,8-Tetrahydro-[1,8]naphthyridin-2-yl)-propylamine dihydrochloride (11-8)

HCl gas was rapidly bubbled through a solution of 11-7 (2.5 g, 8.6 mmol) in EtOAc (100 ml) at 0°C for 10 minutes. After 30 minutes, the solution was purged with argon for 30 minutes. The solution was concentrated and then azeotroped with CH₃CN to give the amine 11-8 as a yellow solid.
¹H NMR (300 MHz, CD₃OD) δ 7.61 (d, J=7.3 Hz, 1H), 6.67 (d, J=7.3 Hz, 1H), 3.52 (t, J=5.6 Hz, 2H), 2.99 (m, 2H), 2.83 (m, 4H), 2.08 (m, 2H),1.96 (m, 2H).

### 3(S)-(3-Fluorophenyl)-3-{3-[3-(5,6,7,8-tetrahydro-[1,8]naphthyridin-2-yl)-propyl]-ureido}-propionic acid ethyl ester (11-9)

To a stirred solution of 9-1 (100 mg, 0.4037 mmol), DIPEA (0.380 ml, 2.42 mmol) and CHCl₃ (5 ml) was added triphosgene (42 mg, 0.1413 mmol). After 30 minutes, 11-8 was added. After 1 hour, the reaction was diluted with EtOAc and then washed with sat NaHCO₃, brine, and dried over MgSO₄. Evaporative removal of the solvent gave 11-9 as a yellow solid.
TLC R_{f} = 0.37 (silica, 75:10:15 chloroform /ethyl acetate /methanol)
¹H NMR (300 MHz, CDCl₃) δ 7.22 (m,2H), 7.11 (m, 2H), 6.99 (m, 1H), 6.36 (d, J=7.1 Hz, 1H), 6.00 (m, 1H), 5.78 (m, 1H), 5.27 (m, 1H), 4.08 (m, 2H), 3.66 (m, 1H), 3.44 (m, 2H), 3.21 (m, 2H), 2.63 to 2.91 (m, 6H), 1.92 (m, 2H), 1.85 (m, 2H), 1.18 (t, J=7.1 Hz,3H).

### 3(S)-(3-Fluorophenyl)-3-{[3-(5,6,7,8-tetrahydro-[1,8]naphthyridin-2-yl)-propyl]-ureido}-propionic acid (11-10)

To a solution of 11-9 (0.4037 mmol) in EtOH (3 mL) was added 1N NaOH (0.600 ml, 0.600 mmol). After stirring for 2 h, the solvents were evaporated and the residue was chromatographed (silica gel, 20:10:1:1 to 10:10:1:1 ethyl acetate /EtOH /water /NH₄OH) to give 11-10 as a white solid.
TLC Rf = 0.21 (10:10:1:1 ethyl acetate/EtOH/water/NH₄OH).
¹H NMR (300 MHz, CD₃OD) δ 7.41 (d, J=7.3 Hz, 1H), 7.31 (m, 1H), 7.17 (d, J=7.5 Hz, 1H), 7.09 (d, J=10.2 Hz, 1H), 6.95 (m,1H), 6.49 (d,J=7.3 Hz, 1H), 5.23 (m, 1H,), 3.45 (t, J=5.6 Hz, 2H), 3.04 (m, 1H), 2.46 to 2.79 (m, 7H), 1.76 to 1.96 (m,4H).

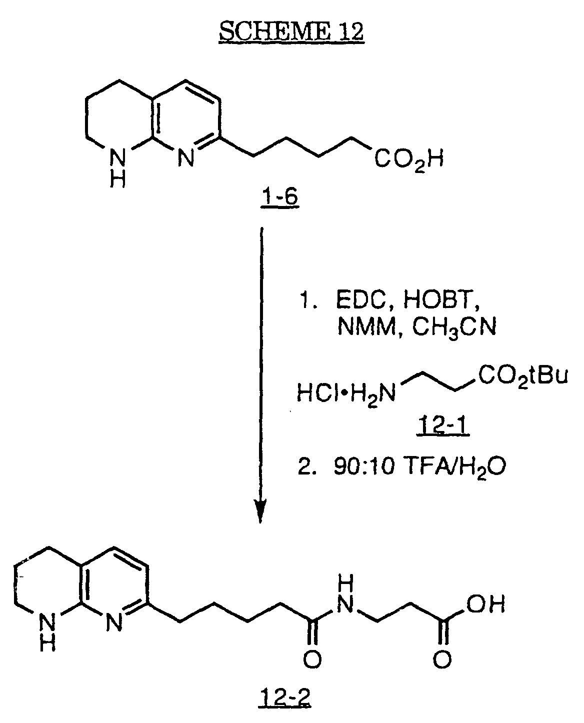

### 3-(5-(5,6,7,8-Tetrahydro-[1,8]naphthyridin-2-yl)-pentanoylamino)-propionic acid (12-2)

A mixture of acid 1-6 (10.8 mg, 0.04 mmol), EDC (7.7 mg, 0.04 mmol), HOBT (5.4 mg, 0.04 mmol) and NMM (0.026 mL, 0.24 mmol) in DMF (1 mL) was agitated until clear solution. After 30 minutes, amine 12-1 was added. The solution was agitated for one minute and then let stand for 18 h. The solution was diluted with ethyl acetate, washed with sat. NaHCO₃ and H₂O. Following evaporative removal of the solvent, the residue was dissolved in 90:10 TFA/H2O (1 ml). After 2 h, the solvents were evaporated to give acid 12-2.
TLC Rf = 0.49 (silica, 10:10:1:1 ethyl acetate/ EtOH/ NH₄OH/ H₂O).
Mass calculated for C₁₆H₂₃N₃O₃= 305, found M+1=306

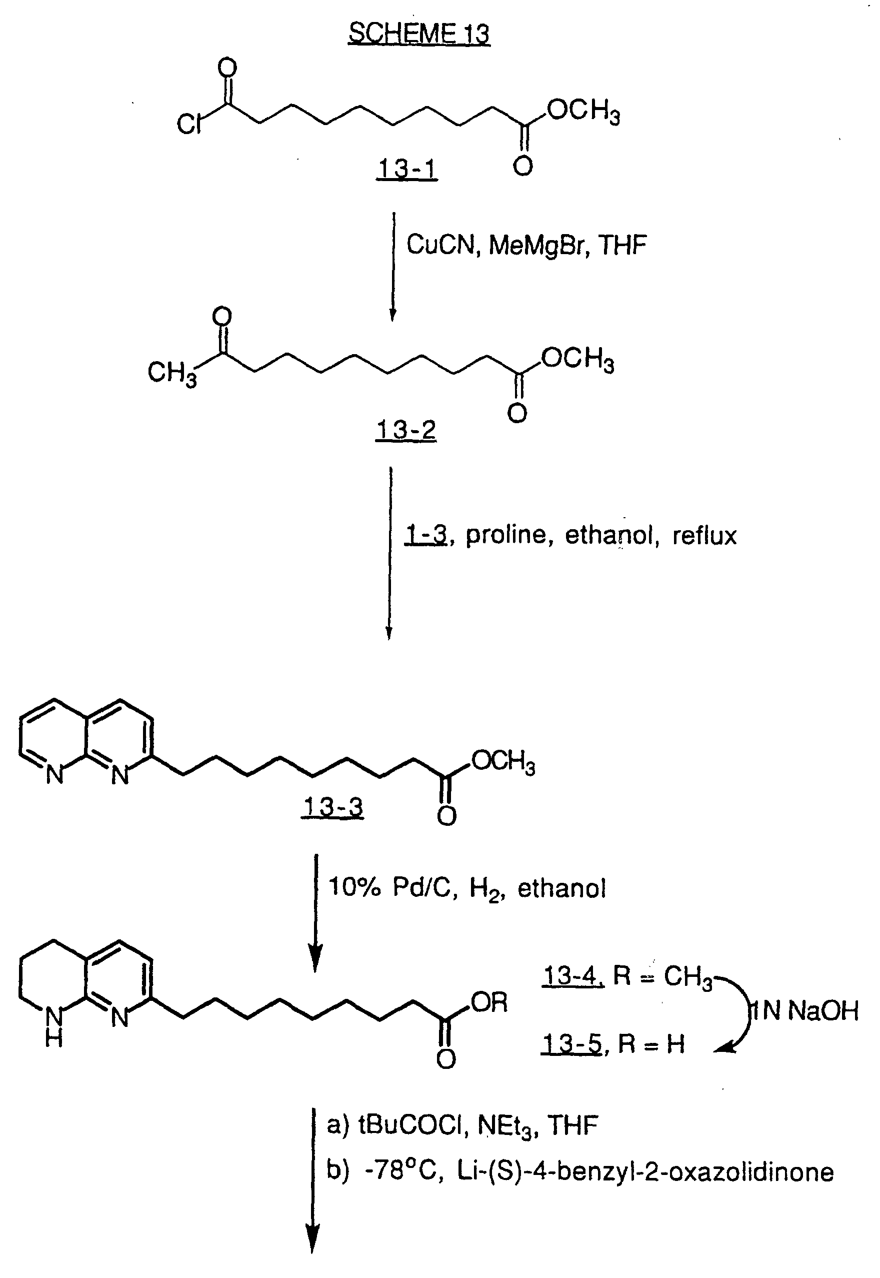

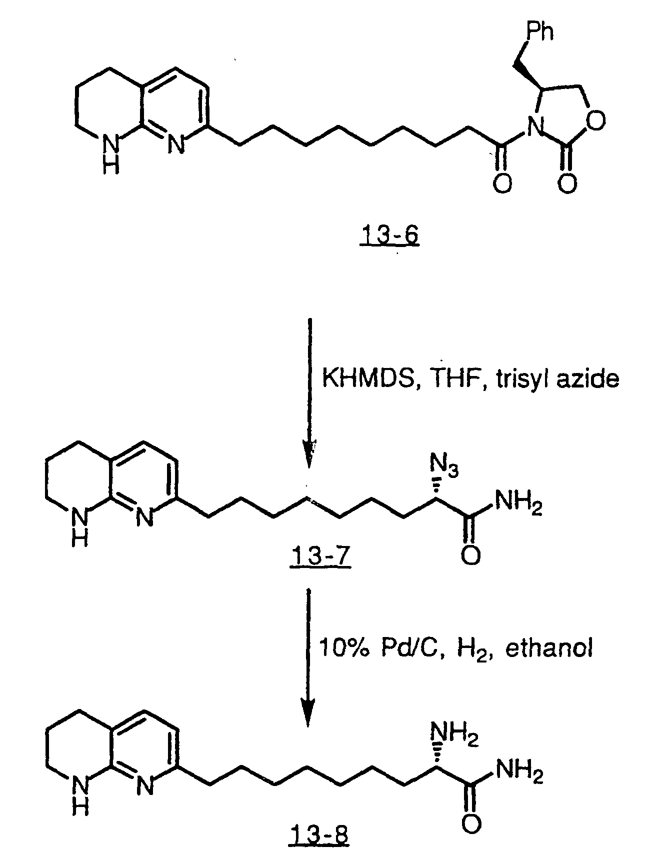

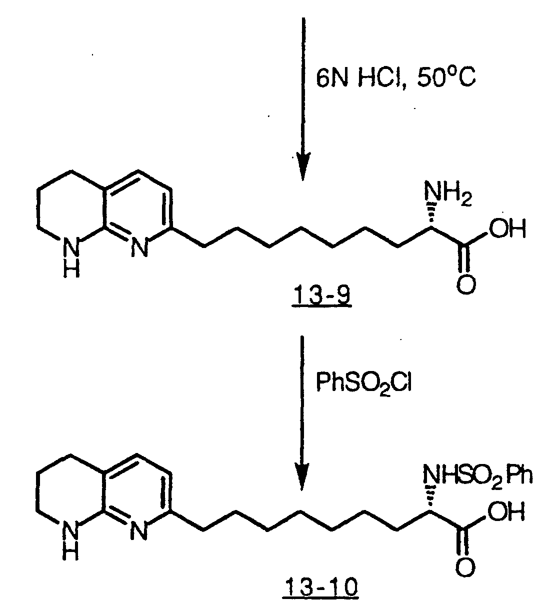

### 10-Oxo-undecanoic methyl ester (13-2)

To a suspension of CuCN (5.0 g, 56 mmol) in THF (200 mL) at -78°C was added MeMgBr (17.4 mL, 3M solution in THF) dropwise. After addition was complete, the reaction mixture was warmed to -15°C for 5 min, recooled to -78°C and then treated dropwise with 13-1 (9.3 mL, 42 mmol). The reaction mixture was then warmed to -10°C for 1.5 h and then quenched with 90% sat. NH₄Cl (300 mL) and EtOAc (400 mL). The organic phase was washed with sat. NH₄Cl, sat. NaHCO₃, and brine, dried (MgSO₄), and concentrated to furnish 13-2 as a pale yellow oil.
TLC Rf = 0.52 (silica, 30% EtOAc/hexanes);
¹H NMR (300 MHz, CDCl₃) δ 3.65 (s, 3H), 2.43 (t, J=7 Hz, 2H), 2.30 (t, J=7 Hz, 2H), 2.13 (s, 3H), 1.60 (m, 4H), 1.29 (m, 8H).

### 9-([1,8]Naphythyridin-2-yl)-nonanoic acid methyl ester (13-3)

A mixture of 13-2 (9.2 g, 43 mmol), 1-3 (5.3 g, 43 mmol), proline (2.5 g, 22 mmol), and ethanol (215 mL) was heated to reflux for 20 h. The cooled reaction mixture was concentrated and the residue purified by flash chromatography (silica, EtOAc) to give 13-3 as a yellow oil.
TLC Rf = 0.37 (silica, EtOAc);
¹H NMR (300 MHz, CDCl₃) δ 9.08 (m, 1H), 8.16 (m, 1H), 8.10 (d, J=8 Hz, 1H), 7.44 (m, 1H), 7.40 (d, J=8 Hz, 1H), 3.68 (s, 3H), 3.04 (m, 2H), 2.30 (m, 2H), 1.90 (m, 2H), 1.60 (m, 2H), 1.50-1.20 (m, 8H).

### 9-(5,6,7,8-Tetrahydro-[1,8]naphthyridin-2-yl)-nonanoic acid methyl ester (13-4)

A mixture of 13-3 (8.5 g, 28 mmol), 10% Pd/C (1.7 g), and ethanol (140 mL) was stirred under a hydrogen atmosphere for 20 h. The reaction mixture was then filtered through a celite pad and concentrated to give 13-4 as a pale yellow oil.
TLC Rf = 0.45 (silica, EtOAc);
¹H NMR (300 MHz, CDCl₃) δ 7.06 (d, J=8 Hz, 1H), 6.33 (d, J=8 Hz, 1H), 3.68 (s, 3H), 3.40 (m, 2H), 2.70 (m, 2H), 2.53 (m, 2H), 2.30 (m, 2H), 1.90 (m, 2H), 1.60 (m, 4H), 1.50-1.20 (m, 8H).

### 9-(5,6,7,8-Tetrahydro-[1,8]naphthyridin-2-yl)-nonanoic acid(13-5)

A solution of 13-4 (8.2 g, 27 mmol), 1N NaOH (30 mL), and methanol (134 mL) was stirred at 0°C for 72 h. The methanol was evaporated and the residue dissolved in H₂O (30 mL) and neutralized with 1N HCl to effect a white precipitate. The solid was collected by filtration and dried at 50°C to give 13-5.
TLC Rf = 0.53 (silica, 20:1:1 ethanol/NH₄OH/H₂O);
¹H NMR (300 MHz, CD₃OD) δ 7.36 (d, J=8 Hz, 1H), 6.46 (d, J=8 Hz, 1H), 3.44 (m, 2H), 2.75 (m, 2H), 2.60 (m, 2H), 2.22 (m, 2H), 1.90 (m, 2H), 1.62 (m, 4H), 1.40-1.30 (m, 8H).

### 9-(5,6,7,8-Tetrahydro-[1,8]naphthyridin-2-yl)-nonanoyl [(-)-4-benzyl-2-oxazolidinone (13-6)

To a suspension of 13-5 (5.6 g, 19 mmol), THF (97 mL), and NEt₃ (3.2 mL, 23 mmol) at -78°C was added trimethylacetyl chloride (2.6 mL, 21 mmol) dropwise. After addition was complete, the reaction mixture was warmed to 0°C for 2 h then recooled to -78°C and treated with lithium (S)-(-)-4-benzyl-2-oxazolidinone (18 mL, 29 mmol; 1.6M solution in THF). The reaction mixture was then warmed to 0°C for 1 h and poured into EtOAc (300 mL) and sat. NaHCO₃ (30 mL). The organic phase was washed with sat. NaHCO₃, H₂O, and brine, dried (MgSO₄), and concentrated. Flash chromatography (silica, EtOAc) gave 13-6 as an oil.
TLC Rf = 0.31 (silica, EtOAc); ,
1H NMR (300 MHz, CDCl₃) δ 7.40-7.15 (m, 5H), 7.03 (d, J=8 Hz, 1H), 6.33 (d, J=8 Hz, 1H), 4.67 (m, 1H), 4.13 (m, 2H), 3.40 (m, 2H), 3.29 (1H), 3.00-2.73 (m, 3H), 2.68 (m, 2H), 2.53 (m, 2H), 1.90 (m, 2H), 1.63 (m, 4H), 1.40-1.30 (m, 8H).

### 2-Azido-9-(5,6,7,8-tetrahydro-[1,8]naphthyridin-2-yl)-nonanoyl amide (13-7)

To a solution of 13-6 (19.4 mmol) and THF (65 mL) at -78°C was added KHMDS (89 mL, 44.6 mmol; 0.5 M solution in THF). After 30 min., trisyl azide (9.0 g, 29 mmol) in THF (50 mL) was added via cannula. After 3 min., the reaction was quenched with AcOH (6.9 mL) dropwise followed by removal of the cooling bath. After 20 h, the reaction mixture was poured into EtOAc (300 mL) and sat. NaHCO₃ (60 mL). The organic phase was washed with sat. NaHCO₃, H₂O, and brine, dried (MgSO₄), and concentrated. Flash chromatography (silica, 5-20% isopropanol/EtOAc) gave 13-7 as an oil.
TLC Rf = 0.47 (silica, 5% NH₃ sat. ethanol/EtOAc);
¹H NMR (300 MHz, CDCl₃) δ 7.03 (d, J=8 Hz, 1H), 6.34 (d, J=8 Hz, 1H), 6.30 (bs, 1H), 5.77 (bs, 1H), 4.82 (bs, 1H), 3.97 (m, 1H), 3.40 (m, 2H), 2.68 (m, 2H), 2.50 (m, 2H), 2.00-1.20 (m, 14H).

### 2-Amino-9-(5,6,7,8-tetrahydro-[1,8]naphthyridin-2-yl)-nonanoyl amide 13-8)

A mixture of 13-7 (0.4 g, 1.2 mmol), ethanol (6 mL), and 10% Pd/C (0.4 g) was stirred under a hydrogen atmosphere for 30 min. The reaction mixture was then filtered through a celite pad and concentrated to give 13-8 as an oil.
TLC Rf = 0.82 (silica, 10:1:1 ethanol/NH₄OH/H₂O);
¹H NMR (300 MHz, CDCl₃) δ 7.10 (m, 1H), 7.08 (d, J=8 Hz, 1H), 6.35 (d, J=8 Hz, 1H), 6.30 (bs, 1H), 5.48 (bs, 1H), 5.13 (bs, 1H), 3.40 (m, 4H), 2.70 (m, 2H), 2.53 (m, 2H), 2.00-1.20 (m, 14H).

### 2-Amino-9-(5,6,7,8-tetrahydro-[1,8]naphthyridin-2-yl)-nonanoic acid (13-9)

A solution of 13-8 (0.3 g, 1.0 mmol) and 6N HCl (10 mL) was heated at 50°C for 20 h. The reaction was then treated with conc. HCl (1 mL) and heated for an additional 5 h. The reaction mixture was then concentrated, the residue dissolved in H₂O and neutralized with conc. NH₄OH to form a precipitate. Filtration followed by drying under vacuum at 40°C gave 13-9 as a gray solid.
TLC Rf = 0.90 (silica, 10:1:1 ethanol/NH₄OH/H₂O);
¹H NMR (300 MHz, D₂O) δ 7.00 (d, J=8 Hz, 1H), 6.37 (d, J=8 Hz, 1H), 3.10 (m, 2H), 3.00 (m, 1H), 2.45 (m, 2H), 2.27 (m, 2H), 1.63 (m, 2H), 1.30 (m, 4H), 1.05 (m, 8H).

### 2(S)-(Benzenesulfonylamino)-9-(5,6,7,8-tetrahydro-[1,8]naphthyridin-2-yl)-nonanoic acid (13-10)

A solution of 13-9 (0.27 g, 0.88 mmol) in H₂O/dioxane (2:1, 4.4 mL) was cooled to 0°C and then treated dropwise with 1N NaOH to attained a pH of 10.5. The reaction mixture was then treated with with PhSO₂Cl (0.23 g, 1.3 mmol) in dioxane (750 µL) while maintaining a pH of 10.5 by adding 1N NaOH. After 15 min, the pH was adjusted to 7 with ¹N HCl to effect a white precipitate. The precipitate was collected by filtration and triturated with EtOAc and then ether to give 13-10 as a colorless solid.
¹H NMR (300 MHz, D₂O) δ 7.50 (m, 2H), 7.10 (m, 3H), 6.95 (d, J=8 Hz, 1H), 6.20 (d, J=8 Hz, 1H), 4.60 (m, 1H), 3.04 (m, 2H), 2.40 (m, 2H), 2.23 (m, 2H), 1.63 (m, 2H), 1.20 (m, 4H), 0.08 (m, 8H).

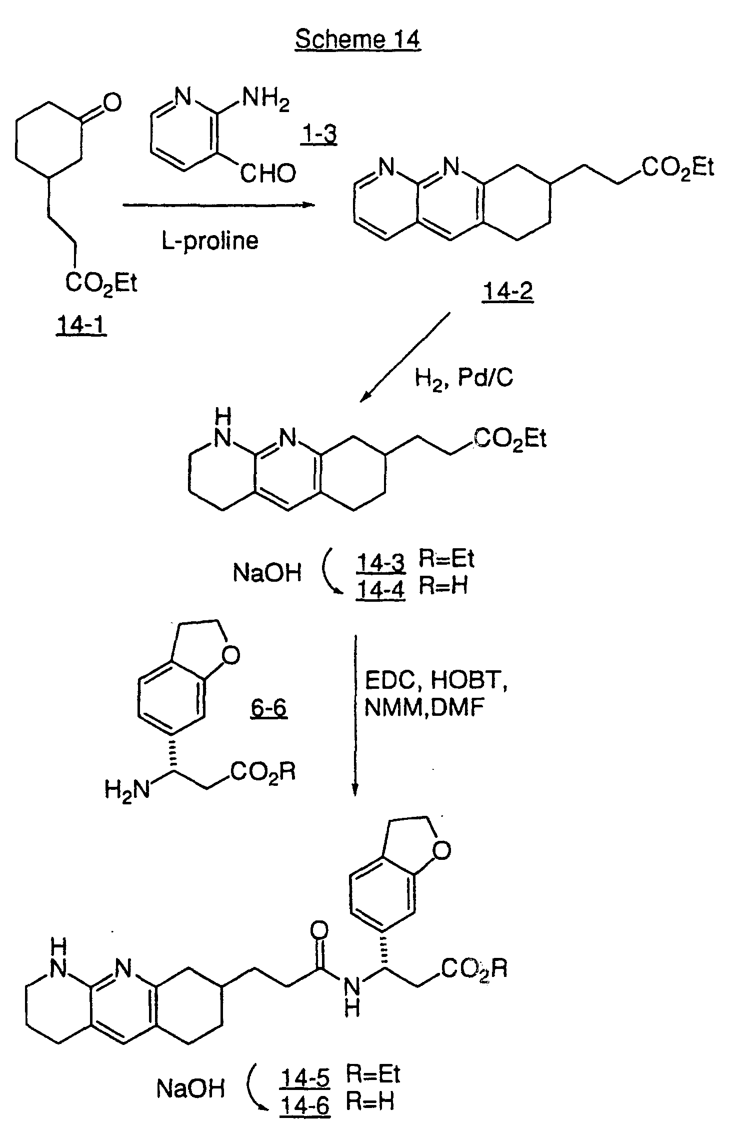

### 3-(6,7,8,9-tetrahydro-benzo[b]-[1,8]naphthyridin-8-yl)-propionic acid ethyl ester (14-2)

A solution of 2-amino-pyridine-3-carbaldehyde (1-3) (0.244 g, 2.0 mmol) and 3-(3-oxo-cyclohexyl)-propionic acid ethyl ester (14-1) (0.245 g, 2.00 mmol; for prep., *see* J. R. Wiseman et.al., *J. Am. Chem. Soc.,* 1970, 92, 956-962) in ethanol (10 mL) was treated with L-proline (0.230 g, 2.00 mmol) and heated at reflux for 12 h. The solution was cooled to ambient temperature and concentrated. The residue was purified by flash chromatography (10% acetone in CH₂Cl₂) to give 14-2.
¹H NMR (300 MHz, CDCl₃) δ 9.02-9.00 (m, 1H), 8.09-8.06 (m, 1H), 7.84-7.37 (m, 1H), 7.27(s, 1H), 4.15 (q, J=7.2 Hz, 2H), 3.43-3.35 (m, 1H), 3.31-2.98 (m, 4H), 2.84-2.75 (dd, J=11, 16 Hz, 1H), 2.47 (t, J=7.8 Hz, 2H), 2.10-1.77 (m, 3H), 1.57-1.51 (m, 1H), 1.27 (t, J=7.2 Hz, 3H) ppm.

### 3-(1,2,3,4,6,7,8,9-octahydro-benzo[b]-[1,8]naphthyridin-8-yl)-propionic acid ethyl ester (14-3)

A mixture of 14-2 (0.298 g, 1.05 mmol) and Pd on carbon (0.060 g) in ethanol (10 mL) was placed under 1 atm of H₂ and stirred for 12 h. The solution was concentrated. The residue was purified by flash chromatography (5% MeOH in CH₂Cl₂) to give 14-3.
¹H NMR (300 MHz, CDCl₃) δ 6.88 (s, 1H),4.70 (s, 1H),4.13 (q, J=7.2 Hz, 2H), 3.37 (m, 2H), 2.80-2.58 (m, 5 H), 2.42-2.27 (m, 3H), 1.93-1.65 (m, 6H), 1.25 (t, J=7.2 Hz, 3H) ppm.

### 3-(1,2,3,4,6,7,8,9-octahydro-benzo[b][1,8]naphthyridin-8-yl)-propionic acid (14-4)

A solution of 14-3 (0.180 g, 0.62 mmol) and NaOH (1.24 mL of a 1N soln, 1.24 mmol) in THF/H₂O (5 mL/5 mL) was stirred at room temperature for 12 h. The solution was concentrated. The residue was triturated with Et₂O to give 14-4.
¹H NMR (300 MHz, CD₃OD) δ 6.91 (s, 1H), 2.75-2.58 (m, 6H), 2.29-2.15 (m, 5H), 1.96-1.62 (m, 5H), 1.34-1.27 (m, 1H) ppm.

### 3(S)-(2,3-Dihydro-benzofuran-6-yl)-3-(3-(1,2,3,4,6,7,8,9-octahydro-benzo[b][1,8]naphthyridin-8-yl)-propionylamino)-propionic acid ethyl ester (14-5)

A solution of 14-4 (0.110 g, 0.39 mmol), β-aminoester 6-6 (0.106 g 0.39 mmol), EDC (0.075 g, 0.39 mmol), HOBT (0.053 g, 0.39 mmol) and N-methylmorpholine (0.164 mL, 1.17 mmol) in degassed DMF (5 mL) was stirred at room temperature for 12 h. The solution was concentrated. The residue was purified by flash chromatography to give 14-5.
¹H NMR (300 MHz, CD₃OD) δ 7.19 (s, 1H), 7.12-7.07 (m, 1H), 6.79-6.76 (m, 1H), 6.69 (br s, 1H), 5.49-5.26 (m, 1H), 4.52-4.45 (m, 2H), 4.05 (q, J=7.0 Hz, 2H), 3.41-3.38 (m, 2H), 3.15-3.10 (t, J= 8.5 Hz, 2H), 2.78-2.68 (m, 3H), 3.59 (m, 2H), 2.37-2.21 (m, 5H), 1.61-1.70 (m, 5H), 1.38 (m, 1H), 1.18-1.14 (t, J=7.0 Hz, 3H) ppm.

### 3(S)-(2,3-Dihydro-benzofuran-6-yl)-3-(3-(1,2,3,4,6,7,8,9-octahydro-benzo[b][1,8]naphthyridin-8-yl)-propionylamino)-propionic acid (14-6)

A solution of 14-5 (0.050 g, 0.105 mmol) and aqueous 1N NaOH (0.210 mL, 0.210 mmol) was stirred at room temperature for 3 h. The solution was concentrated. The residue was triturated with Et₂O and the white solid collected by to give 14-6.
¹H NMR (300 MHz, CD₃OD) δ 7.41 (s, 1H), 7.12-7.10 (d, J=7.3 Hz, 1H), 6.80-6.78 (d, J=7.3 Hz, 1H), 6.70 (s, 1H), 5.38 (m, 1H), 4.43 (m, 2H), 3.43 (m, 4H), 3.17 (m, 2H), 2.78 (m, 3H), 2.61 (m, 2H), 2.36 (m, 3H), 1.95 (m, 2H), 1.77 (m, 4H), 1.40 (m, 1H) ppm.

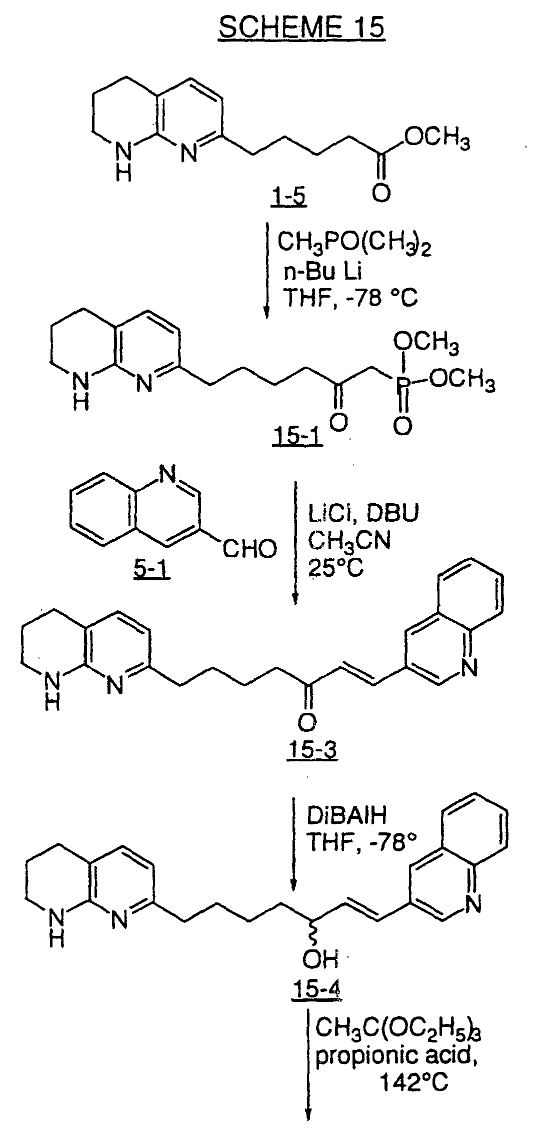

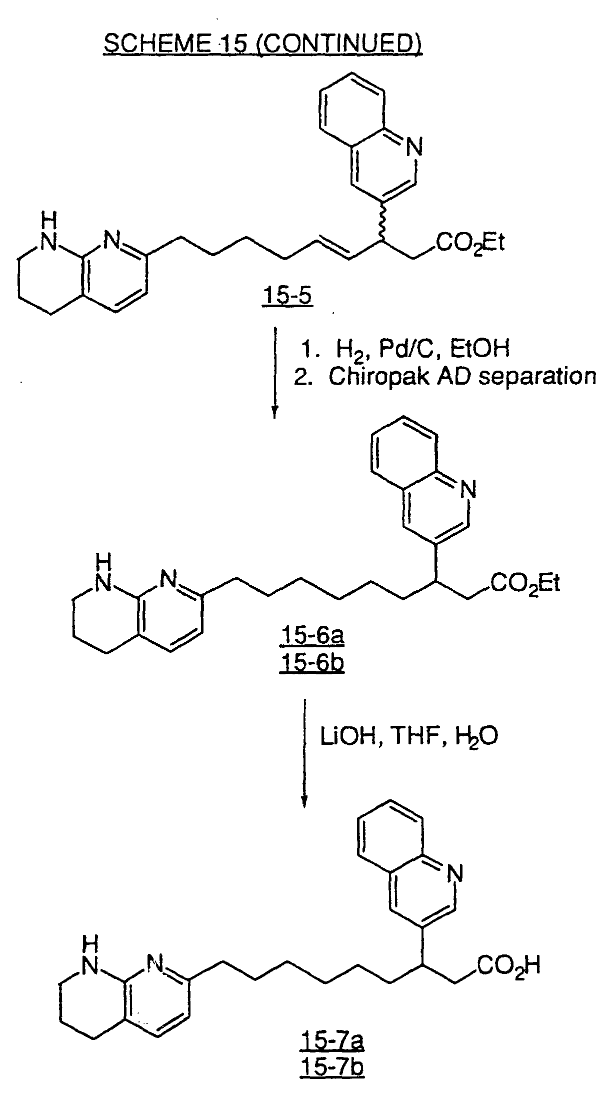

### 2-Oxo-6-(5,6,7,8-tetrahydro-[1,8]-naphthyridin-2-yl)-hexyl-phosphonic acid dimethyl ester (15-1)

A solution of dimethyl methylphosphonate (13.20 g, 106.5 mmol) in anhydrous THF (165 mL) was cooled to -78° and treated dropwise with 2.5 M n-BuLi (42.3 mL). After stirring at -78° for 45 min, a solution of ester 1-5 (6.6 g, 26.6 mmol) in THF (35 mL) was added dropwise and the resulting solution stirred for 30 min -78°, quenched with sat. NH₄Cl (100 mL), then extracted with ethyl acetate (3 X 150 mL). The combined organic extracts were dried (MgSO₄), filtered, and concentrated to afford a yellow oil. Chromatography on silica gel (5% MeOH/CH₂Cl₂) afforded 15-1 as a yellow oil.
R_{f} (silica, 5% MeOH/CH₂Cl₂) = 0.20.
¹H NMR (300 MHz, CDCl₃) δ 7.05 (d, J=7.3 Hz, 1H), 6.34 (d, J=7.32 Hz, 1H), 4.80 (br, s, 1H), 3.81 (s, 3H), 3.75 (s, 3H), 3.4 (m, 2H), 3.08 (d, J=22.7 Hz), 2.7-2.5 (m, 6 H), 1.91 (m, 2H), 1.68 (m, 4H).

### 3-(Quinolin-3-yl)-7-(5,6,7,8-tetrahydro-[1,8]-naphthyridin-2-yl)-(E)-hept-1-en-3-one (15-3)

Ketophosphonate 15-1 (2.0 g, 5.9 mmol), anhydrous LiCl (250 mg, 5.9 mmol), and 3-quinoline-carboxaldehyde 5-1 (0.77 g, 4.9 mmol) in anhydrous acetonitrile (60 mL) were treated with DBU (0.73 mL, 5.88 mmol) and the resulting suspension stirred at room temperature for 1 h. The solvent was removed at reduced pressure and the resulting residue partitioned between brine and methylene chloride. The organic layer was removed, dried, and concentrated to afford a yellow solid which was recrystallized from ethyl acetate/hexanes to afford 15-3 as an off-white solid.
R_{f} (silica, 5% MeOH/CH₂Cl₂) = 0.45.
¹H NMR (300 MHz, CDCl₃) δ 9.05 (d, J= 1.8 Hz, 1H), 8.23 (d, J=1.8 Hz, 1H), 8.05(d, J= 8.5 Hz, 1H), 7.87 (d, J=7.5 Hz, 1H), 7.81 (t, J= 7.5 Hz, 1H), 7.68 (d, J=16 Hz, 1H), 7.58 (t, J=7.5 Hz, 1H), 7.05 (d, J=7.3 Hz, 1H), 6.95 (d, J=16 Hz, 1H), 6.34 (d, J=7.32 Hz, 1H), 4.80 (br, s, 1H), 3.4 (m, 2H), 2.7-2.5 (m, 6 H), 1.91 (m, 2H), 1.68 (m, 4H).

### 3-(Quinolin-3-yl)-7-(5,6,7,8-tetrahydro-[1,8]-naphthyridin-2-yl)-(E)-hept-1-en-3-ol (15-4)

A solution of 15-3 (1.33 g, 3.58 mmol) in anhydrous THF (150 mL) was cooled to -78°, then treated dropwise with i-Bu₂AlH (10.75 mL, 10.75 mmol). The resulting solution was stirred at -78° for 20 min., then quenched with ethyl acetate (20 mL), warmed to room temperature, treated with 1 M potassium sodium tartrate (25 mL) and stirred for 4 h. The mixture was extracted with ethyl acetate (2 X 150 mL) dried, filtered and evaporated to afford 15-4 as an off-white solid.
R_{f} (silica, 10% MeOH/CH₂Cl₂) = 0.10.
¹H NMR (300 MHz, CDCl₃) δ 9.05 (d, J= 1.8 Hz, 1H), 8.15 (d, J= 8.5 Hz, 1H), 8.13 (d, J=1.8 Hz, 1H), 7.87 (d, J=7.5 Hz, 1H), 7.81 (t, J= 7.5 Hz, 1H), 7.58 (t, J=7.5 Hz, 1H), 7.05 (d, J=7.3 Hz, 1H), 6.86 (d, J=16 Hz, 1H), 6.45 (dd, J=16,6.5 Hz, 1H) 6.37 (d, J=7.32 Hz, 1H), 4.80 (br, s, 1H), 4.4 (m, 1H) 3.4 (m, 2H),2.75 (m, 2 H), 2.62 (m, 2H), 1.91 (m, 2H), 1.72 (m, 4H) 1.55(m, 2 H).

### 3-(Quinolin-3-yl)-9-(5,6,7,8-tetrahydro-[1,8]-naphthyridin-2-yl)-(E)-non-4-enoic acid ethyl ester (15-5)

A solution of the allylic alcohol 15-4 (1.4 g, 3.75 mmol) in triethyl orthoacetate (64 mL) was treated with propionic acid (0.014 mL, 0.19 mmol) and refluxed for 1.5 h. The cooled mixture was treated with a solution of 1:1 sat. brine/1N HCl (50 mL), then extracted with CH₂Cl₂ (3 X 125 mL). The pooled organic extracts were washed with sat. NaHCO₃, dried, filtered and evaporated. Chromatography on silica gel (5% MeOH/CH₂Cl₂) afforded 15-5 as a yellow glass.
R_{f} (silica, 5% MeOH/CH₂Cl₂) = 0.25.
¹H NMR (300 MHz, CDCl₃) δ 9.08 (br, s, 1H), 8.92 (d, J=1.8 Hz, 1H), 8.15 (d, J= 8.5 Hz, 1H), 7.96 (d, J=1.8 Hz, 1H), 7.82 (d, J=7.5 Hz, 1H), 7.71 (t, J= 7.5 Hz, 1H), 7.54 (t, J=7.5 Hz, 1H), 7.05 (d, J=7.3 Hz, 1H), 6.25 (d, J=7.32 Hz, 1H), 5.6 (m, 2H), 4.05 (m, 2H), 4.05 (m, 1H), 3.40 (m, 2H), 2.75 (m, 2 H), 2.65 (m, 2H),2.58 (m, 2H), 2.01 (m, 2H), 1.91 (m, 2H), 1.65 (m, 2H), 1.45(m, 2 H), 1.08 (t, J=7.5 Hz, 3H).

### 3(S or R)-(Quinolin-3-yl)-9-(5,6,7,8-tetrahydro-[1,8]-naphthyridin-2-yl)-nonanoic acid ethyl ester & 3(R or S)-(quinolin-3-yl)-9-(5,6,7,8-tetrahydro-[1,8]-naphthyridin-2-yl)-nonanoic acid ethyl ester (15-6a & 15-6b)

A solution of 15-5 (1.0 g, 2.25 mmol) in EtOH was treated with 10% Pd on C (200 mg) and the mixture stirred under a hydrogen gas-filled balloon for 3 hours. The catalyst was removed by filtration through celite and the solvent evaporated to afford the mixture of enantiomers as a colorless glass.
R_{f} (silica, 5% MeOH/CH₂Cl₂) = 0.25.
¹H NMR (300 MHz, CDCl₃) δ 8.81 (d, J= 1.8 Hz, 1H), 8.60 (br, s, 1H), 8.15 (d, J= 8.5 Hz, 1H), 7.96 (d, J=1.8 Hz, 1H), 7.82 (d, J=7.5 Hz, 1H), 7.71 (t, J= 7.5 Hz, 1H), 7.54 (t, J=7.5 Hz, 1H), 7.15 (d, J=7.3 Hz, 1H), 6.25 (d, J=7.32 Hz, 1H), 4.05 (m, 2H), 3.40 (m, 2H), 3.25 (m, 1H), 2.75 (m, 2 H), 2.65 (m, 2H),2.58 (m, 2H), 1.87 (m, 2H), 1.81 (m, 2H), 1.75 (m, 2H), 1.25(m, 4 H), 1.08 (t, J=7.5 Hz, 3H).
The enantiomers 15-6a and 15-6b were separated on a 250 X 20 mm Chiralpak AD column (flow = 8 mL/min, A:B = 50:50) (A = 0.1% diethylamine/hexane, B =1-propanol). Rt (15-6a) = 18.8 min, (15-6b)= 20.9 min.

### 3(R or S)-(Quinolih-3-yl)-9-(5,6,7,8-tetrahydro-[1,8]-naphthyridin-2-yl)-nonanoic acid (15-7a)

A solution of 15-6a (193 mg, 0.43 mmol) in 50% aqueous THF (10 mL) was treated with solid LiOH (27 mg, 0.65 mmol), and the mixture stirred at room temperature for 18 h, then neutralized with 1N HCl. Chromatography on silica gel (50% A:50% EtOAc) (A= 20:1:1 EtOH: NH₄OH:H₂O) afforded 15-7a as a colorless glass.
R_{f} (silica, 50% A: EtOAc) = 0.45
¹H NMR (300 MHz, CDCl₃) δ 10.2 (br, s, 1H), 8.92 (d, J=1.8 Hz, 1H), 8.15 (d, J= 8.5 Hz, 1H), 7.96 (d, J=1.8 Hz, 1H), 7.82 (d, J=7.5 Hz, 1H), 7.71 (t, J= 7.5 Hz, 1H), 7.54 (t, J=7.5 Hz, 1H), 7.05 (d, J=7.3 Hz, 1H), 6.25 (d, J=7.32 Hz, 1H), 5.6 (m, 2H), 3.56 (m, 1H), 3.40 (m, 2H), 2.75 (m, 2 H), 2.65 (m, 2H),2.58 (m, 2H), 2.01 (m, 2H), 1.91 (m, 2H), 1.65 (m, 2H), 1.45(m, 2 H).

### 3(S or R)-(Quinolin-3-yl)-9-(5,6,7,8-tetrahydro-[1,8]-naphthyridin-2-yl)-nonanoic acid (15-7b)

This compound was prepared from 15-6b as described above for 15-7a.

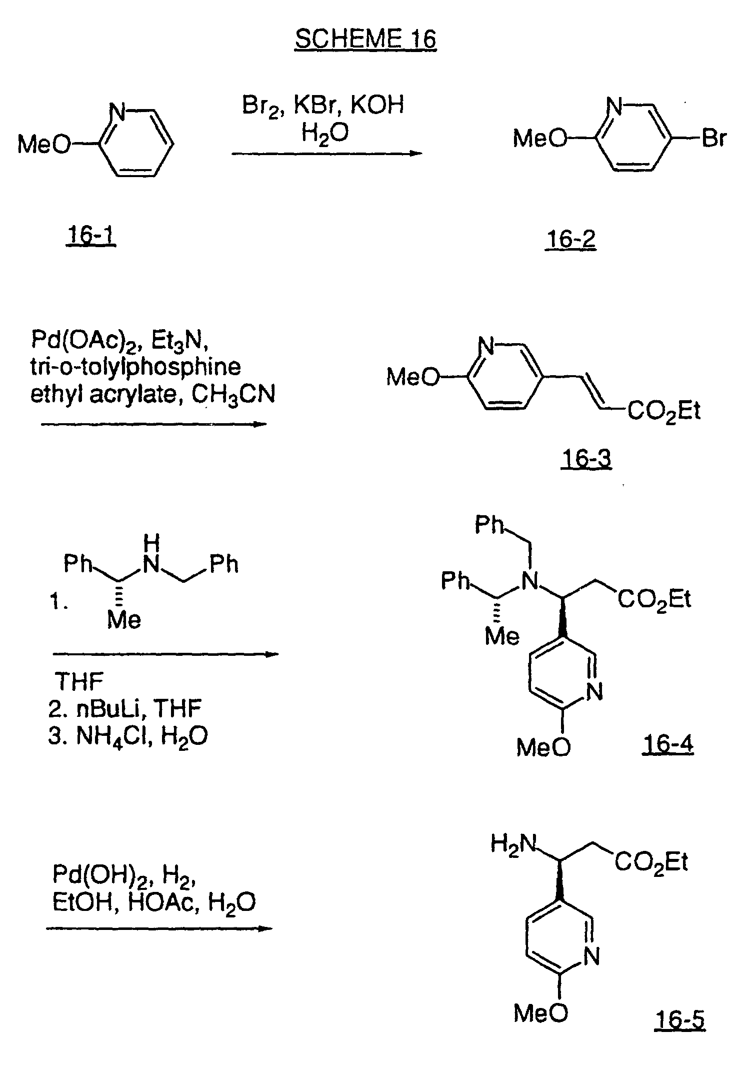

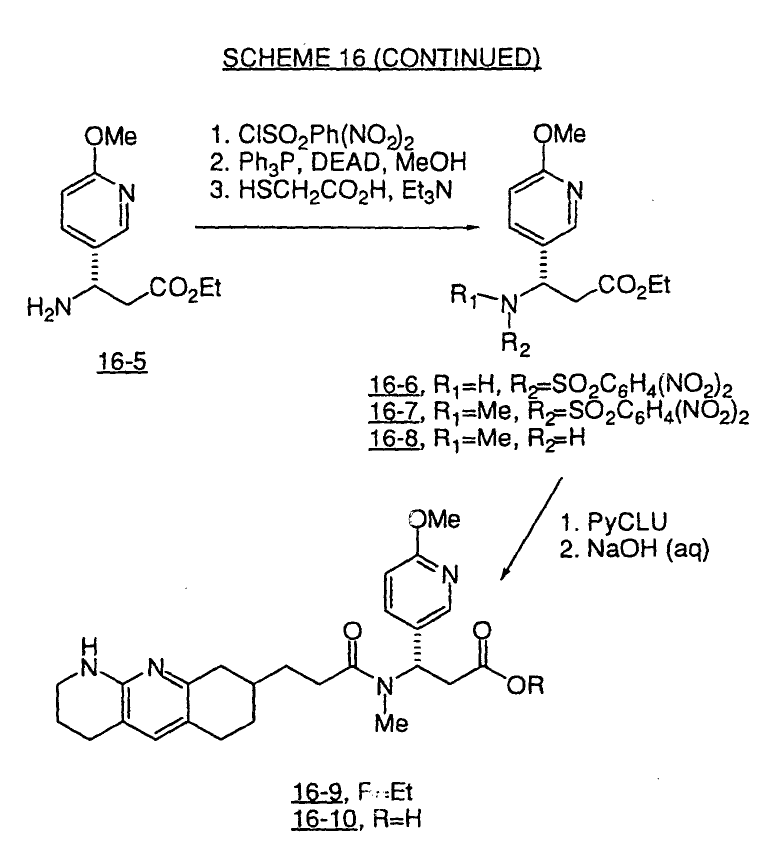

### 5-Bromo-2-methoxypyridine (16-2)

To a solution of KOH (4.2 g, 0.075 mol) in water (750 mL) was added 2-methoxypyridine 16-1 (16.4 g, 0.15 mol) followed by a dropwise addition of bromine (24 g, 0.15 mol) in 1N aqueous KBr (750 mL) and the resulting solution was stirred at room temperature for 5 hr. Solid NaHCO₃ was added until basic and the solution was extracted with CHCl₃ (3x500 mL). The organic layer was washed with 10% NaHSO₃, then brine, dried over Na₂SO₄, filtered, and the solvent removed in vacuo. The resulting dark brown oil was predominantly the desired compound 16-2 and was used as such in the next step.
¹H NMR (300 MHz, CDCl₃) δ 3.91 (3H, s), 6.66 (1H, d), 7.62 (1H, dd), 8.20 (1H, d).

### Ethyl 3-(6-methoxypyridin-3-yl)acrylate (16-3)

A solution of the 5-bromo-2-methoxypyridine 16-2 (74.3 g, 0.4 mol), ethyl acrylate (150 mL, 1.4 mol), triethylamine (150 mL, 1.08 mol), palladium acetate (10 g, 0.045 mol) and tri-o-tolylphosphine (20 g, 0.066 mol) in 100 mL acetonitrile was degassed with argon for 10 minutes. The mixture was heated at 90°C for 12 hr, then the volatiles were removed in vacuo. Toluene (300 mL) was added and the mixture concentrated again. Diethyl ether (300 mL) was added and the mixture filtered through a pad of silica gel eluting with 800 mL of diethyl ether. After removal of the diethyl ether, the residue was chromatographed on silica gel eluting with EtOAc/hexane, 1:19 then 1:14 then 1:9 to give 16-3 as a yellow solid.
1H NMR (300 MHz, CDCl3) δ 1.34 (3H, t), 3.97 (3H, s), 4.26 (2H, q), 6.34 (1H, d),6.76 (1H, d), 7.63 (1H, d), 7.77 (1H, dd),8.27 (1H, d).

### N-Benzyl-(R)-α-methylbenzyl-3(S)-(6-methoxypyridin-3-yl)-β-alanine ethyl ester (16-4)

To a solution of N-benzyl-(R)-α-methylbenzylamine (97.5 g, 462 mmol) in THF (750 mL) at 0°C was added n-butyllithium (2.5M in hexanes; 178.5 mL, 446 mmol). The dark violet solution was stirred at 0°C for 20 minutes, cooled to -78°C, and the ester 16-3 (63.7 g, 308 mmol) in THF (250 mL) was added over 60 minutes. The resulting solution was stirred at -78°C for 1 hr, then cannulated into saturated NH₄Cl and extracted with EtOAc, washed with water then brine, dried and concentrated *in vacuo* to give an oil. Column chromatography (silica gel; hexane/EtOAc 9:1 then 4:1) gave 16-4 as an oil contaminated with N-benzyl-(R)-α-methylbenzylamine. This oil was taken up in 5% AcOH in water and extracted with diethyl ether (4x). The organic layers were dried over MgSO₄ and the solvent removed to give the title compound 16-4.
¹H NMR (300 MHz, CDCl₃) δ 1.08 (3H, t), 1.27 (3H, d), 2.52 (1H, dd), 2.62 (1H, dd), 3.66 (1H, d), 3.70 (1H, d), 3.93 (3H, s), 3.95 (2H, m), 4.41 (1H, dd), 6.74 (1H, d), 7.15-7.45 (10H, m), 7.64 (1H, dd), 8.15 (1H, d).

### 3(S)-(6-methoxypyridin-3-yl)-β-alanine ethyl ester (16-5)

To a degassed (argon) solution of the ester 16-4 (70 g) in EtOH (250 mL), HOAc (25 mL) and water (2 mL) was added 20% Pd(OH)₂ on carbon. The mixture was placed under hydrogen gas using a balloon and the resulting mixture was stirred for 24 hr. After filtration through celite (washing with EtOAc), the solvent was removed in vacuo to afford a waxy solid. This was dissolved in 200 mL water and extracted with diethyl ether (2x200 mL). The aqueous layer was then treated with solid K₂CO₃ until fully saturated and extracted with EtOAc (4x200 mL). After drying over MgSO₄, the solvent was removed in vacuo to give the title compound 16-5 as an oil which solidified in the freezer.
¹H NMR (300 MHz, CDCl₃) δ 1.23 (3H, t), 2.61 (1H, dd), 2.68 (1H, dd), 3.92 (3H, s), 4.15 (2H, q), 4.41 (1H, dd), 6.93 (1H, d), 7.62 (1H, dd), 8.13 (1H, d).

### 3(S)-(6-Methoxy-pyridin-3-yl)-3-(4-nitro-benzenesulfonylamino)-propionic acid ethyl ester (16-6)

A solution of aminoester 16-5 (3.0 g, 13.0 mmol) in CH₂Cl₂ (20 mL) was treated with aq NaHCO₃ (4.4 g in 20 mL H₂O). 2,4-Dinitrobenzenesulfonyl chloride (4.3 g, 16 mmol) was added and the reaction mixture stirred for 12 h. The solution was extracted with CH₂Cl₂ (3 x 40 mL) and the combined organic solutions washed with satd aq NaHCO₃ (40 mL) and brine (40 mL). The solution was dried over MgSO₄, filtered, and concentrated. The residue was purified by flash chromatography (97:3 CH₂Cl₂/MeOH) to give the desired product 16-6.
TLC Rf = 0.45 (5% methanol/dichloromethane).

### 3(S)-(6-Methoxy-pyridin-3-yl)-3-[methyl-(4-nitro-benzenesulfonyl)-amino]-propionicacid ethyl ester (16-7)

Triphenylphosphine (3.9 g, 15 mmol) was added to a solution of sulfonamide 16-6 (4.5 g, 10 mmol) in THF (30 mL). To this solution was added a solution of diethyl azodicarboxylate (2.4 mL, 15 mmol) in THF/MeOH (10 mL/2.02 mL). A vigorous exotherm occurred and the reaction was stirred overnight at room temperature. The dark mixture was concentrated. The dark oily residue was purified by flash chromatography (40% EtOAc/hexanes) to give the desired product 16-7. TLC Rf = 0.37 (40% ethyl acetate/hexanes).

### 3(S)-(6-Methoxy-pyridin-3-yl)-3-methylamino-propionic acid ethyl ester (16-8)

A solution of sulfonamide 16-7 (4.7 gm, 10 mmol) in CH₂Cl₂ (50 mL) was treated with triethylamine (2.8 mL, 20 mmol) and mercaptoacetic acid (1.04 mL, 15 mmol). The reaction was stirred for 90 min at room temperature. The green solution was diluted with EtOAc (500 mL) and washed with satd aq NaHCO₃ (150 mL), water (3 x 100 mL), and brine (3 x 100 mL). The solution was dried over Na₂SO₄, filtered and concentrated to a black oil. The residue was purified by flash chromatography (5% MeOH/CH₂Cl₂) to give the desired product 16-8.
¹HNMR (300 MHz, CDCl₃) δ 8.07 (d, J=2.4 Hz, 1H), 7.57 (m, 1H), 6.73 (m, 1H), 4.11 (q, J=7.3 Hz, 2H), 3.93 (s, 3H), 2.72 (m, 2H), 1.21 (t, J = 7.3 Hz, 3H) ppm

### 3(S)-(6-Methoxy-pyridin-3-yl)-3-[N-methyl-3-(1,2,3,4,6,7,8,9-octahydro-benzo[b][1,8]naphthyridin-8-yl-propionyl)-amino]-propionic acid ethyl ester (16-9)

Racemic tricyclic ester 14-3 was resolved by chiral HPLC (Chiracel OD column; 25x2mm eluting with 95:5 hexanes/isopropanol/0.1% diethylamine at a flow rate of 8 ml/min: RT = 6.48 and 7.21 min.) The more polar enantiomer was hydrolyzed to give carboxylate 14-4a. A solution of 14-4a (0.175 gm, 0.62 mmol) in DMF/1N HCl (10 mL/0.62 mL) was treated with disopropylethylamine (0.540, 3.10 mmol), aminoester 16-8 (0.162 gm, 0.68 mmol) in DMF ( 2 mL), and PyCLU (0.224 gm, 0.62 mmol). After stirring for 12 h at room temperature, the solution was concentrated and the residue partitioned between EtOAc (20 mL) and satd aq NaHCO₃ (20 mL). The organic solution was washed with brine (10 mL), dried over sodium sulfate, filtered and concentrated. The residue was purified by flash chromatography (5% MeOH/CH₂Cl₂) to give the desired amide 16-9.
TLC Rf = 0.28 (5% methanol/dichloromethane).

### 3(S)-(6-Methoxy-pyridin-3-yl)-3-[N-methyl-3-(1,2,3,4,6,7,8,9-octahydro-benzo[b][1,8]naphthyridin-8-yl-propionyl)-amino]propionic acid (16-10)

A solution of ester 16-9 (0.133 gm, 0.28 mmol) in MeOH/THF/H₂O (1 mL/1 mL/1 mL) was treated with 1N aq NaOH (0.56 mL, 0.56 mmol). After stirring for 12 h at room temperature, the mixture was concentrated and the resulting aqueous residue neutralized with 1N aq HCl (0.56 mL). The residue was purified by flash chromatography (15% EtOH/15% EtOAc/1% aq NH₄OH/ 1% H₂O) to give the desired acid 16-10.
¹HNMR (300 MHz, CDCl₃) mixture of rotamers: δ 8.07 (m, 1H), 7.62 (m, 1H), 7.35 (m, 1H), 6.80 (m, 1H), 6.38 (m, 1H), 4.90 (s, 3H), 3.45 (m, 1H), 3.78 (m, 13H), 1.93 (m, 7H), 1.42 (m, 1H) ppm.

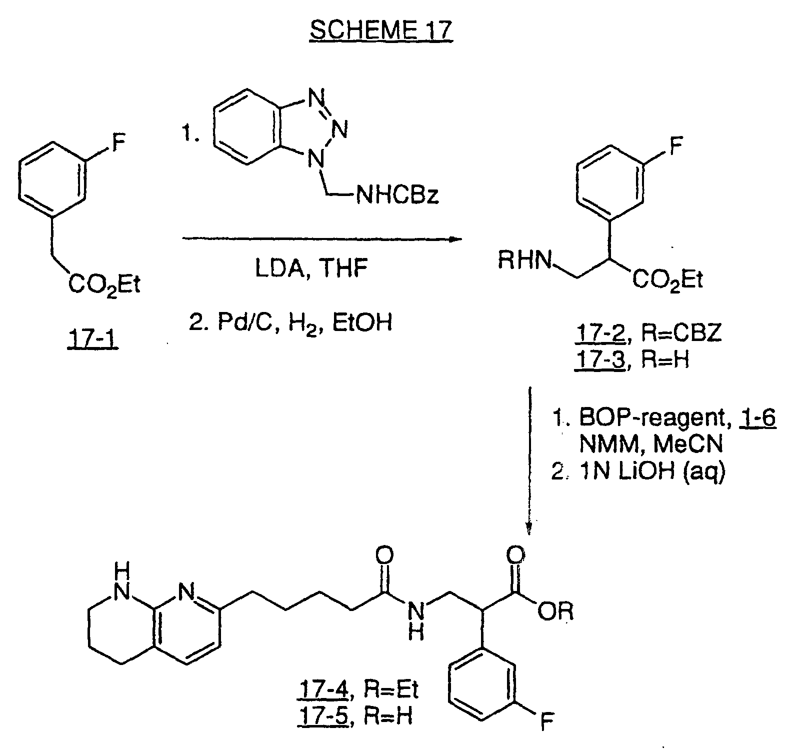

### 3-Benzyloxycarbonylamino-2-(3-fluoro-phenyl)-propionic acid ethyl ester (17-2)

To a stirred solution of LDA (9.43 mL of a 2.0 M solution in THF, 18.86 mmol) in THF (80 mL) at -78°C was added a solution of 3-fluorophenylacetic acid ethyl ester 17-1 (3.12 g, 17.15 mmol) in THF (5 mL). After 10 min, a solution of the aminomethylbenzotriazole (4.8 gm, 17.15 mmol) in THF (5 mL) was added and the solution was slowly warmed to room temperature over 5 h. The reaction was quenched with satd aq NH₄Cl, extracted with EtOAc (3 x 40 mL) and the combined organic solutions washed with brine (50 mL). The organic solution was dried over MgSO₄, filtered, and concentrated. The residue was purified by flash chromatography (20% ethyl acetate/hexanes) to give the desired product 17-2.
TLC Rf = 0.19 (20% ethyl acetate/hexanes).

### 2-(3-Fluorophenyl)-3-(5-(5,6,7,8-tetrahydro-[1,8]naphthyridin-2-yl)-pentanoylamino)-propionic acid (17-5)

A solution of ester 17-2 (0.47 gm, 1.36 mmol) in EtOH (15 mL) was purged with argon and treated with Pd/C ( 0.047 gm). The heterogeneous mixture was placed under 1 atm of H₂ for 12 h. The mixture was filtered through Celite and concentrated to give amine 17-3 (0.30 g, 100%) as a pale yellow oil. A stirred solution of acid 1-6 (0.09 g, 0.33 mmol), amine 17-3 (0.071, 0.33 mmol), and N-methylmorpholine (0.11 mL, 0.99 mmol) in CH₃CN (5 mL) was treated with BOP-reagent (0.15 g, 0.33 mmol). After stirring for 12 h at room temperature, the mixture was concentrated and the residue redissolved in CH₂Cl₂ (30 mL). The organic solution was washed with satd aq NaHCO₃ (10 mL) and brine (10 mL). The solution was dried over Na₂SO₄, filtered and concentrated to give 0.140 gm of the crude adduct 17-4. Ester 17-4 (0.140 gm, 0.33 mmol) was dissolved in MeOH/THF (2 mL/5 mL) and treated with aq 1N LiOH (1.0 mL). The solution was stirred for 12 h at room temperature. The desired product was purified by preparative HPLC (95:5 to 5:95 H₂O/MeCN gradient) to give the acid 17-5.
¹H NMR (300 MHz, CD₃OD) δ 7.58 (d, J= 7.6 Hz, 1H), 7.33 (m, 1H), 7.13 (m, 3H), 6.59 (d, J=7.6 Hz, 1H), 3.89 (t, J= 7.3 Hz, 1H), 3.64 (m, 2H), 3.56 (m, 2H), 2.80 (t, J=6.1 Hz), 2.65 (m, 2H), 2.19 (m, 2H), 1.96 (m, 2H), 1.58 (m, 4H) ppm.

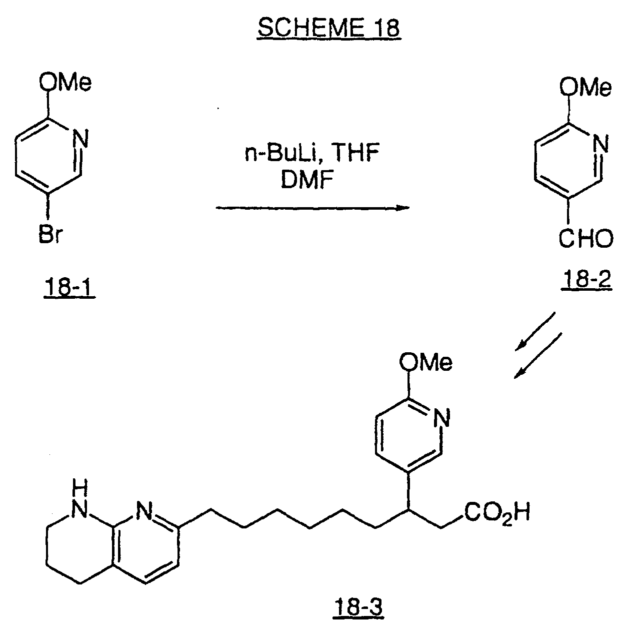

### 6-Methoxy-pyridine-3-carboxaldehyde (18-2)

A solution of n-butyllithium (3.46 mL of a 1.6 M solution in hexanes) in THF (18 mL) was cooled to -78 °C and treated with a solution of 5-bromo-2-methoxypyridine (Johnson, C. R.; Sirisoma, N. S. *Tetrahedron Lett*. **1998**, 39, 2059) 18-1 (1.04 g, 5.53 mmol) in THF (2 mL). The heterogeneous mixture was stirred for 40 min and neat DMF (1.5 mL) was added. The solution was stirred for 90 min at -78°C and quenched with satd aq NH₄Cl solution (2 mL). The cold bath was removed and the mixture warmed to room temperature. The mixture was extracted with EtOAc (2 x 30 mL) and the combined organic solutions washed with brine, dried over MgSO₄, filtered, and concentrated to the desired aldehyde 18-2.
TLC Rf = 0.45 (10% ethyl acetate/hexanes).

### 3-(6-Methoxy-pyridin-3-yl)-9-(5,6,7,8-tetrahydro-[1,8]naphthyridin-2-yl)-nonanoic acid (18-3)

The 6-methoxypyridine carboxaldehyde 18-2 was transformed to acid 18-3 as per Scheme 15.
¹H NMR (300 MHz, CD₃OD) δ 8.31 (m, 1H), 8.19 (s, 1H), 7.59 (m, 1H), 7.45 (m, 1H), 6.60(m, 1H), 4.16 (s, 3H), 3.51 (m, 2H), 3.20 (m, 1H), 2.79 (m, 6H), 1.95 (m, 2H), 1.71 (m, 4H), 1.37 (m, 3H) ppm.

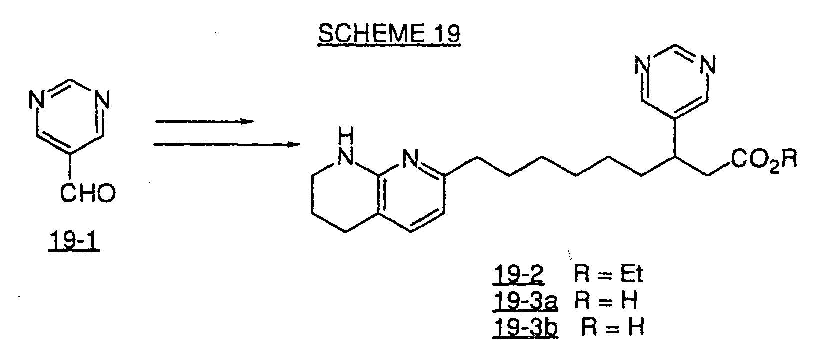

### 3(R or S)-(Pyrimidin-5-yl)-9-(5,6,7,8-tetrahydro-[1,8]naphthyridin-2-yl)-nonanoic acid and 3(S or R)-(pyrimidin-5-yl)-9-(5,6,7,8-tetrahydro-[1,8]naphthyridin-2-yl)-nonanoic acid (19-3a and 19-3b)

The 5-pyrimidine carboxaldehyde 19-1 (Rho, T.; Abuh, Y. F., *Synthetic Comm.* **1994,** *24*, 253) was converted into to acid 19-2 as per Scheme 15. Separation of the enantiomers of racemic ethyl ester 19-2 was accomplished by HPLC (Chiralcel OD; 25x2 mm column; 90/10 to 40/60 hexanes/isopropanol/0.1% diethylamine over 60 minutes at a flow rate of 7.0 mL/min) to give the two enantiomers (R_{T} = 7.79 min and 8.72 min). Hydrolysis of the resulting optically active esters as per Scheme 15 provided acids 19-3a and 19-3b.
¹H NMR (300 MHz, CD₃OD) δ 9.01 (s, 1H), 8.71 (s, 2H), 7.57 (d, J=7.4 Hz, 1H), 6.59 (d, J=7.4 Hz, 1H), 3.49 (m, 2H), 3.12 (m, 1H), 2.72 (m, 6 H), 1.96 (m, 2H) 1.72 (m, 4H) 1.30 (m, 6H) ppm.

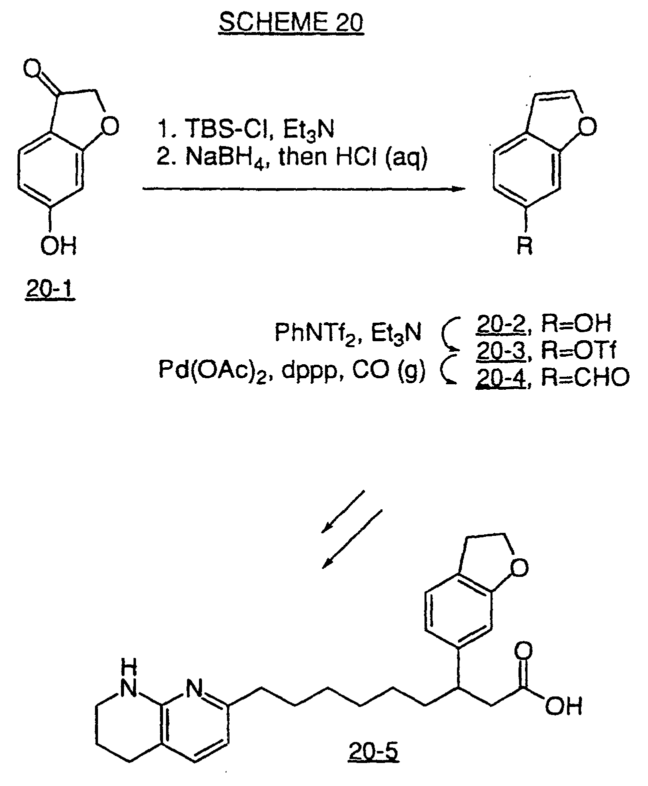

### Benzofuran-6-ol (20-2)

To a solution of 6-hydroxy-[2*H*]-benzofuran-3-one 20-1 (7.84 g, 62.2 mmol) in DMF (100 mL) at room temperature was added triethylamine (8.17 g, 80.9 mmol) and *tert*-butyldimethylsilyl chloride (10.32 g, 68.4 mmol). After stirring for 2 h, the solution was diluted with Et₂O (300 mL) and washed with satd aq NH₄Cl (150 mL) and brine (100 mL). The solution was dried over MgSO₄, filtered and concentrated to give the benzofuranone as a yellow oil which solidifed upon standing and was not further purified. A solution of this ketone (44.2 g, 167 mmol) in 400 mL MeOH was treated with NaBH₄ (9.5 g, 251 mmol, 1.5 equiv) in four equivalent portions at room temperature until complete by TLC (∼1 h). The reaction mixture was quenched by the addition of acetone (10 mL). This mixture was then treated with 3 N aq HCl (200 mL) at room temperature until complete by TLC (∼24 h). The resulting solution was concentrated in vacuo to 150 mL and was extracted with EtOAc (2 x 250 mL). The combined organic extracts were dried (Na₂SO₄), filtered and concentrated in vacuo. The residue was purified by flash chromatography (30% EtOAc/hexanes) affording phenol 20-2 (99%).
TLC Rf = 0.35 (30% ethyl acetate/hexanes).
¹H NMR (300 MHz, CDCl₃) δ 7.53 (d, J=1.5 Hz, 1H); 7.41 (d, J=6.3 Hz, 1H); 7.1 (br s, 1H), 6.81-6.78 (dd, J=1.5, 6.3 Hz, 1H), 6.69 (d, J=1.5 Hz, 1H) ppm.

### Trifluoromethanesulfonic acid benzofuran-6-yl ester (20-3)

A solution of benzofuran-6-01 20-2 (4.00 g, 29.85 mmol) and N-phenyltriflimide (10.66 g, 29.85 mmol) in CH₂Cl₂ (150 mL) cooled to 0°C was treated with triethylamine (5.37 mL, 3.92 g, 38.81 mmol). The reaction was warmed to room temperature over 90 min and diluted with Et₂O (200 mL). The organic solution was washed with satd aq NH₄Cl (100 mL) and brine (100 mL). The solution was dried over MgSO₄, filtered and concentrated. The residue was purified by flash chromatography (5% EtOAc/hexanes) to give the triflate 20-3 as a colorless oil which solidified upon standing.
TLC Rf = 0.39 (10% ethyl acetate/hexanes).
¹H NMR (300 MHz, CDCl₃) δ 7.72 (d, J=1.0 Hz, 1H), 7.63 (d, J=8.4 Hz, 1H), 7.48 (br s, 1H), 7.20 (dd, J=8.4, 1.0 Hz, 1H), 6.82 (br s, 1H) ppm

### Benzofuran-6-carbaldehyde (20-4)

A solution of triflate 20-3 (0.798 g, 3.0 mmol), Pd(OAc)₂ (13.5 mg, 0.060 mmol), and diphenylphosphinopropane (24 mg, 0.060 mmol) in DMF (15 mL) was heated to 70°C with a gentle flow of CO (g) passing through it. Triethylamine (1.66 mL, 12 mmol) was added followed by trioctylsilane (2.70 mL, 6.0 mmol). The solution was maintained at 70°C for 2 h, and cooled to room temperature. The solution was diluted with water (10 mL). The mixture was extracted with Et₂O (2 x 30 mL). The combined organic extracts were washed with brine (10 mL), dried over MgSO₄, filtered and concentrated. The residue was purified by flash chromatography (5% acetone/hexanes) to give the desired aldehyde 20-4. TLC Rf = 0.39 (10% ethyl acetate/hexanes).

### 3-(2,3-Dihydro-benzofuran-6-yl)-9-(5,6,7,8-tetrahydro-[1,8]naphthyridin-2-yl)-nonanoic acid (20-5)

The aldehyde 20-4 was converted into acid 20-5 as per Scheme 15.
¹H NMR (300 MHz, CDCl₃) δ 7.3 (m, 1H); 7.11 (m, 1H); 6.8 (m, 1H), 6.62 (s, 1H), 6.35 (m, 1H), 4.5 (m, 2H), 3.51 (m, 2H), 3.15 (m, 3H), 2.61 (m, 5H), 1.91 (m, 3H), 1,72 (m, 4H), 1.4 (m, 6H) ppm.

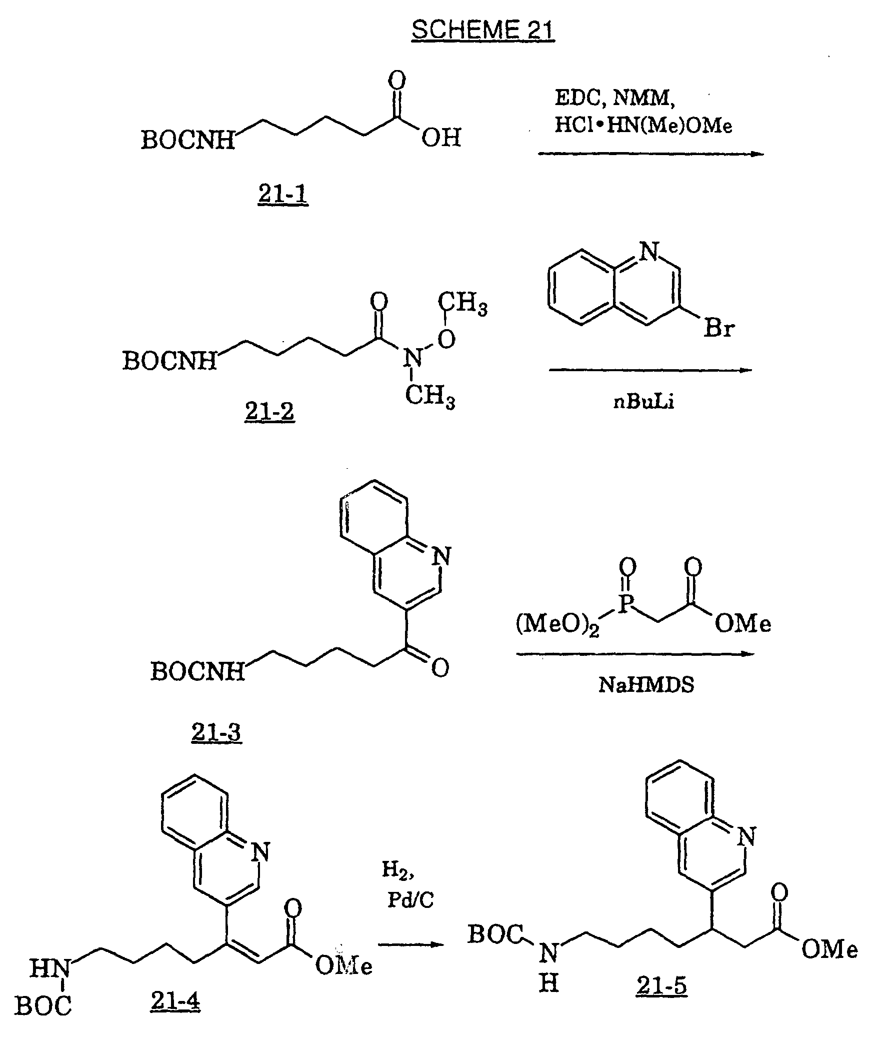

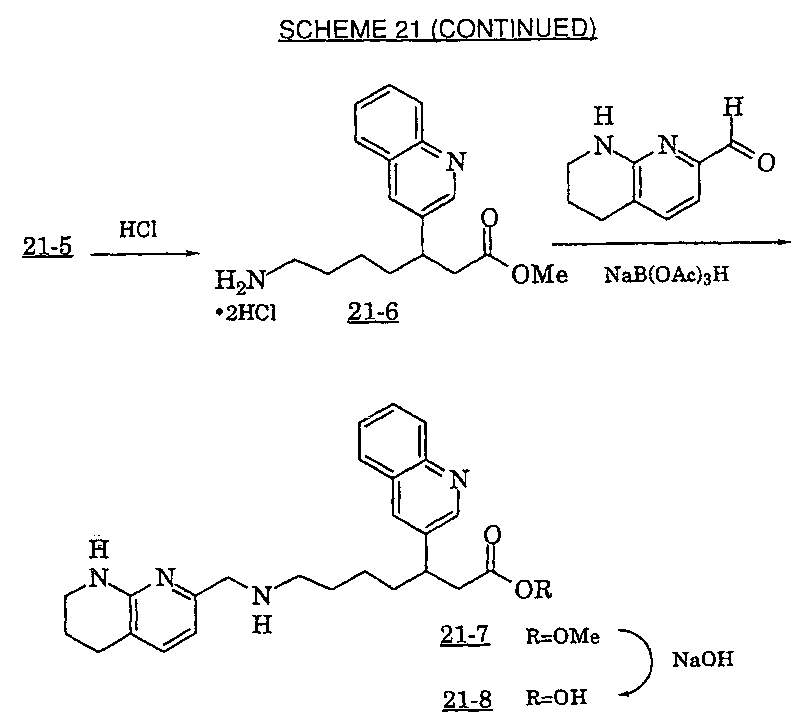

### [4-(Methoxy-methyl-carbamoyl)-butyl]-carbamic acid tert-butyl ester (21-2)

N-Boc-aminovaleric acid 21-1 (92 mmol) was dissolved in CH₃CN (300ml) and then treated with HCl•HN(Me)OMe (10.8 g, 110 mmol), EDC (17.6g, 92 mmol), HOBT (12.4 g, 92 mmol) and NMM (61 mL, 552 mmol). The mixture was stirred for 18 hours and then concentrated. The residue was dissolved in ethyl acetate, washed with H₂O, 10% KHSO₄, sat. NaHCO₃, brine, dried (MgSO₄), and concentrated to give amide 21-2 as a brown oil.
TLC R_{f}= 0.67 (silica, ethyl acetate)
¹H NMR (300 MHz, CDCl₃) δ 4.66 (s, 1H), 3.68 (s, 3H), 3.18 (s, 3H), 3.13 (t, 2H, 6Hz), 2.45 (m, 2H), 1.65 (m, 2H), 1.53 (m, 2H), 1.44 (s, 9H).

### (5-Oxo-5-quinolin-3-yl-pentyl)-carbamic acid tert-butyl ester (21-3)

To a stirred solution of 3-bromoquinoline (25 g, 120 mmol) in diethyl ether -78°C was added nBuLi (2.5M THF, 48 ml, 120 mmol) dropwise over 30 minutes. After 30 minutes, 21-2 (3.9 g, 15 mmol), dissolved in 50 ml ether was added dropwise over 10 minutes. After 30 minutes, the cooling bath was removed. After 1.0 hour, the reaction was quenched with sat. NH₄Cl. The organic portion was separated, washed with brine, dried (MgSO₄) and concentrated. The residue was chromatographed (silica gel, 20-50% ethyl acetate/ hexanes) to give 21-3 as a yellow solid.
TLC R_{f} = 0.33 (silica, 50% ethyl acetate/ hexanes)

### 7-tert-butoxycarbonylamino-3-quinolin-3-yl-heptanoic acid methyl ester (21-5)

To a stirred solution of trimethylphosphonoacetate (6.7 g, 36.5 mmol) and THF at 0°C was added NaHMDS (1.0M THF, 37 ml, 37 mmol) dropwise over 30 minutes. After 30 minutes, 21-3 (3.0 g, 9.13 mmol), dissolved in 100 ml THF, was added. The reaction was heated to reflux. After 1.0 hour, the reaction was diluted with diethyl ether and then washed with sat. NaHCO₃, brine, dried (MgSO₄) and concentrated. The residue was chromatographed (silica gel, 50%ethyl acetate/hexanes) to give 21-4 as a yellow oil. A mixture of 21-4 (3.5 g, 9.13 mmol) and 10% Pd/carbon (1.0 g) in CH₃OH (50 mL) was stirred under a balloon of hydrogen for 6 hours. Following filtration and evaporative removal of the solvent, the residue was chromatographed (silica gel, 30-50% ethyl acetate/ hexanes) to give 21-5 as a yellow oil.
TLC R_{f}= 0.43 (silica, 50% ethyl acetate/ hexanes)
¹H NMR (300 MHz, CDCl₃) δ 8.79 (d, 1H, J=2Hz), 8.08 (d, 1H, J=9Hz), 7.93 (s, 1H), 7.79 (d,1H, J=8Hz), 7.68 (m,1H), 7.54 (m, 1H), 4.47 (s,1H), 3.57 (s, 3H), 3.30 (m, 1H), 3.04 (m,2H), 2.73 (m, 2H), 1.78 (m,2H), 1.45 (m, 2H),1.39 (s, 9H), 1.26 (m, 2H).

### 7-[(5,6,7,8-Tetrahydro-[1,8]naphthyridin-2-ylmethyl)-amino]-3-(quinolin-3-yl)-heptanoic acid methyl ester (21-7)

Ester 21-5 (9.1 mmol) was dissolved in 4M HCl/dioxane (10 ml), stirred for 30 minutes and then concentrated to give the amine 21-6 as a yellow oil. A mixture of 21-6 (900 mg, 2.5 mmol), 3-formyl-5,6,7,8-tetrahydro-[1,8]naphthyridine (405 mg, 2.5 mmol), powdered molecular sieves (2 g), DIPEA (0.35 ml, 2.5 mmol) and DCE (100 mL) was stirred for 30 minutes. The mixture was cooled to 0°C and then Na(OAc)₃BH (730 mg, 3.5 mmol) was added. After 1 hour, the reaction was diluted with EtOAc and then washed with sat NaHCO₃, brine, and dried over MgSO₄. Following evaporative removal of the solvent, the residue was chromatographed (silica gel, 10% [10:10:1 EtOH/ NH₄OH/ H₂O]/ 70:20:10 chloroform/ ethyl acetate/ MeOH] to give 21-7 as a yellow oil.
¹H NMR (300 MHz, CDCl₃) δ 8.79 (d, 1H, J=2Hz), 8.08 (d, 1H, J=9Hz), 7.93 (d, 1H, J=2Hz), 7.78 (d, 1H, J=8Hz), 7.67 (m, 1H), 7.54 (m, 1H), 7.05 (d, 1H, J=7Hz), 6.39 (d, 1H, J=7Hz), 4.83 (s, 1H), 3.58 (s, 2H), 3.57 (s, 3H), 3.34 (m, 3H), 2.73 (m, 4H), 2.53 (t, 2H, J=7Hz), 1.89 (m, 2H),1.78 (m, 2H), 1.52 (m, 2H), 1.24 (m, 2H).

### 7-[(5,6,7,8-Tetrahydro-[1,8]naphthyridin-2-ylmethyl)-amino]-3-(quinolin-3-yl)-heptanoic acid (21-8)

To a solution of 21-7 (0.8255 mmol) in EtOH (5 mL) was added 1N NaOH (1.0 ml, 1.0 mmol). After stirring for 1 hour, the solvents were evaporated and the residue was chromatographed (silica gel, 20:10:1:1 followed by 15:10:1:1 ethyl acetate /EtOH /water /NH₄OH) to give 21-8 as a white solid.
TLC Rf = 0.10 (10:10:1:1 ethyl acetate/EtOH/water/NH₄OH).
¹H NMR (300 MHz, CD₃OD) δ 8.78 (d, 1H, J=2Hz), 8.20 (d, 1H, J=2Hz), 7.97 (d, 1H, J=9Hz), 7.89 (d, 1H, J=8Hz), 7.71 (m, 1H), 7.58 (m, 1H), 7.16 (d, 1H, J=7Hz), 6.43 (d, 1H, J=7Hz), 3.96 (s, 2H), 3.36 (m, 2H), 2.94 (m, 2H), 2.65 (m, 2H), 1.83 (m, 5H),1.69 (t, 2H, J=8Hz), 1.30 (m, 2H).

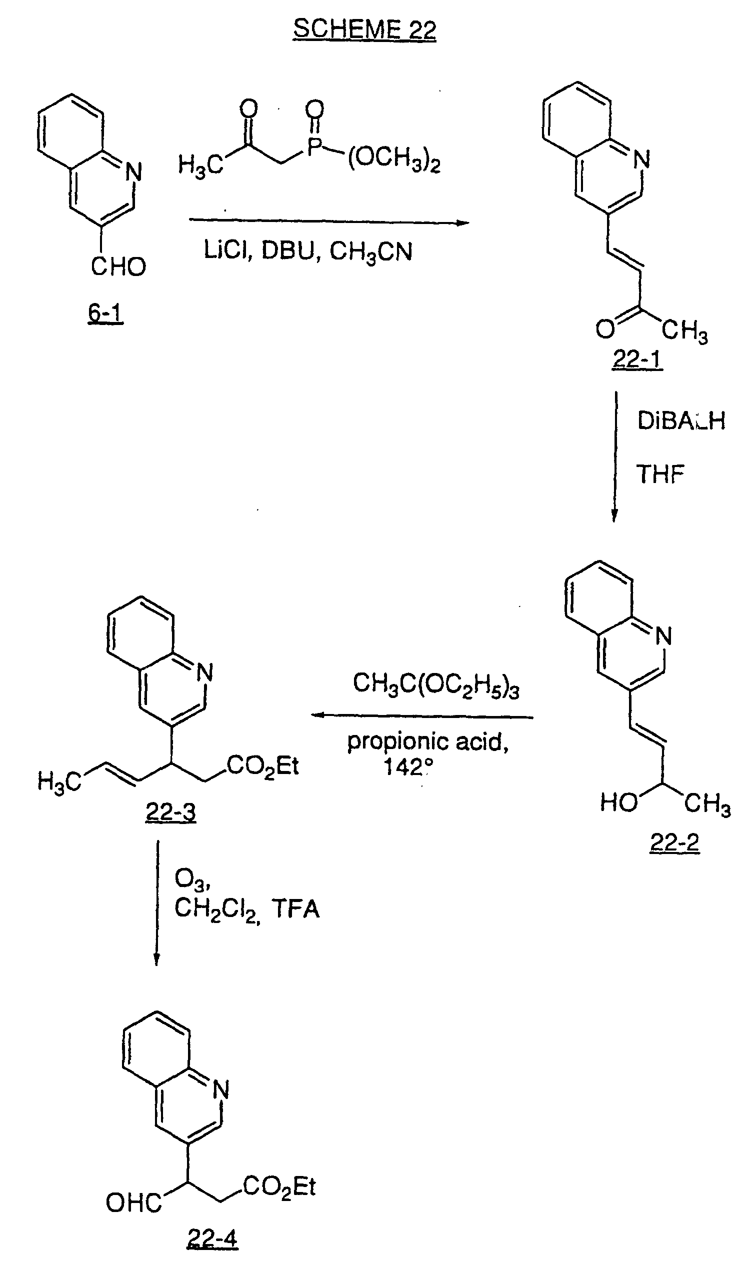

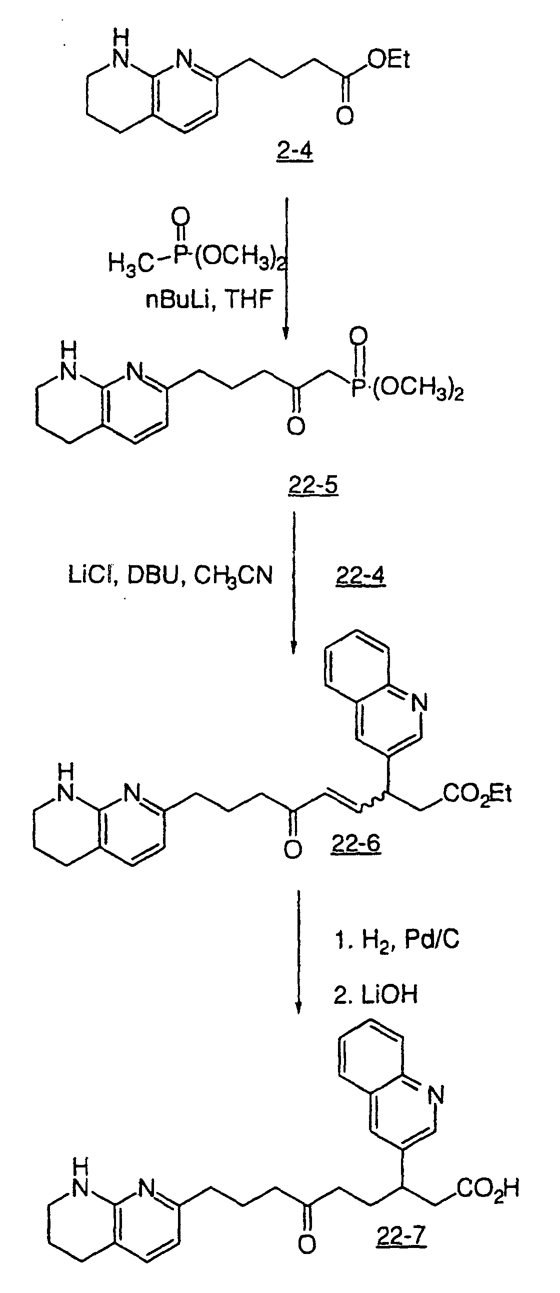

### 4-(Quinolin-3-yl)-but-3-en-2-one (22-1)

Dimethyl (2-oxopropyl)phosphonate (3.1 g,19.1 mmol), anhydrous LiCl (972 mg, 23 mmol), and 3-quinoline carboxaldehyde 6-1 (3.0 g,19.1 mmol) in anhydrous acetonitrile (70 mL), was treated with DBU (2.9 mL, 19.1 mmol) and the resulting suspension stirred at room temperature for 1 h. The solvent was removed at reduced pressure and the resulting residue partitioned between brine and methylene chloride. The organic layer was removed, dried, and concentrated to afford a yellow solid which was recrystallized from ethyl acetate/hexanes to afford 22-1 as an off-white solid.
¹H NMR (300 MHz, CDCl₃) δ 9.05 (d, J= 1.8 Hz, 1H), 8.35 (d, J=1.8 Hz, 1H), 8.09 (d, J= 8.5 Hz, 1H), 7.86 (d, J=7.5 Hz, 1H), 7.75 (t, J= 7.5 Hz, 1H), 7.65 (d, J=16 Hz, 1H), 7.55 (t, J=7.5 Hz, 1H), 6.53 (d, J=16 Hz, 1H), 1.44 (s, 3H). ¹H NMR (300 MHz, CDCl₃) d 9.05 (d, J= 1.8 Hz, 1H), 8.13 (d, J= 8.5 Hz, 1H), 8.09 (d, J=1.8 Hz, 1H), 7.81 (d, J=7.5 Hz, 1H), 7.73 (t, J= 7.5 Hz, 1H), 7.55 (t, J=7.5 Hz, 1H), 6.75 (d, J=16 Hz, 1H), 6.53 (dd, J=16, 5.8 Hz, 1H),4.56 (m, 1H), 1.44 (d, J = 4.6 Hz, 3H).

### 4-(Quinolin-3-yl)-but-3-en-2-ol (22-2)

A solution of 22-1 (1.2 g, 6.1 mmol) in anhydrous THF (50 mL) was cooled to -78°, then treated dropwise with i-Bu₂AlH (12.75 mL, 12.2 mmol). The resulting solution was stirred at -78° for 20 min, then quenched with ethyl acetate (20 mL), warmed to room temperature, treated with 1 M potassium sodium tartrate (25 mL) and stirred for 4 h. The mixture was extracted with ethyl acetate (2 X 150 mL) dried, filtered and evaporated to afford 22-2 as a yellow oil.
R_{f} (silica, EtOAc) = 0.30.
¹H NMR (300 MHz, CDCl₃) δ 9.05 (d, J= 1.8 Hz, 1H), 8.13 (d, J= 8.5 Hz, 1H), 8.09 (d, J=1.8 Hz, 1H), 7.81 (d, J=7.5 Hz, 1H), 7.73 (t, J= 7.5 Hz, 1H), 7.55 (t, J=7.5 Hz, 1H), 6.75 (d, J=16 Hz, 1H), 6.53 (dd, J=16, 5.8 Hz, 1H),4.56 (m, 1H), 1.44 (d, J = 4.6 Hz, 3H).

### 3-(Quinolin-3-yl)-hex-4-enoic acid ethyl ester (22-3)

A solution of the allylic alcohol 22-2 (1.2 g, 6.1 mmol) in triethyl orthoacetate (50 mL) was treated with propionic acid (0.022 mL, 0.31 mmol) and refluxed for 2 h. The cooled mixture was treated with a solution of 1:1 sat. brine/1N HCl (150 mL), then extracted with CH₂Cl₂ (3 X 125 mL). The pooled organic extracts were washed with sat. NaHCO₃, dried, filtered and evaporated. Chromatography on silica gel (EtOAc) afforded 22-3 as a yellow glass.
R_{f} (silica,EtOAc) = 0.65.
¹H NMR (300 MHz, CDCl₃) δ 8.92 (d, J= 1.8 Hz, 1H), 8.15 (d, J=8.5 Hz, 1H), 7.96 (d, J=1.8 Hz, 1H), 7.82 (d, J=7.5 Hz, 1H), 7.71 (t, J=7.5 Hz, 1H), 7.54 (t, J=7.5 Hz, 1H), 5.6 (m, 2H), 4.21 (m, 1H), 4.05 (t, J=7.4 Hz, 2H), 2.79 (m, 2 H),1.91 (d, J=6 Hz, 2H), 1.08 (t, J=7.4 Hz, 3H).

### 4-Oxo-3-(quinolin-3-yl)-butyric acid ethyl ester (22-4)

A solution of 22-3 (1.0 g, 3.7 mmol), TFA (0.06 mL, 3.9 mmol), and sudan red (0.5 mg) in anhydrous CH₂Cl₂ (50 mL) was cooled to -78° and treated with O₃ until the sudan red color disappeared (5 min). Solid Ph₃P (1.4 g, 5.6 mmol) was added and the solution warmed to room temperature. After 30 min., the solution was washed with sat. NaHCO₃ , dried, filtered, and evaporated. Chromatography on silica gel (10% acetone/EtOAc) afforded 22-4 as colorless glass.
R_{f}(silica,EtOAc) = 0.25.
¹H NMR (300 MHz, CDCl₃) δ 9.88( s, 1H), 8.82 (d, J= 1.8 Hz, 1H), 8.13 (d, J= 8.5 Hz, 1H), 7.96 (d, J=1.8 Hz, 1H), 7.82 (d, J=7.5 Hz, 1H), 7.76 (t, J= 7.5 Hz, 1H), 7.58 (t, J = 7.5 Hz, 1H), 4.4 (m, 1H), 4.05 (t, J = 7.4 Hz, 1H), 4.13 (m, 2H), 3.30 (dd, J= 7.6, 16 Hz, 1H), 2.87 (dd, J = 7.2, 16 Hz, 1H), 1.20 (m, 3H).

### 2-Oxo-5-(5,6,7,8-tetrahydro-[1,8]-naphthyridin-2-yl)-pentyl-phosphonic acid dimethyl ester (22-5)

A solution of dimethyl methylphosphonate (10.0 g, 80.5 mmol) in anhydrous THF (125 mL) was cooled to -78° and treated dropwise with 2.5 M n-BuLi (32.2 mL). After stirring at -78° for 45 min, a solution of ester 22-4 (6.6 g, 26.6 mmol) in THF (35 mL) was added dropwise and the resulting solution stirred for 30 min at -78°, quenched with sat. NH₄Cl (100 mL), then extracted with ethyl acetate (3 X 150 mL). The combined organic extracts were dried (MgSO₄), filtered, and concentrated to afford a yellow oil. Chromatography on silica gel (5% MeOH/CH₂Cl₂) afforded 22-5 as a yellow oil.
R_{f} (silica, 5% MeOH/CH₂Cl₂) = 0.20.
¹H NMR (300 MHz, CDCl₃) δ 7.05 (d, J=7.3 Hz, 1H), 6.34 (d, J=7.32 Hz, 1H), 4.80 (br, s, 1H), 3.81 (s, 3H), 3.75 (s, 3H), 3.4 (m, 2H), 3.08 (d, J=22.7 Hz), 2.72 (m, 6 H), 2.56 (t, 2 H), 1.91 (m, 2H).

### 6-Oxo-(3-quinolin-3-yl)-9-(5,6,7,8-tetrahydro-[1,8]naphthyridin-2-yl)-nonanoic acid (22-7)

Ketophosphonate 22-5 (1.0 g, 3.1 mmol), anhydrous LiCl (170 mg, 4.0 mmol), and 22-4 (0.797 g, 3.1 mmol) in anhydrous acetonitrile (60 mL), was treated with DBU (0.52 mL, 3.3 mmol) and the resulting suspension stirred at room temperature for 1 h. The solvent was removed at reduced pressure and the resulting residue partitioned between brine and methylene chloride. The organic layer was removed, dried, and concentrated to afford 22-6 as mixture of E and Z olefins. The crude mixture was dissolved in EtOH (50 mL), treated with 10% Pd/C (200 mg) and stirred under a hydrogen filled balloon for 4 h, then filtered. The filrate was treated with LiOH (2.0 eq) and water (20 mL) and stirred at room temperature for 12 h, then neutralized with 1N HCl and evaporated Chromatography on silica gel (10% MeOH/methylene chloride) afforded 22-7 as a white solid.
R_{f} (silica, 10% MeOH/CH₂Cl₂) = 0.20.
¹H NMR (300 MHz, CDCl₃) δ 10.9 ( br,s, 1H), 8.92 (d, J= 1.8 Hz, 1H), 8.21(d, J= 7.5 Hz, 1H), 8.05 (d, J=1.8 Hz, 1H), 7.87 (d, J=7.5 Hz, 1H), 7.81 (t, J= 7.5 Hz, 1H), 7.58 (t, J=7.5 Hz, 1H), 7.25 (d, J = 7.3 Hz, 1H), 6.34 (d, J=7.3 Hz, 1H), 3.81 (m, 1H), 3.44 (m, 2H), 3.0-2.2 (m, 12 H), 1.92 (m, 2H), 1.68 (m, 1H) 1.42 (m, 1H).

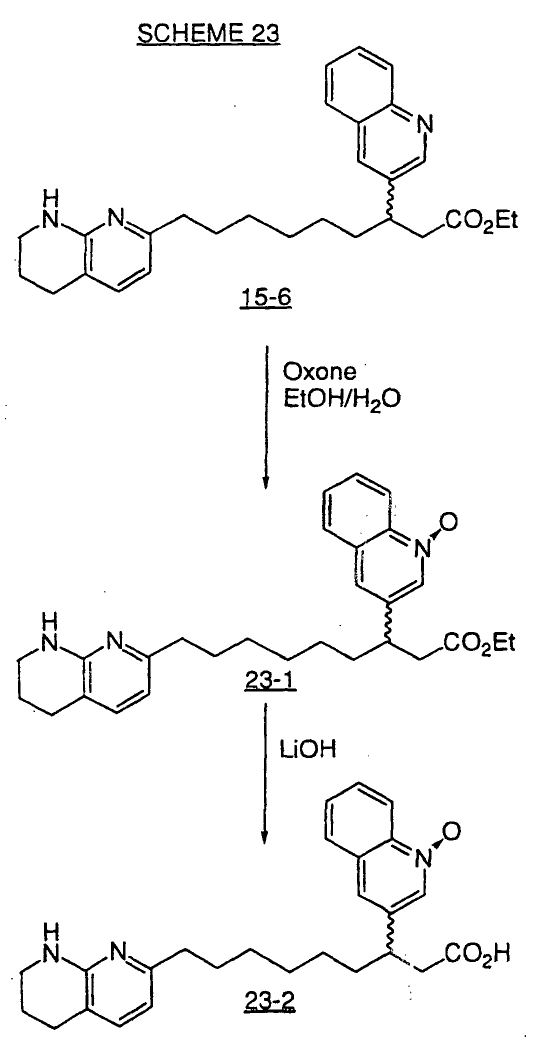

### 3-(N-Oxo-quinolin-3-yl)-9-(5,6,7,8-tetrahydro-[1,8]naphthyridin-2-yl)nonanoic acid (23-2)

A solution of 15-6 (100 mg, 0.22 mmol) in 50 aqueous ethanol (30 mg) was treated with Oxone® (414 mg, 0.66 mmol) and the resulting solution stirred at room temperature for 18 h, extracted with CH₂Cl₂ ( 3 x 60 mL), dried, filtered and evaporated to afford 23-1 as a yellow film. Crude 23-1 was hydrolyzed with LiOH and chromatographed on silica gel (50%A:50EtOAc) (A= 20:1:1 EtOH: NH₄OH:H₂O) giving 23-2 as a colorless glass.
R_{f} (silica, 50% A: EtOAc) = 0.30
¹H NMR (300 MHz, CD₃OD) δ 8.66 (d, J= 1.8 Hz, 1H), 8.63 (d, J= 8.5 Hz, 1H), 8.09 (d, J=1.8 Hz, 1H), 8.03 (d, J=7.5 Hz, 1H), 7.82 (t, J= 7.5 Hz, 1H), 7.74 (t, J=7.5 Hz, 1H), 7.30 (d, J=7.3 Hz, 1H), 6.41 (d, J=7.32 Hz, 1H), 5.6 (m, 2H), 3.56 (m, 1H), 3.40 (m, 2H), 2.75 (m, 2 H), 2.65 (m, 2H),2.58 (m, 2H), 2.01 (m, 2H), 1.91 (m, 2H), 1.65 (m, 2H), 1.45(m, 2 H).

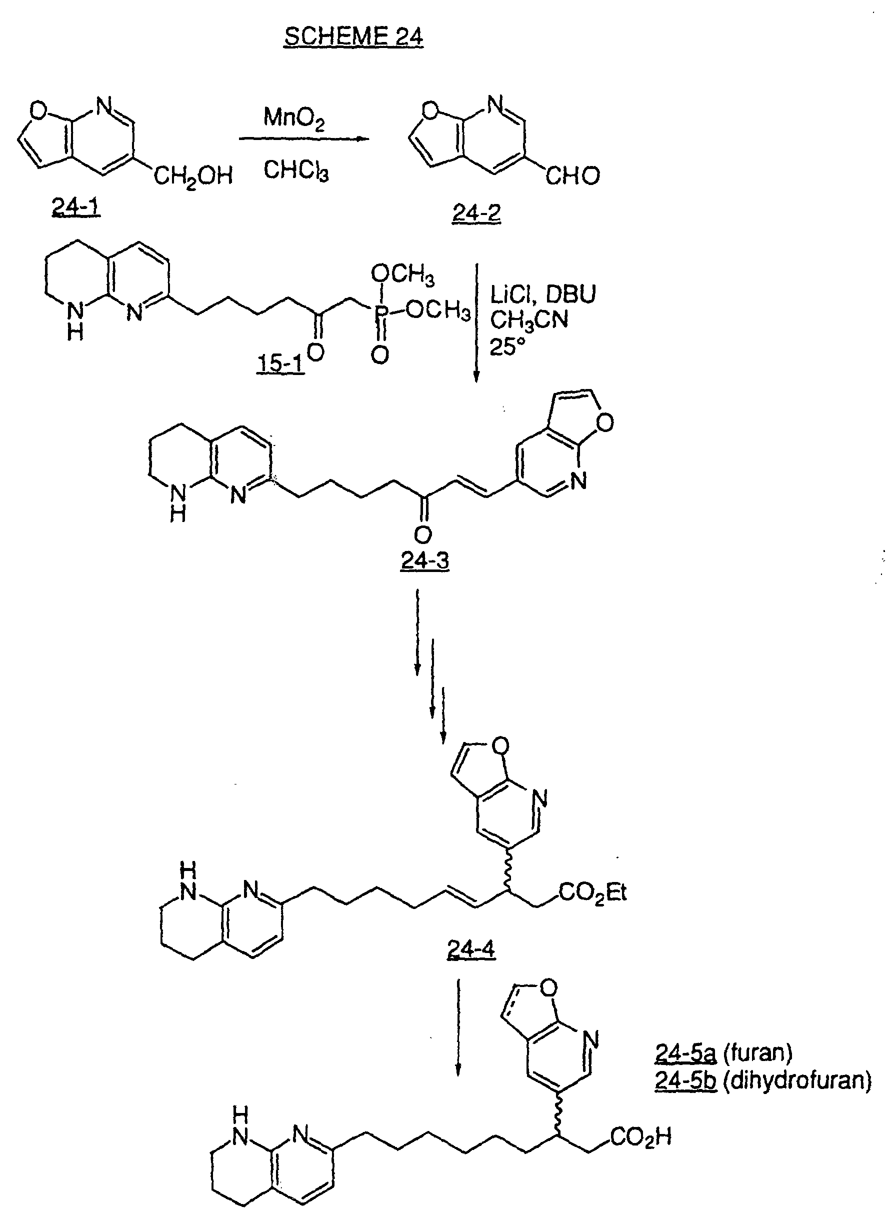

### Furo-[2,3-b]pyridine-5-carboxaldehyde (24-2)

A solution of alcohol 24-1 (Bhupathy, M.; et al. J. Heterocycl. Chem. (1995), *32*, 1283-1287) was treated with excess MnO₂ (10 eq) and the mixture stirred at room temperature for 16 h, then filtered through Celite and evaporated to afford 24-2 as a white solid.
TLC Rf= 0.40 (25% EtOAc/ Hexane)
¹H NMR (300 MHz, CDCl₃) δ 10.22 (s, 1H), 9.05 (d, J= 1.8 Hz, 1H), 8.27 (d, J=1.7 Hz, 1H) 8.08 (d, J=1.8 Hz, 1H), 7.10 (d, J=1.7 Hz, 1H).

### 3-Furo-[2,3-b]pyridin-5-yl-7-(5,6,7,8-tetrahydro-[1,8]-naphthyridin-2-yl)-(E)-hept-1-en-3-one (24-3)

Ketophosphonate 15-1 (0.69 g, 2.0 mmol), anhydrous LiCl (86 mg, 2.0 mmol), and 24-2 (0.25 g, 1.7 mmol) in anhydrous acetonitrile (25 mL), was treated with DBU (0.25 mL, 1.8 mmol) and the resulting suspension stirred at room temperature for 1 h. The solvent was removed at reduced pressure and the resulting residue partitioned between brine and methylene chloride. The organic layer was removed, dried, and concentrated to afford a yellow solid which was chromatographed on silica (5% isopropanol/chloroform) to give 24-3 as an off-white solid.
R_{f} (silica, 5% isopropanol/CH₂Cl₂) = 0.45.
¹H NMR (300 MHz, CDCl₃) δ 8.50 (d, J=2.1 Hz, 1H), 8.13 (d, J=2.1 Hz, 1H), 7.75(d, J=2.4 Hz, 1H),7.60 (d, J= 16 Hz, 1H), 7.06 (d, J=7.3 Hz, 1H), 6.83 (d, J= 2.4 Hz, 1H), 6.77 (d, J=16 Hz, 1H), 6.36 (d, J=7.3 Hz, 1H), 4.85 (br, s, 1H), 3.4 (m, 2H), 2.7-2.5 (m, 6 H), 1.91 (m, 2H), 1.68 (m, 4H).

### 3-(Furo[2,3-b]pyridin-5-yl)-9-(5,6,7,8-tetrahydro-[1,8]naphthyridin-2-yl)-(E)-non-4-enoic acid ethyl ester (24-4)

24-3 was converted to 24-4 via the methods taught in the conversion of 15-4 to 15-5.
R_{f} (silica, 5% isopropanol/CH₂Cl₂) = 0.40.
¹H NMR (300 MHz, CDCl₃) δ 8.18 (d, J=2.1 Hz, 1H), 7.91 (d, J=2.1 Hz, 1H), 7.72 (d, J=2.4 Hz, 1H), 7.05 (d, J=7.3 Hz, 1H), 6.53 (d, J= 2.4 Hz, 1H), 6.36 (d, J=7.3 Hz, 1H), 5.6 (m, 2H), 4.85 (br, s, 1H), 4.05 (q, J =7.5Hz, 2H), 4.05 (m, 1H), 3.40 (m, 2H), 2.75 (m, 2 H), 2.65 (m, 2H),2.58 (m, 2H), 2.01 (m, 2H), 1.91 (m, 2H), 1.65 (m, 2H), 1.45(m, 2 H), 1.08 (t, J=7.5 Hz, 3H).

### 3-(Furo[2,3b]pyridin-5-yl)-9-(5,6,7,8-tetrahydro-[1,8]naphthyridin-2-yl)-nonanoic acid (24-5a)

A solution of 24-4 (108 mg, 0.25 mmol) in ethanol was treated with 10% Pd/C (30 mg) and stirred under a H₂ filled balloon for 6 h, then filtered through Celite and evaporated. Hydrolysis of the crude product with LiOH and chromatography on silica ((50%A:50EtOAc)(A=20:1:1EtOH: NH₄OH:H₂O) afforded 24-5a and 24-5b as white solids.
R_{f} (silica, 50% A: EtOAc) = 0.45
¹H NMR (300 MHz, CDCl₃) δ 10.7 (br, s, 1H), 8.25 (d, J= 1.8 Hz, 1H), 7.81 (d, J= 1.8 Hz, 1H),7.68 (d, J=1.8 Hz, 1H), 7.21 (d, J=7.3 Hz, 1H), 6.75 (d, J=1.7 Hz, 1H), 6.25 (d, J=7.3 Hz, 1H), 3.56 (m, 1H), 3.40 (m, 2H), 2.75 (m, 2 H), 2.65 (m, 2H),2.58 (m, 2H), 2.01 (m, 2H), 1.91 (m, 2H), 1.65 (m, 2H), 1.45(m, 2 H).

### 3-(2,3-Dihydro-furo[2,3-b]pyridin-5-yl)-9-(5,6,7,8-tetrahydro-[1,8] naphthyridin-2-yl)-nonanoic acid (24-5b)

R_{f} (silica, 50% A: EtOAc) = 0.45
¹H NMR (300 MHz, CDCl₃) δ 10.8 (br, s, 1H), 7.85 (d, J=1.8 Hz, 1H),7.40 (d, J=1.8 Hz, 1H), 7.21 (d, J=7.3 Hz, 1H), 6.25 (d, J=7.3 Hz, 1H), 4.60 (t, J= 7.4 Hz, 2 H), 3.7 (m, 1H), 3.40 (m, 2H),3.20 (t, J=7,4 Hz, 2H), 2.75 (m, 2 H), 2.65 (m, 2H),2.58 (m, 2H), 2.01 (m, 2H), 1.91 (m, 2H), 1.65 (m, 2H), 1.45(m, 2 H).

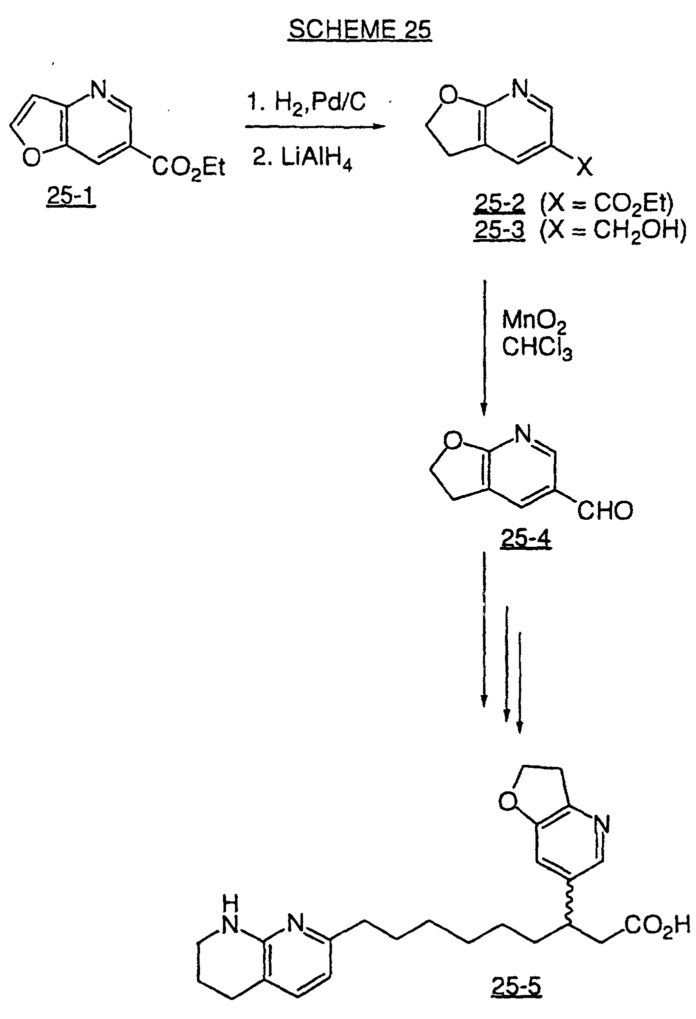

### 2,3-Dihydro-furo[3,2-b]pyridine-5-ethylcarboxylate (25-2)

A solution of ester 25-1 (Hoffman, Jacob M., Jr. US patent No. 4,808,595) in ethanol was treated with 10% Pd/C (30 wt%) and stirred under a hydrogen atmosphere for 22 h, then filtered and evaporated to afford 25-2 as a tan solid.
TLC Rf = 0.40 (25% EtOAc/ Hexane).
¹H NMR (300 MHz, CDCl₃) δ 8.45 (d, J= 1.8 Hz, 1H), 7.23 (d, J=1.8 Hz, 1H). 4.72 (t, J=7.5 HZ, 2H), 4.01 (q, J=7.4 Hz, 2H), 3.42 (t, J=7.5 Hz, 2H), 1.02 (t, J= 7.4 Hz,3H).

### 2,3-Dihydro-furo[3,2b]pyridine-5-methanol (25-3)

A solution of ester 25-2 (1.93 g, 10 mmol) in anhydrous THF (150 mL) was cooled to -78°, then treated dropwise with LAH (10.75 mL, 10.75 mmol). The resulting solution was stirred at -78° for 20 min, then warmed to 25° and stirred for 4 h, then quenched with ethyl acetate (20 mL), treated with 1 M potassium sodium tartrate (25 mL) and stirred for 4 h. The mixture was extracted with ethyl acetate (2 X 150 mL), dried, filtered, and evaporated to afford 25-3 as a white solid which was carried on without further purification.
TLC Rf = 0.6 (EtOAc).
¹H NMR (300 MHz, CDCl₃) δ 8.10 (d, J=1.8 Hz, 1H), 7.13 (d, J=1.8 Hz, 1H). 5,10 (s, 2H), 4.65 (t, J=7.5 HZ, 2H), 3.36 (t, J=7.5 Hz, 2H).

### 2,3-Dihydro-furo[3,2b]pyridine-5-carboxaldehyde (25-4).

A solution of alcohol 25-3 was treated with excess MnO₂ (10 eq) and the mixture stirred at room temperature for 16 h and then filtered through Celite and evaporated to afford 25-4 as a white solid.
TLC Rf = 0.35 (25% EtOAc/ Hexane)
¹H NMR (300 MHz, CDCl₃) δ 10.08 (s, 1H),8.43 (d, J=1.8 Hz, 1H), 7.45 (d, J=1.8 Hz, 1H). 4.72 (t, J=7.5 Hz, 2H), 3.42 (t, J=7.5 Hz, 2H).

### 3-(2,3-Dihydro-furo[3,2-b]pyridin-5-yl)-9-(5,6,7,8-tetrahydro-[1,8] naphthyridin-2-yl)-nonanoic acid (25-5)

Prepared from aldehyde 25-4 using the method described for the preparation of 15-7.
¹H NMR (300 MHz, CD₃OD) δ 7.80 (d, J=1.5 Hz, 1H), 7.41 (d, J=7.3 Hz, 1H), 7.02 (d, J=1.5 Hz, 1H), 6.45 (d, J=7.3 Hz, 1H), 4.72 (t, J=7.5 Hz, 2H), 3.56 (m, 1H), 3.46 (m, 2H), 3.42 (t, J=7.5 Hz, 2H), 2.75 (m, 2H), 2.62 (m, 2H), 2.50 (m, 2H), 1.95 (m, 2H), 1.91 (m, 2H), 1.65 (m, 2H), 1.45 (m, 2 H).

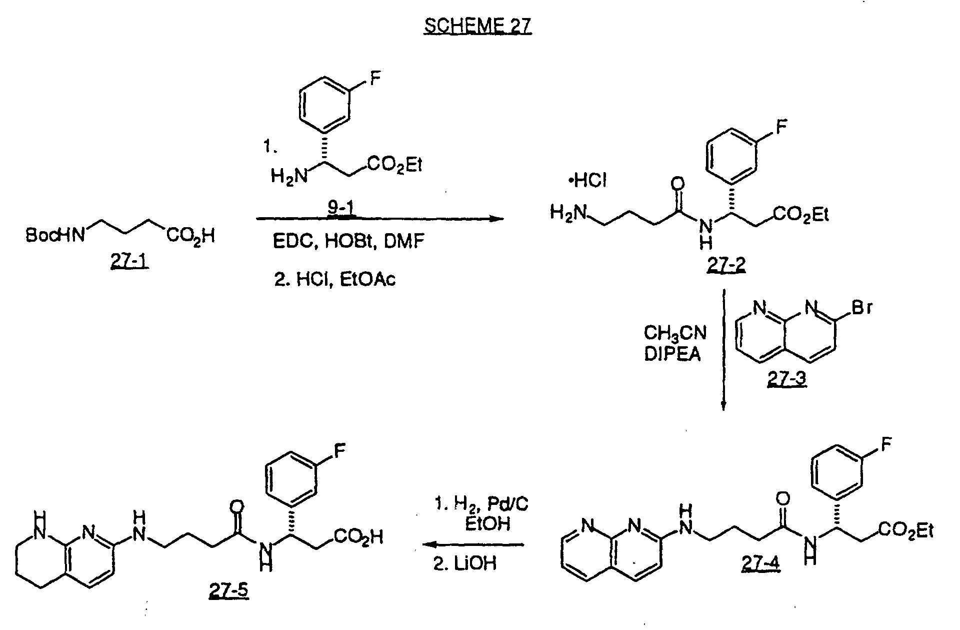

### 3-(4-Aminbutyrylamino)-3-(S)-(3-fluorophenyl)-propionic acid ethyl ester hydrochloride (27-2)

A mixture of 27-1 (245 mg, 1.2mmol), 9-1 (300 mg, 1.21 mmol), EDC (300 mg, 1.57 mmol), NMM (490 mg, 4.84 mmol), and HOBT (213 mg,157 mmol) in DMF (7 mL) was stirred under argon for 16 h, then diluted with EtOAc (50 mL ) and washed successively with sat. NaHCO₃, H₂O, 10% KHSO₄, and brine (20 mL), dried and concentrated to give the Boc-protected amino ester as an oil.
TLC Rf = 0.7 ( 90%EtOAc/ Hexane).
¹H NMR (300 MHz, CDCl₃) δ 7.3 (t, J=7.8 Hz, 1H),7.1 (d, J=7.8 Hz, 1H). 7.07 (d, J=7.8 Hz, 1H). 6.92 (t, J=7.8 Hz, 1H), 5.40 (m, 1H), 4.80 (br, t, 1H), 4.18 (q, J=7.3 Hz, 2H), 3.20 (m, 2H), 2.82 (t, J=7.4 Hz, 2H), 2.35 (t, J=7.4 Hz, 2H),1.7 (4H), 1.25 (t, J=7.3 Hz, 3H).
The Boc protected amino ester obtained above (440 mg, 1.1 mmol) was dissolved in 2.3N ethanolic HCl (10 mL) and stirred at ambient temperature for 3 h then concentrated to afford 27-2 as its HCl salt.
¹H NMR (300 MHz, CD₃OD) δ 7.3 (t, J=7.8 Hz, 1H),7.1 (d, J=7.8 Hz, 1H). 7.07 (d, J=7.8 Hz, 1H). 6.92 (t, J=7.8 Hz, 1H), 5.40 (m, 1H), 4.18 (q, J=7.3 Hz, 2H), 3.20 (m, 2H), 2.82 (t, J=7.4 Hz, 2H), 2.35 (t, J=7.4 Hz, 2H),1.7 (4H), 1.25 (t, J=7.3 Hz, 3H).

### 3(S)-(3-Fluorophenyl)-3-[4-([1,8]naphthyridin-2-ylamino)butyrylamino] propionic acid ethyl ester (27-4)

A mixture of amine hydrochloride 27-2 (350 mg, 1.1 mmol), bromide 27-3 (230 mg, 1.1 mmol) (for preparation see: Roszkiewicz, W.; Wozniak, M.; Synthesis 1976, 691-2), and DIPEA (355 mg, 2.75 mmol) in acetonitrile (5.5 mmol) was heated at reflux for 20 h. The solution was concentrated and the brown residue partitioned between EtOAc and sat. NaHCO₃. The organic layer was washed with brine, dried, filtered, concentrated and the oil chromatographed on silica (5% EtOH/ EtOAc) to afford 27-4 as a colorless glass.
TLC Rf = 0.45 (5% EtOH/ EtOAc).
¹H NMR (300 MHz, CDCl₃) δ 8.80 (d, J=1.8 HZ, 1H), 8.50 (br,d, J-7 Hz, 1H), 7.85 (d,d, J=1.8, 7.0 Hz, 1H), 7.75 (d, J=7.5 Hz, 1H). 7.2-6.8 (5H), 6.62 (d, J= 7.5 Hz, 1H), 5.61 (m, 1H), 5.31 (br, t, 1H), 4.18 (q, J=7.3 Hz, 2H), 3.60 (m, 2H), 3:2 (dd, J=6.0, 10.5 Hz, 1H) 3.02 (dd, J=5.6,10.5 Hz, 1H) 2.22 (t, J=7.4 Hz, 2H),1.92 (m, 2H), 1.25 (t, J=7.3 Hz, 3H).

### 3(S)-(3-Fluorophenyl)-3-[4-(5,6,7,8-tetrahydro-[1,8]naphthyridin-2-ylamino)-butyrylamino]-propionic acid bis trifluoroacetate (27-5)

A solution of 27-4 (90 mg, 0.21 mmol) in EtOH (3 ml) was treated with 10% Pd/C (60 mg) and the mixture stirred under a H₂ filled balloon for 24 h. The catalyst was removed by filtration and the filtrate concentrated to afford crude reduced ester. This material was hydrolyzed with LiOH , neutralized with 1N HCl, concentrated and the residue purified by reverse phase chromatography to afford 27-5 as its bis-TFA salt.
High resolution Ms Calc'd.=401.1977, Observed = 401.1983 .

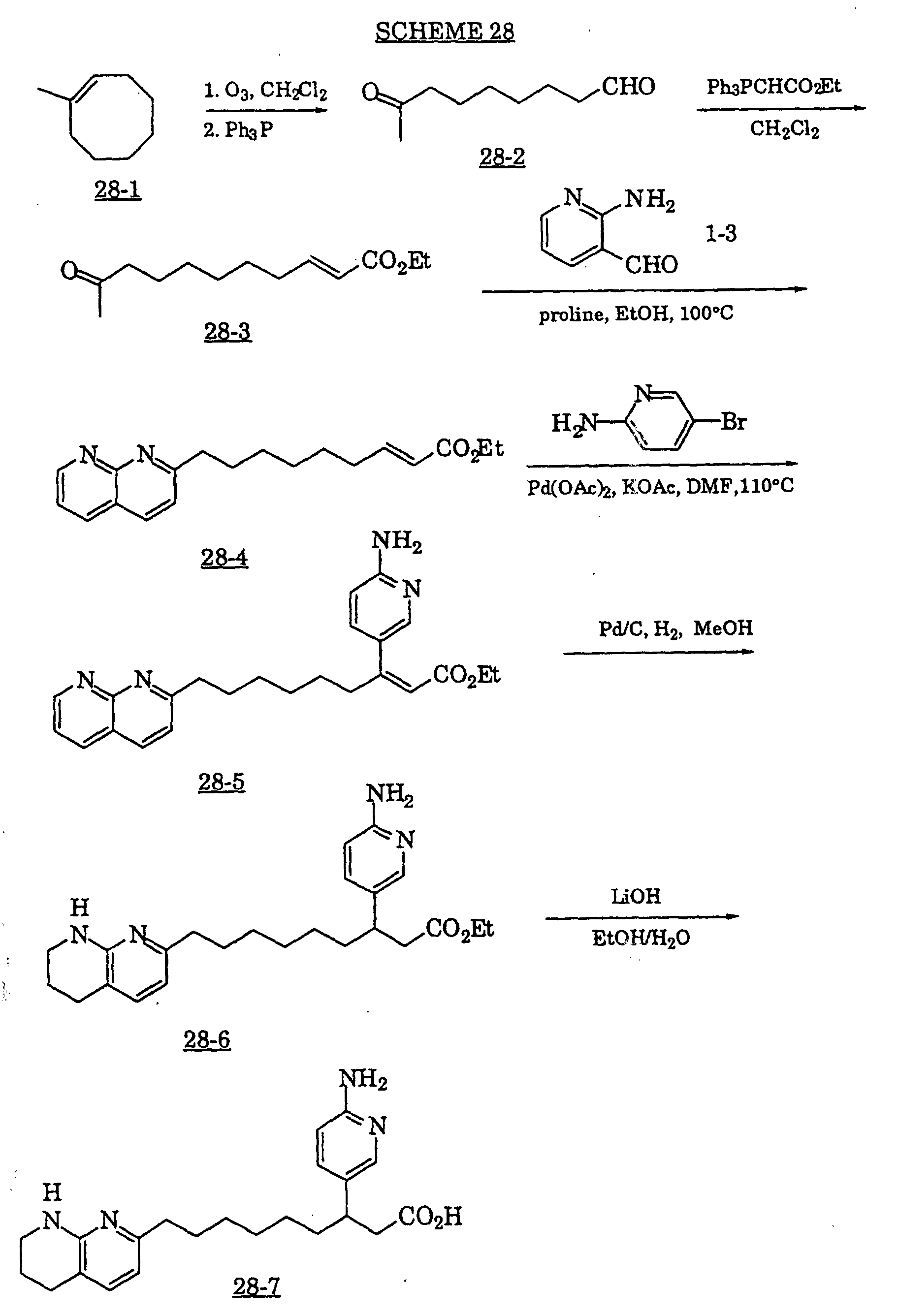

### 8-Oxo-noixanal (28-2)

To a cooled (-78°C) solution of 1-methyl-cyclooctene 28-1 (5.2 g, 41.9 mmol) in 200 mL CH₂Cl₂ was introduced ozone for 30 min. The mixture was stirred for 1 hr and purged with argon. It was then treated with Ph₃P. The reaction mixture was concentrated and purified by silica gel flash chromatography (EtOAc/hexanxes 1:10) to afford the desired product 28-2 as an oil.
¹H NMR (400 MHz, CDCl₃) δ 9.72 (t, J=2.0 Hz, 1H), 2.38 (m, 4H), 2.09 (s, 3H), 1.61-1.52 (m, 4H), 1.28 (m, 4H).

### 10-Oxo-undec-2-enoic acid ethyl ester (28-3)

To a cooled (-40°C) solution of 28-2 (10.8 g, 69.2 mmol) in 150 mL CH₂Cl₂ was added (carbethoxymethylene)triphenylphosphorane (24.1 g, 69.2 mmol) in 100 mL CH₂Cl₂ gradually over 15 min. The reaction mixture was stirred for 12 hr while it was warmed up to room temperature. After solvent removal, the residue was purified using silica gel flash chromatography (EtOAc/hexanes: 1:8 to 1:6) to afford the desired product 28-3 as an oil.
¹H NMR (400 MHz, CDCl₃) δ 6.95 (m, 1H), 5.80 (d, J=15.6 Hz, 1H), 4.18 (d, J=7.2 Hz, 2H), 2.42 (t, J=7.2, 2H), 2.20 (m, 2H), 2.13 (s, 3H), 1.57 (m, 2H), 1.46 (m, 2H), 1.29 (m, 7H).

### 9-[1,8]Naphthyridin-2-yl-non-2-enoic acid ethyl ester (28-4)

A mixture of 28-3 (4.1 g, 18.3 mmol), proline (2.7 g, 23.8 mmol), and 1-3 (2.9 g, 23.8 mmol ) in 50 mL EtOH was heated at 110 °C for 24 hr. The reaction mixture was concentrated and purified by silica gel chromatography (EtOAc, 100%) to afford the desired product 28-4 as a solid.
¹H NMR (400 MHz, CDCl₃) δ 9.08 (dd, J=4.4, 2.0 Hz, 1H), 8.15 (dd, J=8.0, 2.0 Hz, 1H), 8.09 (d, J=8.4 Hz, 1 H), 7.44 (dd, J=8.0, 4.4, 1H), 7.38 (d, J=8.4 Hz, 1H), 6.95 (dt, J=15.6, 7.2 Hz, 1H), 5.80 (dt, J=15.6, 1.6Hz, 1H), 4.15 (q, J=7.2 Hz, 2H), 3.05 (t, J=7.6 Hz, 2H), 2.18 (m, 2H), 1.90 (m, 2H), 1.43 (m, 6H), 1.28 (t, J=7.2 Hz, 3H).
3-(6-Amino-pyridin-3-yl)-9-[1,8]naphthyridin-2-yl-non-2-enoic acid ethyl ester (28-5)

A mixture of 28-4 (0.3 g, 1.0 mmol), 2-amino-5-bromopyridine (0.3 g, 1.9 mmol), KOAc (0.3 g, 2.4 mmol) and Pd(OAc)₂ (0.02 g, 0.1 mmol) in 6 mL DMF was purged with argon for 5 min and then heated at 90°C for 7 hr and 115°C for 48 hr. It was cooled, treated with 50 mL water and extracted with EtOAc (x3). The combined organic layers were washed with brine and dried over Na₂SO₄. After solvent removal, the residue was purified using silica gel flash chromatography (100% EtOAc to EtOAc/MeOH 10:1) to afford the desired product 28-5 as an oil.
¹H NMR (400 MHz, CDCl₃) δ 9.07 (dd, J=4.4, 2.0 Hz, 1H), 8.18 (d, J=2.0 Hz, 1H), 8.14 (dd, J=8.0, 2.0 Hz, 1H), 8.07 (d, J=8.4 Hz, 1 H), 7.53 (dd, J=8.8, 2.0 Hz, 1H), 7.44 (dd, J=8.0, 4.4, 1H), 7.38 (d, J=8.4 Hz, 1H), 6.50 (d, J=8.8 Hz, 1H), 5.96 (s, 1H), 4.83 (bs, 2H), 4.18 (q, J=7.2 Hz, 2H), 3.02 (m, 4H), 1.85 (m, 2H), 1.43 (m, 6H), 1.29 (t, J=7.2 Hz, 3H).

### 3-(6-Amino-pyridin-3-yl)-9-(5,6,7,8-tetrahydro-[1,8]naphthyridin-2-yl)-nonanoic acid ethyl ester (28-6)

A mixture of 28-5 (0.1 g, 0.3 mmol) and 10% Pd/C (0.1 g) in 10 mL methanol was purged with argon under vacuum and then treated under balloon hydrogenation conditions for 40 hr. It was filtered through a pad of celite. The solution was concentrated. The residue was purified by silica gel chromatography (100% CHCl₃ to CHCl₃/MeOH 5:1) to afford the desired product 28-6 as an oil.
¹H NMR (400 MHz, CDCl₃) δ 7.88 (d, J=2.0 Hz, 1H), 7.25 (dd, J=8.4, 2.0 Hz, 1H), 7.05 (d, J=7.6 Hz, 1H), 6.45 (d, J=8.4 Hz, 1 H), 6.31 (d, J=7.6 Hz 1H), 4.98 (bs, 1H), 4.34 (bs, 2H), 4.04 (q, J=7.2 Hz, 2H), 3.39 (m, 2H), 2.95 (m, 1H), 2.68 (m, 2H), 2.60-2.43 (m, 4H), 1.89 (m, 2H), 1.64-1.44 (m, 4H), 1.27 (m, 6H), 1.29 (t, J=7.2 Hz, 3H).

### 3-(6-Amino-pyridin-3-yl)-9-(5,6,7,8-tetrahydro-[1,8]naphthyridia-2-yl)-nonanoic acid (28-7)

A mixture of 28-6 (0.04 g, 0.1 mmol) and LiOH (1M, 0.3 mL, 0.3 mmol) in 1 mL EtOH and 0.5 mL H₂O was stirred for 12 hr at room temperature. It was concentrated and diluted with 1N HCl (2 mL). The mixture was purified by reverse phase HPLC (C18 column; gradient: H₂O /CH₃CN/TFA from 95:5:0.1 to 5:95:0.1 over 45 min) to give pure 28-7 as the TFA salt.
¹H NMR (400 MHz, CD₃OD) δ 7.90 (dd, J=9.2, 2.0 Hz, 1H), 7.67 (d, J=2.0 Hz, 1H), 7.54 (d, J=7.6 Hz, 1H), 6.99 (d, J=9.2 Hz, 1 H), 6.55 (d, J=7.6 Hz, 1H), 3.48 (m, 2H),3.00 (m, 1H), 2.80 (m, 2H), 2.66 (m, 5H), 2.53 (m, 1H), 1.94 (m, 2H), 1.64 (m, 4H), 1.27 (m, 4H).

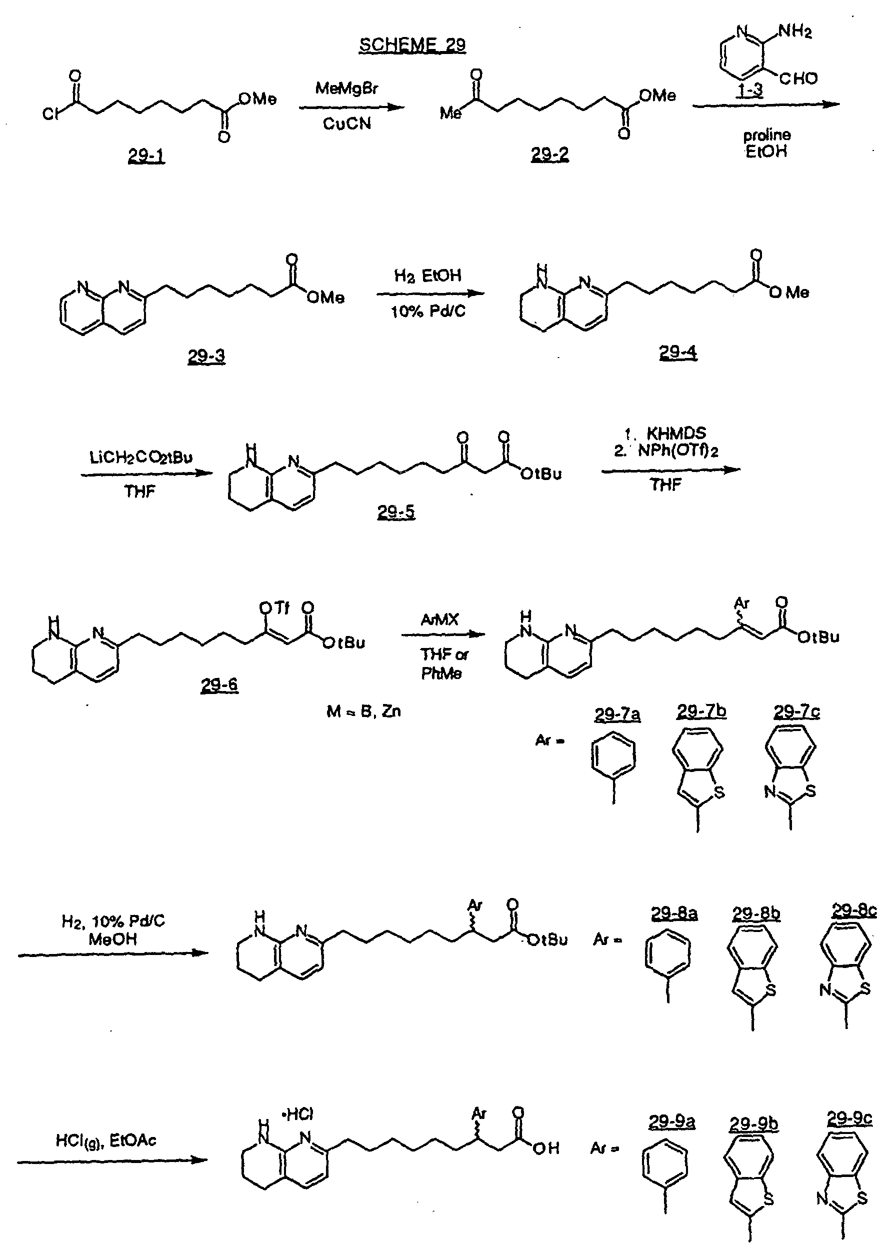

### Methyl 8-oxo-nonanoate (29-2)

To a stirred suspension of cuprous cyanide (2.87 g, 32.0 mmol) in tetrahydrofuran (100 mL) at -78°C was added a solution of methylmagnesium bromide (9.9 mL of a 3.0 M solution in tetrahydrofuran). The reaction mixture was allowed to warm to -15°C for 5 minutes and then cooled to -78°C. To this was added a solution of methyl 7-(chloroformyl)-heptanoate 29-1 (4.9 g, 23.7 mmol) in tetrahydrofuran (20 mL) and the reaction mixture was allowed to warm to -10°C for 1.5 hours. To the mixture was added a 9:1 solution of saturated aqueous ammonium chloride and concentrated aqueous ammonium hydroxide (200 mL) and the resulting mixture was extracted with ethyl acetate (2 x 200 mL). The organic extracts were washed successively with saturated aqueous ammonium chloride, saturated aqueous sodium hydrogen carbonate, and saturated aqueous sodium chloride. The organic extracts were then dried with anhydrous magnesium sulfate, filtered, and concentrated at reduced pressure to give 29-1 as an oil which was used in the next step without further purification.

### 7-[1,8]Naphthyridin-2-yl-heptanoic acid methyl ester (29-3)

To a stirred solution of methyl 8-oxo-nananoate 29-2 (3.8 g, 20.4 mmol) in absolute ethanol (100 mL) was added 2-aminopyridine-3-carboxaldehyde (2.49 g, 20.4 mmol) and L-proline (1.17 g, 10.2 mmol) and the mixture was heated to 95°C for 18 hours, after which the mixture was cooled to ambient temperature and then concentrated at reduced pressure. The resulting solid was purified by flash column chromatography over silica gel with 95:5 ethyl acetate/methanol to give 29-3 as a white solid.
¹H NMR (300 MHz, CDCl₃) δ 9.11 (dd, J = 2.0, 4.2 Hz, 1 H), 8.17 (dd, J = 1.9,8.1 Hz, 1 H), 8.14 (d, J = 8.4 Hz, 1H), 7.44 (dd, J = 4.4,8.1 Hz, 1H), 7.39 (d, J = 8.3 Hz, 1H), 3.65 (s, 3H), 3.03 (app t, J = 6.2 Hz, 2H), 2.32 (app t, J = 7.7 Hz, 2H), 1.98-1.84 (m, 2H), 1.70-1.57 (m, 2H), 1.50-1.33 (m, 4H).

### 7-(5,6,7,8-Tetrahydro-[1,8]naphthyridin-2-yl)-heptanoic acid methyl ester (29-4)

To a stirred suspension of 10% palladium on carbon (600 mg) in ethanol (25 mL) was added a solution of 7-[1,8]naphthyridin-2-yl-heptanoic acid methyl ester 29-3 (3.6 g) in ethanol (75 mL) and the mixture was subjected to an atmosphere of hydrogen for 18 hours. The reaction mixture was filtered through Celite and concentrated at reduced pressure to give 29-4 as an oil.
¹H NMR (300 MHz, CDCl₃) δ 7.06 (d, J = 7.3 Hz, 1H), 6.33 (d, J = 7.3 Hz, 1H), 5.01 (br s, 1H), 3.66 (s, 3H), 3.42-3.37 (m, 2H), 2.69 (app t, J = 6.3 Hz, 2H), 2.53 (app t, J = 7.6 Hz, 2H), ), 2.29 (app t, J = 7.5 Hz, 2H), 1.94-1.86 (m, 2H), 1.67-1.59 (m, 2H), 1.37-1.33 (m, 4H).

### 3-Qxo-9-(5,6,7,8-tetrahydro-[1,8]naphthyridin-2-yl)-nonanoic- acid tert-butyl ester (29-5)

To a stirred solution of diisopropylamine (5.17 mL, 36.9 mmol) in anhydrous tetrahydrofuran (100 mL) at -78°C was added a solution of n-butyllithium in hexanes (16.2 mL of a 2.5 M solution). After 5 minutes, *t*-butyl acetate (4.97 mL, 36.9 mmol) was added. After an additional 5 minutes, 7-(5,6,7,8-tetrahydro-[1,8]naphthyridin-2-yl)-heptanoic acid methyl ester 29-4 (3.4 g, 12.3 mmol) in tetrahydrofuran (30 mL) was added and the solution was warmed to -40°C for one hour. The reaction mixture was then poured into saturated aqueous sodium hydrogen carbonate, and extracted with ethyl acetate. The organic layer was washed with saturated aqueous sodium chloride, dried with anhydrous magnesium sulfate, filtered, and concentrated at reduced pressure. The resulting oil was purified by flash column chromatography over silica gel with ethyl acetate to give 29-5 as a yellowish oil.
¹H NMR (300 MHz, CDCl₃) δ 7.04 (d, J = 7.3 Hz, 1H), 6.33 (d, J = 7.3 Hz, 1H), 4.84 (br s, 1H), 3.42-3.37 (m, 2H), 3.33 (s, 2H), 2.69 (app t, J = 6.4 Hz, 2H), 2.58-2.44 (m, 2H), 1.94-1.86 (m, 2H), 1.72-1.52 (m, 4H), 1.47 (s, 9H), 1.39-1.28 (m, 4H).

### 9-(5,6,7,8-Tetrahydro-[1,8]nayphthyridin-2-yl)-3-(trifluoromethanesulfonyloxy)-non-2-enoic acid tert-butyl ester (29-6)

To a stirred solution of 3-oxo-9-(5,6,7,8-tetrahydro-[1,8]naphthyridin-2-yl)-nonanoic acid *tert*-butyl ester 29-5 (1.0 g, 2.77 mmol) in anhydrous tetrahydrofuran (25 mL) at 0°C was added a solution of potassium bis(trimethylsilyl)amide in toluene (7.2 mL of a 0.5 M solution). After 5 minutes, N-phenyltrifluoromethanesulfonimide (1.49 g, 4.17 mmol) was added in one portion and the resulting solution was allowed to warm to ambient temperature for 2 hours. The reaction mixture was then poured into saturated aqueous sodium hydrogen carbonate and extracted with ethyl acetate. The organic layer was washed with saturated aqueous sodium chloride, dried with anhydrous magnesium sulfate, filtered, and concentrated at reduced pressure. The resulting oil was purified by flash column chromatography over silica gel with 3:2 ethyl acetate/hexanes to give 29-6 as a yellowish semi-solid.
¹H NMR (300 MHz, CDCl₃) δ 7.08 (d, J = 7.3 Hz, 1H), 6.34 (d, J = 7.3 Hz, 1H), 5.66 (s, 1H), 5.09 (br s, 1H), 3.44-3.37 (m, 2H), 2.68 (app t, J = 6.2 Hz, 2H), 2.56 (app t, J = 7.7 Hz, 2H), ), 2.33 (app t, J = 7.6 Hz, 2H), 1.94-1.83 (m, 2H), 1.70-1.48 (m, 4H), 1.49 (s, 9H), 1.37-1.29 (m, 4H).

### 3-Phenyl-9-(5,6,7,8-tetrahydro-[1,8]naphthyridin-2-yl)-non-2-enoic acid tert-butyl ester (29-7a)

To a stirred solution of 9-(5,6,7,8-Tetrahydro-[1,8]naphthyridin-2-yl)-3-(trifluoromethanesulfonyloxy)-non-2-enoic acid *tert*-butyl ester 29-6 (100 mg, 0.20 mmol) in toluene (2.5 mL) was added palladium(tetrakis)-triphenylphosphine (23 mg, 0.020 mmol), phenylboronic acid (50 mg, 0.41 mmol), and potassium carbonate (56 mg, 0.41 mmol). The resulting suspension was heated at 90-100°C for 2 hours, and then was allowed to cool to ambient temperature. The reaction mixture was then poured into saturated aqueous sodium hydrogen carbonate and extracted with ethyl acetate. The organic layer was washed with saturated aqueous sodium chloride, dried with anhydrous magnesium sulfate, filtered, and concentrated at reduced pressure. The resulting oil was purified by flash column chromatography over silica gel with 4:1 ethyl acetate/hexanes to give 29-7a as a colorless oil.
¹H NMR (300 MHz, CDCl₃) δ 7.41-7.15 (m, 5H), 7.02 (d, J = 7.3 Hz, 1H), 6.31 (d, J = 7.2 Hz, 1H), 5.77 (br s, 1H), 4.77 (br s, 1H), 3.42-3.34 (m, 2H), 2.69 (app t, J = 6.3 Hz, 2H), 2.50 (app t, J = 7.6 Hz, 2H), 2.39 (app t, J = 7.8 Hz, 2H), 1.95-1.84 (m, 2H), 1.66-1.54 (m, 4H), 1.42-1.26 (m, 8H), 1.21 (s, 9H).

### 3-Phenyl-9-(5,6,7,8-tetrahydro-[1,8]naphthyridin-2-yl)-nonanoic acid tert-butyl ester (29-8a)

To a stirred suspension of 10% palladium on carbon (15 mg) in ethanol (2 mL) was added a solution of 3-phenyl-9-(5,6,7,8-tetrahydro-[1,8]naphthyridin-2-yl)-non-2-enoic acid *tert*-butyl ester 29-7a (62 mg) in ethanol (3 mL) and the mixture was subjected to an atmosphere of hydrogen for 6 hours. The reaction mixture was filtered through Celite and concentrated at reduced pressure to give 29-8a as an oil.
¹H NMR (300 MHz, CDCl₃) d 7.31-7.13 (m, 5H), 7.04 (d, J = 7.3 Hz, 1H), 6.31 (d, J = 7.2 Hz, 1H), 4.92 (br s, 1H), 3.42-3.35 (m, 2H), 3.06-2.94 (m, 1H), 2.69 (app t, J = 6.2 Hz, 2H), 2.58-2.40 (m, 4H), 1.94-1.85 (m, 2H), 1.66-1.48 (m, 4H), 1.36-1.18 (m, 17H).

### 3-Phenyl-9-(5,6,7,8-tetrahydro-[1,8]naphthyridin-2-yl)-nonanoic acid hydrochloride (29-9a)

Hydrogen chloride gas was bubbled into a stirred solution of 3-phenyl-9-(5,6,7,8-tetrahydro-[1,8]naphthyridin-2-yl)-nonanoic acid *tert*-butyl ester 29-8a (60 mg) in ethyl acetate at 0°C for one hour. The reaction mixture was allowed to warm to ambient temperature and then concentrated at reduced pressure. The resulting solid 29-9a was dried in vacuo overnight.
¹H NMR (300 MHz, CD₃OD) δ 7.78 (d, J = 7.1 Hz, 1H), 7.33-7.11 (m, 5H), 6.58 (d, J = 7.2 Hz, 1H), 3.52-3.42 (m, 2H), 3.10-2.96 (m, 1H), 2.82 (app t, J = 7.0 Hz, 2H), 2.68-2.43 (m, 4H), 1.98-1.89 (m, 2H), 1.76-1.50 (m, 4H), 1.40-1.05 (m, 8H).

### 3-(benzo[b]thiophen-2-yl)-9-(5,6,7,8-tetrahydro-[1,8]naphthyridin-2-yl)-non-2-enoic acid tert-butyl ester (29-7b)

To a stirred solution of 9-(5,6,7,8-tetrahydro-[1,8]naphthyridin-2-yl)-3-(trifluoromethanesulfonyloxy)-non-2-enoic acid *tert*-butyl ester 29-6 (100 mg, 0.20 mmol) in toluene (2.5 mL) was added palladium(tetrakis)-triphenylphosphine (23 mg, 0.020 mmol), benzo[b]thiophene-2-boronic acid (50 mg, 0.41 mmol) and potassium carbonate (56 mg, 0.41 mmol). The resulting suspension was heated at 90-100°C for 2 hours, and then was allowed to cool to ambient temperature. The reaction mixture was then poured into saturated aqueous sodium hydrogen carbonate and extracted with ethyl acetate. The organic layer was washed with saturated aqueous sodium chloride, dried with anhydrous magnesium sulfate, filtered, and concentrated at reduced pressure. The resulting oil was purified by flash column chromatography over silica gel with 4:1 ethyl acetate/hexanes to give 29-7b as a colorless oil.
¹H NMR (300 MHz, CDCl₃) δ 7.82-7.70 (m, 2H), 7.53 (s, 1H), 7.39-7.22 (m, 2H), 7.02 (d, J = 7.3 Hz, 1H), 6.33 (d, J = 7.3 Hz, 1H), 5.89 (br s, 1H), 4.78 (br s, 1H), 3.42-3.36 (m, 2H), 2.74 (app t, J = 6.2 Hz, 2H), 2.58-2.42 (m, 2H), 1.95-1.77 (m, 4H), 1.70-1.10 (m, 17H).

### 3-(Benzo[b]thiophen-2-yl)-9-(5,6,7,8-tetrahydro-[1,8]naphthyridin-2-yl)-nonanoic acid tert-butyl ester (29-8b)

To a stirred suspension of 10% palladium on carbon (45 mg) in methanol (5 mL) was added a solution of 3-(benzo[b]thiophen-2-yl)-9-(5,6,7,8-tetrahydro-[1,8]naphthyridin-2-yl)-non-2-enoic acid *tert*-butyl ester 29-7b (220 mg) in ethanol (5 mL) and the mixture was subjected to an atmosphere of hydrogen for 48 hours. The reaction mixture was filtered through Celite and concentrated at reduced pressure. The resulting oil was purified by flash column chromatography over silica gel with 9:1 ethyl acetate / methanol to give 29-8b as a colorless oil.
¹H NMR (300 MHz, CDCl₃) δ 7.89-7.64 (m, 4H),7.03 (s, 1H), 7.02 (d, J = 7.3 Hz, 1H), 6.29 (d, J = 7.3 Hz, 1H), 4.85 (br s, 1H), 3.44-3.34 (m, 3H), 2.72-2.44 (m, 6H), 1.94-1.84 (m, 2H), 1.72-1.52 (m, 4H), 1.34 (s, 9H), 1.33-1.22 (m, 8H).

### 3-(Benzo[b]thiophen-2-yl)-9-(5,6,7,8-tetrahydro-[1,8]naphthyridin-2-yl)-nonanoic acid (29-9b)

Hydrogen chloride gas was bubbled into a stirred solution of 3-(benzo[b]thiophen-2-yl)-9-(5,6,7,8-tetrahydro-[1,8]naphthyridin-2-yl)-nonanoic acid *tert*-butyl ester 29-8b (85 mg) in ethyl acetate at 0°C for one hour. The reaction mixture was allowed to warm to ambient temperature and then concentrated at reduced pressure. The resulting solid was neutralized with concentrated ammonium hydroxide, then the resulting free base was purified by flash column chromatography over silica gel with 25:3:2 chloroform/ethyl acetate/methanol to give 29-9b as a colorless oil.
¹H NMR (300 MHz, CD₃OD) d 7.77-7.62 (m, 2H), 7.38 (d, J = 7.3 Hz, 1H), 7.30-7.18 (m, 2H), 7.09 (s, 1H), 6.45 (d, J = 7.2 Hz, 1H),3.60-3.51 (m, 1H), 3.41 (app t, J = 6.4 Hz, 2H),2.78-2.52 (m, 6H), 1.94-1.56 (m, 8H), 1.44-1.28 (m, 8H).

### 3-(Benzothiazol-2-yl)-9-(5,6,7,8-tetrahydro-[1,8]naphthyridin-2-yl)-non-2-enoic acid tert-butyl ester (29-7c)

To a stirred solution of benzthiazole (165 mg, 1.22 mmol) in anhydrous tetrahydrofuran (5 mL) at -78°C was added a solution of n-butyllithium in hexanes (0.52 mL of a 2.5 M solution). After 5 minutes, a solution of zinc chloride in tetrahydrofuran (2.6 mL of a 0.50 M solution) was added and the reaction mixture was allowed to warm to ambient temperature. To the resulting solution was added 9-(5,6,7,8-tetrahydro-[1,8]naphthyridin-2-yl)-3-(trifluoromethanesulfonyloxy)-non-2-enoic acid *tert*-butyl ester 29-6 (400 mg, 0.81 mmol) and palladium(tetrakis)-triphenylphosphine (94 mg, 0.081 mmol) and the mixture was stirred at ambient temperature for 1 hour. The reaction mixture was then poured into saturated aqueous sodium hydrogen carbonate and extracted with ethyl acetate. The organic layer was washed with saturated aqueous sodium chloride, dried with anhydrous magnesium sulfate, filtered, and concentrated at reduced pressure. The resulting oil was purified by flash column chromatography over silica gel with 4:1 ethyl acetate/hexanes to give 29-7c as a colorless oil.
¹H NMR (300 MHz, CDCl₃): δ 8.10-7.85 (m, 2H), 7.50-7.35 (m, 2H), 7.02 (d, J = 7.2 Hz, 1H), 6.35 (d, J = 7.2 Hz, 1H), 6.05 (br s, 1H), 5.55 (br s, 1H), 3.45-3.36 (m, 2H), 2.74-2.65 (m, 2H), 2.62-2.42 (m, 2H), 2.28 (app t, J = 7.2 Hz, 2H), 1.95-1.82 (m, 2H), 1.70-1.15 (m, 17H).

### 3-(Benzothiazol-2-yl)-9-(5,6,7,8-tetrahydro-[1,8]naphthyridin-2-yl)-nonanoic acid tert-butyl ester (29-8c)

To a stirred suspension of 10% palladium on carbon (50 mg) in methanol (5 mL) was added a solution of 3-(benzothiazol-2-yl)-9-(5,6,7,8-tetrahydro-[1,8]naphthyridin-2-yl)-non-2-enoic acid *tert*-butyl ester 29-7c (190 mg) in ethanol (5 mL) and the mixture was subjected to an atmosphere of hydrogen for 48 hours. The reaction mixture was filtered through Celite and concentrated at reduced pressure. The resulting oil was purified by flash column chromatography over silica gel with 4:1 hexanes / acetone to give 29-8c as a colorless oil.
¹H NMR (300 MHz, CDCl₃) δ 7.99-7.81 (m, 2H), 7.48-7.31 (m, 2H), 7.04 (d, J = 7.3 Hz, 1H), 6.30 (d, J = 7.2 Hz, 1H), 4.95 (br s, 1H), 3.66-3.56 (m, 1H), 3.38 (app t, J = 6.3 Hz, 2H), 2.90-2.63 (m, 6H), 2.48 (app t, J = 7.6 Hz, 2H), 1.94-1.74 (m, 4H), 1.64-1.52 (m, 2H), 1.36 (s, 9H), 1.34-1.22 (m, 8H).

### 3-(Benzothiazol-2-yl)-9-(5,6,7,8-tetrahydro-[1,8]naphthyridin-2-yl)-nonanoic acid hydrochloride (29-9c)

Hydrogen chloride gas was bubbled into a stirred solution of 3-(benzothiazol-2-yl)-9-(5,6,7,8-tetrahydro-[1,8]naphthyridin-2-yl)-nonanoic acid *tert*-butyl ester 29-9b (80 mg) in ethyl acetate at 0°C for one hour. The reaction mixture was allowed to warm to ambient temperature and then concentrated at reduced pressure. The resulting solid 29-9c was pumped in vacuo overnight.
¹H NMR (300 MHz, CD₃OD) δ 8.01-7.90 (m, 2H), 7.60-7.41 (m, 3H), 6.58 (d, J = 7.3 Hz, 1H), 3.78-3.62 (m, 1H), 3.49 (app t, J = 6.4 Hz, 2H), 2.98-2.59 (m, 6H), 1.97-1.82 (m, 4H), 1.70-1.56 (m, 2H), 1.44-1.28 (m, 6H).

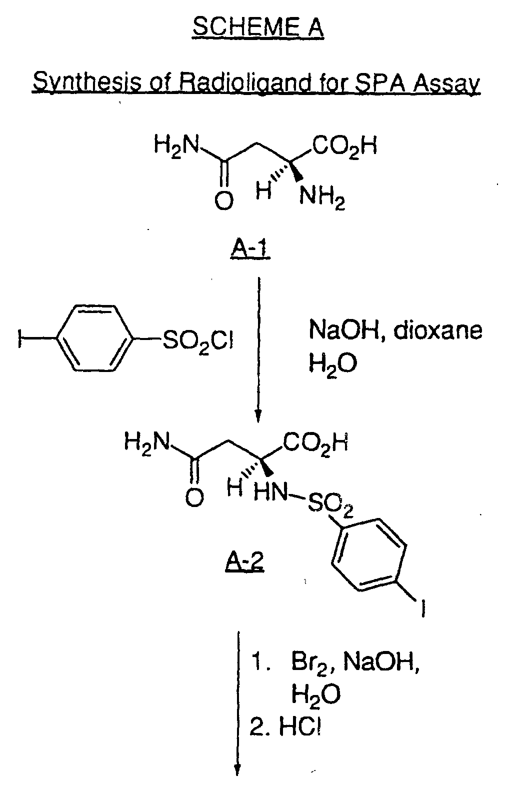

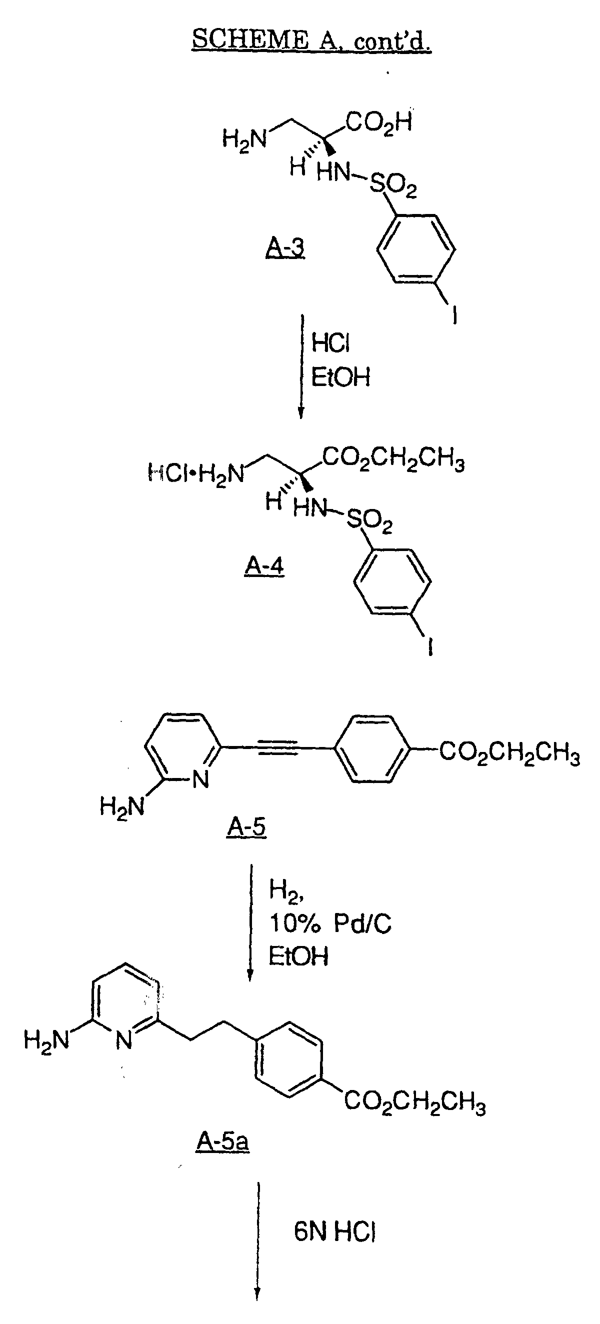

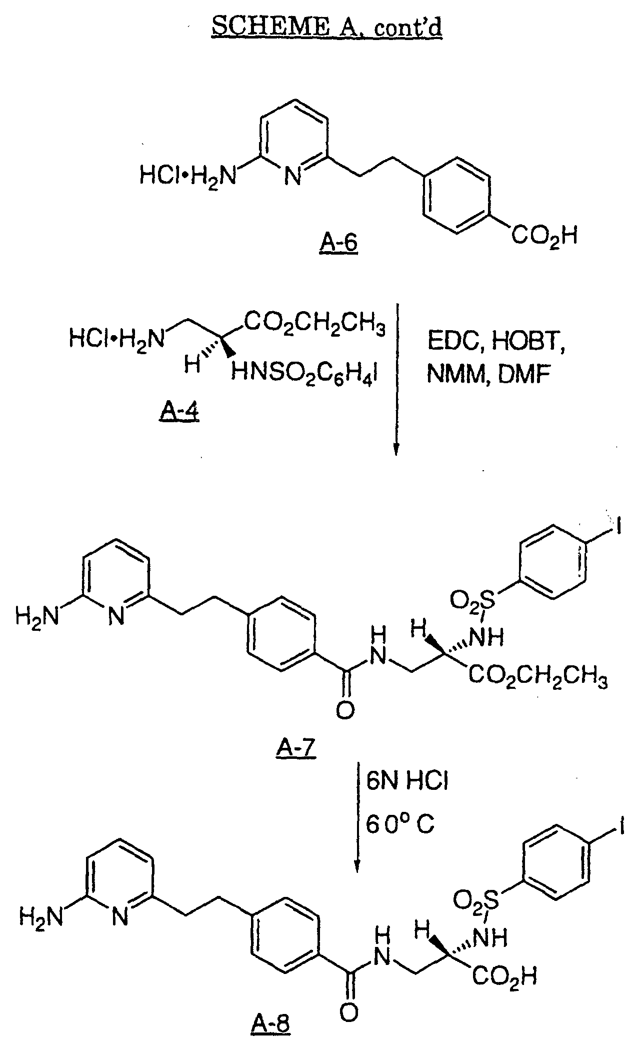

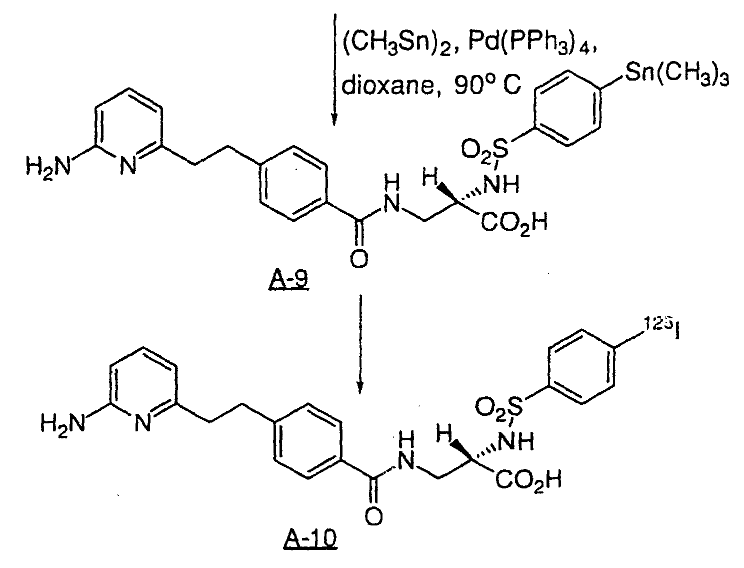

### N-(4-Iodo-phenylsulfonylamino)-L-asparagine (A-2)

To a stirred solution of acid A-1 (4.39 g, 33.2 mmol), NaOH (1.49 g, 37.2 mmol), dioxane (30 ml) and H₂O (30 ml) at 0°C was added pipsyl chloride (10.34 g, 34.2 mmol). After ∼5 minutes, NaOH (1.49, 37.2 mmol) dissolved in 15 ml H₂O, was added followed by the removal of the cooling bath. After 2.0 h, the reaction mixture was concentrated. The residue was dissolved in H₂O (300 ml) and then washed with EtOAc. The aqueous portion was cooled to 0°C and then acidified with concentrated HCl. The solid was collected and then washed with Et₂O to provide acid A-2 as a white solid.
¹H NMR (300 MHz, D₂O) δ 7.86 (d, 2H, J=8Hz ), 7.48 (d, 2H, J=8Hz) 3.70 (m, 1H), 2.39 (m, 2H).

### 2(S)-(4-Iodo-phenylsulfonylamino)-β-alanine (A-3)

To a stirred solution of NaOH (7.14 g, 181.8 mmol) and H₂O (40 ml) at 0°C was added Br₂ (1.30 ml, 24.9 mmol) dropwise over a ten minute period. After ∼5 minutes, acid A-2 (9.9 g, 24.9 mmol), NaOH (2.00 g, 49.8 mmol) and H₂O (35 ml) were combined, cooled to 0°C and then added in a single portion to the reaction. After stirring for 20 minutes at 0°C, the reaction was heated to 90°C for 30 minutes and then recooled to 0°C. The pH was adjusted to ∼7 by dropwise addition of concentrated HCl. The solid was collected, washed with EtOAc, and then dried *in vacuo* to provide acid A-3 as a white solid.
¹H NMR (300 MHz, D₂O) δ 8.02 (d, 2H, J=8Hz), 7.63 (d, 2H, J=8Hz), 4.36 (m, 1H), 3.51 (dd, 1H, J=5Hz, 13Hz) 3.21 (m, 1H).

### Ethyl 2(S)-(4-iodo-phenylsulfonylamino)-β-alanine-hydrochloride (A-4)

HCl gas was rapidly bubbled through a suspension of acid A-3 (4.0 g, 10.81 mmol) in EtOH (50 ml) at 0°C for 10 minutes. The cooling bath was removed and the reaction was heated to 60°C. After 18 h, the reaction was concentrated to provide ester A-4 as a white solid.
¹H NMR (300 MHz, CD₃OD) δ 7.98 (d, 2H, J=8Hz), 7.63 (d, 2H, J=8Hz), 4.25 (q, 1H, J=5Hz), 3.92 (m, 2H), 3.33 (m, 1H), 3.06 (m, 1H), 1.01 (t, 3H, J=7Hz).

### Ethyl 4-[2-(2-Aminopyridin-6-yl]ethyl]benzoate (A-5a)

A mixture of ester A-5 (700 mg, 2.63 mmol), (for preparation, see: Scheme 29 of PCT International Application Publication No. WO 95/32710, published December 7,1995) 10% Pd/C (350 mg) and EtOH were stirred under 1 atm H₂. After 20 h, the reaction was filtered through a celite pad and then concentrated to provide ester A-5a as a brown oil.
TLC R_{f} = 0.23 (silica, 40% EtOAc/hexanes)
¹H NMR (300 MHz, CDCl₃) δ 7.95 (d, 2H, J=8Hz), 7.26 (m, 3H), 6.43 (d, 1H, J=7Hz), 6.35 (d, 1H, J=8Hz), 4.37 (m, 4H), 3.05 (m, 2H), 2.91 (m, 2H), 1.39 (t, 3H, J=7Hz).

### 4-[2-(2-Aminopyridin-6-yl)ethyl]benzoic acid hydrochloride (A-6)

A suspension of ester A-5a (625 mg, 2.31 mmol) in 6N HCl (12 ml) was heated to 60°C. After ∼20 h, the reaction was concentrated to give acid A-6 as a tan solid.
¹H NMR (300 MHz, CD₃OD) δ 7.96 (d, 2H, J=8Hz), 7.80 (m, 1H), 7.33 (d, 2H, J=8Hz), 6.84 (d, 1H, J=9Hz), 6.69 (d, 1H, J=7Hz), 3.09 (m, 4H).

### Ethyl 4-[2-(2-Aminopyridin-6-yl)ethyl]benzoyl-2(S)-(4-iodophenylsulfonylamino)-β-alanine (A-7)

A solution of acid 15-6 (400 mg, 1.43 mmol), amine A-4 (686 mg, 1.57 mmol), EDC (358 mg, 1.86 mmol), HOBT (252 mg, 1.86 mmol), NMM (632 µl, 5.72 mmol) in DMF (10 ml) was stirred for ∼20 h. The reaction was diluted with EtOAc and then washed with sat. NaHCO₃, brine, dried (MgSO₄) and concentrated. Flash chromatography (silica, EtOAc then 5% isopropanol/EtOAc) provided amide A-7 as a white solid.
TLC R_{f} = 0.4 (silica, 10% isopropanol/EtOAc)
¹H NMR (300 MHz, CD₃OD) δ 7.79 (d, 2H, J=9Hz) 7.61 (d, 2H, J=8Hz), 7.52 (d, 2H, J=9Hz), 7.29 (m, 1H), 7.27 (d, 2H, J=8Hz), 4.20 (m, 1H), 3.95 (q, 2H, J=7Hz), 3.66 (dd, 1H, J=6Hz, 14Hz), 3.49 (dd, 1H, J=8Hz, 13Hz), 3.01 (m, 2H), 2.86 (m, 2H), 1.08 (t, 3H, J=7Hz).

### 4-[2-(2-Aminopyridin-6-yl)ethyl]benzoyl-2(S)-(4-iodophenylsulfonylamino)-β-alanine (A-8)

A solution of ester A-7 (200 mg, 0.3213 mmol) and 6N HCl (30 ml) was heated to 60°C. After ∼20 h, the reaction mixture was concentrated. Flash chromatography (silica, 20:20:1:1 EtOAc/EtOH/NH₄OH/H₂O) provided acid A-8 as a white solid.
TLC R_{f} = 0.45 (silica, 20:20:1:1 EtOAc/EtOH/NH₄OH/H₂O)
¹H NMR (400 MHz, DMSO) δ 8.40 (m, 1H), 8.14 (Bs, 1H), 7.81 (d, 2H, J=8Hz), 7.62 (d, 2H, J=8Hz), 7.48 (d, 2H, J=8Hz), 7.27 (m, 3H), 6.34 (d, 1H, J=7Hz), 6.25 (d, 1H, J=8Hz), 5.85 (bs, 2H), 3.89 (bs, 1H), 3.35 (m, 2H), 2.97 (m, 2H), 2.79 (m, 2H).

### 4-[2-(2-Aminopyridin-6-yl)ethyl)benzoyl-2(S)-(4-trimethylstannylphenylsulfonylamino-β-alanine (A-9)

A solution of iodide A-8 (70 mg, 0.1178 mmol), [(CH₃)₃Sn]₂ (49 µl, 0.2356 mmol), Pd(PPh₃)₄ (5 mg) and dioxane (7 ml) was heated to 90°C. After 2 h, the reaction was concentrated and then purified by preparative HPLC (Delta-Pak C₁₈ 15 µM 100A°, 40 x 100 mm; 95:5 then 5:95 H₂O/CH₃CN) to provide the trifluoroacetate salt. The salt was suspended in H₂O (10 ml), treated with NH₄OH (5 drops) and then lyophilized to provide amide A-9 as a white solid.
¹H NMR (400 MHz, DMSO) δ 8.40 (m, 1H), 8.18 (d, 1H, J=8Hz), 7.67 (m, 5H), 7.56 (d, 2H, J=8Hz), 7.29 (d, 2H, J=8Hz), 6.95-7.52 (m, 2H), 6.45 (bs, 2H), 4.00 (m, 1H), 3.50 (m, 1H), 3.33 (m, 1H), 2.97 (m, 2H), 2.86 (m, 2H).

### 4-[2-(2-Aminopyridin-6-yl)ethyl]benzoyl-2(S)-4-¹²⁵iodophenylsulfonylamino-β-alanine (A-10)

An iodobead (Pierce) was added to a shipping vial of 5 mCi of Na¹²⁵I (Amersham, IMS30) and stirred for five minutes at room temperature. A solution of 0.1 mg of A-9 in 0.05 mL of 10% H₂SO₄/MeOH was made and immediately added to the Na¹²⁵I/iodobead vial. After stirring for three minutes at room temperature, approximately 0.04-0.05 mL of NH₄OH was added so the reaction mixture was at pH 6-7. The entire reaction mixture was injected onto the HPLC for purification [Vydac peptide-protein C-18 column, 4.6 x 250 mm, linear gradient of 10% acetonitrile (0.1% (TFA):H₂O (0.1% TFA) to 90% acetonitrile (0.1% TFA):H₂O (0.1% TFA) over 30 minutes, 1 mL/min]. The retention time of A-10 is 17 minutes under these conditions. Fractions containing the majority of the radioactivity were pooled, lyophilized and diluted with ethanol to give approximately 1 mCi of A-10, which coeluted on HPLC analysis with an authentic sample of A-8.

The following additional but non-limiting examples were prepared using the procedures described above and are accompanied by their mass spectral characterization data:

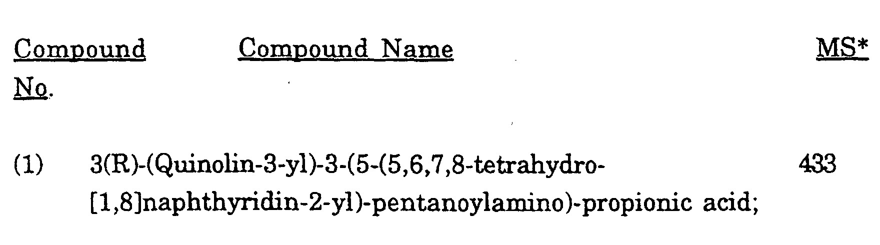

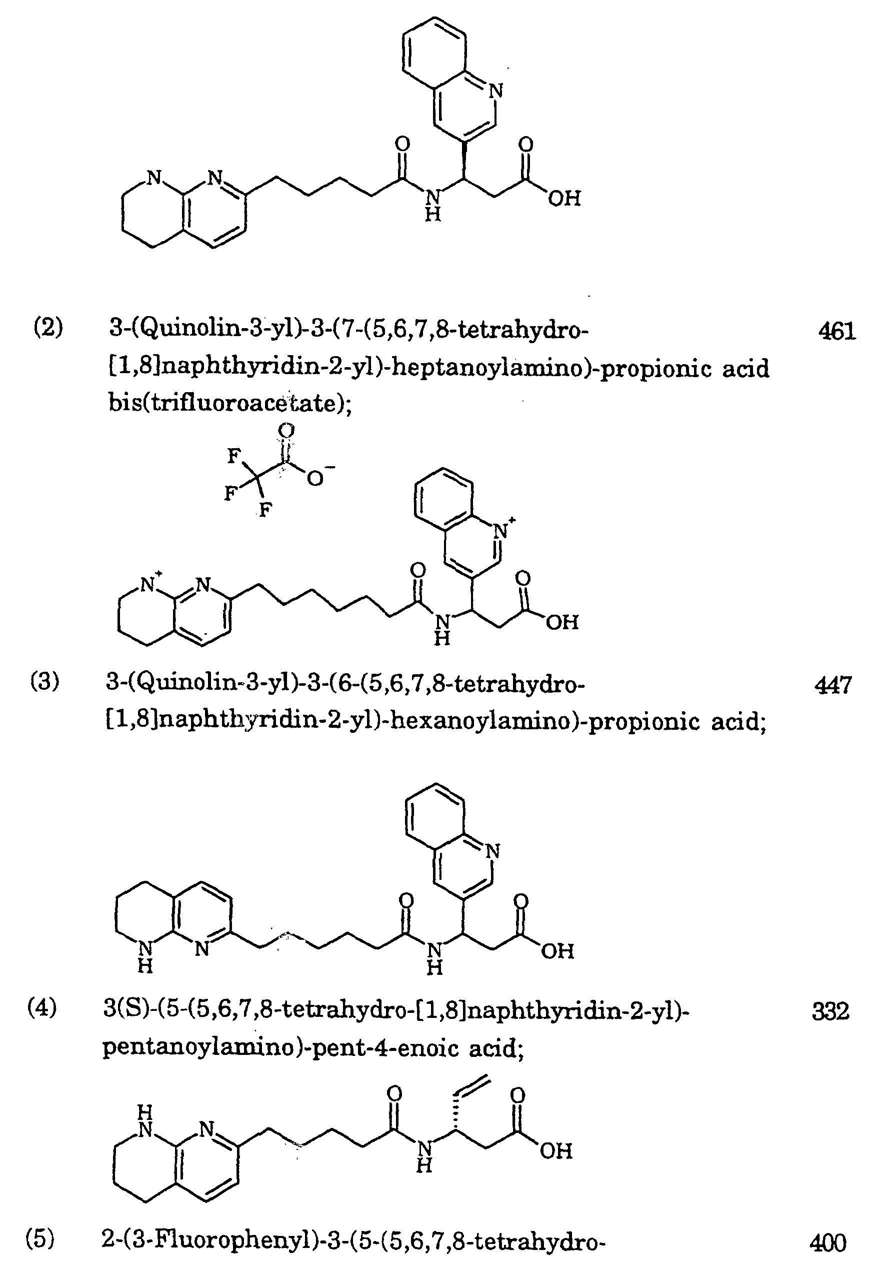

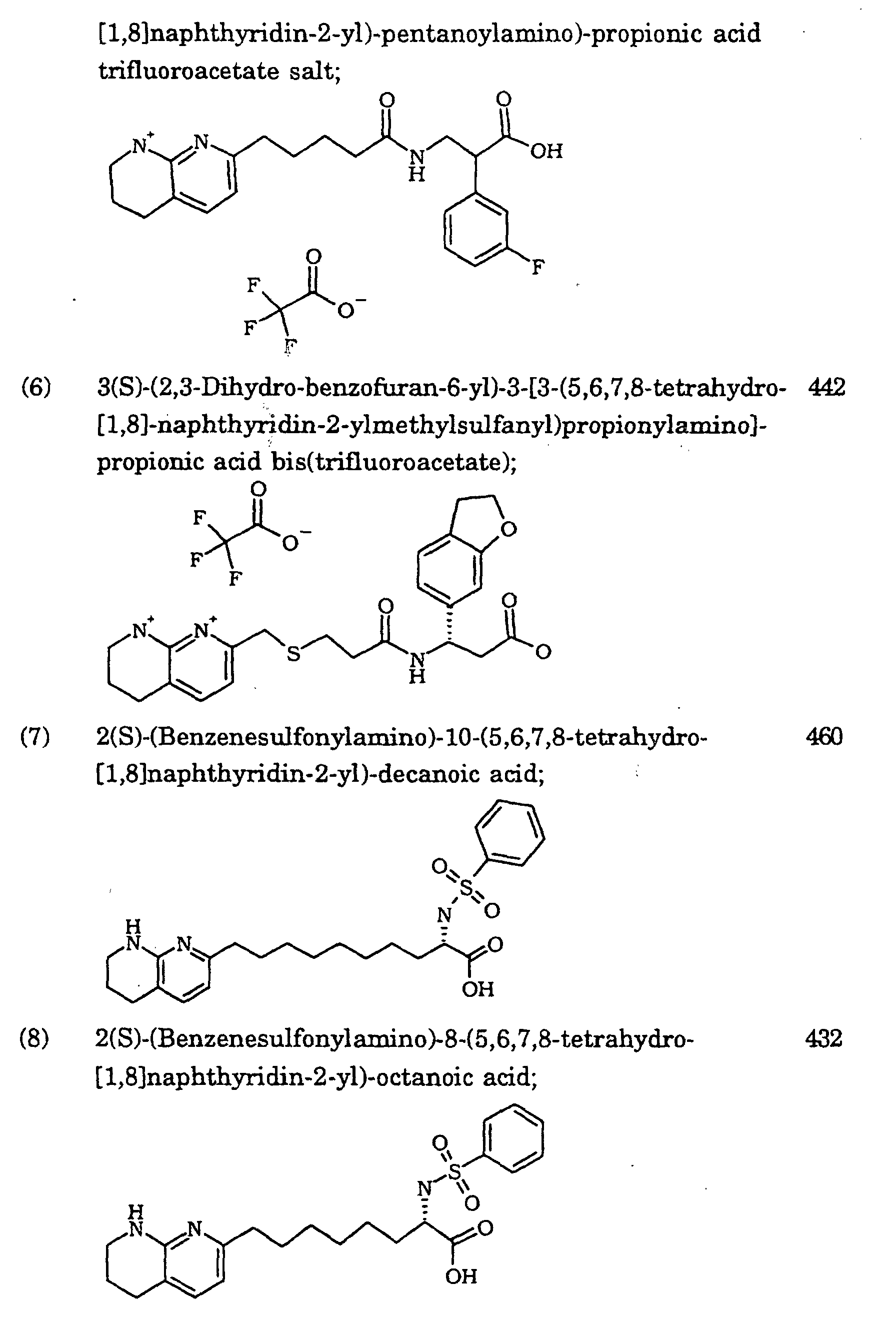

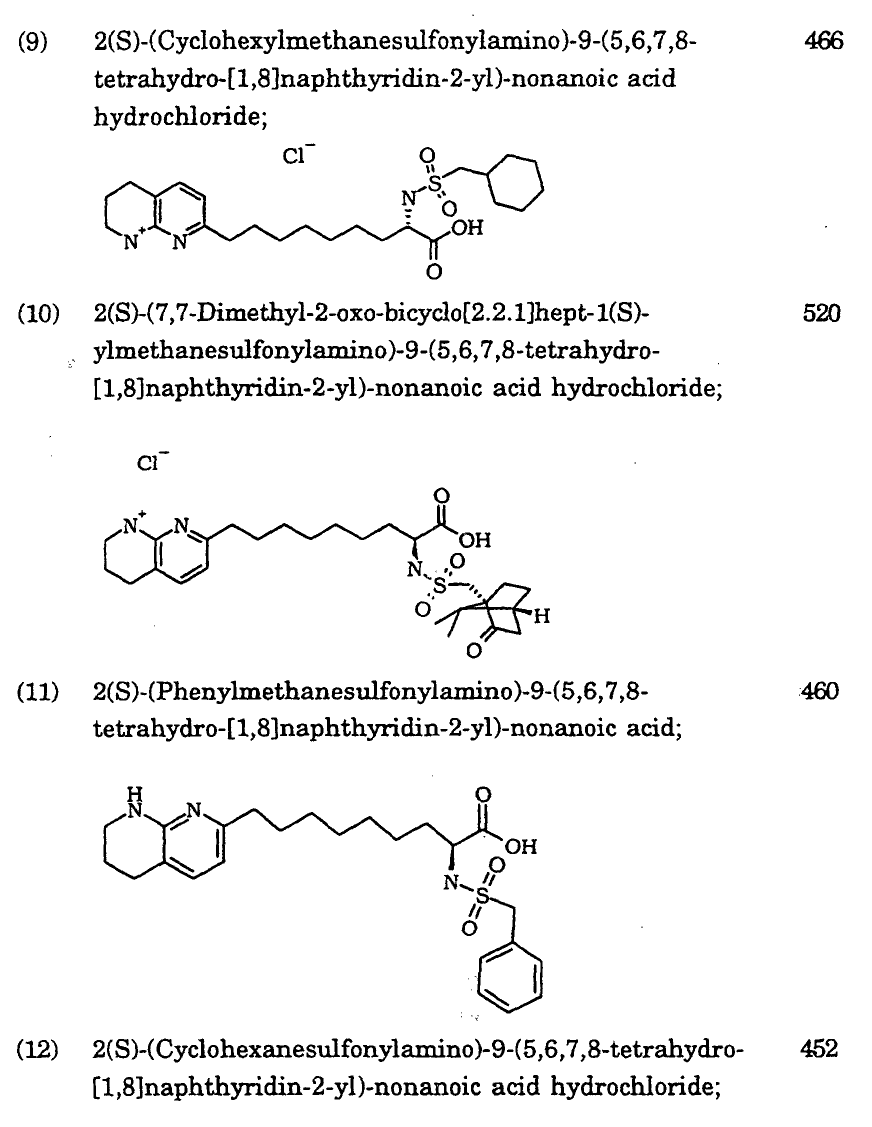

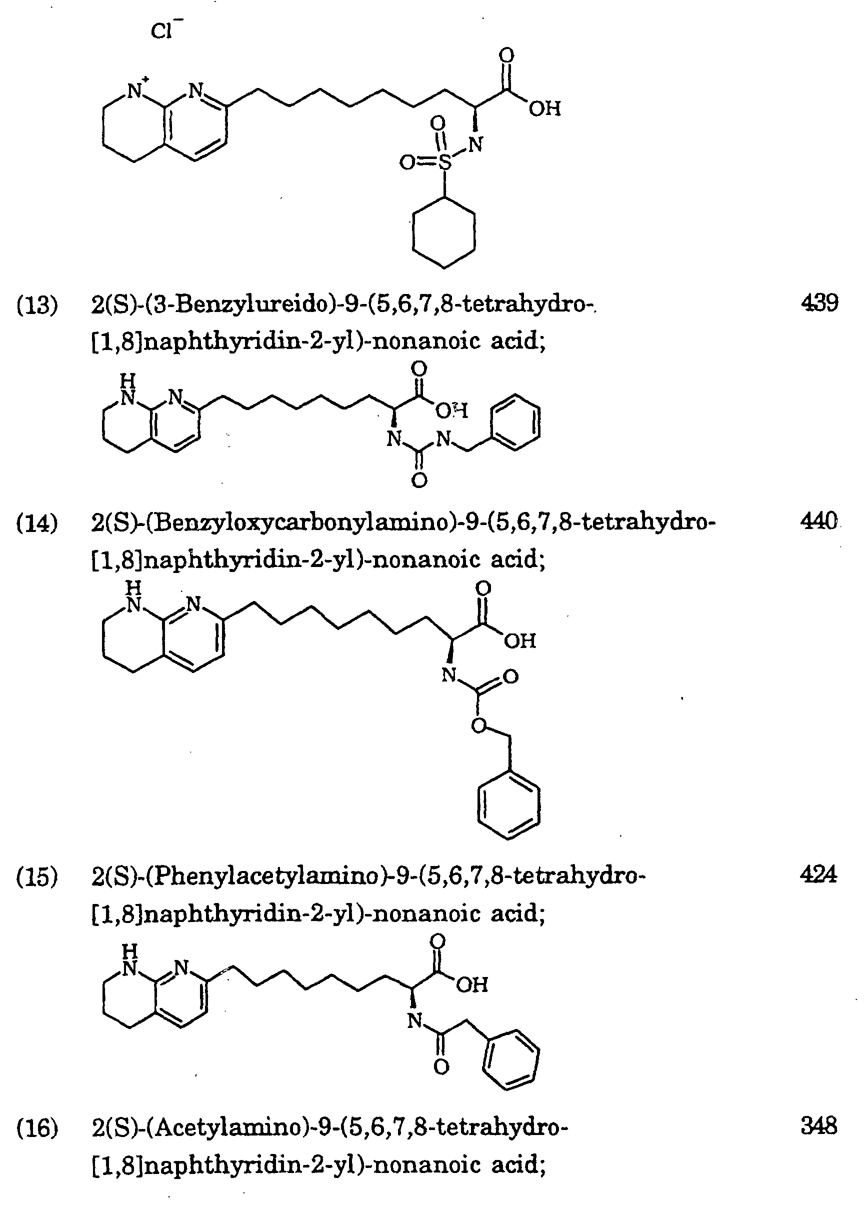

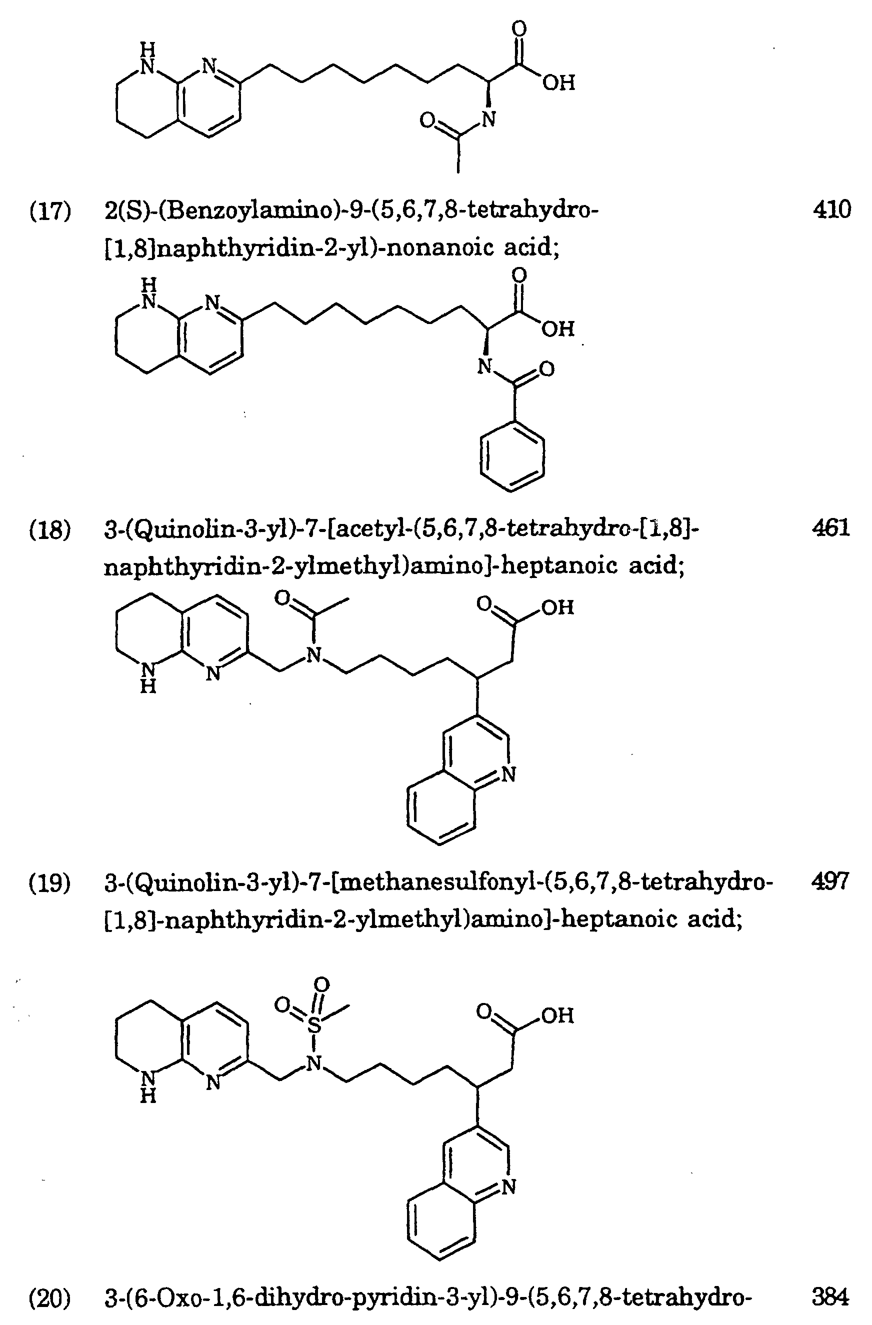

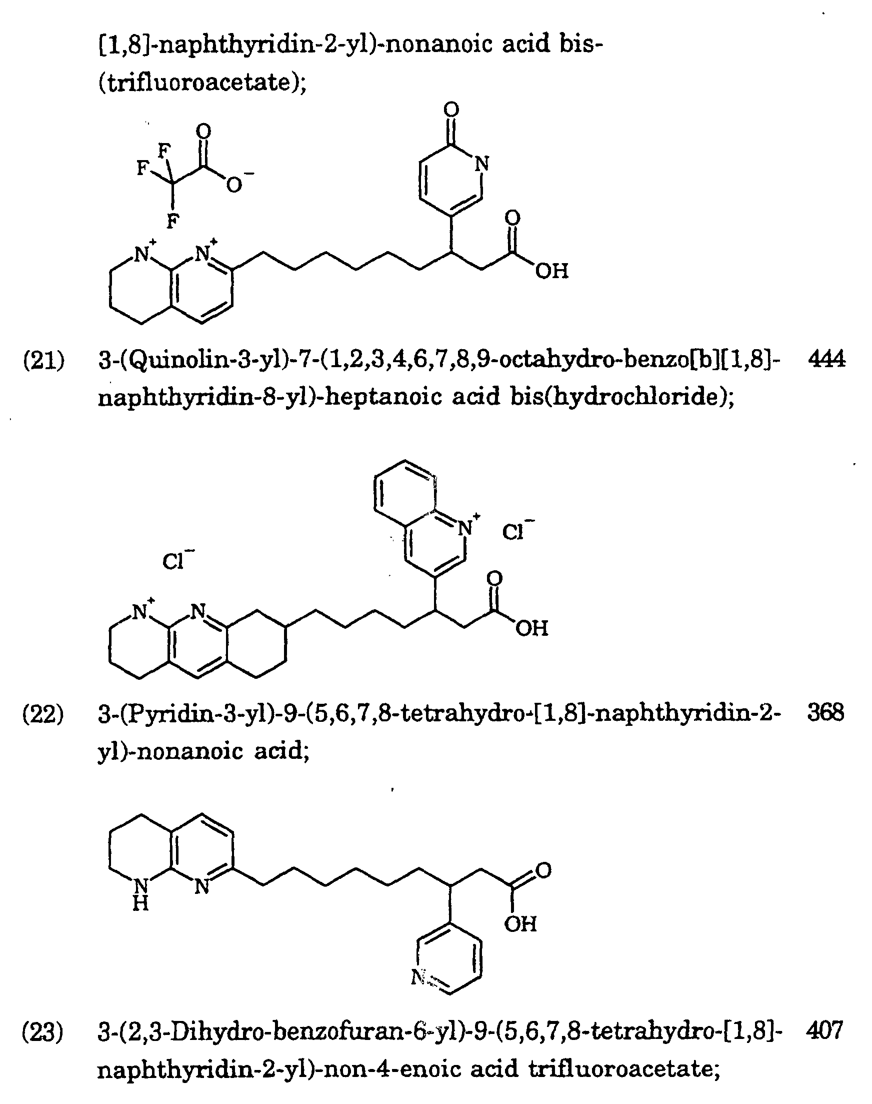

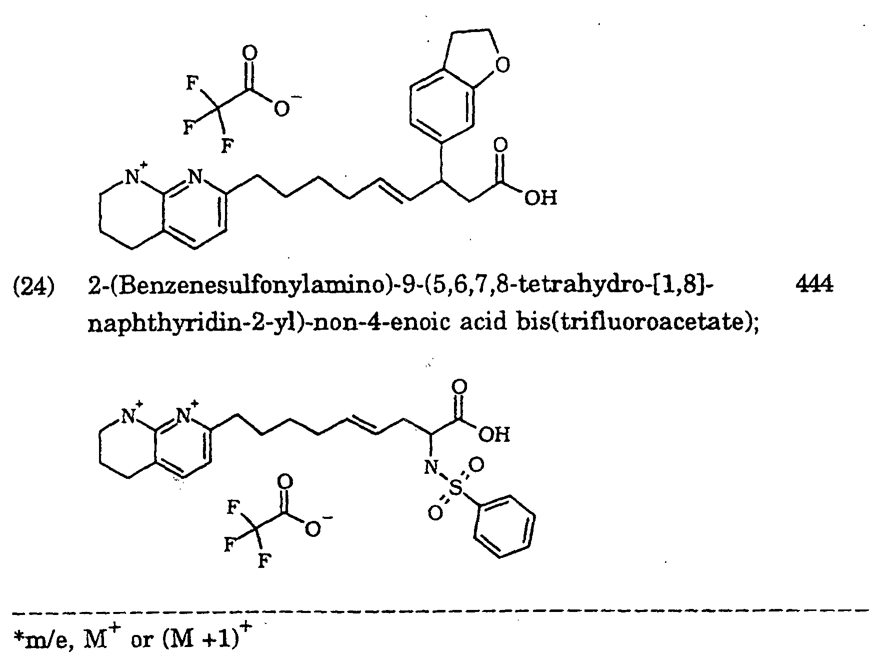

Compounds I-XXX whose structures are shown below can also be prepared as described above and depicted in Schemes 1-29 using synthetic methodologies or variations thereon which are known and understood by those skilled in the art of synthetic organic chemistry:

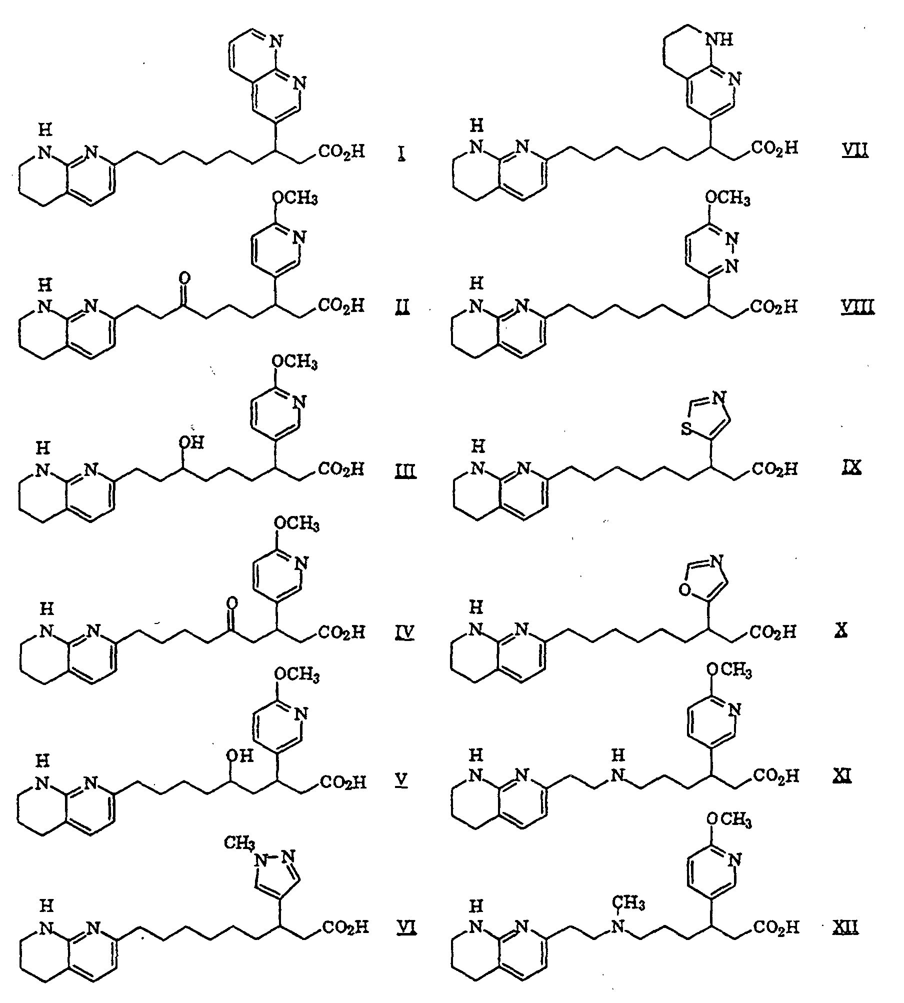

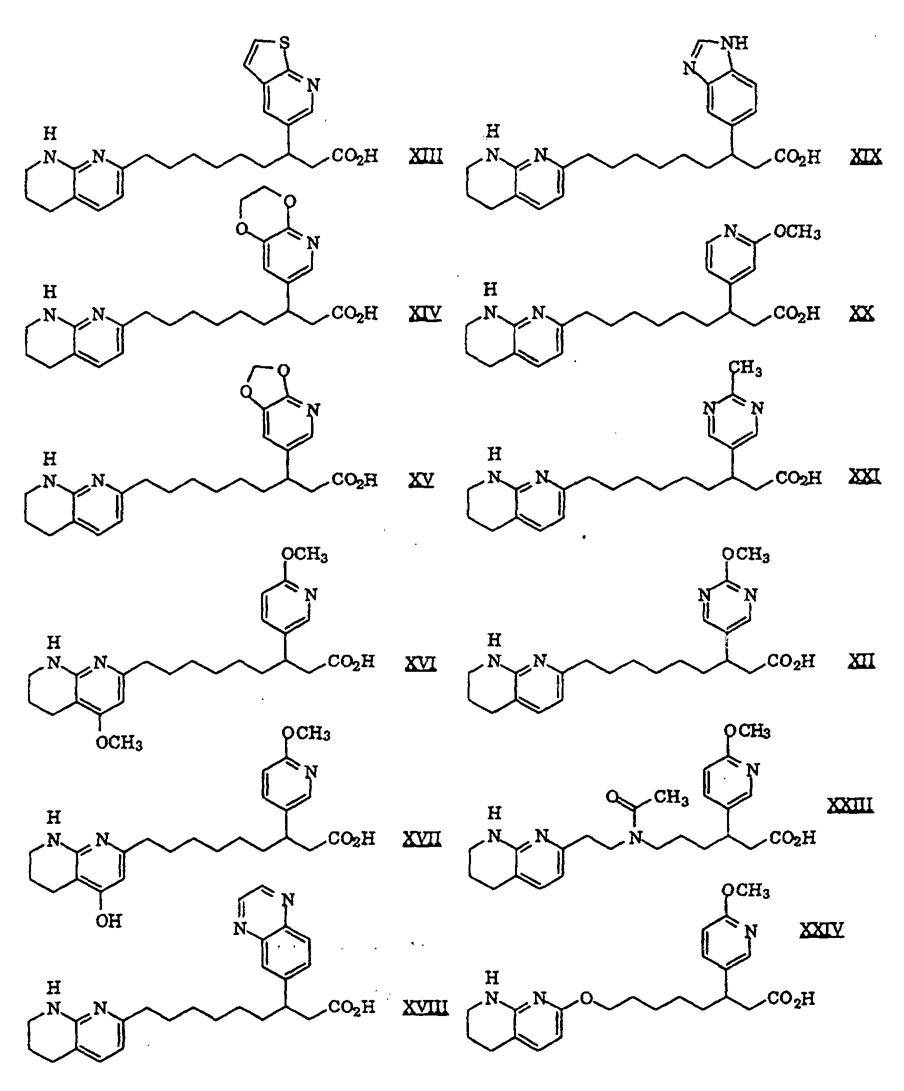

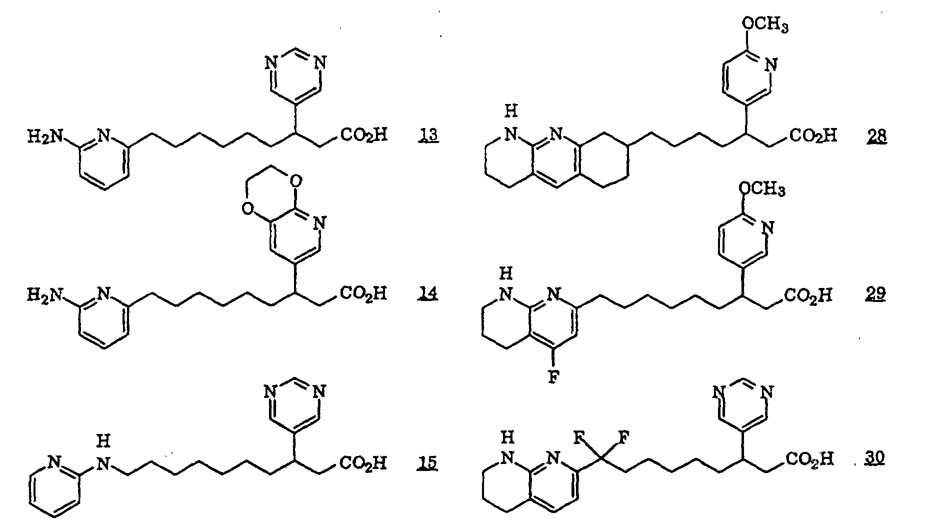

Instrumentation: Analytical and preparative HPLC was carried out using a Waters 600E Powerline Multi Solvent Delivery System with 0.1 mL heads with a Rheodyne 7125 injector and a Waters 990 Photodiode Array Detector with a Gilson FC203 Microfraction collector. For analytical and preparative HPLC, a Vydac peptide-protein C-18 column, 4.6 x 250 mm was used with a C-18 Brownlee modular guard column. The acetonitrile used for the HPLC analyses was Fisher Optima grade. The HPLC radiodetector used was a Beckman 170 Radioisotope detector. A Vydac C-18 protein and peptide column, 3.9 x 250 mm was used for analytical and preparative HPLC. Solutions of radioactivity were concentrated using a Speedvac vacuum centrifuge. Calibration curves and chemical concentrations were determined using a Hewlett Packard Model 8452A UV/Vis Diode Array Spectrophotometer. Sample radioactivities were determined in a Packard A5530 gamma counter.

The test procedures employed to measure αvβ3 and αvβ5 binding and the bone resorption inhibiting activity of the compounds of the present invention are described below.

### BONE RESORPTION-PIT ASSAY

When osteoclasts engage in bone resorption, they can cause the formation of pits in the surface of bone that they are acting upon. Therefore, when testing compounds for their ability to inhibit osteoclasts, it is useful to measure the ability of osteoclasts to excavate these resorption pits when the inhibiting compound is present.

Consecutive 200 micron thick cross sections from a 6 mm cylinder of bovine femur diaphysis are cut with a low speed diamond saw (Isomet, Beuler, Ltd., Lake Bluff, II). Bone slices are pooled, placed in a 10% ethanol solution and refrigerated until further use.

Prior to experimentation, bovine bone slices are ultrasonicated twice, 20 minutes each in H₂O. Cleaned slices are placed in 96 well plates such that two control lanes and one lane for each drug dosage are available. Each lane represents either triplicate or quadruplicate cultures. The bone slices in 96 well plates are sterilized by UV irradiation. Prior to incubation with osteoclasts, the bone slices are hydrated by the addition of 0.1 ml αMEM, pH 6.9 containing 5% fetal bovine serum and 1% penicillin/streptomycin.

Long bones from 7-14 day old rabbits (New Zealand White Hare) are dissected, cleaned of soft tissue and placed in αMEM containing 20 mM HEPES. The bones are minced using scissors until the pieces are <1 mm and transferred to a 50 ml tube in a volume of 25 ml. The tube is rocked gently by hand for 60 cycles, the tissue is sedimented for 1 min., and the supernatant is removed. Another 25 ml of medium is added to the tissue and rocked again. The second supernatant is combined with the first. The number of cells is counted excluding erythrocytes (typically ∼ 2 x 10⁷ cells/ml). A cell suspension consisting of 5 x 10⁶/ml in αMEM containing 5% fetal bovine serum, 10 nM 1,25(OH)₂D₃, and pencillin-streptomycin is prepared. 200 ml aliquots are added to bovine bone slices (200 mm x 6 mm) and incubated for 2 hrs. at 37°C in a humidified 5% CO₂ atmosphere. The medium is removed gently with a micropipettor and fresh medium containing test compounds is added. The cultures are incubated for 48 hrs., and assayed for c-telopeptide (fragments of the a1 chain of type I collagen) by Crosslaps for culture media (Herlev, Denmark).

Bovine bone slices are exposed to osteoclasts for 20-24 hrs and are processed for staining. Tissue culture media is removed from each bone slice. Each well is washed with 200 ml of H₂O, and the bone slices are then fixed for 20 minutes in 2.5% glutaraldehyde, 0.1 M cacodylate, pH 7.4. After fixation, any remaining cellular debris is removed by 2 min. ultrasonication in the presence of 0.25 M NH₄OH followed by 2 X 15 min ultrasonication in H₂O. The bone slices are immediately stained for 6-8 min with filtered 1% toluidine blue and 1% borax.

After the bone slices have dried, resorption pits are counted in test and control slices. Resorption pits are viewed in a Microphot Fx (Nikon) fluorescence microscope using a polarizing Nikon IGS filter cube. Test dosage results are compared with controls and resulting IC₅₀ values are determined for each compound tested.

The appropriateness of extrapolating data from this assay to mammalian (including human) disease states is supported by the teaching found in Sato, M., et al., Journal of Bone and Mineral Research, Vol. 5, No. 1, pp. 31-40, 1990, which is incorporated by reference herein in its entirety. This article teaches that certain bisphosphonates have been used clinically and appear to be effective in the treatment of Paget's disease, hypercalcemia of malignancy, osteolytic lesions produced by bone metastases, and bone loss due to immobilization or sex hormone deficiency. These same bisphosphonates are then tested in the resorption pit assay described above to confirm a correlation between their known utility and positive performance in the assay.

### EIB ASSAY

Duong et al., J. Bone Miner. Res., 8: S378 (1993), describes a system for expressing the human integrin αvβ3. It has been suggested that the integrin stimulates attachment of osteoclasts to bone matrix, since antibodies against the integrin, or RGD-containing molecules, such as echistatin (European Publication 382 451), can effectively block bone resorption.

### Reaction Mixture:

1. 175 µl TBS buffer (50 mM Tris•HCl pH 7.2, 150 mM NaCl, 1% BSA, 1 mM CaCl₂, 1 mM MgCl₂).
2. 25 ml cell extract (dilute with 100 mM octylglucoside buffer to give 2000 cpm/25 µl).
3. ¹²⁵I-echistatin (25 µl/50,000 cpm) (see EP 382 451).
4. 25 µl buffer (total binding) or unlabeled echistatin (non-specific binding).

The reaction mixture was then incubated for 1 h at room temp. The unbound and the bound αvβ3 were separated by filtration using a Skatron Cell Harvester. The filters (prewet in 1.5% polyethyleneimine for 10 mins) were then washed with the wash buffer (50 mM Tris HCl, 1mM CaCl₂/MgCl₂, pH 7.2). The filter was then counted in a gamma counter.

### SPA ASSAY

### MATERIALS:

1. Wheat germ agglutinin Scintillation Proximity Beads (SPA): Amersham
2. Octylglucopyranoside: Calbiochem
3. HEPES: Calbiochem
4. NaCl: Fisher
5. CaCl₂: Fisher
6. MgCl₂: SIGMA
7. Phenylmethylsulfonylfluoride (PMSF): SIGMA
8. Optiplate: PACKARD
9. Compound A-10 (specific activity 500-1000 Ci/mmole)
10. test compound
11. Purified integrin receptor: αvβ3 was purified from 293 cells overexpressing αvβ3 (Duong et al., J. Bone Min. Res., 8:S378, 1993) according to Pytela (Methods in Enzymology, 144:475, 1987)
12. Binding buffer: 50 mM HEPES, pH 7.8, 100 mM NaCl, 1 mM Ca^{2+/}Mg²⁺, 0.5 mM PMSF
13. 50 mM octylglucoside in binding buffer: 50-OG buffer

### PROCEDURE:

1. Pretreatment of SPA beads:
   500 mg of lyophilized SPA beads were first washed four times with 200 ml of 50-OG buffer and once with 100 ml of binding buffer, and then resuspended in 12.5 ml of binding buffer.
2. Preparation of SPA beads and receptor mixture
   In each assay tube, 2.5 µl (40 mg/ml) of pretreated beads were suspended in 97.5 µl of binding buffer and 20 ml of 50-OG buffer. 5 ml (∼30 ng/µl) of purified receptor was added to the beads in suspension with stirring at room temperature for 30 minutes. The mixture was then centrifuged at 2,500 rpm in a Beckman GPR Benchtop centrifuge for 10 minutes at 4°C. The pellets were then resuspended in 50 µl of binding buffer and 25 µl of 50-OG buffer.
3. Reaction
   The following were sequentially added into Optiplate in corresponding wells:
   (i) Receptor/beads mixture (75 µl)
   (ii) 25 µl of each of the following: compound to be tested, binding buffer for total binding or A-8 for non-specific binding (final concentration 1 µM)
   (iii) A-10 in binding buffer (25 µl, final concentration 40 pM)
   (iv) Binding buffer (125 µl)
   (v) Each plate was sealed with plate sealer from PACKARD and incubated overnight with rocking at 4°C
4. Plates were counted using PACKARD TOPCOUNT
5. % inhibition was calculated as follows:
   A = total counts
   B = nonspecific counts
   C = sample counts
   % inhibition = [((A-B)-(C-B)}/(A-B)]/(A-B) x 100

### OCFORM ASSAY

Osteoblast-like cells (1.8 cells), originally derived from mouse calvaria, were plated in CORNING 24 well tissue culture plates in αMEM medium containing ribo- and deoxyribonucleosides, 10% fetal bovine serum and penicillin-streptomycin. Cells were seeded at 40,000/well in the morning. In the afternoon, bone marrow cells were prepared from six week old male Balb/C mice as follows:

Mice were sacrificed, tibiae removed and placed in the above medium. The ends were cut off and the marrow was flushed out of the cavity into a tube with a 1 mL syringe with a 27.5 gauge needle. The marrow was suspended by pipetting up and down. The suspension was passed through >100 mm nylon cell strainer. The resulting suspension was centrifuged at 350 x g for seven minutes. The pellet was resuspended, and a sample was diluted in 2% acetic acid to lyse the red cells. The remaining cells were counted in a hemacytometer. The cells were pelleted and resuspended at 1 x 10⁶ cells/mL. 50 µL was added to each well of 1.8 cells to yield 50,000 cells/well and 1,25-dihydroxy-vitamin D₃ (D₃) was added to each well to a final concentration of 10 nM. The cultures were incubated at 37°C in a humidified, 5% CO₂ atmosphere. After 48 h, the medium was changed. 72 h after the addition of bone marrow, test compounds were added with fresh medium containing D₃ to quadruplicate wells. Compounds were added again after 48 h with fresh medium containing D₃. After an additional 48 h., the medium was removed, cells were fixed with 10% formaldehyde in phosphate buffered saline for 10 minutes at room temperature, followed by a 1-2 minute treatment with ethanol:acetone (1:1) and air dried. The cells were then stained for tartrate resistant acid phosphatase as follows:

The cells were stained for 10-15 minutes at room temperature with 50 mM acetate buffer, pH 5.0 containing 30 mM sodium tartrate, 0.3 mg/mL Fast Red Violet LB Salt and 0.1 mg/mL Naphthol AS -MX phosphate. After staining, the plates were washed extensively with deionized water and air dried. The number of multinucleated, positive staining cells was counted in each well.

### αvβ5 ATTACHMENT ASSAY

Duong et al., J. Bone Miner. Res., 11: S290 (1996), describes a system for expressing the human αvβ5 integrin receptor.

### Materials:

1. Media and solutions used in this assay are purchased from BRL/Gibco, except BSA and the chemicals are from Sigma.
2. Attachment medium: HBSS with 1 mg/ml heat-inactivated fatty acid free BSA and 2 mM CaCl₂.
3. Glucosaminidase substrate solution: 3.75 mM p-nitrophenyl N-acetyl-beta-D-glucosaminide, 0.1 M sodium citrate, 0.25% Triton, pH 5.0.
4. Glycine-EDTA developing solution: 50 mM glycine, 5 mM EDTA, pH 10.5.

### Methods:

1. Plates (96 well, Nunc Maxi Sorp) were coated overnight at 4 °C with human vitronectin (3 µg/ml) in 50 mM carbonate buffer (pH 9/.6), using 100 µl/well. Plates were then washed 2X with DPBS and blocked with 2% BSA in DPBS for 2h at room temperature. After additional washes (2X) with DPBS, plates were used for cell attachment assay.
2. 293 (αvβ5) cells were grown in αMEM media in presence of 10% fetal calf serum to 90% confluence. Cells were then lifted from dishes with 1X Trypsin/EDTA and washed 3X with serum free αMEM. Cells were resuspended in attachment medium (3 X 10⁵ cells/ml).
3. Test compounds were prepared as a series of dilutions at 2X concentrations and added as 50 µl/well. Cell suspension was then added as 50 ml/well. Plates were incubated at 37 °C with 55 CO₂ for 1 hour to allow attachment.
4. Non-adherent cells were removed by gently washing the plates (3X) with DPBS and then incubated with glucosaminidase substrate solution (100 µl/well), overnight at room temperature in the dark. To quantitate cell numbers, standard curve of glucosaminidase activity was determined for each experiment by adding samples of cell suspension directly to wells containing the enzyme substrate solution.
5. The next day, the reaction was developed by addition of 185 µl/well of glycine/EDTA solution and reading absorbance at 405 nm using a Molecular Devices V-Max plate reader. Average test absorbance values (4 wells per test samples) were calculated. Then, the number of attached cells at each drug concentration was quantitated versus the standard curve of cells using the Softmax program.

### EXAMPLE OF A PHARMACEUTICAL FORMULATION

As a specific embodiment of an oral composition, 100 mg of any of the compounds of the present invention are formulated with sufficient finely divided lactose to provide a total amount of 580 to 590 mg to fill a size O hard gel capsule.

The exemplified compounds of the present invention were tested and found to bind to human αvβ3 integrin. These compounds were generally found to have IC₅₀ values less than about 100 nM in the SPA assay.

The exemplified compounds of the present invention were tested and generally found to inhibit ≥ 50% the attachment of αvβ5 expressing cells to plates coated with vitronectin at concentrations of about 1 µM.

While the invention has been described and illustrated in reference to certain preferred embodiments thereof, those skilled in the art will appreciate that various changes, modifications and substitutions can be made therein without departing from the spirit and scope of the invention. For example, effective dosages other than the preferred doses as set forth hereinabove may be applicable as a consequence of variations in the responsiveness of the mammal being treated for severity of bone disorders caused by resorption, or for other indications for the compounds of the invention indicated above. Likewise, the specific pharmacological responses observed may vary according to and depending upon the particular active compound selected or whether there are present pharmaceutical carriers, as well as the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of the formula 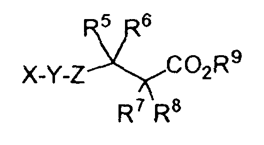 wherein X is 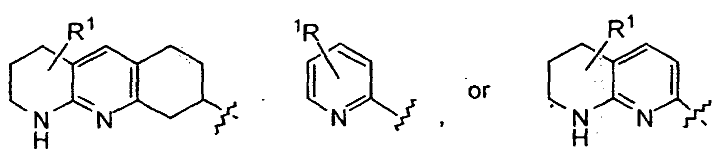 Y is selected from the group consisting of
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH₂)ₘ-O-(CH₂)ₙ-O-(CH₂)ₚ-,
-(CH₂)ₘ-O-(CH₂)ₙ-NR⁴-(CH₂)ₚ-,
-(CH₂)ₘ-NR⁴-(CH₂)ₙ-NR⁴-(CH₂)ₚ-,
-(CH₂)ₘ-O-(CH₂)ₙ-S-(CH₂)ₚ-,
-(CH₂)ₘ-S-(CH₂)ₙ-S-(CH₂)ₚ-,
-(CH₂)ₘ-NR⁴-(CH₂)ₙ-S-(CH₂)ₚ-,
-(CH₂)ₘ-NR⁴-(CH₂)ₙ-O-(CH₂)ₚ-,
-(CH₂)ₘ-S-(CH₂)ₙ-O-(CH₂)ₚ-, and
-(CH₂)ₘ-S-(CH₂)ₙ-NR⁴-(CH₂)ₚ-,
wherein any methylene (CH₂) carbon atom in Y, other than in R⁴, can be substituted by one or two R³ substituents, with the proviso that when Y is -(CH₂)ₘ-NR⁴-(CH₂)ₙ- and n is 1, then the R³ substituent on the methylene carbon in -(CH₂)ₘ- adjacent to the nitrogen cannot be oxo;
Z is selected from the group consisting of 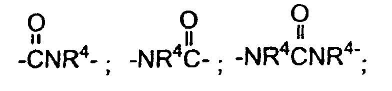 -CH₂CH₂-, and -CH=CH-, wherein either carbon atom can be substituted by one or two R³ substituents;
R¹ is selected from the group consisting of
hydrogen, halogen, C₁₋₁₀ alkyl, C₃₋₈ cycloalkyl, C₃₋₈ cycloheteroalkyl, C₃₋₈ cycloalkyl C₁₋₆ alkyl, C₃₋₈ cycloheteroalkyl C₁₋₆ alkyl, aryl, aryl C₁₋₈ alkyl, amino, amino C₁₋₈ alkyl, C₁₋₃ acylamino, C₁₋₃ acylamino C₁₋₈ alkyl, (C₁₋₆ alkyl)ₚamino, (C₁₋₆ alkyl)ₚamino C₁₋₈ alkyl, C₁₋₄ alkoxy, C₁₋₄ alkoxy C₁₋₆ alkyl, hydroxycarbonyl, hydroxycarbonyl C₁₋₆ alkyl, C₁₋₃ alkoxycarbonyl, C₁₋₃ alkoxycarbonyl C₁₋₆ alkyl, hydroxycarbonyl-C₁₋₆ alkyloxy, hydroxy, hydroxy C₁₋₆ alkyl, C₁₋₆ alkyloxy-C₁₋₆ alkyl, nitro, cyano, trifluoromethyl, trifluoromethoxy, trifluoroethoxy, C₁₋₈ alkyl-S(O)ₚ, (C₁₋₈ alkyl)ₚaminocarbonyl, C₁₋₈ alkyloxycarbonylamino, (C₁₋₈ alkyl)ₚaminocarbonyloxy, (aryl C₁₋₈ alkyl)ₚamino, (aryl)ₚamino, aryl C₁₋₈ alkylsulfonylamino, and C₁₋₈ alkylsulfonylamino;
each R³, R⁵, R⁶, R⁷ and R⁸ is independently selected from the group consisting of
hydrogen,
aryl,
C₁₋₁₀ alkyl,
aryl-(CH₂)ᵣ-O-(CH₂)ₛ-,
aryl-(CH₂)ᵣS(O)ₚ-(CH₂)ₛ-,
aryl-(CH₂)ᵣ-C(O)-(CH₂)ₛ-,
aryl-(CH₂)ᵣ-C(O)-N(R⁴)-(CH₂)ₛ-,
aryl-(CH₂)ᵣ-N(R⁴)-C(O)-(CH₂)ₛ-,
aryl-(CH₂)ᵣ-N(R⁴)-(CH₂)ₛ-,
halogen,
hydroxyl,
C₁₋₈ alkylcarbonylamino,
aryl C₁₋₅ alkoxy,
C₁₋₅ alkoxycarbonyl,
(C₁₋₈ alkyl)ₚaminocarbonyl,
C₁₋₆ alkylcarbonyloxy,
C₃₋₈ cycloalkyl,
(C₁₋₆ alkyl)ₚamino,
arylaminocarbonyl,
aryl C₁₋₅ alkylaminocarbonyl,
aminocarbonyl,
aminocarbonyl C₁₋₆ alkyl,
hydroxycarbonyl,
hydroxycarbonyl C₁₋₆ alkyl,
HC≡C-(CH₂)ₜ-,
C₁₋₆ alkyl-C≡C-(CH₂)ₜ-,
C₃₋₇ cycloalkyl-C≡C-(CH₂)ₜ-,
aryl-C≡C-(CH₂)ₜ-,
C₁₋₆ alkylaryl-C≡C-(CH₂)ₜ-,
CH₂=CH-(CH₂)ₜ-,
C₁₋₆ alkyl-CH=CH-(CH₂)ₜ-,
C₃₋₇ cycloalkyl-CH=CH-(CH₂)ₜ-,
aryl-CH=CH-(CH₂)ₜ-,
C₁₋₆ alkylaryl-CH=CH-(CH₂)ₜ-,
C₁₋₆ alkyl-SO₂-(CH₂)ₜ-,
C₁₋₆ alkylaryl-SO₂-(CH₂)ₜ-,
C₁₋₆ alkoxy,
aryl C₁₋₆ alkoxy,
aryl C₁₋₆ alkyl,
(C₁₋₆ alkyl)ₚamino C₁₋₆ alkyl,
(aryl)ₚamino,
(aryl)ₚamino C₁₋₆ alkyl,
(aryl C₁₋₆ alkyl)ₚamino,
(aryl C₁₋₆ alkyl)ₚamino C₁₋₆ alkyl,
arylcarbonyloxy,
aryl C₁₋₆ alkylcarbonyloxy,
(C₁₋₆ alkyl)ₚaminocarbonyloxy,
C₁₋₈ alkylsulfonylamino,
arylsulfonylamino,
C₁₋₈ alkylsulfonylamino C₁₋₆ alkyl,
aryl C₁₋₆ alkylsulfonylamino,
aryl C₁₋₆ alkylsulfonylamino C₁₋₆ alkyl,
C₁₋₈ alkoxycarbonylamino,
C₁₋₈ alkoxycarbonylamino C₁₋₈ alkyl,
aryloxycarbonylamino C₁₋₈ alkyl,
aryl C₁₋₈ alkoxycarbonylamino,
aryl C₁₋₈ alkoxycarbonylamino C₁₋₈ alkyl,
C₁₋₈ alkylcarbonylamino C₁₋₆ alkyl,
arylcarbonylamino C₁₋₆ alkyl,
aryl C₁₋₆ alkylcarbonylamino,
aryl C₁₋₆ alkylcarbonylamino C₁₋₆ alkyl,
aminocarbonylamino C₁₋₆ alkyl,
(C₁₋₈ alkyl)ₚaminocarbonylamino,
(C₁₋₈ alkyl)ₚaminocarbonylamino C₁₋₆ alkyl,
(aryl)ₚaminocarbonylamino C₁₋₆ alkyl,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylsulfonyl,
C₁₋₆ alkylsulfonyl C₁₋₆ alkyl,
arylsulfonyl C₁₋₆ alkyl,
aryl C₁₋₆ alkylsulfonyl,
aryl C₁₋₆ alkylsulfonyl C₁₋₆ alkyl,
C₁₋₆ alkylcarbonyl,
C₁₋₆ alkylcarbonyl C₁₋₆ alkyl,
arylcarbonyl C₁₋₆ alkyl,
aryl C₁₋₆ alkylcarbonyl,
aryl C₁₋₆ alkylcarbonyl C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino,
aryl C₁₋₆ alkylthiocarbonylamino C₁₋₆ alkyl,
(C₁₋₈ alkyl)ₚaminocarbonyl C₁₋₆ alkyl,
(aryl)ₚaminocarbonyl C₁₋₆ alkyl,
(aryl C₁₋₈ alkyl)ₚaminocarbonyl, and
(aryl C₁₋₈ alkyl)ₚaminocarbonyl C₁₋₆ alkyl;
or R³ is oxo, trifluoromethyl or C₁₋₈ alkylcarbonylamino;
or R⁵ and R⁶ are independently amino C₁₋₆ alkyl, arylsulfonylamino C₁₋₆ alkyl, or C₁₋₈ alkylcarbonylamino;
or R⁷ and R⁸ are independently amino C₁₋₆ alkyl, arylcarbonylamino, arylsulfonylamino C₁₋₆ alkyl, arylaminocarbonylamino or C₇₋₂₀ polycyclyl C₀₋₈ alkylsulfonylamino;
or two R³ substituents, when on the same carbon atom are taken together with the carbon atom to which they are attached to form a carbonyl group or a cyclopropyl group,
or R⁵ and R⁶ when on the same carbon atom are taken together with the carbon atom to which they are attached to form a carbonyl group,
wherein any of the alkyl groups of R³, R⁵, R⁶, R⁷ and R⁸ are either unsubstituted or substituted with one to three R¹ substituents, and provided that each R³, each R⁵ and R⁶, and each R⁷ and R⁸, are selected such that in the resultant compound the carbon atom or atoms to which R³, R⁵ and R⁶, and R⁷ and R⁸, are attached are themselves attached to no more than one heteroatom;
each R⁴ is independently selected from the group consisting of
hydrogen,
aryl,
aminocarbonyl,
C₃₋₈ cycloalkyl,
amino C₁₋₆ alkyl,
(aryl)ₚaminocarbonyl,
(aryl C₁₋₅ alkyl)ₚaminocarbonyl,
hydroxycarbonyl C₁₋₆ alkyl,
C₁₋₈ alkyl,
aryl C₁₋₆ alkyl,
(C₁₋₆ alkyl)ₚamino C₂₋₆ alkyl,
(aryl C₁₋₆ alkyl)ₚamino C₂₋₆ alkyl,
C₁₋₈ alkylsulfonyl,
C₁₋₈ alkoxycarbonyl,
aryloxycarbonyl,
aryl C₁₋₈ alkoxycarbonyl,
C₁₋₈ alkylcarbonyl,
arylcarbonyl,
aryl C₁₋₆ alkylcarbonyl,
(C₁₋₈ alkyl)ₚaminocarbonyl,
aminosulfonyl,
C₁₋₈ alkylaminosulfonyl,
(aryl)ₚaminosulfonyl,
(aryl C₁₋₈ alkyl)ₚaminosulfonyl,
arylsulfonyl,
arylC1-6 alkylsulfonyl,
C₁₋₆ alkylthiocarbonyl,
arylthiocarbonyl, and
aryl C₁₋₆ alkylthiocarbonyl,
wherein any of the alkyl groups of R⁴ are either unsubstituted or substituted with one to three R¹ substituents;
R⁹ is selected from the group consisting of
hydrogen,
C₁₋₈ alkyl,
aryl,
aryl C₁₋₈ alkyl,
C₁₋₈ alkylcarbonyloxy C₁₋₄ alkyl,
aryl C₁₋₈ alkylcarbonyloxy C₁₋₄ alkyl,
C₁₋₈ alkylaminocarbonylmethylene, and
C₁₋₈ dialkylaminocarbonylmethylene;
wherein
each aryl as a group or part of a group is independently selected from the group consisting of: phenyl, naphthyl, pyridyl, pyrryl, pyrazolyl, pyrazinyl, pyrimidinyl, imidazolyl, benzimidazolyl, benzthiazolyl, benzoxazolyl, indolyl, thienyl, furyl, dihydrobenzofuryl, benzo(1,3) dioxolane, oxazolyl, isoxazolyl and thiazolyl;
each m is independently an integer from 2 to 4;
each n is independently an integer from 0 to 6;
each p is independently an integer from 0 to 2;
each r is independently an integer from 1 to 3;
each s is independently an integer from 0 to 3; and
each t is independently an integer from 0 to 3;
and the pharmaceutically acceptable salts thereof.

2. The compound of Claim 1 wherein Y is selected from the group consisting of
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH2)m-O-(CH2)n-O-(CH2)p-,
-(CH2)m-O-(CH2)n-N-R⁴-(CH2)p-,
-(CH2)m-NR⁴-(CH2)n-NR⁴-(CH2)p-, and
-(CH2)m-NR⁴-(CH2)n-O-(CH2)p-,
wherein any methylene (CH₂) carbon atom in Y, other than in R⁴, can be substituted by one or two R³ substituents;
with the proviso that when Y is -(CH₂)ₘ-NR⁴-(CH₂)ₙ- and n is 1, then the R³ substituent on the methylene carbon in -(CH₂)ₘ- adjacent to the nitrogen cannot be oxo;
and Z is selected from the group consisting of 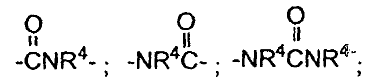 -CH₂CH₂-, and -CH=CH-, wherein either carbon atom can be substituted by one or two R³ substituents.

3. The compound of Claim 2 wherein
Y is selected from the group consisting of
(CH₂)ₘ, (CH₂)ₘ-S-(CH₂)ₙ, and (CH₂)ₘ-NR⁴-(CH₂)ₙ,
wherein any methylene (CH₂) carbon atom in Y, other than in R⁴, can be substituted by one or two R³ substituents;
with the proviso that when Y is -(CH₂)ₘ-NR⁴-(CH₂)ₙ- and n is 1, then the R³ substituent on the methylene carbon in -(CH₂)ₘ- adjacent to the nitrogen cannot be oxo;
and Z is selected from the group consisting of 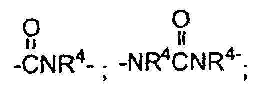 and
-CH₂CH₂-, wherein either carbon atom can be substituted by one or two R³ substituents.

4. The compound of Claim 3 wherein each R³ is independently selected from the group consisting of
hydrogen,
fluoro,
trifluoromethyl,
aryl,
C₁₋₈ alkyl,
arylC₁₋₆ alkyl
hydroxyl,
oxo,
arylaminocarbonyl,
aryl C₁₋₅ alkylaminocarbonyl,
aminocarbonyl, and
aminocarbonyl C₁₋₆ alkyl;
and each R⁴ is independently selected from the group consisting of
hydrogen,
aryl,
C₃₋₈ cycloalkyl,
C₁₋₈ alkyl,
C₁₋₈ alkylcarbonyl,
arylcarbonyl,
C₁₋₆ alkylsulfonyl,
arylsulfonyl,
arylC₁₋₆alkylsulfonyl,
arylC₁₋₆alkylcarbonyl,
C₁₋₈alkylaminocarbonyl,
arylC₁₋₅alkylaminocarbonyl,
arylC₁₋₈alkoxycarbonyl, and
C₁₋₈alkoxycarbonyl.

5. The compound of Claim 4 wherein R⁶, R⁷, and R⁸ are each hydrogen and R⁵ is selected from the group consisting of
hydrogen,
aryl,
C₁₋₈ alkyl,
aryl-C≡C-(CH₂)ₜ-,
aryl C₁₋₆ alkyl,
CH₂=CH-(CH₂)ₜ-, and
HC≡C-(CH₂)ₜ-.

6. The compound of Claim 5 wherein R⁹ is selected from the group consisting of hydrogen, methyl, and ethyl.

7. The compound of Claim 6 wherein R⁹ is hydrogen.

8. The compound of Claim 4 wherein R⁵, R⁶, and R⁸ are each hydrogen and R⁷ is selected from the group consisting of
hydrogen,
aryl,
C₁₋₈ alkylcarbonylamino,
C₁₋₈ alkylsulfonylamino,
arylcarbonylamino,
arylsulfonylamino,
C₁₋₈ alkylsulfonylamino C₁₋₆ alkyl,
arylsulfonylamino C₁₋₆ alkyl,
aryl C₁₋₆ alkylsulfonylamino,
aryl C₁₋₆ alkylsulfonylamino C₁₋₆ alkyl,
C₁₋₈ alkoxycarbonylamino,
C₁₋₈ alkoxycarbonylamino C₁₋₈ alkyl,
aryloxycarbonylamino C₁₋₈ alkyl,
aryl C₁₋₈ alkoxycarbonylamino,
aryl C₁₋₈ alkoxycarbonylamino C₁₋₈ alkyl,
C₁₋₈ alkylcarbonylamino C₁₋₆ alkyl,
arylcarbonylamino C₁₋₆ alkyl,
aryl C₁₋₆ alkylcarbonylamino,
aryl C₁₋₆ alkylcarbonylamino C₁₋₆ alkyl,
aminocarbonylamino C₁₋₆ alkyl,
(C₁₋₈ alkyl)paminocarbonylamino,
(C₁₋₈ alkyl)paminocarbonylamino C₁₋₆ alkyl,
(aryl)ₚaminocarbonylamino C₁₋₆ alkyl,
arylaminocarbonylamino,
(aryl C₁₋₈ alkyl)ₚaminocarbonylamino,
(aryl C₁₋₈ alkyl)ₚaminocarbonylamino C₁₋₆ alkyl,
aminosulfonylamino C₁₋₆ alkyl,
(C₁₋₈ alkyl)ₚaminosulfonylamino,
(C₁₋₈ alkyl)ₚaminosulfonylamino C₁₋₆ alkyl,
(aryl)ₚaminosulfonylamino C₁₋₆ alkyl,
(aryl C₁₋₈ alkyl)ₚaminosulfonylamino,
(aryl C₁₋₈ alkyl)ₚaminosulfonylamino C₁₋₆ alkyl,
C₁₋₆ alkylthiocarbonylamino,
C₁₋₆ alkylthiocarbonylamino C₁₋₆ alkyl,
arylthiocarbonylamino C₁₋₆ alkyl,
aryl C₁₋₆ alkylthiocarbonylamino,
aryl C₁₋₆ alkylthiocarbonylamino C₁₋₆ alkyl, and
C₇₋₂₀ polycyclyl C₀₋₈ alkylsulfonylamino.

9. The compound of Claim 8 wherein R⁵, R⁶, and R⁸ are each hydrogen and R⁷ is selected from the group consisting of
hydrogen,
aryl,
C₁₋₈ alkylcarbonylamino,
aryl C₁₋₆ alkylcarbonylamino,
arylcarbonylamino,
C₁₋₈ alkylsulfonylamino,
aryl C₁₋₆ alkylsulfonylamino,
arylsulfonylamino,
C₁₋₈ alkoxycarbonylamino,
aryl C₁₋₈ alkoxycarbonylamino,
arylaminocarbonylamino,
(C₁₋₈ alkyl)ₚaminocarbonylamino,
(aryl C₁₋₈ alkyl)ₚaminocarbonylamino,
(C₁₋₈ alkyl)ₚaminosulfonylamino, and
(aryl C₁₋₈ alkyl)ₚaminosulfonylamino.

10. The compound according to Claim 9 wherein R⁹ is selected from the group consisting of hydrogen, methyl, and ethyl.

11. The compound according to Claim 10 wherein R⁹ is hydrogen.

12. The compound of Claim 4 selected from the group consisting of:
3-(5-(5,6,7,8-Tetrahydro[1,8]naphthyridin-2-yl)-pentanoylamino)-propionic acid;
3(S)-(Pyridin-3-yl)-3-(5-(5,6,7,8-tetrahydro[1,8]naphthyridin-2-yl)-pentanoylamino)-propionic acid;
3(S)-(5,6,7,8-Tetrahydroquinolin-3-yl)-3-(5-(5,6,7,8-tetrahydro[1,8]naphthyridin-2-yl)-pentanoylamino)-propionic acid (trifluoroacetate);
2(S)-Benzenesulfonylamino-3-(5-(5,6,7,8-tetrahydro-[1,8]naphthyridin-2-yl)-pentanoylamino)-propionic acid trifluoroacetate;
3(S)-(Quinolin-3-yl)-3-(5-(5,6,7,8-tetrahydro-[1,8]naphthyridin-2-yl)-pentanoylamino)-propionic acid;
3(R)-(Quinolin-3-yl)-3-(5-(5,6,7,8-tetrahydro-[1,8]naphthyridin-2-yl)-pentanoylamino)-propionic acid;
3-(Quinolin-3-yl)-3-(7-(5,6,7,8-tetrahydro-[1,8]naphthyridin-2-yl)-heptanoylamino)-propionic acid bis(trifluoroacetate);
3-(Quinolin-3-yl)-3-(6-(5,6,7,8-tetrahydro-[1,8]naphthyridin-2-yl)-hexanoylamino)-propionic acid;
3(S)-(3-Fluorophenyl)-3-(4-(5,6,7,8-tetrahydro-[1,8]naphthyridin-2-ylamino)-butyrylamino)-propionic acid bis(trifluoroacetate);
3(S)-(5-(5,6,7,8-Tetrahydro-[1,8]naphthyridin-2-yl)-pentanoylamino)-pent-4-enoic acid;
3(S)-(3-Fluorophenyl)-3-(5-(5,6,7,8-tetrahydro-[1,8]naphthyridin-2-yl)-pentanoylamino)-propionic acid;
2-(3-Fluorophenyl)-3-(5-(5,6,7,8-tetrahydro-[1,8]naphthyridin-2-yl)-pentanoylamino)-propionic acid trifluoroacetate salt;
3(S)-(Benzo[1,3]dioxol-5-yl)-3-(5-(5,6,7,8-tetrahydro-[1,8]naphthyridin-2-yl)-pentanoylamino)-propionic acid;
3(S)-(2,3-Dihydro-benzofuran-6-yl)-3-(5-(5,6,7,8-tetrahydro-[1,8]naphthyridin-2-yl)-pentanoylamino)-propionic acid;
3(S)-(2-Oxo-2,3-dihydro-benzoxazol-6-yl)-3-(5-(5,6,7,8-tetrahydro-[1,8]naphthyridin-2-yl)-pentanoylamino)-propionic acid trifluoroacetate;
3(S)-(3-Fluorophenyl)-3-{3-[(5,6,7,8-tetrahydro-[1,8]naphthyridin-2-ylmethyl)-amino]-propionylamino}-propionic acid;
3(S)-(3-Fluorophenyl)-3-(2-{propyl-[2-(5,6,7,8-tetrahydro-[1,8]naphthyridin-2-yl)-ethyl]-amino}-acetylamino)-propionic acid trifluoroacetate;
3(S)-(3-Fluorophenyl)-3-(2-{phenethyl-[2-(5,6,7,8-tetrahydro-[1,8]naphthyridin-2-yl)-ethyl]-amino}-acetylamino)-propionic acid trifluoroacetate;
3(S)-(3-Fluorophenyl)-3-{3(S)-[(5,6,7,8-tetrahydro-[1,8]naphthyridin-2-ylmethyl)-amino)-pent-4-ynoylamino}-propionic acid;
3(S)-(3-Fluorophenyl)-3-{3(S)-(3-fluorophenyl)-3-[(5,6,7,8-tetrahydro-[1,8]naphthyridin-2-ylmethyl)-amino]-propionylamino}-propionic acid bis(trifluoroacetate);
3(S)-(3-Fluoro-4-phenyl-phenyl)-3-(5-(5,6,7,8-tetrahydro-[1,8]naphthyridin-2-yl)-pentanoylamino)-propionic acid trifluoroacetate;
2(S)-(2-Thienylsulfonylamino)-3-(5-(5,6,7,8-tetrahydro-[1,8]naphthyridin-2-yl)-pentanoylamino)-propionic acid trifluoroacetate;
3(S)-(3-Fluorophenyl)-3-{3-methyl-3-[(5,6,7,8-tetrahydro-[1,8]naphthyridin-2-ylmethyl)-amino]-propionylamino}-propionic acid;
3(S)-(3-Fluorophenyl)-3-{2-[2-(5,6,7,8-tetrahydro-[1,8]naphthyridin-2-yl)-ethylamino]-acetylamino}-propionic acid;
3(S)-(3-Fluorophenyl)-3{[3-(5,6,7,8-tetrahydro-[1,8]naphthyridin-2-yl)-propyl]-ureido}-propionic acid;
2(S)-(Methanesulfonylamino)-3-(5-(5,6,7,8-tetrahydro-[1,8]naphthyridin-2-yl)-pentanoylamino)-propionic acid;
3(S)-(2,3-Dihydro-benzofuran-6-yl)-3-[3-(1,2,3,4,6,7,8(R, or S),9-octahydro-benzo[*b*][1,8]naphthyridin-8-yl)-propionylamino]-propionic acid;
3(S)-(2,3-Dihydro-benzofuran-6-yl)-3-[3-(1,2,3,4,6,7,8(S or R),9-octahydro-benzo[*b*][1,8]naphthyridin-8-yl)-propionylamino]-propionic acid;
3(S)-(6-Methoxy-pyridin-3-yl)-3-[N-methyl-3-(1,2,3,4,6,7,8,9-octahydro-benzo[b][1,8]naphthyridin-8-yl-propionyl)-amino]propionic acid;
3(S)-(2,3-Dihydro-benzofuran-6-yl)-3-[3-(5,6,7,8-tetrahydro-[1,8]-naphthyridin-2-ylmethylsulfanyl)propionylamino]-propionic acid bis(trifluoroacetate);
3-(Quinolin-3-yl)-7-[(5,6,7,8-tetrahydro-[1,8]-naphthyridin-2-ylmethyl)amino]-heptanoic acid;
3-(Quinolin-3-yl)-7-[acetyl-(5,6,7,8-tetrahydro-[1,8]-naphthyridin-2-ylmethyl)amino]-heptanoic acid;
3-(Quinolin-3-yl)-7-[methanesulfonyl-(5,6,7,8-tetrahydro-[1,8]-naphthyridin-2-ylmethyl)amino]-heptanoic acid;
9-(5,6,7,8-Tetrahydro-[1,8]-naphthyridin-2-yl)-nonanoic acid;
2-(Benzenesulfonylamino)-9-(5,6,7,8-tetrahydro-[1,8]-naphthyridin-2-yl)-non-4-enoic acid bis(trifluoroacetate);
2(S)-(Benzenesulfonylamino)-9-(5,6,7,8-tetrahydro-[1,8]naphthyridin-2-yl)-nonanoic acid;
2(R)-(Benzenesulfonylamino)-9-(5,6,7,8-tetrahydro-[1,8]naphthyridin-2-yl)-nonanoic acid;
2(S)-(Banzenesulfonylamino)-10-(5,6,7,8-tetrahydro-[1,8]naphthyridin-2-yl)-decanoic acid;
2(S)-(Benzenesulfonylamino)-8-(5,6,7,8-tetrahydro-[1,8]naphthyridin-2-yl)-octanoic acid;
2(S)-(Cyclohexylmethanesulfonylamino)-9-(5,6,7,8-tetrahydro-[1,8]naphthyridin-2-yl)-nonanoic acid hydrochloride;
2(S)-(7,7-Dimethyl-2-oxo-bicyclo[2.2.1]hept-1(S)-ylmethanesulfonylamino)-9-(5,6,7,8-tetrahydro-[1,8]naphthyridin-2-yl)-nonanoic acid hydrochloride;
2(S)-(Phenylmethanesulfonylamino)-9-(5,6,7,8-tetrahydro-[1,8]naphthyridin-2-yl)-nonanoic acid;
2(S)-(Cyclohexanesulfonylamino)-9-(5,6,7,8-tetrahydro-[L,BJnaphthyridin-2-yl)-nonanoic acid hydrochloride;
2(S)-(Butane-1-sulfonylamino)-9-(5,6,7,8-tetrahydro-[1,8]naphthyridin-2-yl)-nonanoic acid hydrochloride;
2(S)-(3-Benzylureido)-9-(5,6,7,8-tetrahydro-[1,8]naphthyridin-2-yl)-nonanoic acid;
2(S)-(Benzyloxycarbonylamino)-9-(5,6,7,8-tetrahydro-[1,8]naphthyridin-2-yl)-nonanoic acid;
2(S)-(Phenylacetylamino)-9-(5,6,7,8-tetrahydro-[1,8]naphthyrictin-2-yl)-nonanoic acid;
2(S)-(Acetylamino)-9-(5,6,7,8-tetrahydro-[1,8]naphthyridin-2-yl)-nonanoic acid;
2(S)-(Benzoylamino)-9-(5,6,7,8-tetrahydro-[1,8]naphthyridin-2-yl)-nonanoic acid;
3-(Quinolin-3-yl)-9-(5,6,7,8-tetrahydro-[1,8]-naphthyridin-2-yl)-nonanoic acid;
3(S)-(Quinolin-3-yl)-9-(5,6,7,8-tetrahydro-[1,8]-naphthyridin-2-yl)-nonanoic acid;
3(R)-(Quinolin-3-yl)-9-(5,6,7,8-tetrahydro-[1,8]-naphthyridin-2-yl)-nonanoic acid;
3-(Quinolin-3-yl)-7-(1,2,3,4,6,7,8,9-octahydro-benzo[b][1,8]-naphthyridin-8-yl)-heptanoic acid bis(hydrochloride);
6-Oxo-3-(quinolin-3-yl)-9-(5,6,7,8-tetrahydro-[1,8]-naphthyridin-2-yl)-nonanoic acid;
3-(N-Oxo-quinolin-3-yl)-9-(5,6,7,8-tetrahydro-[1,8]-naphthyridin-2-yl)-nonanoic acid;
3-(Phenyl)-9-(5,6,7,8-tetrahydro-[1,8]-naphthyridin-2-yl)-nonanoic acid;
3-(Benzo[b]thiophen-2-yl)-9-(5,6,7,8-tetrahydro-[1,8]-naphthyridin-2-yl)-nonanoic acid;
3(R)-(Benzo[b]thiophen-2-yl)-9-(5,6,7,8-tetrahydro-[1,8]-naphthyridin-2-yl)-nonanoic acid;
3(S)-(Benzo[b]thiophen-2-yl)-9-(5,6,7,8-tetrahydro-[1,8]-naphthyridin-2-yl)-nonanoic acid;
3-(Pyridin-3-yl)-9-(5,6,7,8-tetrahydro-[1,8]-naphthyridin-2-yl)-nonanoic acid;
3(R)-(Pyridin-3-yl)-9-(5,6, 7,8-tetrahydro-[1,8]-naphthyridin-2-yl)-nonanoic acid;
3(S)-(Pyridin-3-yl)-9-(5,6,7,8-tetrahydro-[1,8]-naphthyridin-2-yl)-nonanoic acid;
3-(3-Fluorophenyl)-9-(5,6,7,8-tetrahydro-[1,8]-naphthyridin-2-yl)-nonanoic acid;
3(R)-(3-Fluorophenyl)-9-(5,6,7,8-tetrahydro-[1,8]-naphthyridin-2-yl)-nonanoic acid;
3(S)-(3-Fluorophenyl)-9-(5,6,7,8-tetrahydro-[1,8]-naphthyridin-2-yl)-nonanoic acid;
3-(2,3-Dihydro-benzofuran-6-yl)-9-(5,6,7,8-tetrahydro-[1,8]-naphthyridin-2-yl)-nonanoic acid;
3(S)-(2,3-Dihydro-benzofuran-6-yl)-9-(5,6,7,8-tetrahydro[1,8]-naphthyridin-2-yl)-nonanoic acid;
3(R)-(2,3-Dihydro-benzofuran-6-yl)-9-(5,6,7,8-tetrahydro-[1,8]-naphthyridin-2-yl)-nonanoic acid;
3-(2,3-Dihydro-benzofuran-6-yl)-9-(5,6,7,8-tetrahydro-[1,8]-naphthyridin-2-yl)-non-4-enoic acid trifluoroacetate;
3-(2,3-Dihydro-furo[3,2-b]pyridin-5-yl)-9-(5,6,7,8-tetrahydro-[1.8] naphthyridin-2-yl)-nonanoic acid;
3(R)-(2,3-Dihydro-furo[3,2-b]pyridin-5-yl)-9-(5,6,7,8-tetrahydro-[1,8] naphthyridin-2-yl)-nonanoic acid;
3(S)-(2,3-Dihydro-furo[3,2-b]pyridin-5-yl)-9-(5,6,7,8-tetrahydro-[1,8] naphthyridin-2-yl)-nonanoic acid;
3-(Furo[2,3b]pyridin-5-yl)-9-(5,6,7,8-tetrahydro-[1,8]naphthyridin-2-yl)-nonanoic acid;
3(R)-(Furo[2,3b]pyridin-5-yl)-9-(5,6,7,8-tetrahydro-[1,8]naphthyridin-2-yl)-nonanoic acid;
3(S)-(Furo[2,3b]pyridin-5-yl)-9-(5,6,7,8-tetrahydro-[1,8]naphthyridin-2-yl)-nonanoic acid;
3-(2,3-Dihydro-furo[2,3-b]pyriclin-5-yl)-9-(5,6,7,8-tetrahydro-[1,8] naphthyridin-2-yl)-nonanoic acid;
3(R)-(2,3-Dihydro-furo[2,3-b]pyridin-5-yl)-9-(5,6,7,8-tetrahydro-[1,8] naphthyridin-2-yl)-nonanoic acid;
3(S)-(2,3-Dihydro-furo[2,3-b]pyridin-5-yl)-9-(5,6,7,8-tetrahydro-[1,8] naphthyridin-2-yl)-nonanoic acid;
3-(6-Methoxy-pyridin-3-yl)-9-(5,6,7,8-tetrahydro-[1,8]-naphthyridin-2-yl)-nonanoic acid;
3(S)-(6-Methoxy-pyridin-3-yl)-9-(5,6,7,8-tetrahydro-[1,8]-naphthyridin-2-yl)-nonanoic acid;
3(R)-(6-Methoxy-pyridin-3-yl)-9-(5,6,7,8-tetrahydro-[1,8]-naphthyridin-2-yl)-nonanoic acid;
3-(Pyrimidin-5-yl)-9-(5,6,7,8-tetrahydro-[1,8]-naphthyridin-2-yl)-nonanoic acid trifluoroacetate;
3(R)-(Pyrimidin-5-yl)-9-(5,6,7,8-tetrahydro-[1,8]-naphthyridin-2-yl)-nonanoic acid trifluoroacetate;
3(S)-(Pyrimidin-5-yl)-9-(5,6,7,8-tetrahydro-[1,8]-naphthyridin-2-yl)-nonanoic acid trifluoroacetate;
3-(6-Amino-pyridin-3-yl)-9-(5,6,7,8-tetrahydro-[1,8]-naphthyridin-2-yl)-nonanoic acid;
3(R)-(6-Amino-pyridin-3-yl)-9-(5,6,7,8-tetrahydro-[1,8]-naphthyridin-2-yl)-nonanoic acid;
3(S)-(6-Amino-pyridin-3-yl)-9-(5,6,7,8-tetrahydro-[1,8]-naphthyridin-2-yl)-nonanoic acid;
3-(Benzo[b]thiazol-2-yl)-9-(5,6,7,8-tetrahydro-[1,8]-naphthyridin-2-yl)-nonanoic acid hydrochloride;
3(R)-(Benzo[b]thiazol-2-yl)-9-(5,6,7,8-tetrahydro-[1,8]-naphthyridin-2-yl)-nonanoic acid hydrochloride;
3(S)-(Benzo[b]thiazol-2-yl)-9-(5,6,7,8-tetrahydro-[1,8]-naphthyridin-2-yl)-nonanoic acid hydrochloride;
3-(6-Oxo-1,6-dihydro-pyridin-3-yl)-9-(5,6,7,8-tetrahydro-[1,8]-naphthyridin-2-yl)-nonanoic acid bis-(trifluoroacetate);
and the pharmaceutically acceptable salts thereof.

13. The compound of Claim 12 selected from the group consisting of:
3(S)-(Pyridin-3-yl)-3-(5-(5,6,7,8-tetrahydro[1,8]naphthyridin-2-yl)-pentanoylamino)-propionic acid;
2(S)-Benzenesulfonylamino-3-(5-(5,6,7,8-tetrahydro-[1,8]naphthyridin-2-yl)-pentanoylamino)-propionic acid trifluoroacetate;
3(S)-(Quinolin-3-yl)-3-(5-(5,6,7,8-tetrahydro-[1,8]naphthyridin-2-yl)-pentanoylamino)-propionic acid;
3(R)-(Quinolin-3-yl)-3-(5-(5,6,7,8-tetrahydro-[1,8]naphthyridin-2-yl)-pentanoylamino)-propionic acid;
2(S)-(2-Thienylsulfonylamino)-3-(5-(5,6,7,8-tetrahydro-[1,8]naphthyridin-2-yl)-pentanoylamino)-propionic acid trifluoroacetate;
3(S)-(2,3-Dihydro-benzofuran-6-yl)-3-[3-(1,2,3,4,6,7,8(R or S),9-octahydro-benzo[*b*][1,8]naphthyridin-8-yl)-propionylamino]-propionic acid;
3(S)-(2,3-Dihydro-benzofuran-6-yl)-3-[3-(1,2,3,4,6,7,8(S or R),9-octahydro-benzo[*b*][1,8]naphthyridin-8-yl)-propionylamino]-propionic acid;
3(S)-(6-Methoxy-pyridin-3-yl)-3-[N-methyl-3-(1,2,3,4,6,7,8,9-octahydro-benzo[b][1,8]naphthyridin-8-yl-propionyl)-amino]propionic acid;
2-(Benzenesulfonylamino)-9-(5,6,7,8-tetrahydro-[1,8]-naphthyridin-2-yl)-non-4-enoic acid bis(trifluoroacetate);
and the pharmaceutically acceptable salts thereof.

14. The compound of Claim 12 selected from the group consisting of:
3(R)-(Quinolin-3-yl)-9-(5,6,7,8-tetrahydro-[1,8]-naphthyridin-2-yl)-nonanoic acid;
3(S)-(Quinolin-3-yl)-9-(5,6,7,8-tetrahydro-[1,8]-naphthyridin-2-yl)-nonanoic acid;
3(R)-(Benzo[b]thiophen-2-yl)-9-(5,6,7,8-tetrahydro-[1,8]-naphthyridin-2-yl)-nonanoic acid;
3(S)-(Benzo[b]thiophen-2-yl)-9-(5,6,7,8-tetrahydro-[1,8]-naphthyridin-2-yl)-nonanoic acid;
3(R)-(Pyridin-3-yl)-9-(5,6,7,8-tetrahydro-[1,8]-naphthyridin-2-yl)-nonanoic acid;
3(S)-(Pyridin-3-yl)-9-(5,6,7,8-tetrahydro-[1,8]-naphthyridin-2-yl)-nonanoic acid;
3(R)-(3-Fluorophenyl)-9-(5,6,7,8-tetrahydro-[1,8]-naphthyridin-2-yl)-nonanoic acid;
3(S)-(3-Fluorophenyl)-9-(5,6,7,8-tetrahydro-[1,8]-naphthyridin-2-yl)-nonanoic acid;
3(R)-(2,3-Dihydro-benzofuran-6-yl)-9-(5,6,7,8-tetrahydro-[1,8]-naphthyridin-2-yl)-nonanoic acid;
3(S)-(2,3-Dihydro-benzofuran-6-yl)-9-(5,6,7,8-tetrahydro-[1,8]-naphthyridin-2-yl)-nonanoic acid;
3(R)-(2,3-Dihydro-furo[3,2-b]pyridin-5-yl)-9-(5,6,7,8-tetrahydro-[1,8] naphthyridin-2-yl)-nonanoic acid;
3(S)-(2,3-Dihydro-furo[3,2-b]pyridin-5-yl)-9-(5,6,7,8-tetrahydro-[1,8] naphthyridin-2-yl)-nonanoic acid;
3(R)-(Furo[2,3b]pyridin-5-yl)-9-(5,6,7,8-tetrahydro-[1,8]naphthyridin-2-yl)-nonanoic acid;
3(S)-(Furo[2,3b]pyridin-5-yl)-9-(5,6,7,8-tetrahydro-[1,8]naphthyridin-2-yl)-nonanoic acid;
3(R)-(2,3-Dihydro-furo[2,3-b]pyridin-5-yl)-9-(5,6,7,8-tetrahydro-[1,8] naphthyridin-2-yl)-nonanoic acid;
3(S)-(2,3-Dihydro-furo[2,3-b]pyridin-5-yl)-9-(5,6,7,8-tetrahydro-[1,8] naphthyridin-2-yl)-nonanoic acid;
3(R)-(6-Methoxy-pyridin-3-yl)-9-(5,6,7,8-tetrahydro-[1,8]-naphthyridin-2-yl)-nonanoic acid;
3(S)-(6-Methoxy-pyridin-3-yl)-9-(5,6,7,8-tetrahydro-[1,8]-naphthyridin-2-yl)-nonanoic acid;
3(R)-(Pyrimidin-5-yl)-9-(5,6,7,8-tetrahydro-[1,8]-naphthyridin-2-yl)-nonanoic acid;
3(S)-(Pyrimidin-5-yl)-9-(5,6,7,8-tetrahydro-[1,8]-naphthyridin-2-yl)-nonanoic acid;
3(R)-(6-Amino-pyridin-3-yl)-9-(5,6,7,8-tetrahydro-[1,8]-naphthyridin-2-yl)-nonanoic acid;
3(S)-(6-Amino-pyridin-3-yl)-9-(5,6,7,8-tetrahydro-[1,8]-naphthyridin-2-yl)-nonanoic acid;
3(R)-(Benzo[b]thiazol-2-yl)-9-(5,6,7,8-tetrahydro-[1,8]-naphthyridin-2-yl)-nonanoic acid hydrochloride;
3(S)-(Benzo[b]thiazol-2-yl)-9-(5,6,7,8-tetrahydro-[1,8]-naphthyridin-2-yl)-nonanoic acid hydrochloride;
and the pharmaceutically acceptable salts thereof.

15. The compound of Claim 1 which is:
3(S)-(Pyrimidin-5-yl)-9-(5,6,7,8-tetrahydro-[1,8]-naphthyridin-2-yl)-nonanoic acid;
or a pharmaceutically acceptable salt thereof.

16. The compound of Claim 1 which is:
3(R)-(Pyrimidin-5-yl)-9-(5,6,7,8-tetrahydro-[1,8]-naphthyridin-2-yl)-nonanoic acid;
or a pharmaceutically acceptable salt thereof.

17. A pharmaceutical composition comprising a compound according to any one of Claims 1 to 16 and a pharmaceutically acceptable carrier.

18. A pharmaceutical composition made by combining a compound according to any one of Claims 1 to 16 and a pharmaceutically acceptable carrier.

19. A process for making a pharmaceutical composition comprising combining a compound according to any one of Claims 1 to 16 and a pharmaceutically acceptable carrier.

20. The composition of Claim 17 which further comprises an active ingredient selected from the group consisting of
a) an organic bisphosphonate or a pharmaceutically acceptable salt or ester thereof,
b) an estrogen receptor modulator,
c) a cytotoxic/antiproliferative agent,
d) a matrix metalloproteinase inhibitor,
e) an inhibitor of epidermal-derived, fibroblast-derived, or platelet-derived growth factors,
f) an inhibitor of VEGF,
g) an inhibitor of Flk-1/KDR, Flt-1, Tck/Tie-2, or Tie-1,
h) a cathepsin K inhibitor, and
i) a prenylation inhibitor, such as a farnesyl transferase inhibitor or a geranylgeranyl transferase inhibitor or a dual farnesyl/geranylgeranyl transferase inhibitor; and mixtures thereof.

21. The composition of Claim 20 wherein said active ingredient is selected from the group consisting of
a) an organic bisphosphonate or a pharmaceutically acceptable salt or ester thereof,
b) an estrogen receptor modulator, and
c) a cathepsin K inhibitor; and mixtures thereof.

22. The composition of Claim 21 wherein said organic bisphosphonate or pharmaceutically acceptable salt or ester thereof is alendronate monosodium trihydrate.

23. The composition of Claim 20 wherein said active ingredient is selected from the group consisting of
a) a cytotoxic/antiproliferative agent,
b) a matrix metalloproteinase inhibitor,
c) an inhibitor of epidermal-derived, fibroblast-derived, or platelet-derived growth factors,
d) an inhibitor of VEGF,
e) an inhibitor of Flk-1/KDR, Flt-1, Tck/Tie-2, or Tie-1, and
f) a cathepsin K inhibitor;
and mixtures thereof.

24. The use of a compound according to any one of Claims 1 to 16 for the manufacture of a medicament for eliciting an integrin receptor antagonizing effect.

25. The use as claimed in Claim 24 wherein the integrin receptor antagonizing effect is an αvβ3 antagonizing effect.

26. The use as claimed in Claim 25 wherein the αvβ3 antagonizing effect is selected from the group consisting of inhibition of bone resorption, restenosis, angiogenesis, diabetic retinopathy, macular degeneration, inflammation, viral disease, tumor growth, and metastasis.

27. The use as claimed in Claim 26 wherein the αvβ3 antagonizing effect is the inhibition of bone resorption.

28. The use as claimed in Claim 24 wherein the integrin receptor antagonizing effect is an αvβ5 antagonizing effect.

29. The use as claimed in Claim 28 wherein the αvβ5 antagonizing effect is selected from the group consisting of inhibition of restenosis, angiogenesis, diabetic retinopathy, macular degeneration, inflammation, tumor growth, and metastasis. '

30. The use as claimed in Claim 24 wherein the integrin receptor antagonizing effect is a dual αvβ3/αvβ5 antagonizing effect.

31. The use as claimed in Claim 30 wherein the dual αvβ3/αvβ5 antagonizing effect is selected from the group consisting of inhibition of bone resorption, restenosis, angiogenesis, disbetic retinopathy, macular degeneration, inflammation, viral disease, tumor growth, and metastasis.

32. The use as claimed in Claim 24 wherein the integrin receptor antagonizing effect is an ανβ6 antagonizing effect.

33. The use as claimed in Claim 32 wherein the ανβ6 antagonizing effect is selected from the group consisting of angiogenesis, inflammatory response, and wound healing.

34. The use of the composition of Claim 17 for the manufacture of a medicament for eliciting an integrin receptor antagonizing effect.

35. The use of the composition of Claim 17 for the manufacture of a medicament for treating or preventing a condition mediated by antagonism of an integrin receptor.

36. The use of the composition of Claim 17 or Claim 21 for the manufacture of a medicament for inhibiting bone resorption.

37. The use of the composition of Claim 23 for the manufacture of a medicament for treating tumor growth.

38. The use, for the manufacture of a medicament for treating tumor growth in combination with radiation therapy, of a compound according to any one of Claims 1 to 16.

## Patentansprüche

1. Eine Verbindung der Formel 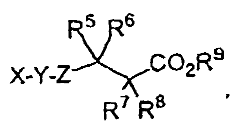 wobei X ist 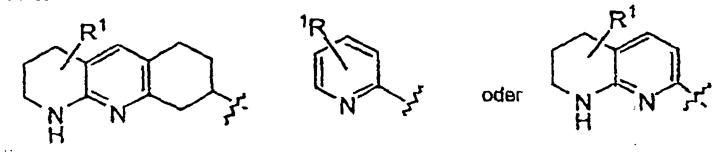 Y ausgewählt ist aus der Gruppe, bestehend aus
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH₂)ₘ-O-(CH₂)ₙ-O-(CH₂)ₚ-,
-(CH₂)ₘ-O-(CH₂)ₙ-NR⁴-(CH₂)ₚ-,
-(CH₂)ₘ-NR⁴-(CH₂)ₙ-NR⁴-(CH₂)ₚ-,
-(CH₂)ₘ-O-(CH₂)ₙ-S-(CH₂)ₚ-,
-(CH₂)ₘ-S-(CH₂)ₙ-S-(CH₂)ₚ-,
-(CH₂)ₘ-NR⁴-(CH₂)ₙ-S-(CH₂)ₚ-,
-(CH₂)ₘ-NR⁴-(CH₂)ₙ-O-(CH₂)ₚ-,
-(CH₂)ₘ-S-(CH₂)ₙ-O-(CH₂)ₚ- und
-(CH₂)ₘ-S-(CH₂)ₙ-NR⁴-(CH₂)ₚ-,
wobei jedes beliebige Methylen(CH₂)-Kohlenstoffatom in Y, anders als in R⁴, durch ein oder zwei R³-Substituenten substituiert sein kann, mit der Maßgabe, daß, wenn Y -(CH₂)ₘ-NR⁴-(CH₂)ₙ- ist und n 1 ist, der R³-Substituent am Methylenkohlenstoff in -(CH₂)ₘ- neben dem Stickstoff dann nicht Oxo sein kann,
Z ausgewählt ist aus der Gruppe, bestehend aus 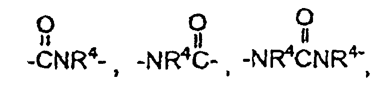 -CH₂CH₂- und -CH=CH-, wobei jedes Kohlenstoffatom durch ein oder zwei R³-Substituenten substituiert sein kann,
R¹ ausgewählt ist aus der Gruppe, bestehend aus
Wasserstoff, Halogen, C₁₋₁₀-Alkyl, C₃₋₈-Cycloalkyl, C₃₋₈-Cycloheteroalkyl, C₃₋₈-Cycloalkyl-C₁₋₆alkyl, C₃₋₈-Cycloheteroalkyl-C₁₋₆-alkyl, Aryl, Aryl-C₁₋₈-alkyl, Amino, Amino-C₁₋₈-alkyl, C₁₋₃-Acylamino, C₁₋₃-Acylamino-C₁₋₈-alkyl, (C₁₋₆-Alkyl)ₚamino, (C₁₋₈-Alkyl)ₚamino-C₁₋₈-alkyl, C₁₋₄-Alkoxy, C₁₋₄-Alkoxy-C₁₋₆-alkyl, Hydroxycarbonyl, Hydroxycarbonyl-C₁₋₆-alkyl, C₁₋₃-Alkoxycarbonyl, C₁₋₃-Alkoxycarbonyl-C₁₋₆-alkyl, Hydroxycarbonyl-C₁₋₆-alkyloxy, Hydroxy, Hydroxy-C₁₋₅-alkyl, C₁₋₆-Alkyloxy-C₁₋₆-alkyl, Nitro, Cyano, Trifluormethyl, Trifluormethoxy, Trifluorethoxy, C₁₋₈-Alkyl-S(O)ₚ, (C₁₋₈-Alkyl)ₚaminocarbonyl, C₁₋₈-Alkyloxycarbonylamino, (C₁₋₈-Alkyl)ₚaminocarbonyloxy, (Aryl-C₁₋₈-alkyl)ₚamino, (Aryl)ₚamino, Aryl-C₁₋₈-alkylsulfonylamino und C₁₋₈-Alkylsulfonylamino,
jeder der Reste R³, R⁵, R⁶, R⁷ und R⁸ unabhängig ausgewählt ist aus der Gruppe, bestehend aus Wasserstoff,
Aryl,
C₁₋₁₀-Alkyl,
Aryl-(CH₂)ᵣ-O-(CH₂)ₛ-,
Aryl-(CH₂)ᵣS(O)ₚ-(CH₂)ₛ-,
Aryl-(CH₂)ᵣ-C(O)-(CH₂)ₛ-,
Aryl-(CH₂)ᵣ-C(O)-N(R⁴)-(CH₂)ₛ-,
Aryl-(CH₂)ᵣ-N(R⁴)-C(O)-(CH₂)ₛ-,
Aryl-(CH₂)ᵣ-N(R⁴)-(CH₂)ₛ-,
Halogen,
Hydroxyl,
C₁₋₈-Alkylcarbonylamino,
Aryl-C₁₋₅-alkoxy,
C₁₋₅-Alkoxycarbonyl,
(C₁₋₈-Alkyl)ₚaminocarbonyl,
C₁₋₆-Alkylcarbonyloxy,
C₃₋₈-Cycloalkyl,
(C₁₋₈-Alkyl)ₚamino,
Arylaminocarbonyl,
Aryl-C₁₋₅-alkylaminocarbonyl,
Aminocarbonyl,
Aminocarbonyl-C₁₋₆-alkyl,
Hydroxycarbonyl,
Hydroxycarbonyl-C₁₋₆-alkyl,
HC≡C-(CH₂)ₜ-,
C₁₋₆-Alkyl-C≡C-(CH₂)ₜ-,
C₃₋₇-Cycloalkyl-C≡C-(CH₂)ₜ-,
Aryl-C≡C-(CH₂)ₜ-,
C₁₋₆-Alkylaryl-C≡C-(CH₂)ₜ-,
CH₂=CH-(CH₂)ₜ-,
C₁₋₆-Alkyl-CH=CH-(CH₂)ₜ-,
C₃₋₇-Cycloalkyl-CH=CH-(CH₂)ₜ-,
Aryl-CH=CH-(CH₂)ₜ-,
C₁₋₆-Alkylaryl-CH=CH-(CH₂)ₜ-,
C₁₋₆-Alkyl-SO₂-(CH₂)ₜ-,
C₁₋₆-Alkylaryl-SO₂-(CH₂)ₜ-,
C₁₋₆-Alkoxy,
Aryl-C₁₋₆-alkoxy,
Aryl-C₁₋₆-alkyl,
(C₁₋₆-Alkyl)ₚamino-C₁₋₆-alkyl,
(Aryl)ₚamino,
(Aryl)ₚamino-C₁₋₆-alkyl,
(Aryl-C₁₋₆-alkyl)ₚamino,
(Aryl-C₁₋₆-alkyl)ₚamino-C₁₋₆-alkyl,
Arylcarbonyloxy,
Aryl-C₁₋₆-alkylcarbonyloxy,
(C₁₋₆-Alkyl)ₚaminocarbonyloxy,
C₁₋₆-Alkylsulfonylamino,
Arylsulfonylamino,
C₁₋₈-Alkylsulfonylamino-C₁₋₆-alkyl,
Aryl-C₁₋₆-alkylsulfonylamino,
Aryl-C₁₋₆-alkylsulfonylamino-C₁₋₆-alkyl,
C₁₋₈-Alkoxycarbonylamino,
C₁₋₈-Alkoxycarbonylamino-C₁₋₈-alkyl,
Aryloxycarbonylamino-C₁₋₈-alkyl,
Aryl-C₁₋₈-alkoxycarbonylamino,
Aryl-C₁₋₈-alkoxycarbonylamino-C₁₋₈-alkyl,
C₁₋₈-Alkylcarbonylamino-C₁₋₆-alkyl,
Arylcarbonylamino-C₁₋₆-alkyl,
Aryl-C₁₋₆-alkylcarbonylamino,
Aryl-C₁₋₆-alkylcarbonylamino-C₁₋₆-alkyl,
Aminocarbonylamino-C₁₋₆-alkyl,
(C₁₋₈-Alkyl)ₚaminocarbonylamino,
(C₁₋₈-Alkyl)ₚaminocarbonylamino-C₁₋₆-alkyl,
(Aryl)ₚaminocarbonylamino-C₁₋₆-alkyl,
(Aryl-C₁₋₈-alkyl)ₚaminocarbonylamino,
(Aryl-C₁₋₈-alkyl)ₚaminocarbonylamino-C₁₋₆-alkyl,
Aminosulfonylamino-C₁₋₆-alkyl,
(C₁₋₈-Akyl)ₚaminosulfonylamino,
(C₁₋₈-Alkyl)ₚaminosulfonylamino-C₁₋₆-alkyl,
(Aryl)ₚaminosulfonylamino-C₁₋₆-alkyl,
(Aryl-C₁₋₈-alkyl)ₚaminosulfonylamino,
(Aryl-C₁₋₈-alkyl)ₚaminosulfonylamino-C₁₋₆-alkyl,
C₁₋₆Alkylsulfonyl,
C₁₋₆-Alkylsulfonyl-C₁₋₆-alkyl,
Arylsulfonyl-C₁₋₆-alkyl,
Aryl-C₁₋₆-alkylsulfonyl,
Aryl-C₁₋₆-alkylsulfonyl-C₁₋₆-alkyl,
C₁₋₆-Alkylcarbonyl,
C₁₋₆-Alkylcarbonyl-C₁₋₆-alkyl,
Arylcarbonyl-C₁₋₆-alkyl,
Aryl-C₁₋₆-alkylcarbonyl,
Aryl-C₁₋₆-alkylcarbonyl-C₁₋₆-alkyl,
C₁₋₆-Alkylthiocarbonylamino,
C₁₋₆-Alkylthiocarbonylamino-C₁₋₆-alkyl,
Arylthiocarbonylamino-C₁₋₆-alkyl,
Aryl-C₁₋₆-alkylthiocarbonylamino,
Aryl-C₁₋₆-alkylthiocarbonylamino-C₁₋₆-alkyl,
(C₁₋₈-Alkyl)ₚaminocarbonyl-C₁₋₆-alkyl
(Ary)ₚaminocarbonyl-C₁₋₆-alkyl,
(Aryl-C₁₋₈-alkyl)ₚaminocarbonyl und
(Aryl-C₁₋₈-alkyl)ₚaminocarbonyl-C₁₋₆-alkyl,
oder R³ Oxo, Trifluormethyl oder C₁₋₈-Alkylcarbonylamino ist,
oder R⁵ und R⁶ unabhängig Amino-C₁₋₆-alkyl, Arylsulfonylamino-C₁₋₆-alkyl oder C₁₋₈-Alkylcarbonylamino sind,
oder R⁷ und R⁸ unabhängig Amino-C₁₋₆-alkyl, Arylcarbonylamino, Arylsulfonylamino-C₁₋₆-alkyl, Arylaminocarbonylamino oder C₇₋₂₀-Polycyclyl-C₀₋₈-alkylsulfonylamino sind, oder zwei R³-Substituenten, wenn sie sich am selben Kohlenstoffatom befinden, mit dem Kohlenstoffatom, an das sie gebunden sind, zusammengenommen werden, um eine Carbonylgruppe oder eine Cyclopropylgruppe zu bilden,
oder R⁵ und R⁶, wenn sie sich am selben Kohlenstoffatome befinden, mit dem Kohlenstoffatom, an das sie gebunden sind, zusammengenommen werden, um eine Carbonylgruppe zu bilden,
wobei beliebige der Alkylgruppen von R³, R⁵, R⁶, R⁷ und R⁸ entweder unsubsfituiert oder substituiert sind mit ein bis drei R¹-Substituenten, und mit der Maßgabe, daß jedes R³, jedes R⁵ und R⁶ und jedes R⁷ und R⁸ derart ausgewählt sind, daß in der resultierenden Verbindung das Kohlenstoffatom oder die Kohlenstoffatome, an das/die R³, R⁵ und R⁶ und R⁷ und R⁸ gebunden sind, ihrerseits an nicht mehr als ein Heteroatom gebunden sind,
jedes R⁴ unabhängig ausgewählt ist aus der Gruppe, bestehend aus
Wasserstoff,
Aryl,
Aminocarbonyl,
C₃₋₈-Cycloalkyl,
Amino-C₁₋₆-alkyl,
(Aryl)ₚaminocarbonyl,
(Aryl-C₁₋₅-alkyl)ₚaminocarbonyl,
Hydroxycarbonyl-C₁₋₆-alkyl,
C₁₋₈-Alkyl,
Aryl-C₁₋₆-alkyl,
(C₁₋₆-Alkyl)ₚamino-C₂₋₆-alkyl,
(Aryl-C₁₋₆-alkyl)ₚamino-C₂₋₆-alkyl,
C₁₋₈-Alkylsulfonyl,
C₁₋₈-Alkoxycarbonyl,
Aryloxycarbonyl,
Aryl-C₁₋₈-alkoxycarbonyl,
C₁₋₈-Alkylcarbonyl,
Arylcarbonyl,
Aryl-C₁₋₆-alkylcarbonyl,
(C₁₋₈-Alkyl)ₚaminocarbonyl,
Aminosulfonyl,
C₁₋₈-Alkylaminosulfonyl,
(Aryl)ₚaminosulfonyl,
(Aryl-C₁₋₈-alkyl)ₚaminosulfonyl,
Arylsulfonyl,
Aryl-C₁₋₆-alkylsulfonyl,
C₁₋₆-Alkylthiocarbonyl,
Arylthiocarbonyl und
Aryl-C₁₋₆-alkylthiocarbonyl,
wobei beliebige der Alkylgruppen von R⁴ entweder unsubstituiert oder substituiert sind mit ein bis drei R¹-Substituenten,
R⁹ ausgewählt ist aus der Gruppe, bestehend aus
Wasserstoff,
C₁₋₈-Alkyl,
Aryl,
Aryl-C₁₋₈-alkyl,
C₁₋₈-Alkylcarbonyloxy-C₁₋₄-alkyl,
Aryl-C₁₋₈-alkylcarbonyloxy-C₁₋₄-alkyl
C₁₋₈-Alkylaminocarbonylmethylen und
C₁₋₈-Dialkylaminocarbonylmethylen.
wobei
jedes Aryl als eine Gruppe oder Teil einer Gruppe unabhängig ausgewählt ist aus der Gruppe, bestehend aus: Phenyl, Naphthyl, Pyridyl, Pyrryl, Pyrazolyl, Pyrazinyl, Pyrimidinyl, Imidazolyl, Benzimidazolyl, Benzthiazolyl, Benzoxazolyl, Indolyl, Thienyl, Furyl, Dihydrobenzofuryl, Benzo(1,3)dioxolan, Oxazolyl, Isoxazolyl und Thiazolyl,
jedes m unabhängig eine ganze Zahl von 2 bis 4 ist,
jedes n unabhängig eine ganze Zahl von 0 bis 6 ist,
jedes p unabhängig eine ganze Zahl von 0 bis 2 ist,
jedes r unabhängig eine ganze Zahl von 1 bis 3 ist,
jedes s unabhängig eine ganze Zahl von 0 bis 3 ist und
jedes t unabhängig eine ganze Zahl von 0 bis 3 ist,
und die pharmazeutisch annehmbaren Salze davon.

2. Die Verbindung nach Anspruch 1, wobei Y ausgewählt ist aus der Gruppe, bestehend aus
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH2)m-O-(CH2)n-O-(CH2)p-,
-(CH2)m-O-(CH2)n-NR⁴-(CH2)p-,
-(CH2)m-NR⁴-(CH2)n-NR⁴-(CH2)p- und
-(CH2)m-NR⁴-(CH2)n-O-(CH2)p-,
wobei jedes beliebige Methylen(CH₂)-Kohlenstoffatom in Y, anders als in R⁴, durch ein oder zwei R³-Substituenten substituiert sein kann,
mit der Maßgabe, daß, wenn Y -(CH₂)ₘ-NR⁴-(CH₂)ₙ- ist und n 1 ist, der R³-Substituent am Methylenkohlenstoff in -(CH₂)ₘ- neben dem Stickstoff dann nicht Oxo sein kann,
und Z ausgewählt ist aus der Gruppe, bestehend aus 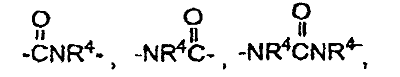 -CH₂CH₂- und -CH=CH-, wobei jedes Kohlenstoffatom durch ein oder zwei R³-Substituenten substituiert sein kann.

3. Die Verbindung nach Anspruch 2, wobei
Y ausgewählt ist aus der Gruppe, bestehend aus
(CH₂)ₘ, (CH₂)ₘ-S-(CH₂)ₙ und (CH₂)ₘ-NR⁴-(CH₂)ₙ, wobei jedes beliebige Nfethyten(CH₂)-Kohlenstoffatom in Y, anders als in R⁴, durch ein oder zwei R³-Substituenten substituiert sein kann, mit der Maßgabe, daß, wenn Y -(CH₂)ₘ-NR⁴-(CH₂)ₙ- ist und n 1 ist, der R³-Substituent am Methylenkohlenstoff in -(CH₂)ₘ- neben dem Stickstoff dann nicht Oxo sein kann,
und Z ausgewählt ist aus der Gruppe, bestehend aus 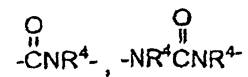 und
-CH₂CH₂-, wobei jedes Kohlenstoffatom durch ein oder zwei R³-Substituenten substituiert sein kann.

4. Die Verbindung nach Anspruch 3, wobei jedes R³ unabhängig ausgewählt ist aus der Gruppe, bestehend aus
Wasserstoff,
Fluor,
Trifluormethyl,
Aryl,
C₁₋₈-Alkyl,
Aryl-C₁₋₆-alkyl,
Hydroxyl,
Oxo,
Arylaminocarbonyl,
Aryl-C₁₋₅-alkylaminocarbonyl,
Aminocarbonyl und
Aminocarbonyl-C₁₋₆-alkyl,
und wobei jedes R⁴ unabhängig ausgewählt ist aus der Gruppe, bestehend aus
Wasserstoff,
Aryl,
C₃₋₈-Cycloalkyl,
C₁₋₈-Alkyl,
C₁₋₈-Alkylcarbonyl,
Arylcarbonyl,
C₁₋₆-Alkylsulfonyl,
Arylsulfonyl,
Aryl-C₁₋₆-alkylsulfonyl,
Aryl-C₁₋₆-alkylcarbonyl,
C₁₋₈-Alkylaminocarbonyl,
Aryl-C₁₋₅-alkylaminocarbonyl,
Aryl-C₁₋₈-alkoxycarbonyl und
C₁₋₈-Alkoxycarbonyl.

5. Die Verbindung nach Anspruch 4, wobei R⁶, R⁷ und R⁸ jeweils Wasserstoff sind und R⁵ ausgewählt ist aus der Gruppe, bestehend aus
Wasserstoff,
Aryl,
C₁₋₈-Alkyl,
Aryl-C≡C-(CH₂)ₜ-,
Aryl-C₁₋₆-alkyl,
CH₂=CH-(CH₂)ₜ- und
HC≡C-(CH₂)ₜ-.

6. Die Verbindung nach Anspruch 5, wobei R⁹ ausgewählt ist aus der Gruppe, bestehend aus Wasserstoff, Methyl und Ethyl.

7. Die Verbindung nach Anspruch 6, wobei R⁹ Wasserstoff ist.

8. Die Verbindung nach Anspruch 4, wobei R⁵, R⁶ und R⁸ jeweils Wasserstoff sind und R⁷ ausgewählt ist aus der Gruppe, bestehend aus
Wasserstoff,
Aryl,
C₁₋₈-Alkylcarbonylamino,
C₁₋₈-Alkylsulfonylamino,
Arylcarbonylamino,
Arylsulfonylamino,
C₁₋₈-Alkylsulfonylamino-C₁₋₆-alkyl,
Arylsulfonylamino-C₁₋₆-alkyl,
Aryl-C₁₋₆-alkylsulfonylamino,
Aryl-C₁₋₆-alkylsulfonylamino-C₁₋₆-alkyl,
C₁₋₈-Alkoxycarbonylamino,
C₁₋₈-Alkoxycarborlylamino-C₁₋₈-alkyl,
Aryloxycarbonylamino-C₁₋₈-alkyl,
Aryl-C₁₋₈-alkoxycarbonylamino,
Aryl-C₁₋₈-alkoxycarbonylamino-C₁₋₈-alkyl,
C₁₋₈-Alkylcarbonylamino-C₁₋₆-alkyl,
Arylcarbonylamino-C₁₋₆-alkyl,
Aryl-C₁₋₆-alkylcarbonylamino,
Aryl-C₁₋₆-alkylcarbonylamino-C₁₋₆-alkyl,
Aminocarbonylamino-C₁₋₆-alkyl,
(C₁₋₈-Alkyl)ₚaminocarbonylamino,
(C₁₋₈-Alkyl)ₚaminocarbonylamino-C₁₋₆-alkyl,
(Aryl)ₚaminocarbonylamino-C₁₋₆-alkyl,
Arylaminocarbonylamino,
(Aryl-C₁₋₈-alkyl)ₚaminocarbonylamino,
(Aryl-C₁₋₈-alkyl)ₚaminocarbonylamino-C₁₋₆-alkyl,
Aminosulfonylamino-C₁₋₆-alkyl,
(C₁₋₈-Alkyl)ₚaminosulfonylamino,
(C₁₋₈-Alkyl)ₚaminosulfonylamino-C₁₋₆-alkyl,
(Aryl)ₚaminosulfonylamino-C₁₋₆-alkyl,
(Aryl-C₁₋₈-alkyl)ₚaminosulfonylamino,
(Aryl-C₁₋₈-alkyl)ₚaminosulfonylamino-C₁₋₆-alkyl,
C₁₋₆-Alkylthiocarbonylamino,
C₁₋₆-Alkylthiocarbonylamino-C₁₋₆-alkyl,
Arylthiocarbonylamino-C₁₋₆-alkyl,
Aryl-C₁₋₆-alkylthiocarbonylamino,
Aryl-C₁₋₆-alkylthiocarbonylamino-C₁₋₆-alkyl und
C₇₋₂₀-Polycyclyl-C₀₋₈-alkylsulfonylamino.

9. Die Verbindung nach Anspruch 8, wobei R⁵, R⁶ und R⁸ jeweils Wasserstoff sind und R⁷ ausgewählt ist aus der Gruppe, bestehend aus
Wasserstoff,
Aryl,
C₁₋₈-Alkylcarbonylamino,
Aryl-C₁₋₆-alkylcarbonylamino,
Arylcarbonylamino,
C₁₋₈-Alkylsulfonylamino,
Aryl-C₁₋₆-alkylsulfonylamino,
Arylsulfonylamino,
C₁₋₈-Alkoxycarbonylamino,
Aryl-C₁₋₈-alkoxycarbonylamino,
Arylaminocarbonylamino,
(C₁₋₈-Alkyl)paminocarbonylamino,
(Aryl-C₁₋₈-alkyl)paminocarbonylamino,
(C₁₋₈-Alkyl)ₚaminosulfonylamino und
(Aryl-C₁₋₈alkyl)ₚaminosulfonylamino.

10. Die Verbindung gemäß Anspruch 9, wobei R⁹ ausgewählt ist aus der Gruppe, bestehend aus Wasserstoff, Methyl und Ethyl.

11. Die Verbindung gemäß Anspruch 10, wobei R⁹ Wasserstoff ist.

12. Die Verbindung nach Anspruch 4, ausgewählt aus der Gruppe, bestehend aus:
3-(5-(5,6,7,8-Tetrahydro[1,8]naphthyridin-2-yl)pentanoylamino)propionsäure,
3(S)-(Pyridin-3-yl)-3-(5-(5,6,7,8-tetrahydro[1,8]naphthyridin-2-yl)pentanoylamino)propionsäure,
3(S)-(5,6,7,8-Tetrahydrochinolin-3-yl)-3-(5-(5,6,7,8-tetrahydro[1,8]naphthyridin-2-yl)pentanoylaminopropionsäure(trifluoracetat),
2(S)-Benzolsulfonylamino-3-(5-(5,6,7,8-tetrahydro[1,8]naphthyridin-2-yl)pentanoylamino)propionsäuretrifluoracetat,
3(S)-(Chinolin-3-yl)-3-(5-(5,6,7,8-tetrahydro[1,8]naphthyridin-2-yl)pentanoylamino)propionsäure,
3(R)-(Chinolin-3-yl)-3-(5-(5,6,7,8-tetrahydro[1,8]naphthyridin-2-yl)pentanoylamino)propionsäure,
3-(Chinolin-3-yl)-3-(7-(5,6,7,8-tetrahydro[1,8]naphthyridin-2-yl)heptanoylamino)propionsäurebis(trifluoracetat),
3-(Chinolin-3-yl)-3-(6-(5,6,7,8-tetrahydro[1,8]naphthyridin-2-yl)hexanoylamino)propionsäure,
3(S)-(3-Fluorphenyl)-3-(4-(5,6,7,8-tetrahydro[1,8]naphthyridin-2-ylamino)butyrylamino)propionsäurebis(trifluoracetat),
3(S)-(5-(5,6,7,8-Tetrahydro[1,8]naphthyridin-2-yl)pentanoylamino)pent-4-ensäure,
3(S)-(3-Fluorphenyl)-3-(5-(5,6,7,8-tetrahydro[1,8]naphthyridin-2-yl)pentanoylamino)propionsäure,
2-(3-Fluorphenyl)-3-(5-(5,6,7,8-tetrahydro[1,8]naphthyridin-2-yl)pentanoylamino)propionsäuretrifluoracetatsalz,
3(S)-(Benzo[1,3]dioxol-5-yl)-3-(5-(5,6,7,8-tetrahydro[1,8]naphthyridin-2-yl)pentanoylamino)propionsäure,
3(S)-(2,3-Dihydrobenzofuran-6-yl)-3-(5-(5,6,7,8-tetrahydro[1,8]naphthyridin-2-yl)pentanoylamino)-propionsäure.
3(S)-(2-Oxo-2,3-dihydrobenzoxazol-6-yl)-3-(5-(5,6,7,8-tetrahydro[1,8]naphthyridin-2-yl)pentanoylamino)propionsäuretrifluoracetat,
3(S)-(3-Fluorophenyl)-3-{3-[(5,6,7,8-tetrahydro[1,8]naphthyridin-2-ylmethyl)amino]propionylamino}propionsäure,
3(S)-(3-Fluorphenyl)-3-(2-{propyl-[2-(5,6,7,8-tetrahydro[1,8]naphthyridin-2-yl)ethyl]amino}acetylamino)propionsäuretrifluoracetat,
3(S)-(3-Fluorphenyl)-3-(2-{phenethyl-[2-(5,6,7,8-tetrahydro[1,8]naphthyridin-2-yl)ethyl]amino}-acetylamino)propionsäuretrifluoracetat,
3(S)-(3-Fluorphenyl)-3-{3(S)-[(5,6,7,8-tetrahydro[1,8]naphthyridin-2-ylmethyl)amino]pent-4-inoylamino}propionsäure,
3(S)-(3-Fluorphenyl)-3-{3(S)-(3-fluorphenyl)-3-[(5,6,7,8-tetrahydro[1,8]naphthyridin-2-ylmethyl)-amino]propionylamino}propionsäurebis(trifluoracetat).
3(S)-(3-Fluor-4-phenylphenyl)-3-(5-(5,6,7,8-tetrahydro[1,8]naphthyridin-2-yl)pentanoylamino)-propionsäuretrifluoracetat,
2(S)-(2-Thienylsulfonylamino)-3-(5-(5,6,7,8-tetrahydro[1,8]naphthyridin-2-yl)pentanoylamino)-propionsäuretrifluoracetat,
3(S)-(3-Fluorphenyl)-3-{3-methyl-3-[(5,6,7,8-tetrahydro[1,8]naphthyridin-2-ylmethyl)amino]propionylamino}propionsäure,
3(S)-(3-Fluorphenyl)-3-{2-[2-(5,6,7,8-tetrahydro[1,8]naphthyridin-2-yl)ethylamino]acetylamino}propionsäure,
3(S)-(3-Fluorphenyl)-3-{(3-(5,6,7,8-tetrahydro[1,8]naphthyridin-2-yl)propyl]ureido}propionsäure,
2(S)-(Methansulfonylamino)-3-(5-(5,6,7,8-tetrahydro[1,8]naphthyridin-2-yl)pentanoylamino)propionsäure,
3(S)-(2,3-Dihydrobenzofuran-6-yl)-3-[3-(1,2,3,4,6,7,8(R oder S),9-octahydrobenzo[b][1,8]-naphthyridin-8-yl)propionylamino]propionsäure,
3(S)-(2,3-Dihydrobenzofuran-6-yl)-3-[3-(1,2,3,4,6,7,8(S oder R),9-octahydropenzo[b][1,8]-naphthyridin-8-yl)propionylamino]propionsäure,
3(S)-(6-Methoxypyridin-3-yl)-3-[N-methyl-3-(1,2,3,4,6,7,8,9-octahydrobenzo[b][1,8]naphthyridin-8-ylpropionyl)amino]propionsäure,
3(S)-(2,3-Dihydrobenzofuran-6-yl)-3-[3-(5,6,7,8-tetrahydro-[1,8]-naphthyridin-2-ylmethylsulfanyl)-propionylamino]propionsäurebis(trifluoracetat),
3-(Chinolin-3-yl)-7-[(5,6,7,8-tetrahydro[1,8]naphthyridin-2-ylmethyl)amino]heptansäure,
3-(Chinolin-3-yl)-7-[acetyl-[5,6,7,8-tetrahydro[1,8]naphthyridin-2-ylmethyl)amino]heptansäure,
3-(Chinolin-3-yl)-7-[methansulfonyl-(5,6,7,8-tetrahydro[1,8]naphthyridin-2-ylmethyl)amino]heptansäure.
9-(5,6,7,8-Tetrahydro[1,8]naphthyridin-2-yl)nonansäure,
2-(Benzolsulfonylamino)-9-(5,6,7,8-tetrahydro[1,8]naphthyridin-2-yl)non-4-ensäurebis(trifluoracetat),
2(S)-(Benzolsulfonylamino)-9-(5,6,7,8-tetrahydro[1,8]naphthyridin-2-yl)nonansäure,
2(R)-(Benzolsulfonylamino)-9-(5,6,7,8-tetrahydro-[1,8]naphthyridin-2-yl)nonansäure,
2(S)-(Benzolsulfonylamino)-10-(5,6,7,8-tetrahydro-[1,8]naphthyridin-2-yl)decansäure,
2(S)-(Benzolsulfonylamino)-8-(5,6,7,8-tetrahydro[1,8]naphthyridin-2-yl)octansäure,
2(S)-(Cyclohexylmethansulfonylamino)-9-(5,6,7,8-tetrahydro[1,8]naphthyridin-2-yl)nonansäurehydrochlorid,
2(S)-(7,7-Dimethyl-2-oxobicyclo[2.2.1]hept-1(S)-ylmethansulfonylamino)-9-(5,6,7,8-tetrahydro[1,8]-naphthyridin-2-yl)nonansäurehydrochlorid,
2(S)-(Phenylmethansulfonylamino)-9-(5,6,7,8-tetrahydro[1,8]naphthyridin-2-yl)nonansäure,
2(S)-(Cyclohexansulfonylamino)-9-(5,6,7,8-tetrahydro[1,8]naphthyridin-2-yl)nonansäurehydrochlorid,
2(S)-(Butan-1-sulfonylamino)-9-(5,6,7,8-tetrahydro[1,8]naphthyridin-2-yl)nonansäurehydrochlorid,
2(S)-(3-Benzylureido)-9-(5,6,7,8-tetrahydro[1,8]naphthyridin-2-yl)nonansäure,
2(S)-(Benzyloxycarbonylamino)-9-(5,6,7,8-tetrahydro[1,8]naphthyridin-2-yl)nonansäure,
2(S)-(Phenylacetylamino)-9-(5,6,7,8-tetrahydro[1,8]naphthyridin-2-yl)nonansäure,
2(S)-(Acetylamino)-9-(5,6,7,8-tetrahydro[1,8]naphthyridin-2-yl)nonansäure,
2(S)-(Benzoylamino)-9-(5,6,7,8-tetrahydro[1,8]naphthyridin-2-yl)nonansäure,
3-(Chinolin-3-yl)-9-(5,6,7,8-tetrahydro[1,8]naphthyridin-2-yl)nonansäure,
3(S)-(Chinolin-3-yl)-9-(5,6,7,8-tetrahydro[1,8]naphthyridin-2-yl)nonansäure,
3(R)-(Chinolin-3-yl)-9-(5,6,7,8-tetrahytiro[1,8]naphthyridin-2-yl)nonansäure,
3-(Chinolin-3-yl)-7-(1,2,3,4,6,7,8,9-octahydrobenzo[b][1,8]naphthyridin-8-yl)heptansäurebis-(hydrochlorid),
6-Oxo-3-(chinolin-3-yl)-9-(5,6,7,8-tetrahydro[1,8]naphthyridin-2-yl)nonansäure,
3-(N-Oxochinolin-3-yl)-9-(5,6,7,8-tetrahydro[1,8]naphthyridin-2-yl)nonansäure,
3-Phenyl)-9-(5,6,7,8-tetrahydro[1,8]naphthyridin-2-yl)nonansäure,
3-(Benzo[b]thiophen-2-yl)-9-(5,6,7,8-tetrahydro[1,8]naphthyridin-2-yl)nonansäure,
3(R)-(Benzo[b]thiopnen-2-yl)-9-(5,6,7,8-tetrahydro[1,8]naphthyridin-2-yl)nonansäure,
3(S)-(Benzo[b]thiophen-2-yl)-9-(5,6,7,8-tetrahydro[1,8]naphthyridin-2-yl)nonansäure,
3-(Pyridin-3-yl)-9-(5,6,7,8-tetrahydro[1,8]naphthyridin-2-yl)nonansäure,
3(R)-(Pyridin-3-yl)-9-(5,6,7,8-tetrahydro[1,8]naphthyridin-2-yl)nonansäure,
3(S)-(Pyridin-3-yl)-9-(5,6,7,8-tetrahydro[1,8]naphthyridin-2-yl)nonansäure,
3-(3-Fluorphenyl)-9-(5,6,7,8-tetrahydro[1,8]naphthyridin-2-yl)nonansäure,
3(R)-(3-Fluorphenyl)-9-(5,6,7,8-tetrahyaro[1,8]naphthyridin-2-yl)nonansäure,
3(S)-(3-Fluorphenyl)-9-(5,6,7,8-tetrahydro[1,8]naphthyridin-2-yl)nonansäure,
3-(2,3-Dihydrobenzofuran-6-yl)-9-(5,6,7,8-tetrahydro[1,8]naphthyridin-2-yl)nonansäure,
3(S)-(2,3-Dihydrobenzofuran-6-yl)-9-(5,6,7,8-tetrahydro[1,8]naphthyridin-2-yl)nonansäure,
3(R)-(2,3-Dihydrobenzofuran-6-yl)-9-(5,6,7,8-tetrahydro[1,8]naphthyridin-2-yl)nonansäure,
3-(2,3-Dihydrobenzofuran-6-yl)-9-(5,6,7,8-tetrahydro[1,8]naphthyridin-2-yl)non-4-ensäuretrifluoracetat,
3-(2,3-Dihydrofuro[3,2-b]pyridin-5-yl)-9-(5,6,7,8-tetrahydro[1,8]naphthyridin-2-yl)nonansäure,
3(R)-(2,3-Dihydrofuro[3,2-b]pyridin-5-yl)-9-(5,6,7,8-tetrahydro[1,8]naphthyridin-2-yl)nonansäure,
3(S)-(2,3-Dinydrofuro[3,2]pyridin-5-yl)-9-(5,6,7,8-tetrahydro[1,8]naphthyridin-2-yl)nonansäure,
3-(Furo[2,3-b]pyridin-5-yl)-9-(5,6,7,8-tetrahydro[1,8]naphthyridin-2-yl)nonansäure,
3(R)-(Furo[2,3-b]pyridin-5-yl)-9-(5,6,7,8-tetrahydro[1,8]naphthyridin-2-yl)nonensäure,
3(S)-(Furo[2,3-b]pyridin-5-yl)-9-(5,6,7,8-tetrahydro[1,8]naphthyridin-2-yl)nonansäure,
3-(2,3-Dihydrofuro[2,3-b]pyridin-5-yl)-9-(5,6,7,8-tetrahydro[1,8]naphthyridin-2-yl)nonansäure,
3(R)-(2,3-Dihydrofuro[2,3-b]pyridin-5-yl)-9-(5,6,7,8-tetrahydro[1,8]-naphthyridin-2-yl)nonansäure,
3(S)-(2,3-Dihydrofuro[2,3-b]pyridin-5-yl)-9-(5,6,7,8-tetrahydro[1,8]-naphthyridin-2-yl)nonansäure,
3-(6-Methoxypyridin-3-yl)-9-(5,6,7,8-tetrahydro[1,8]naphthyridin-2-yl)nonansäure,
3(S)-(6-Methoxypyridin-3-yl)-9-(5,6,7,8-tetrahydro[1,8]naphthyridin-2-yl)nonansäure,
3(R)-(6-Methoxypyridin-3-yl)-9-(5,6,7,8-tetrahydro[1,8]naphthyridin-2-yl)nonansäure,
3-(Pyrimidin-5-yl)-9-(5,6,7,8-tetrahydro[1,8]naphthyridin-2-yl)nonansäuretrifluoracetat,
3(R)-(Pyrimidin-5-yl)-9-(5,6,7,8-tetrahydro[1,8]naphthyridin-2-yl)nonansäuretrifluoracetat,
3(S)-(Pyrimidin-5-yl)-9-(5,6,7,8-tetrahydro[1,8]naphthyridin-2-yl)nonansäuretrifluoracetat,
3-(6-Aminopyridin-3-yl)-9-(5,6,7,8-tetrahhydro[1,8]naphthyridin-2-yl)nonansäure,
3(R)-(6-Aminopyridin-3-yl)-9-(5,6,7,8-tetrahydro[1,8]naphthyridin-2-yl)nonansäure,
3(S)-(6-Aminopyridin-3-yl)-9-(5,6,7,8-tetrahydro[1,8]naphthyridin-2-yl)nonansäure,
3-(Benzo[b]thiazol-2-yl)-9-(5,6,7,8-tetrahydro[1,8]naphthyridin-2-yl)nonansäurehydrochlorid,
3(R)-(Benzo[b]thiazol-2-yl)-9-(5,6,7,8-tetrahydro[1,8]naphthyridin-2-yl)nonansäurehydrochlorid,
3(S)-(Benzo[b]thiazol-2-yl)-9-(5,6,7,8-tetrahydro[1,8]naphthyridin-2-yl)-nonansäurehydrochlorid,
3-(6-Oxo-1,6-dihydropyridin-3-yl)-9-(5,6,7,8-tetrahydro[1,8]naphthyridin-2-yl)nonansäurebis(trifluoracetat)
und den pharmazeutisch annehmbaren Salzen davon.

13. Die Verbindung nach Anspruch 12, ausgewählt aus der Gruppe, bestehend aus:
3(S)-(Pyridin-3-yl)-3-(5-(5,6,7,8-tetrahydro[1,8]naphthyridin-2-yl)pentanoylamino)propionsäure,
2(S)-Benzolsulfonylamino-3-(5-(5,6,7,8-tetrahydro[1,8]naphthyridin-2-yl)pentanoylamino)propionsäuretrifluoracetat,
3(S)-(Chinolin-3-yl)-3-(5,6,7,8-tetrahydro[1,8]naphthyridin-2-yl)pentanoylamino)propionsäure,
3(R)-(Chinolin-3-yl)-3-(5-(5,6,7,8-tetrahydro[1,8]naphthyridin-2-yl)pentanoylamino)propionsäure,
2(S)-(2-Thienylsulfonylamino)-3-(5-(5,6,7,8-tetrahydro[1,8]naphthyridin-2-yl)pentanoylamino)-propionsäuretrifluoracetat,
3(S)-(2,3-Dihydrobenzofuran-6-yl)-3-[3-(1,2,3,4,6,7,8(R oder S),9-octahydrobenzo[b][1,8]-naphthyridin-8-yl)propionylamino]propionsäure,
3(S)-(2,3-Dihydrobenzofuran-6-yl)-3-[3-(1,2,3,4,6,7,8(S oder R),9-octahydrobenzo[b][1,8]-naphthyridin-8-yl)propionylamino]propionsäure,
3(S)-(6-Methoxypyridin-3-yl)-3-[N-methyl-3-(1,2,3,4,6,7,8,9-octahydrobenzo[b][1,8]naphthyridin-8-ylpropionyl)amino]propionsäure,
2-(Benzolsulfonylamino)-9-(5,6,7,8-tetrahydro[1,8]naphthyridin-2-yl)non-4-ensäurebis(trifluoracetat)
und den pharmazeutisch annehmbaren Salzen davon.

14. Die Verbindung nach Anspruch 12, ausgewählt aus der Gruppe, bestehend aus:
3(R)-(Chinolin-3-yl)-9-(5,6,7,8-tetrahydro[1,8]naphthyridin-2-yl)nonansäure,
3(S)-(Chinolin-3-yl)-9-(5,6,7,8-tetrahydro[1,8]naphthyridin-2-yl)nonansäure,
3(R)-(Benzo[b]thiophen-2-yl)-9-(5,6,7,8-tetrahydro[1,8]naphthyridin-2-yl)nonansäure,
3(S)-(Benzo[b]thiophen-2-yl)-9-(5,6,7,8-tetrahydro[1,8]naphthyridin-2-yl)nonansäure,
3(R)-(Pyridin-3-yl)-9-(5,6,7,8-tetrahydro[1,8]naphthyridin-2-yl)nonansäure,
3(S)-(Pyridin-3-yl)-9-(5,6,7,8-tetrahydro[1,8]naphthyridin-2-yl)nonansäure,
3(R)-(3-Fluorphenyl)-9-(5,6,7,8-tetrahydro[1,8]naphthyridin-2-yl)nonansäure,
3(S)-(3-Fluorphenyl)-9-(5,6,7,8-tetrahydro[1,8]naphthyridin-2-yl)nonansäure,
3(R)-(2,3-Dihydrobenzofuran-6-yl)-9-(5,6,7,8-tetrahydro[1,8]naphthyridin-2-yl)nonansäure,
3(S)-(2,3-Dihydrobenzofuran-6-yl)-9-(5,6,7,8-tetrahydro[1,8]naphthyridin-2-yl)nonansäure,
3(R)-(2,3-Dihydrofuro[3,2-b]pyridin-5-yl)-9-(5,6,7,8-tetrahydro[1,8]naphthyridin-2-yl)nonansäure,
3(S)-(2,3-Dihydrofuro[3,2-b]pyridin-5-yl)-9-(5,6,7,8-tetrahydro[1,8]naphthyridin-2-yl)nonansäure,
3(R)-(Furo[2,3-b]pyridin-5-yl)-9-(5,6,7,8-tetrahydro[1,8]naphthyridin-2-yl)nonansäure,
3(S)-(Furo[2,3-b]pyridin-5-yl)-9-(5,6,7,8-tetrahydro[1,8]naphthyridin-2-yl)nonansäure,
3(R)-(2,3-Dihydrofuro[2,3-b]pyridin-5-yl)-9-(5,6,7,8-tetrahydro[1,8]-naphthyridin-2-yl)nonansäure,
3(S)-(2,3-Dihydrofuro[2,3-b]pyridin-5-yl)-9-(5,6,7,8-tetrahydro[1,8]-naphthyridin-2-yl)nonansäure,
3(R)-(6-Methoxypyridin-3-yl)-9-(5,6,7,8-tetrahydro[1,8]naphthyridin-2-yl)nonansäure,
3(S)-(6-Methoxypyridin-3-yl)-9-(5,6,7,8-tetrahydro[1,8]naphthyridin-2-yl)nonansäure,
3(R)-(Pyrimidin-5-yl)-9-(5,6,7,8-tetrahydro[1,8]naphthyridin-2-yl)nonansäure,
3(S)-(Pyrimidin-5-yl)-9-(5,6,7,8-tetrahydro[1,8]naphthyridin-2-yl)nonansäure,
3(R)-(6-Aminopyridin-3-yl)-9-(5,6,7,8-tetrahydro[1,8]naphthyridin-2-yl)nonansäure,
3(S)-(6-Aminopyridin-3-yl)-9-(5,6,7,8-tetrahydro[1,8]naphthyridin-2-yl)nonansäure,
3(R)-(Benzo[b]thiazol-2-yl)-9-(5,6,7,8-tetrahydro[1,8]naphthyridin-2-yl)-nonansäurehydrochlorid,
3(S)-(Benzo[b]thiazol-2-yl)-9-(5,6,7,8-tetrahydro[1,8]naphthyridin-2-yl)nonansäurehydrochlorid
und den pharmazeutisch annehmbaren Salzen davon.

15. Die Verbindung nacn Anspruch 1, die ist:
3(S)-(Pyrimidin-5-yl)-9-(5,6,7,8-tetrahydro[1,8]naphthyridin-2-yl)nonansäure,
oder ein pharmazeutisch annehmbares Salz davon.

16. Die Verbindung nach Anspruch 1, die ist:
3(R)-(Pyrimidin-5-yl)-9-(5,6,7,8-tetrahydro[1,8]naphthyridin-2-yl)nonansäure,
oder ein pharmazeutisch annehmbares Salz davon.

17. Eine pharmazeutische Zusammensetzung, die eine Verbindung gemäß irgendeinem der Ansprüche 1 bis 16 und einen pharmazeutisch annehmbaren Träger enthält.

18. Eine pharmazeutische Zusammensetzung, die durch Zusammengeben einer Verbindung gemäß irgendeinem der Ansprüche 1 bis 16 und eines pharmazeutisch annehmbaren Trägers erzeugt wird.

19. Ein Verfahren zur Herstellung einer pharmazeutisch annehmbaren Zusammensetzung, umfassend das Zusammengeben einer Verbindung gemäß irgendeinem der Ansprüche 1 bis 16 und eines pharmazeutisch annehmbaren Trägers.

20. Die Zusammensetzung nach Anspruch 17, die ferner einen Wirkstoff enthält, ausgewählt aus der Gruppe, bestehend aus
a) einem organischen Bisphosphonat oder einem pharmazeutisch annehmbaren Salz oder Ester davon,
b) einem Östrogenrezeptormodulator,
c) einem zytotoxischen/antiproliferativen Mittel.
d) einem Matrixmetalloproteinaseinhibitor,
e) einem Inhibitor der epidermalen, Fibroblasten- oder Thrombozyten-Wachstumsfaktoren,
f) einem VEGF-Inhibitor,
g) einem Flk-1/KDR-, Flt-1-, Tck/Tie-2- oder Tie-1-Inhibitor,
h) einem Kathepsin-K-Inhibitor und
i) einem Prenylierungsinhibitor, wie z.B. einem Famesyitransferaseinhibitor oder einem Geranytgeranyltransferaseinhibitor oder einem doppelten Farnesyl/Geranylgeranyl-Transferaseinhibitor und Mischungen davon.

21. Die Zusammensetzung nach Anspruch 20, wobei der genannte Wirkstoff ausgewählt ist aus der Gruppe, bestehend aus
a) einem organischen Bisphosphonat oder einem pharmazeutisch annehmbaren Satz oder Ester davon,
b) einem Östrogenrezeptormodulator und
c) einem Kathepsin-K-Inhibitor und Mischungen davon.

22. Die Zusammensetzung nach Anspruch 21, wobei das organische Bisphosphonat oder das pharmazeutisch annehmbare Salz oder der pharmazeutisch annehmbare Ester davon Alendronat-Mononatrium-Trihydrat ist.

23. Die Zusammensetzung nach Anspruch 20, wobei der Wirkstoff ausgewählt ist aus der Gruppe, bestehend aus
a) einem zytotoxischen/antiproliferativen Mittel,
b) einem Matrixmetalloproteinaseinhibitor,
c) einem Inhibitor der epidermalen, Fibroblasten- oder Thrombozyten-Wachstumsfaktoren,
d) einem VEGF-Inhibitor,
e) einem Flk-1/KDR-, Flt-1-, Tck/Tie-2- oder Tie-1-Inhibitor und
f) einem Kathepsin-K-Inhibitor und Mischungen davon.

24. Die Verwendung einer Verbindung gemäß irgendeinem der Ansprüche 1 bis 16 zur Herstellung eines Medikaments zur Herbeiführung einer Integrinrezeptorantagonisierungswirkung.

25. Die wie in Anspruch 24 beanspruchte Verwendung, wobei die integrinrezeptorantagonisierungswirkung eine αvβ3-Antagonisierungswirkung ist.

26. Die wie in Anspruch 25 beanspruchte Verwendung, wobei die αvβ3-Antagonisierungswirkung ausgewählt ist aus der Gruppe, bestehend aus der Inhibierung von Knochenresorption. Restenose, Angiogenese, diabetischer Retinopathie, Makufadegeneration, Entzündung, Viruserkrankung, Tumorwachstum und Metastase.

27. Die wie in Anspruch 26 beanspruchte Verwendung, wobei die αvβ3-Antagonisierungswirkung die Inhibierung der Knochenresorption ist.

28. Die wie in Anspruch 24 beanspruchte Verwendung, wobei die Integrinrezeptorantagonisierungswirkung eine αvβ5-Antagonisierungswirkung ist.

29. Die wie in Anspruch 28 beanspruchte Verwendung, wobei die αvβ5-Antagonisierungswirkung ausgewählt ist aus der Gruppe, bestehend aus der inhibierung von Restenose, Angiogenese, diabetischer Retinopathie, Makuladegeneration, Entzündung, Tumorwachstum und Metastase.

30. Die wie in Anspruch 24 beanspruchte Verwendung, wobei die Integrinrezeptorantagonisierungswirkung eine doppelte αvβ3/αvβ5-Antagonisierungswirkung ist.

31. Die wie in Anspruch 30 beanspruchte Verwendung, wobei die doppelte αvβ3/αvβ5-Antagonisietungswirkung ausgewählt ist aus der Gruppe, bestehend aus der Inhibierung von Knochenresorption, Restenose, Angiogenese, diabetischer Retinopathie, Makuladegeneration, Entzündung, Viruserkrankung, Tumorwachstum und Metastase.

32. Die wie in Anspruch 24 beanspruche Verwendung, wobei die Integrinrezeptorantagonisierungswirkung eine αvβ6-Antagonisierungswirkung ist.

33. Die wie in Anspruch 32 beanspruchte Verwendung, wobei die αvβ6-Antagonisierungswirkung ausgewählt ist aus der Gruppe, bestehend aus Angiogenese, Entzündungserkrankung und Wundheilung.

34. Die Verwendung der Zusammensetzung nach Anspruch 17 zur Herstellung eines Medikaments zur Herbeiführung einer Integrinrezeptorantagonisierungswirkung.

35. Die Verwendung der Zusammensetzung nach Anspruch 17 zur Herstellung eines Medikaments zur Behandlung oder Prävention eines Zustandes, der durch den Antagonismus eines Integtinrazeptors vermittelt wird.

36. Die Verwendung der Zusammensetzung nach Anspruch 17 oder Anspruch 21 zur Herstellung eines Medikaments zur Inhibierung von Knochenresorption.

37. Die Verwendung der Zusammensetzung nach Anspruch 23 zur Herstellung eines Medikaments zur Behandlung von Tumorwachstum.

38. Die Verwendung einer Verbindung gemäß irgendeinem der Ansprüche 1 bis 16 zur Herstellung eines Medikaments zur Behandlung von Tumorwachstum in Verbindung mit einer Strahientherapie.

## Revendications

1. Composé répondant à la formule 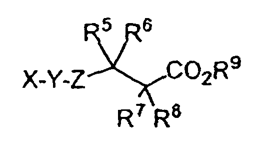 dans laquelle X représente 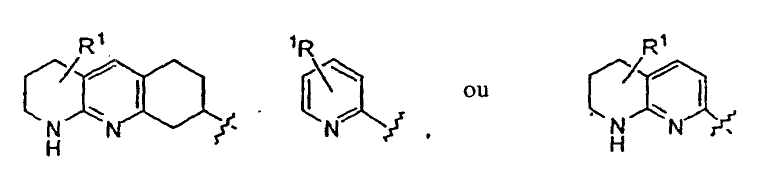 Y est choisi parmi le groupe constitué de
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH₂)ₘ-O-(CH₂)ₙ-O-(CH₂)ₚ-,
-(CH₂)ₘ-O-(CH₂)ₙ-NR⁴-(CH₂)ₚ-,
-(CH₂)ₘ-NR⁴-(CH₂)ₙ-NR⁴-(CH₂)ₚ-,
-(CH₂)ₘ-O-(CH₂)ₙ-S-(CH₂)ₚ-,
-(CH₂)ₘ-S-(CH₂)ₙ-S-(CH₂)ₚ-,
-(CH₂)ₘ-NR⁴-(CH₂)ₙ-S-(CH₂)ₚ-,
-(CH₂)ₘ-NR⁴-(CH₂)ₙ-O-(CH₂)ₚ-,
-(CH₂)ₘ-S-(CH₂)ₙ-O-(CH₂)ₚ-, et
-(CH₂)ₘ-S-(CH₂)ₙ-NR⁴-(CH₂)ₚ-,
dans lequel un quelconque atome de carbone des groupes méthylène (CH₂) dans Y, autre que dans R⁴, peut être substitué par un ou deux substituants R³, à la condition que si Y est -(CH₂)ₘ-NR⁴-(CH₂)ₙ- et n est égal à 1, alors le substituant R³ sur l'atome de carbone du groupe méthylène dans -(CH₂)ₘ- adjacent à l'atome d'azote ne peut pas être oxo;
Z est choisi parmi le groupe constitué de 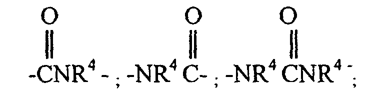 -CH₂ CH₂ -, et -CH = CH-, dans lesquels l'un ou l'autre atome de carbone peut être substitué par un ou deux substituants R³;
R¹ est choisi parmi le groupe constitué de
un atome d'hydrogène, d'un atome d'halogène, les groupes alkyle en C₁ à C₁₀, cycloalkyle en C₃ à C₈, cyclohétéroalkyle en C₃ à C₈, (cycloalkyl en C₃ à C₈ )alkyle en C₁ à C₆, (cyclohétéroalkyl en C₃ à C₈ )alkyle en C1 à C6 , aryle, arylalkyle en C₁ à C₈, amino, aminoalkyle en C₁ à C₈, acylamino en C₁ à C₃ , (acylamino en C₁ à C₃)alkyle en C₁ à C₈, (alkyl en C₁ à C₆ )ₚ amino, (alkyl en C₁ à C₆ )ₚ aminoalkyle en C₁ à C₈, alkoxy en C₁ à C₄, (alkoxy en C₁ à C₄ )alkyle en C₁ à C₆, hydroxycarbonyle, hydroxycarbonylalkyle en C₁ à C₆, alkoxycarbonyle en C₁ à C₃, (alkoxycarbonyl en C₁ à C₃ )alkyle en C₁ à C₆, hydroxycarbonylalkyloxy en C₁ à C₆, hydroxy, hydroxyalkyle en C₁ à C₆, (alkyloxy en C₁ à C₆ )alkyle en C₁ à C₆, nitro, cyano, trifluorométhyle, trifluorométhoxy, trifluoroéthoxy, (alkyl en C₁ à C₈)S(O)ₚ, (alkyle en C₁ à C₈)ₚ aminocarbonyle, alkyloxycarbonylamino en C₁ à C₈, (alkyle en C₁ à C₈)ₚ aminocarbonyloxy, (aryl(alkyl en C₁ à C₈))ₚ amino, (aryl)ₚ amino, (aryl en C₁ à C₈)alkylsulfonylamino, et alkylsulfonylamino en C₁ à C₈ ;
chaque R³, R⁵, R⁶, R⁷ et R⁸ est indépendamment choisi parmi le groupe constitué de un atome d'hydrogène,
les groupes aryle,
alkyle en C₁ à C₁₀,
aryl-(CH₂)ᵣ-O-(CH₂)ₛ-,
aryl-(CH₂)ᵣS(O)ₚ-(CH₂)ₛ-,
aryl-(CH₂)ᵣ-C(O)-(CH₂)ₛ-,
aryl-(CH₂)ᵣ-C(O)-N(R⁴)-(CH₂)ₛ-,
aryl-(CH₂)ᵣ-N(R⁴)-C(O)-(CH₂)ₛ -,
aryl-(CH₂)ᵣ-N(R⁴)-(CH₂)ₛ-,
un atome d'halogène,
les groupes hydroxyle,
(alkyl en C₁ à C₈ )carbonylamino ,
arylalkoxy en C₁ à C₅,
(alkoxy en C₁ à C₅ )carbonyle,
(alkyl en C₁ à C₈ )ₚ aminocarbonyle,
(alkyl en C₁ à C₆ )carbonyloxy,
cycloalkyle en C₃ à C₈ ,
(alkyl en C₁ à C₆ )ₚ amino,
arylaminocarbonyle,
aryl(alkyl en C₁ à C₅ )aminocarbonyle,
aminocarbonyle,
aminocarbonylalkyle en C₁ à C₆,
hydroxycarbonyle,
hydroxycarbonylalkyle en C₁ à C₆,
HC ≡C-(CH₂ )ₜ -,
(alkyl en C₁ à C₆ )-C ≡C-(CH₂ )ₜ -,
(cycloalkyl en C₃ à C₇ )-C ≡C-(CH₂ )ₜ -,
aryl-C ≡C-(CH₂ )ₜ -,
(alkylaryl en C₁ à C₆ )-C ≡C-(CH₂ )ₜ -,
CH₂ = CH-(CH₂ )ₜ -,
(alkyl en C₁ à C₆ )-CH = CH-(CH₂ )ₜ -,
(cycloalkyl en C₃ à C₇ )-CH = CH-(CH₂ )ₜ -,
aryl-CH = CH-(CH₂ )ₜ -,
(alkylaryl en C₁ à C₆ )-CH = CH-(CH₂ )ₜ -,
(alkyl en C₁ à C₆ )-SO₂ -(CH₂ )ₜ -,
(alkylaryl en C₁ à C₆)-SO₂ -(CH₂ )ₜ -,
alkoxy en C₁ à C₆,
arylalkoxy en C₁ à C₆ ,
arylalkyle en C₁ à C₆ ,
(alkyl en C₁ à C₆ )ₚ aminoalkyle en C₁ à C₆,
(aryl)ₚ amino,
(aryl)ₚ aminoalkyle en C₁ à C₆,
(aryl(alkyl en C₁ à C₆ ))ₚ amino,
(aryl(alkyl en C₁ à C₆ ))ₚ aminoalkyle en C₁ à C₆,
arylcarbonyloxy,
aryl(alkyl en C₁ à C₆)carbonyloxy,
(alkyl en C₁ à C₆)ₚaminocarbonyloxy,
(alkyl en C₁ à C₈ )sulfonylamino,
arylsulfonylamino,
(alkyl en C₁ à C₈ )sulfonylaminoalkyle en C₁ à C₆,
aryl(alkyl en C₁ à C₆ )sulfonylamino,
aryl(alkyl en C₁ à C₆ )sulfonylaminoalkyle en C₁ à C₆,
(alkoxy en C₁ à C₈ )carbonylamino,
(alkoxy C₁ à C₈ )carbonylaminoalkyle en C₁ à C₈,
aryloxycarbonylaminoalkyle en C₁ à C₈,
aryl(alkoxy en C₁ à C₈ )carbonylamino,
aryl(alkoxy C₁ à C₈ )carbonylaminoalkyle en C₁ à C₈,
(alkyl C₁ à C₈ )carbonylaminoalkyle en C₁ à C₆,
arylcarbonylaminoalkyle en C₁ à C₆,
aryl(alkyl en C₁ à C₆ )carbonylamino,
aryl(alkyl en C₁ à C₆ )carbonylaminoalkyle en C₁ à C₆,
aminocarbonylaminoalkyle en C₁ à C₆,
(alkyl en C₁ à C₈ )_{P} aminocarbonylamino,
(alkyl en C₁ à C₈ )ₚ aminocarbonylaminoalkyle en C₁ à C₆,
(aryl)ₚaminocarbonylaminoalkyle en C₁ à C₆,
(aryl(alkyl en C₁ à C₈ ))ₚ aminocarbonylamino,
(aryl(alkyl en C₁ à C₈ ))ₚ aminocarbonylaminoalkyle en C₁ à C₆,
aminosulfonylaminoalkyle en C₁ à C₆,
(alkyl en C₁ à C₈ )ₚ aminosulfonylamino,
(alkyl en C₁ à C₈ )ₚ aminosulfonylaminoalkyle en C₁ à C₆,
(aryl)ₚ aminosulfonylaminoalkyle en C₁ à C₆,
(aryl(alkyl en C₁ à C₈ ))ₚ aminosulfonylamino,
(aryl(alkyl en C₁ à C₈ )ₚ aminosulfonylaminoalkyle en C₁ à C₆,
(alkyl en C₁ à C₆ )sulfonyle,
(alkyl en C₁ à C₆ )sulfonylalkyle en C₁ à C₆,
arylsulfonylalkyle en C₁ à C₆,
aryl(alkyle en C₁ à C₆ )sulfonyle,
aryl(alkyle en C₁ à C₆)sulfonylalkyle en C₁ à C₆,
(alkyle en C₁ à C₆ )carbonyle,
(alkyl en C₁ à C₆ )carbonylalkyle en C₁ à C₆,
arylcarbonylalkyle en C₁ à C₆,
aryl(alkyle en C₁ à C₆)carbonyle,
aryl(alkyle en C₁ à C₆)carbonylalkyle en C₁ à C₆,
(alkyl en C₁ à C₆ )thiocarbonylamino,
(alkyl en C₁ à C₆)thiocarbonylaminoalkyle en C₁ à C₆,
arylthiocarbonylaminoalkyle en C₁ à C₆,
aryl(alkyl en C₁ à C₆)thiocarbonylamino,
aryl(alkyl en C₁ à C₆)thiocarbonylaminoalkyle en C₁ à C₆,
(alkyle en C₁ à C₈ )p aminocarbonylalkyle en C₁ à C₆,
(aryl)ₚaminocarbonylalkyle en C₁ à C₆,
(aryl(alkyl en C₁ à C₈))ₚaminocarbonyle, et
(aryl(alkyl en C₁ à C₈))ₚaminocarbonylalkyle en C₁ à C₆;
ou bien R³ est oxo, trifiuorométhyle ou (alkyl en C₁ à C₈)carbonylamino;
ou R⁵ et R⁶ sont indépendamment aminoalkyle en C₁ à C₆, arylsulfonylaminoalkyle en C₁ à C₆, ou (alkyl en C₁ à C₈)carbonylamino;
ou R⁷ et R⁸ sont indépendamment aminoalkyle en C₁ à C₆, arylcarbonylamino, arylsulfonylaminoalkyle en C₁ à C₆, arylaminocarbonylamino ou (polycyclyl en C₇ à C₂₀)(alkyl en C₀ à C₈)sulfonylamino;
ou deux substituants R³; lorsqu'ils se trouvent sur le même atome de carbone, sont pris ensemble avec l'atome de carbone auquel ils sont attachés pour former un groupe carbonyle ou un groupe cyclopropyle,
ou R⁵ et R⁶, lorsqu'ils se trouvent sur le même atome de carbone, sont pris ensemble avec l'atome de carbone auquel ils sont attachés pour former un groupe carbonyle,
où l'un quelconque des groupes alkyle de R³, R⁵, R⁶, R⁷, et R⁸ sont soit non substitués, soit substitués par un à trois substituants R¹, et à la condition que chaque R³, chaque R⁵ et R⁶, et chaque R⁷ et R⁸ soient choisis de telle sorte que, dans le composé résultant, l'atome de carbone ou les atomes auxquels R³, R⁵ et R⁶, et R⁷ et R⁸ sont attachés sont eux-mêmes attachés à pas plus d'un hétéroatome; chaque R⁴ est indépendamment choisi parmi le groupe constitué de
un atome d'hydrogène,
les groupes aryle,
aminocarbonyle,
cycloalkyle en C₃ à C₈,
aminoalkyle en C₁ à C₆,
(aryl)ₚ aminocarbonyle,
(arylalkyle en C₁ à C₅ )ₚ aminocarbonyle,
hydroxycarbonylalkyle en C₁ à C₆,
alkyle en C₁ à C₈,
arylalkyle en C₁ à C₆,
(alkyl en C₁ à C₆ )ₚ aminoalkyle C₂ à C₆,
(arylalkyle en C₁ à C₆ )ₚ aminoalkyle en C₂ à C₆,
(alkyl en C₁ à C₈ )sulfonyle,
(alkoxy en C₁ à C₈ )carbonyle,
aryloxycarbonyle,
aryl(alkoxy en C₁ à C₈)carbonyle,
(alkyle en C₁ à C₈ )carbonyle,
arylcarbonyle,
aryl(alkyl en C₁ à C₆ )carbonyle,
(alkyl en C₁ à C₈ )ₚ aminocarbonyle,
aminosulfonyle,
(alkyl en C₁ à C₈ )aminosulfonyle,
(aryl)p aminosulfonyle,
(aryl(alkyl en C₁ à C₈ ))ₚ aminosulfonyle,
arylsulfonyle,
aryl(alkyl en C₁ à C₆ )sulfonyle,
(alkyl en C₁ à C₆)thiocarbonyle,
arylthiocarbonyle, et
aryl(alkyl en C₁ à C₆ )thiocarbonyle,
dans lequel l'un quelconque des groupes alkyle de R⁴ sont soit non substitués, soit substitués par un à trois substituants R¹;
R9 est choisi parmi le groupe constitué de
un atome d'hydrogène,
les groupes alkyle en C₁ à C₈,
aryle,
arylalkyle en C₁ à C₈,
(alkyl en C₁ à C₈)carbonyloxyalkyle en C₁ à C₄,
aryl(alkyl en C₁ à C₈ )carbonyloxyalkyle en C₁ à C₄,
(alkyl en C₁ à C₈)aminocarbonylméthylène, et
di(alkyl en C₁ à C₈)aminocarbonylméthylène;
dans lequel
chaque groupe aryle en tant qu'un groupe ou une partie d'un groupe est indépendamment choisi parmi le groupe constitué de: phényle, naphtyle, pyridyle, pyrryle, pyrazolyle, pyrazinyle, pyrimidinyle, imidazolyle, benzimidazolyle, benzothiazolyle, benzoxazolyle, indolyle, thiényle, furyle, dihydrobenzofuryle, benzo(1,3)dioxolane, oxazolyle, isoxazolyle et thiazolyle;
chaque m est indépendamment un nombre entier valant de 2 à 4;
chaque n est indépendamment un nombre entier valant de 0 à 6;
chaque p est indépendamment un nombre entier valant de 0 à 2;
chaque r est indépendamment un nombre entier valant de 1 à 3;
chaque s est indépendamment un nombre entier valant de 0 à 3; et
chaque t est indépendamment un nombre entier valant de 0 à 3;
et les sels pharmaceutiquement acceptables de ces composés.

2. Composé selon la revendication 1, dans lequel Y est choisi parmi le groupe constitué de
-(CH₂)ₘ-,
-(CH₂)ₘ-O-(CH₂)ₙ-,
-(CH₂)ₘ-NR⁴-(CH₂)ₙ-,
-(CH₂)ₘ-S-(CH₂)ₙ-,
-(CH₂)ₘ-SO-(CH₂)ₙ-,
-(CH₂)ₘ-SO₂-(CH₂)ₙ-,
-(CH₂)ₘ-O-(CH₂)ₙ-O-(CH₂)ₚ-,
-(CH₂)ₘ-O-(CH₂)ₙ-NR⁴-(CH₂)ₚ-,
-(CH₂)ₘ-NR⁴-(CH₂)ₙ-NR⁴-(CH₂)ₚ-, et
-(CH₂)ₘ-NR⁴-(CH₂)ₙ-O-(CH₂)ₚ-,
dans lequel l'un quelconque des atomes de carbone des groupes méthylène (CH₂) dans Y, autre que dans R⁴, peut être substitué par un ou deux substituants R³; à la condition que si Y est -(CH₂)ₘ-NR⁴ -(CH₂)ₙ- et n est égal à 1, alors le substituant R³ sur l'atome de carbone du groupe méthylène dans -(CH₂)ₘ- adjacent à l'atome d'azote ne peut pas être oxo; et Z est choisi parmi le groupe constitué de 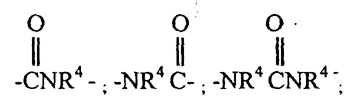 -CH₂CH₂ -, et -CH =CH-, dans lequel l'un ou l'autre atome de carbone peut être substitué par un ou deux substituants R³.

3. Composé selon la revendication 2, dans lequel Y est choisi parmi le groupe constitué de
(CH₂)ₘ, (CH₂)ₘ-S-(CH₂)ₙ, et (CH₂)ₘ-NR⁴-(CH₂)ₙ,
dans lequel l'un quelconque des atomes de carbone des groupes méthylène (CH₂) dans Y, autre que dans R⁴, peut être substitué par un ou deux substituants R³; à la condition que si Y est -(CH₂)ₘ -NR⁴ -(CH₂)ₙ - et n est égal à 1, alors le substituant R³ sur l'atome de carbone du groupe méthylène dans -(CH₂)ₘ- adjacent à l'atome d'azote ne peut pas être oxo; et Z est choisi parmi le groupe constitué de 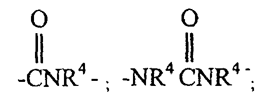 et
-CH₂CH₂-, dans lequel l'un ou l'autre atome de carbone peut être substitué par un ou deux substituants R³.

4. Composé selon la revendication 3, dans lequel chaque R³ est indépendamment choisi parmi le groupe constitué de
un atome d'hydrogène,
les groupes fluoro,
trifluorométhyle,
aryle,
alkyle en C₁ à C₈,
arylalkyle en C₁ à C₆,
hydroxyle,
oxo,
arylaminocarbonyle,
aryl(alkyl en C₁ à C₅ )aminocarbonyle,
aminocarbonyle, et
aminocarbonylalkyle en C₁ à C₆;
et chaque R⁴ est indépendamment choisi parmi le groupe constitué de
un atome d'hydrogène,
les groupes aryle,
cycloalkyle en C₃ à C₈,
alkyle en C₁ à C₈,
(alkyl en C₁ à C₈ )carbonyle,
arylcarbonyle,
(alkyl en C₁ à C₆ )sulfonyle,
arylsulfonyle,
aryl(alkyl en C₁ à C₆ )sulfonyle,
aryl(alkyl en C₁ à C₆ )carbonyle,
(alkyl en C₁ à C₈ )aminocarbonyle,
aryl(alkyl en C₁ à C₅ )aminocarbonyle,
aryl(alkoxy en C₁ à C₈ )carbonyle, et
(alkoxy en C₁ à C₈ )carbonyle.

5. Composé selon la revendication 4 dans lequel R⁶, R⁷ et R⁸ sont chacun un atome d'hydrogène et R⁵ est choisi parmi le groupe constitué de
un atome d'hydrogène,
les groupes aryle,
alkyle en C₁ à C₈,
aryl-C ≡C-(CH₂)ₜ -,
arylalkyle en C₁ à C₆,
CH₂ = CH-(CH₂)ₜ-, et
HC ≡C-(CH₂)ₜ-.

6. Composé selon la revendication 5, dans lequel R⁹ est choisi parmi le groupe constitué d'un atome d'hydrogène, d'un groupe méthyle, et d'un groupe éthyle.

7. Composé selon la revendication 6, dans lequel R⁹ est un atome d'hydrogène.

8. Composé selon la revendication 4, dans lequel R⁵, R⁶ et R⁸ sont chacun un atome d'hydrogène et R⁷ est choisi parmi le groupe constitué de
un atome d'hydrogène,
les groupes aryle,
(alkyl en C₁ à C₈)carbonylamino,
(alkyl en C₁ à C₈ )sulfonylamino,
arylcarbonylamino,
arylsulfonylamino,
(alkyl en C₁ à C₈ )sulfonylaminoalkyle en C₁ à C₆ ,
arylsulfonylaminoalkyle en C₁ à C₆,
aryl(alkyl en C₁ à C₆ )sulfonylamino,
aryl(alkyl en C₁ à C₆ )sulfonylaminoalkyle en C₁ à C₆,
(alkoxy en C₁ à C₈ )carbonylamino,
(alkoxy en C₁ à C₈ )carbonylaminoalkyle en C₁ à C₈,
aryloxycarbonylaminoalkyle en C₁ à C₈,
aryl(alkoxy en C₁ à C₈ )carbonylamino,
aryl(alkoxy en C₁ à C₈ )carbonylaminoalkyle en C₁ à C₈,
(alkyl en C₁ à C₈ )carbonylaminoalkyle en C₁ à C₆,
arylcarbonylaminoalkyle en C₁ à C₆,
aryl(alkyl en C₁ à C₆ )carbonylamino,
aryl(alkyle en C₁ à C₆ )carbonylaminoalkyle en C₁ à C₆,
aminocarbonylaminoalkyle en C₁ à C₆,
(alkyl en C₁ à C₈)ₚ aminocarbonylamino,
(alkyl en C₁ à C₈ )ₚ aminocarbonylaminoalkyle en C₁ à C₆ ,
(aryl)ₚ aminocarbonylaminoalkyle en C₁ à C₆ ,
arylaminocarbonylamino,
(aryl(alkyl en C₁ à C₈))ₚ aminocarbonylamino,
(aryl(alkyl en C₁ à C₈ ))ₚ aminocarbonylaminoalkyle en C₁ à C₆,
aminosulfonylaminoalkyle en C₁ à C₆,
(alkyl en C₁ à C₈ )ₚ aminosulfonylamino,
(alkyl en C₁ à C₈ )ₚ aminosulfonylaminoalkyle en C₁ à C₆,
(aryl)ₚ aminosulfonylaminoalkyle en C₁ à C₆,
(aryl(alkyl en C₁ à C₈))ₚ aminosulfonylamino,
(aryl(alkyl en C₁ à C₈))ₚ aminosulfonylaminoalkyle en C₁ à C₆,
(alkyl en C₁ à C₆ )thiocarbonylamino,
(alkyl en C₁ à C₆ )thiocarbonylaminoalkyle en C₁ à C₆
arylthiocarbonylaminoalkyle en C₁ à C₆,
aryl(alkyl en C₁ à C₆)thiocarbonylamino,
aryl(alkyl en C₁ à C₆)thiocarbonylaminoalkyle en C₁ à C₆, et
(polycyclyl en C₇ à C₂₀ )(alkyle en C₀ à C₈ )sulfonylamino.

9. Composé selon la revendication 8, dans lequel R⁵, R⁶, et R⁸ sont chacun un atome d'hydrogène et R⁷ est choisi parmi le groupe constitué de
un atome d'hydrogène,
les groupes aryle,
(alkyl en C₁ à C₈ )carbonylamino,
aryl(alkyl en en C₁ à C₆)carbonylamino,
arylcarbonylamino,
(alkyl en C₁ à C₈)sulfonylamino,
aryl(alkyl en C₁ à C₆ )sulfonylamino,
arylsulfonylamino,
(alkoxy en C₁ à C₈ )carbonylamino,
aryl(alkoxy en C₁ à C₈ )carbonylamino,
arylaminocarbonylamino,
(alkyl en C₁ à C₈)ₚ aminocarbonylamino,
(aryl(alkyl en C₁ à C₈ ))ₚ aminocarbonylamino,
(alkyl en C₁ à C₈)ₚ aminosulfonylamino, et
(aryl(alkyl en C₁ à C₈ )ₚ aminosulfonylamino.

10. Composé selon la revendication 9, dans lequel R⁹ est choisi parmi le groupe constitué d'un atome d'hydrogène, d'un groupe méthyle, et d'un groupe éthyle.

11. Composé selon la revendication 10, dans lequel R⁹ est un atome d'hydrogène.

12. Composé selon la revendication 4, choisi parmi le groupe constitué de:
acide 3-(5-(5, 6, 7, 8-tétrahydro[1, 8]naphtyridin-2-yl)-pentanoylamino)-propionique;
acide 3(S)-(pyridin-3-yl)-3-(5-(5, 6, 7, 8-tétrahydro[1, 8]naphtyridin-2-yl)-pentanoylamino)-propionique;
(trifluoroacétate) de l'acide 3(S)-(5, 6, 7, 8- tétrahydroquinolin-3-yl)-3-(5-(5, 6, 7, 8-tétrahydro[1, 8]naphtyridin-2-yl)-pentanoylainino)-propionique;
trifluoroacétate de l'acide 2(S)-benzènesulfonylamino-3-(5-(5, 6, 7, 8- tétrahydro-[1, 8]naphtyridin-2-yl)-pentanoylamino)-propionique;
acide 3(S)-(quinolin-3-yl)-3-(5-(5, 6, 7, 8-tétrahydro-[1, 8]naphtyridin-2-yl)-pentanoylamino)-propionique;
acide 3(R)-(quinolin-3-yl)-3-(5-(5, 6, 7, 8- tétrahydro-[1, 8]naphtyridin-2-yl)-pentanoyl amino)-propionique;
bis(trifluoroacétate) de l'acide de 3-(quinolin-3-yl)-3-(7-(5, 6, 7, 8-tétrahydro-[1, 8]naphtyridin-2-yl)-heptanoylamino)-propionique ;
acide 3-(quinolin-3-yl)-3-(6-((5, 6, 7, 8-tétrahydro-[1, 8]naphtyridin-2-yl)-hexanoylamino)-propionique;
bis(trifluoroacétate) de l'acide 3(S)-(3-fluorophényl)-3-(4-(5, 6, 7, 8-tétrahydro-[1, 8]naphtyridin-2-ylamino)-butyrylamino)-propionique;
acide 3(S)-(5-(5, 6, 7, 8-tétrahydro-[1, 8]naphtyridin-2-yl)-pentanoylamino)-pent-4-énoïque;
acide 3(S)-(3-fluorophényl)-3-(5-(5, 6, 7, 8-tétrahydro-[1, 8]naphtyridin-2-yl)-pentanoylamino)-propionique;
sel trifluoroacétate de l'acide 2-(3-fluorophényl)-3-(5-(5, 6, 7, 8-tétrahydro-[1, 8]naphtyridin-2-yl)-pentanoylamino)-propionique;
acide 3(S)-(benzo[1, 3]dioxol-5-yl)-3-(5-(5, 6, 7, 8-tétrahydro-[1, 8]naphtyridin-2-yl)-pentanoylamino)-propionique;
acide 3(S)-(2,3-dihydro-benzofuran-6-yl)-3-(5-(5, 6, 7, 8-tétrahydro-[1, 8]naphtyridin-2-yl)-pentanoylamino)-propionique;
trifluoroacétate de l'acide 3(S)-(2-oxo-2, 3-dihydro-benzoxazol-6-yl)-3-(5-(5, 6, 7, 8-tétrahydro-[1, 8]naphtyridin-2-yl)-pentanoylamino)-propionique;
acide 3(S)-(3-fluorophényl)-3-{3-[(5, 6, 7, 8-tétrahydro-[1, 8]-naphtyridin-2-ylméthyl)-amino]-propionylamino}-propionique;
trifluoroacétate de l'acide 3(S)-(3-fluorophényl)-3-(2-{propyl-[2-(5, 6, 7, 8-tétrahydro-[1,8]naphtyridin-2-yl)-éthyl]-amino}acétylamino)-propionique;
trifluoroacétate de l'acide 3(S)-(3-fluorophényl)-3-(2-{phenéthyl-[2-(5, 6, 7, 8-tétrahydro-[1, 8]naphtyridin-2-yl)-éthyl]-amino}-acétylamino)-propionique;
acide 3(S)-(3-fluorophényl)-3-{3(S)-[(5, 6, 7, 8-tétrahydro-[1, 8]-naphtyridin-2-ylméthyl)-amino]-pent-4-ynoylamino}-propionique;
bis(trifluoroacétate) de l'acide 3(S)-(3-fluorophényl)-3-{3(S)-(3-fluorophényl)-3-[(5, 6, 7, 8-tétrahydro-[1, 8]naphtyridin-2-ylméthyl)-amino]-propionylamino}-propionique;
trifluoroacétate de l'acide 3(S)-(3-fluoro-4-phényl-phényl)-3-(5-(5, 6, 7, 8-tétrahydro-[1, 8]naphtyridin-2-yl)-pentanoylamino)-propionique;
trifluoroacétate de l'acide 2(S)-(2-thiénylsulfonylamino)-3-(5-(5, 6, 7, 8-tétrahydro-[1, 8]naphtyridin-2-yl)-pentanoylamino)-propionique;
acide 3(S)-(3-fluorophényl)-3-{3-méthyl-3-[(5, 6, 7, 8-tétrahydro-[1, 8]-naphtyridin-2-ylméthyl)-amino] propionylamino}-propionique;
acide 3(S)-(3-fluorophényl)-3-{2-[2-(5, 6, 7, 8-tétrahydro-[1, 8]-naphtyridin-2-yl)-éthylamino]-acétylamino}-propionique;
acide 3(S)-(3-fluorophényl)-3{[3-(5, 6, 7, 8-tétrahydro-[1, 8]-naphtyridin-2-yl)-propyl]-uréido}-propionique;
acide 2(S)-(méthanesulfonylamino)-3-(5-(5, 6, 7, 8-tétrahydro-[1, 8]-naphtyridin-2-yl)-pentanoylamino)-propionique;
acide 3(S)-(2,3-dihydro-benzofuran-6-yl)-3-(3-(1, 2, 3, 4, 6, 7, 8 (R ou S), 9-octahydro-benzo(b][1, 8]naphtyridin-8-yl)-propionylamino]-propionique;
acide 3(S)-(2, 3-dihydro-benzofuran-6-yl)-3-[3-(1, 2, 3, 4, 6, 7, 8 (S ou R), 9-octahydro-benzo[b][1, 8]naphtyridin-8-yl)-propionylamino] propionique;
acide 3(S)-(6-méthoxy-pyridin-3-yl)-3-[N-méthyl-3-(1, 2, 3, 4, 6, 7, 8, 9-octahydro-benzo[b][1, 8]naphtyridin-8-yl-propionyl)-amino]-propionique;
bis(trifluoroacétate) de l'acide 3(S)-(2, 3-dihydro-benzofuran-6-yl)-3-[3-(5, 6, 7, 8-tétrahydro-[1, 8]-naphtyridin-2-ylméthylsulfanyl)propionylamino]-propionique;
acide 3-(quinolin-3-yl)-7-[(5, 6, 7, 8-tétrahydro-[1, 8]-naphtyridin-2-ylméthyl)-amino]-heptanoïque;
acide 3-(quinolin-3-yl)-7-(acétyl-(5, 6, 7, 8-tétrahydro-[1, 8]-naphtyridin-2-ylméthyl)-amino]-heptanoïque;
acide 3-(quinolin-3-yl)-7-(méthanesulfonyl-(5, 6, 7, 8-tétrahydro--[1, 8]-naphtyridin-2-ylméthyl)amino]-heptanoïque;
acide 9-(5, 6, 7, 8-tétrahydro-[1, 8]-naphtyridin-2-yl)-nonanoïque;
bis(trifluoroacétate) de l'acide 2-(benzènesulfonylamino)-9-(5, 6, 7, 8-tétrahydro-[1,8]-naphtyridin-2-yl)-non-4-énoïque;
acide 2(S)-(benzènesulfonylamino)-9-(5, 6, 7, 8-tétrahydro-[1, 8]naphtyridin-2-yl)-nonanoïque;
acide 2(R)-(benzènesulfonylamino)-9-(5, 6, 7, 8-tétrahydro-[1, 8]naphtyridin-2-yl)-nonanoïque;
acide 2(S)-(benzènesulfonylamino)-10-(5, 6, 7, 8-tétrahydro-[1, 8]naphtyridin-2-yl)-décanoïque;
acide 2(S)-(benzènesulfonylarnino)-8-(5, 6, 7, 8-tétrahydro-[1, 8]naphtyridin-2-yl)-octanoïque;
chlorhydrate de l'acide 2(S)-(cyclohexylméthanesulfonylamino)-9-(5, 6, 7, 8-tétrahydro-[1, 8]naphthyridin-2-yl)-nonanoïque;
chlorhydrate de l'acide 2(S)-(7, 7-diméthyl-2-oxo-bicyclo[2.2.1]-hept-1(S)-ylméthanesulfonylamino)-9-(5, 6, 7, 8-tétrahydro-[1, 8]naphtyridin-2-yl) nonanoïque;
acide 2(S)-(phénylméthanesulfonylamino)-9-(5, 6, 7, 8-tétrahydro-[1, 8]-naphtyridin-2-yl) nonanoïque;
chlorhydrate de l'acide 2(S)-(cyclohexanesulfonylamino)-9-(5, 6, 7, 8-tétrahydro-(1, 8]naphtyridin-2-yl) nonanoïque;
chlorhydrate de l'acide 2(S)-(butane-1-sulfonylamino)-9-(5, 6, 7, 8-tétrahydro-[1, 8]naphtyridin-2-yl) nonanoïque;
acide 2(S)-(3-benzyluréido)-9-(5, 6, 7, 8-tétrahydro-[1, 8]naphtyridin-2-yl)-nonanoïque;
acide 2(S)-(benzyloxycarbonylamino)-9-(5, 6, 7, 8-tétrahydro-[1, 8]naphtyridin-2-yl)-nonanoïque;
acide 2(S)-(phénylacétylamino)-9-(5, 6, 7, 8-tétrahydro-[1, 8]naphtyridin-2-yl)-nonanoïque;
acide 2(S)-(acétylamino)-9-(5, 6, 7, 8-tétrahydro-[1, 8]naphtyridin-2-yl)-nonanoïque;
acide 2(S)-(benzoylamino)-9-(5, 6, 7, 8-tétrahydro-[1, 8]naphtyridin-2-yl)-nonanoïque;
acide 3-(quinolin-3-yl)-9-(5, 6, 7, 8-tétrahydro-[1, 8]-naphtyridin-2-yl)-nonanoïque;
acide 3(S)-(quinolin-3-yl)-9-(5, 6, 7, 8-tétrahydro-[1, 8]-naphtyridin-2-yl)-nonanoïque;
acide 3(R)-(quinolin-3-yl)-9-(5, 6, 7, 8-tétrahydro-[1, 8]-naphtyridin-2-yl)-nonanoïque;
bis(chlorhydrate) de l'acide 3-(quinolin-3-yl)-7-(1, 2, 3, 4, 6, 7, 8, 9-octahydro-benzo[b][1, 8]-naphtyridin-8-yl)-heptanoïque;
acide 6-oxo-3-(quinolin-3-yl)-9-(5, 6, 7, 8-tétrahydro-[1, 8]-naphtyridin-2-yl)-nonanoïque;
acide 3-(N-oxo-quinolin-3-yl)-9-(5, 6, 7, 8-tétrahydro-[1, 8]-naphtyridin-2-yl)-nonanoïque;
acide 3-(phényl)-9-(5, 6, 7, 8-tétrahydro-[1, 8]-naphtyridin-2-yl) nonanoïque;
acide 3-(benzo[b]thiophén-2-yl)-9-(5, 6, 7, 8-tétrahydro-[1, 8]-naphtyridin-2-yl)-nonanoïque;
acide 3(R)-(benzo[b]thiophén-2-yl)-9-(5, 6, 7, 8-tétrahydro-[1, 8]-naphtyridin-2-yl)-nonanoïque;
acide 3(S)-(benzo[b]thiophén-2-yl)-9-(5, 6, 7, 8-tétrahydro-[1, 8]-naphtyridin-2-yl)-nonanoïque;
acide 3-(pyridin-3-yl)-9-(5, 6, 7, 8-tétrahydro-[1, 8]-naphtyridin-2-yl)-nonanoïque;
acide 3(R)-(pyridin-3-yl)-9-(5, 6, 7, 8-tétrahydro-[1, 8]-naphtyridin-2-yl)-nonanoïque;
acide 3(S)-(pyridin-3-yl)-9-(5, 6, 7, 8-tétrahydro-[1, 8]-naphtyridin-2-yl)-nonanoïque;
acide 3-(3- fluorophényl)-9-(5, 6, 7, 8-tétrahydro-[1, 8]-naphtyridin-2-yl)-nonanoïque;
acide 3(R)-(3-fluorophényl)-9-(5, 6, 7, 8-tétrahydro-[1, 8]-naphtyridin-2-yl)-nonanoïque;
acide 3(S)-(3-fluorophényl)-9-(5, 6, 7, 8-tétrahydro-[1, 8]-naphtyridin-2-yl)-nonanoïque;
acide 3-(2, 3-dihydro-benzofuran-6-yl)-9-(5, 6, 7, 8-tétrahydro-[1, 8]-naphtyridin-2-yl)-nonanoïque;
acide 3(S)-(2, 3-dihydro-benzofuran-6-yl)-9-(5, 6, 7, 8-tétrahydro-[1, 8]-naphtyridin-2-yl)-nonanoïque;
acide 3(R)-(2, 3-dihydro-benzofuran-6-yl)-9-(5, 6, 7, 8-tétrahydro-[1, 8]-naphtyridin-2-yl)-nonanoïque;
trifluoroacétate de l'acide 3-(2, 3-dihydro-benzofuran-6-yl)-9-(5, 6, 7, 8-tétrahydro-[1, 8]-naphtyridin-2-yl)-non-4-énoïque;
acide 3-(2, 3-dihydro-furo[3, 2-b]pyridin-5-yl)-9-(5, 6, 7, 8-tétrahydro-[1, 8]-naphtyridin-2-yl)-nonanoïque;
acide 3(R)-(2, 3-dihydro-furo[3, 2-b]pyridin-5-yl)-9-(5, 6, 7, 8-tétrahydro-[1, 8]-naphtyridin-2-yl)-nonanoïque;
acide 3(S)-(2, 3-dihydro-furo[3, 2-b]pyridin-5-yl)-9-(5, 6, 7, 8-tétrahydro-[1, 8]-naphtyridin-2-yl)-nonanoïque;
acide 3-(furo[2, 3-b]pyridin-5-yl)-9-(5, 6, 7, 8-tétrahydro-[1, 8] naphtyridin-2-yl)-nonanoïque;
acide 3(R)-(furo[2, 3-b]pyridin-5-yl)-9-(5, 6, 7, 8-tétrahydro-[1, 8] naphtyridin-2-yl)-nonanoïque;
acide 3(S)-(furo[2, 3-b]pyridin-5-yl)-9-(5, 6, 7, 8-tétrahydro-[1, 8]-naphtyridin-2-yl)-nonanoïque;
acide 3-(2, 3-dihydro-furo[2, 3-b]pyridin-5-yl)-9-(5, 6, 7, 8-tétrahydro-[1, 8]-naphtyridin-2-yl)-nonanoïque;
acide 3(R)-(2, 3-dihydro-furo[2, 3-b]pyridin-5-yl)-9-(5, 6, 7, 8-tétrahydro-[1, 8]-naphtyridin-2-yl)-nonanoïque;
acide 3(S)-(2, 3-dihydro-furo[2, 3-b]pyridiri-5-yl)-9-(5, 6, 7, 8-tétrahydro-[1, 8]-naphtyridin-2-yl)-nonanoïque;
acide 3-(6-méthoxy-pyridin-3-yl)-9-(5, 6, 7, 8-tétrahydro-[1, 8] naphtyridin-2-yl)-nonanoïque;
acide 3(S)-(6-méthoxy-pyridin-3-yl)-9-(5, 6, 7, 8-tétrahydro-[1, 8]-naphtyridin-2-yl)-nonanoïque;
acide 3(R)-(6-méthoxy-pyridin-3-yl)-9-(5, 6, 7, 8-tétrahydro-[1, 8]-naphtyridin-2-yl)-nonanoïque;
trifluoroacétate de l'acide 3-(pyrimidin-5-yl)-9-(5, 6, 7, 8-tétrahydro-[1, 8]-naphtyridin-2-yl)-nonanoïque;
trifluoroacétate de l'acide 3(R)-(pyrimidin-5-yl)-9-(5, 6, 7, 8-tétrahydro-[1, 8]-naphtyridin-2-yl)-nonanoïque;
trifluoroacétate de l'acide 3(S)-(pyrimidin-5-yl)-9-(5, 6, 7, 8-tétrahydro-[1, 8]-naphtyridin-2-yl)-nonanoïque;
acide 3-(6-amino-pyridin-3-yl)-9-(5, 6, 7, 8-tétrahydro-[1, 8]-naphtyridin-2-yl)-nonanoïque;
acide 3(R)-(6-amino-pyridin-3-yl)-9-(5, 6, 7, 8-tétrahydro-[1, 8]-naphtyridin-2-yl)-nonanoïque;
acide 3(S)-(6-amino-pyridin-3-yl)-9-(5, 6, 7, 8-tétrahydro-[1, 8]-naphtyridin-2-yl)-nonanoïque;
chlorhydrate de l'acide 3-(benzo[b]thiazol-2-yl)-9-(5, 6, 7, 8-tétrahydro-[1, 8]-naphtyridin-2-yl)-nonanoïque;
chlorhydrate de l'acide 3(R)-(benzo[b]thiazol-2-yl)-9-(5, 6, 7, 8-tétrahydro-[1, 8]-naphtyridin-2-yl)-nonanoïque;
chlorhydrate de l'acide 3(S)-(benzo[b]thiazol-2-yl)-9-(5, 6, 7, 8-tétrahydro-[1, 8]-naphtyridin-2-yl)-nonanoïque;
bis-(trifluoroacétate) de l'acide 3-(6-oxo-1, 6-dihydro-pyridin-3-yl)-9-(5, 6, 7, 8-tétrahydro-[1, 8]-naphtyridin-2-yl)-nonanoïque;
et les sels pharmaceutiquement acceptables de ces composés.

13. Composé selon la revendication 12 choisi parmi le groupe constitué de:
acide 3(S)-(pyridin-3-yl)-3-(5-(5, 6, 7, 8-tétrahydro[1, 8]naphtyridin-2-yl)-pentanoylamino)-propionique;
trifluoroacétate de l'acide 2(S)-benzènesulfonylamino-3-(5-(5, 6, 7, 8-tétrahydro-[1, 8]naphtyridin-2-yl)-pentanrylamino)-propionique;
acide 3(S)-(quinolin-3-yl)-3-(5-(5, 6, 7, 8-tétrahydro-[1, 8]naphtyridin-2-yl)-pentanoylamino)-propionique;
acide 3(R)-(quinolin-3-yl)-3-(5-(5, 6, 7, 8-tétrahydro-[1, 8]naphtyridin-2-yl)-pentanoylamino)-propionique;
trifluoroacétate de l'acide 2(S)-(2-thiénylsulfonylamino)-3-(5-(5, 6, 7, 8-tétrahydro-[1, 8]naphtyridin-2-yl)-pentanoylamino)-propionique;
acide 3(S)-(2, 3-dihydro-benzofuran-6-yl)-3-[3-(1, 2, 3, 4, 6, 7, 8 (R ou S), 9-octahydro-benzo[b][1, 8]naphtyridin-8-yl)-propionylamino]-propionique;
acide 3(S)-(2, 3-dihydro-benzofuran-6-yl)-3-[3-(1, 2, 3, 4, 6, 7, 8(S ou R), 9-octahydro-benzo[b][1, 8]naphtyridin-8-yl)-propionylamino]-propionique;
acide 3(S)-(6-méthoxy-pyridin-3-yl)-3-[N-méthyl-3-(1, 2, 3, 4, 6, 7, 8, 9-octahydro-benzo[b][1, 8]naphtyridin-8-yl-propionyl)-amino]propionique;
bis(trifluoroacétate) de l'acide 2-(benzènesulfonylamino)-9-(5, 6, 7, 8-tétrahydro-[1, 8]-naphtyridin-2-yl)-non-4-énoïque;
et les sels pharmaceutiquement acceptables de ces composés.

14. Composé selon la revendication 12, choisi parmi le groupe constitué de:
acide 3(R)-(quinolin-3-yl)-9-(5, 6, 7, 8-tétrahydro-[1, 8]-naphtyridin-2-yl)-nonanoïque;
acide 3(S)-(quinolin-3-yl)-9-(5, 6, 7, 8-tétrahydro-[1, 8]-naphtyridin-2-yl)-nonanoïque;
acide 3(R)-(benzo[b]thiophén-2-yl)-9-(5, 6, 7, 8-tétrahydro-[1, 8]-naphtyridin-2-yl)-nonanoïque;
acide 3(S)-(benzo[b]thiophén-2-yl)-9-(5, 6, 7, 8-tétrahydro-[1, 8]-naphtyridin-2-yl)-nonanoïque;
acide 3(R)-(pyridin-3-yl)-9-(5, 6, 7, 8-tétrahydro-[1, 8]-naphtyridin-2-yl)-nonanoïque;
acide 3(S)-(pyridin-3-yl)-9-(5, 6, 7, 8-tétrahydro-[1, 8]-naphtyridin-2-yl)-nonanoïque;
acide 3(R)-(3-fluorophényl)-9-(5, 6, 7, 8-tétrahydro-[1, 8]-naphtyridin-2-yl)-nonanoïque;
acide 3(S)-(3-fluorophényl)-9-(5, 6, 7, 8-tétrahydro-[1, 8]-naphtyridin-2-yl)-nonanoïque;
acide 3(R)-(2, 3-dihydro-benzofuran-6-yl)-9-(5, 6, 7, 8-tétrahydro-[1, 8]-naphtyridin-2-yl)-nonanoïque;
acide 3(S)-(2, 3-dihydro-benzofuran-6-yl)-9-(5, 6, 7, 8-tétrahydro-[1, 8]-naphtyridin-2-yl)-nonanoïque;
acide 3(R)-(2, 3-dihydro-furo[3, 2-b]pyridin-5-yl)-9-(5, 6, 7, 8-tétrahydro-[1, 8]-naphtyridin-2-yl)-nonanoïque;
acide 3(S)-(2, 3-dihydro-furo[3, 2-b]pyridin-5-yl)-9-(5, 6, 7, 8-tétrahydro-[1, 8]-naphtyridin-2-yl)-nonanoïque;
acide 3(R)-(furo[2, 3-b]pyridin-5-yl)-9-(5, 6, 7, 8-tétrahydro-[1, 8]-naphtyridin-2-yl)-nonanoïque;
acide 3(S)-(furo[2, 3-b]pyridin-5-yl)-9-(5, 6, 7, 8-tétrahydro-[1, 8]-naphtyridin-2-yl)-nonanoïque;
acide 3(R)-(2, 3-dihydro-furo[2, 3-b]pyridin-5-yl)-9-(5, 6, 7, 8-tétrahydro-[1,8]-naphtyridin-2-yl)-nonanoïque;
acide 3(S)-(2, 3-dihydro-furo[2, 3-b]pyridin-5-yl)-9-(5, 6, 7, 8-tétrahydro-[1,8]-naphtyridin-2-yl)-nonanoïque;
acide 3(R)-(6-méthoxy-pyridin-3-yl)-9-(5, 6, 7, 8-tétrahydro-[1, 8]-naphtyridin-2-yl)-nonanoïque;
acide 3(S)-(6-méthoxy-pyridin-3-yl)-9-(5, 6, 7, 8-tétrahydro-[1, 8]-naphtyridin-2-yl)-nonanoïque;
acide 3(R)-(pyrimidin-5-yl)-9-(5, 6, 7, 8-tétrahydro-[1, 8]-naphtyridin-2-yl)-nonanoïque;
acide 3(S)-(pyrimidin-5-yl)-9-(5, 6, 7, 8-tétrahydro-[1, 8]-naphtyridin-2-yl)-nonanoïque;
acide 3(R)-(6-amino-pyridin-3-yl)-9-(5, 6, 7, 8-tétrahydro-[1, 8]-naphtyridin-2-yl)-nonanoïque;
acide 3(S)-(6-amino-pyridin-3-yl)-9-(5, 6, 7, 8-tétrahydro-[1, 8]-naphtyridin-2-yl)-nonanoïque;
chlorhydrate de l'acide 3(R)-(benzo[b]thiazol-2-yl)-9-(5, 6, 7, 8-tétrahydro-[1, 8]-naphtyridin-2-yl)-nonanoïque;
chlorhydrate de l'acide 3(S)-(benzo[b]thiazol-2-yl)-9-(5, 6, 7, 8-tétrahydro-[1, 8]-naphtyridin-2-yl)-nonanoïque;
et les sels pharmaceutiquement acceptables de ces composés.

15. Composé selon la revendication 1, qui est :
acide 3(S)-(pyrimidin-5-yl)-9-(5, 6, 7,8-tétrahydro-[1, 8]-naphtyridin-2-yl)-nonanoïque;
ou un sel pharmaceutiquement acceptable de ce composé.

16. Composé selon la revendication 1, qui est :
acide 3(R)-(pyrimidin-5-yl)-9-(5, 6, 7,8-tétrahydro-[1, 8]-naphtyridin-2-yl)-nonanoïque;
ou un sel pharmaceutiquement acceptable de ce composé.

17. Composition pharmaceutique comprenant un composé selon l'une quelconque des revendications 1 à 16 et un support pharmaceutiquement acceptable.

18. Composition pharmaceutique fabriquée en combinant un composé selon l'une quelconque des revendications 1 à 16 et un support pharmaceutiquement acceptable.

19. Procédé pour fabriquer une composition pharmaceutique comprenant la combinaison d'un composé selon l'une quelconque des revendications 1 à 16 et un support pharmaceutiquement acceptable.

20. Composition selon la revendication 17, qui comprend en outre un ingrédient actif choisi parmi le groupe constitué de:
a) un bisphosphonate organique ou un sel ou un ester pharmaceutiquement acceptable de ce composé,
b) un modulateur des récepteurs d'estrogène,
c) un agent cytotoxique / antiproliférateur,
d) un inhibiteur de métalloprotéinase de la matrice,
e) un inhibiteur des facteurs de croissance dérivés de l'épiderme, dérivés des fibroblastes, ou dérivés des plaquettes,
f) un inhibiteur du VEGF,
g) un inhibiteur des Flk-1/KDR, Flt-1, Tck/Tie-2, ou Tie-1
h) un inhibiteur de la cathepsine K, et
i) un inhibiteur de la prénylation, tel que l'inhibiteur de la farnésyle transférase ou un inhibiteur de la géranylgéranyltransférase ou un inhibiteur double farnésyl/géranylgéranyltransférase; et des mélanges des ces substances.

21. Composition selon la revendication 20, dans laquelle ledit ingrédient actif est choisi parmi le groupe constitué de
a) un bisphosphonate organique ou un sel ou un ester pharmaceutiquement acceptable de ce composé,
b) un modulateur des récepteurs d'estrogène, et
c) un inhibiteur de la cathepsine K ;
et des mélanges de ces substances.

22. Composition selon la revendication 21, dans laquelle ledit bisphosphonate organique ou le sel ou l'ester pharmaceutiquement acceptable de ce composé est l'alendronate monosodique trihydraté.

23. Composition selon la revendication 20, dans laquelle ledit ingrédient actif est choisi parmi le groupe constitué de:
a) un agent cytotoxique / antiproliférateur,
b) un inhibiteur de métalloprotéinase de la matrice,
c) un inhibiteur des facteurs de croissance dérivés de l'épiderme, dérivés des fibroblastes, ou dérivés des plaquettes,
d) un inhibiteur du VEGF,
e) un inhibiteur des Flk-1/KDR, Flt-1, Tck/Tie-2, ou Tie-1, et
f) un inhibiteur de la cathepsine K;
et des mélanges de ces substances.

24. Utilisation d'un composé selon l'une quelconque des revendications 1 à 16 pour la fabrication d'un médicament destiné à obtenir un effet d'antagonisation des récepteurs des intégrines.

25. Utilisation telle que décrite dans la revendication 24, dans laquelle l'effet d'antagonisation du récepteur des intégrines est un effet d'antagonisation des αvβ3.

26. Utilisation telle que décrite dans la revendication 25, dans laquelle l'effet d'antagonisation de l'αvβ3 est choisi parmi le groupe constitué d'une inhibition de la résorption osseuse, d'une resténose, d'une angiogénèse, d'une rétinopathie diabétique, d'une dégénérescence maculaire, d'une inflammation, d'une maladie virale, du développement d'une tumeur, et d'une métastase.

27. Utilisation telle que décrite dans la revendication 26, dans laquelle l'effet d'antagonisation des αvβ3 est l'inhibition de la résorption osseuse.

28. Utilisation telle que décrite dans la revendication 24, dans laquelle l'effet d'antagonisation des récepteurs des intégrines est un effet d'antagonisation des αvβ5.

29. Utilisation telle que décrite dans la revendication 28, dans laquelle l'effet d'antagonisation des αvβ5 est choisi parmi le groupe constitué d'une inhibition d'une resténose, d'une angiogénèse, d'une rétinopathie diabétique, d'une dégénérescence maculaire, d'une inflammation, du développement d'une tumeur, et d'une métastase.

30. Utilisation telle que décrite dans la revendication 24, dans laquelle l'effet d'antagonisation des récepteurs des intégrines est un double effet d'antagonisation des αvβ3 / αvβ5 .

31. Utilisation telle que décrite dans la revendication 30, dans laquelle le double effet d'antagonisation des αvβ3 / αvβ5 est choisi parmi le groupe constitué d'une une inhibition de la résorption osseuse, d'une resténose, d'une angiogénèse, d'une rétinopathie diabétique, d'une dégénérescence maculaire, d'une inflammation, d'une maladie virale, du développement d'une tumeur, et d'une métastase.

32. Utilisation telle que décrite dans la revendication 24, dans laquelle l'effet d'antagonisation des récepteurs des intégrines est un effet d'antagonisation des αvβ6.

33. Utilisation telle que décrite dans la revendication 32, dans laquelle l'effet d'antagonisation des αvβ6 est choisi parmi le groupe constitué d'une angiogénèse, d'une réponse inflammatoire, et de la guérison d'une blessure.

34. Utilisation de la composition selon la revendication 17 pour la fabrication d'un médicament destiné à obtenir un effet d'antagonisation des récepteurs des intégrines.

35. Utilisation de la composition selon la revendication 17 pour la fabrication d'un médicament destiné à traiter ou à prévenir un état physique médié par un antagonisme d'un récepteur des intégrines.

36. Utilisation de la composition selon la revendication 17 ou la revendication 21 pour la fabrication d'un médicament destiné à inhiber une résorption osseuse.

37. Utilisation de la composition selon la revendication 23 pour la fabrication d'un médicament destiné à traiter le développement d'une tumeur.

38. Utilisation, pour la fabrication d'un médicament destiné à traiter le développement d'une tumeur en combinaison avec une radiothérapie, d'un composé selon l'une quelconque des revendications 1 à 16.
